# EUROPEAN PATENT APPLICATION

(11) **EP 1 394 274 A2**
(43) Date of publication of application: **03.03.2004**
(21) Application number: 03254857.0
(22) Date of filing: 04.08.2003
(51) Int. Cl.: C12Q 1/68, C12Q 1/02, C12N 15/11, C12N 15/10

(54) **Methods of testing for bronchial asthma or chronic obstructive pulmonary disease**

(30) Priority: 06.08.2002 JP 2002229312; 20.03.2003 JP 2003077212
(71) Applicant: Genox Research, Inc., Ibaraki 300-2635 (JP)
(72) Inventor: Ohtani, Noriko, Takasaki-shi Gunma 370-1295 (JP); Sugita, Yuji, Tsukuba-shi, Ibaraki 305-8585 (JP); Yamaya, Mutsuo, Aoba-ku, Sendai-shi, Miyagi 980-8574 (JP); Kubo, Hiroshi, Aoba-ku, Sendai-shi, Miyagi 980-8574 (JP); Nagai, Hiroichi, Gifu-shi Gifu 502-8585 (JP); Izuhara, Kenji, Saga-shi Saga 849-8501 (JP)
(74) Representative: Harding, Charles Thomas

(57) **Abstract**

An objective of the present invention is to provide a method of testing for bronchial asthma or chronic obstructive pulmonary disease, a method of screening for candidate compounds for treating bronchial asthma or chronic obstructive pulmonary disease, and a pharmaceutical agent for treating bronchial asthma or chronic obstructive pulmonary disease.

The present invention identified genes whose expression levels varied between respiratory epithelial cells that had been stimulated by IL-13 to induce the goblet cell differentiation, and unstimulated respiratory epithelial cells. The respiratory epithelial cells were cultured according to the air interface method. The genes were revealed to be useful as markers for testing for bronchial asthma or chronic obstructive pulmonary disease and screening for therapeutic agents for such diseases. Specifically, the present invention provides methods of testing for bronchial asthma or chronic obstructive pulmonary disease and methods of screening for compounds to treat the diseases based on the comparison of the expression levels of marker genes identified as described above.

## Description

### FIELD OF THE INVENTION

The present invention relates to methods of testing for bronchial asthma or chronic obstructive pulmonary disease (COPD).

### BACKGROUND OF THE INVENTION

Currently, there are more than one hundred million bronchial asthma patients in the world. The rapid increase in the number of asthma patients is a social problem in Japan as well. In advanced countries, the number has increased by 20-50% in the past decade. Thus, asthma is thought to be one of the diseases that would pose a major health threat in the 21st century.

Pharmaceuticals used today for treating asthma and candidate pharmaceuticals for that purpose, include: inhaled steroids and oral steroids; agents that suppress the release of inflammatory mediators; anti-allergy agents such as histamine H1 antagonists; β2 agonists that act as bronchodilators; and immunosuppressive agents. According to a report describing clinical cases in New Zealand, the widespread use of inhaled steroids and β2 agonists has decreased the mortality rate of patients by 30% compared to 10 years ago. However, both inhaled steroids and β2 agonists have been reported to have side effects. The side effects of inhaled steroids include oral and esophageal candidiasis, olfactory disorders, adrenal suppression, osteoporosis, cataract, glaucoma, skin thinning, and growth inhibition in children. Side effects of β2 agonists include ischemic diseases, hyperthyroidism, and diabetes mellitus. In addition, regular use of β2 agonists has been known to reduce the efficacy of these drugs.

Bronchial asthma is characterized by respiratory inflammation and airflow obstruction resulting from various degrees of respiratory stenosis. Representative symptoms include paroxysmal cough and difficulty in breathing. The degree of airflow obstruction in bronchial asthma ranges from relatively mild to life-threatening obstructions. Furthermore, it has been reported that allergic reactions in the mucous membrane of the respiratory tract and bronchial smooth muscles are closely involved in bronchial asthma development.

Specifically, an atopic disposition accompanied by hyperproduction of IgE antibodies is seen in many bronchial asthma patients. Many causes are thought to lead to bronchial asthma, but there is no doubt that an atopic disposition is one cause of hypersensitivity in many patients. It is predicted that contraction of bronchial smooth muscles, edema of the respiratory tract mucous membrane, or respiratory tract hypersecretion is involved in the mechanism of respiratory obstruction in an asthma attack. Type-I allergic reactions in the respiratory tract due to exposure to pathogenic allergens play an important role in such changes in the respiratory tract.

In bronchial asthma patients, the activity of Th2 helper T cells is enhanced, and so is the production of Th2 cytokines such as interleukin-3 (hereinafter abbreviated as "IL-3"; similarly, interleukin is abbreviated as "IL" ) , IL-4, IL-5 , IL-13 and granulocyte macrophage colony stimulating factor (GM-CSF), and chemokines such as eotaxin and RANTES. IL-4 and IL-13 have the activity of inducing IgE production, and IL-3 and IL-4 have the activity of inducing the proliferation of mast cells. Eosinophils that differentiate and proliferate by IL-5 and GM-CSF infiltrate into the respiratory tract by the action of eotaxin and RANTES (Allergy Asthma. Proc. 20: 141 (1999)).

Eosinophils that infiltrate into the respiratory tract release intracellular granule proteins such as activated major basic protein (MBP) and eosinophil cationic protein (ECP) as a result of degranulation (Compr. Ther. 20: 651 (1994)). These granule proteins exhibit cytotoxic activity, and thus, ablate and damage epithelial cells. The ablation of epithelial cells results in the exposure of sensory nerve endings, enhances the permeability of the epithelium, and causes the loss of the epithelium-derived smooth muscle relaxing factor. Furthermore, eosinophils are known to secrete leukotriene C4 (LTC4) and Platelet activation factor (PAF), which have the activity of enhancing bronchial smooth muscle constriction, and platelet activating factor (PAF). It has been suggested that these reactions are repeated in the body and become chronic resulting in bronchial wall thickening and respiratory hypersensitivity.

Specifically, several reports have suggested the deep involvement of IL-4 and IL-13 in allergic reactions. For example, it is known that respiratory hypersensitivity disappears in IL-4-knockout mice (Yssel, H. and Groux, H., Int. Arch. Allergy Immunol., 121: 10-18, 2000). In a mouse model, IL-13 has been shown to be involved in forming an asthma-like pathology regardless of IgE production and the Th2 type (Wills-Karp, M. et al., Science, 2822: 2258-2261, 1998; Grunig, G. et al., Science, 282: 2261-2263, 1998; Zhu, Z. et al. , J. Clin. Invest., 103: 779-788, 1999). In addition, IL-4 receptors and IL-13 receptors are highly expressed in human respiratory epithelial cells and bronchial smooth muscles (Heinzmann, A. et al. , Hum. Mol. Genet., 9: 549-559, 2000). Accordingly, these tissues are thought to be the targets of IL-4 and IL-13. On the other hand, SNPs present in IL-4 receptor α and IL-13 have been shown to be one of the genetic causes of allergic diseases (Mitsuyasu, H. et al., Nature Genet., 19: 119-120, 1998; Mitsuyasu, H. et al., J. Immunol. , 162: 1227-1231, 1999; Kruse, S. et al . , Immnol., 96: 365-371, 1999; Heinzmann, A. et al., Hum. Mol. Genet., 9: 549-559, 2000).

Furthermore, IL-4 and IL-13 have been reported to suppress the expression of the β and γ subunits of amiloride-sensitive epithelial sodium channel (ENaC) and increase the expression of cystic fibrosis transmembrane conductance regulator (CFTR) in tracheal epithelial cells. This suppresses Na⁺ release and enhances Cl⁻ secretion. As a result, water secretion is assumed to increase in the bronchial lumen (Galietta L. J. V. et al., J. Immunol. 168: 839-45 (2002)). Therapeutic agents that target the signaling molecules of IL-4 or IL-13, such as IL-4 agonists, soluble IL-4 receptor α (Borish L. C. et al. , Am. J. Respir. Crit. Care Med. 160: 912-22 (1999) ) , soluble IL-13 receptor α2, anti-IL-13 antibodies, and anti-IL-4 antibodies, have already been clinically applied and are expected to be effective in treating bronchial asthma.

Inflammation in the respiratory tract is known to elevate the expression levels of cytokines and adhesion molecules. Genes encoding such cytokines and adhesion molecules, which participate in the onset of allergic diseases such as bronchial asthma, can be targets in drug discovery. Specifically, patients can be diagnosed for the onset of symptoms, seriousness, response to medical treatments, or such, by detecting variations in the expression levels of these genes. Furthermore, patients can be treated using a substance that controls the expression level of such genes or regulates protein activity.

There are several commercially available expectorants for removing sputum, the cause of death by suffocation in asthma. However, until recently, available expectorant types were restricted to those that contain an active SH group, and those that hydrolyze or lubricate the mucus. However, "fudosteine" (a low-molecular-weight oral drug), which was jointly developed by two Japanese pharmaceutical companies, SS Pharmaceutical Co. Ltd.,and Mitsubishi Pharma Corporation, and released last December, is a pharmaceutical agent having an activity to suppress goblet cell hyperplasia.

In addition, Genaera Corporation in the United States has reported that the hCLCA1 gene is closely associated with the production of IL-9 and mucus in the mucosal epithelia in asthma patients (J. Allergy Clin. Immunol. 109: 246-50 (2002) ) ; the hCLCA1 gene is the human counterpart of Gob-5 reported by Takeda Chemical Industries LTD. , Japan (Proc. Natl. Acad. Sci. USA 98: 5175-80 (2001)). Furthermore, clinical trials have already been launched for the low-molecular-weight oral drug "LOMUCIN" that inhibits the function of this gene.

In the bronchia of asthma patients, the aggravation of the disease state induces differentiation of respiratory epithelial cells into goblet cells and proliferation of these cells. Goblet cells produce a huge glycoprotein called mucin. This protein contributes to the production of sputum, which causes breathing difficulties and is a leading cause of death in chronic bronchial asthma. The increase in the number of goblet cells, which are secretory cells, enhances secretions in the respiratory tract. Thus, such secreted material enhances the obstruction of the respiratory tract and largely contributes to the worsening of asthma symptoms. However, the mechanism underlying goblet cell differentiation in the respiratory epithelium is still unknown.

The term "chronic obstructive pulmonary disease" refers to mainly pulmonary emphysema and chronic bronchitis. Shortness of breath is a main symptom of pulmonary emphysema; cough and sputum are main symptoms of chronic bronchitis. These are the major subjective symptoms of respiratory diseases in aged patients. In addition to aging, smoking is deeply involved in the onset of chronic obstructive pulmonary diseases. In pulmonary emphysema, the walls of pulmonary alveoli at the end of bronchioles are damaged and greatly swollen; the elasticity and contractility of the walls are impaired, and thus, the lungs have difficulty contracting during exhalation. This often causes shortness of breath. In addition, bronchial disorders result in bronchial obstruction, which is caused by swollen mucous membranes, sputum, and such. In chronic bronchitis, chronic inflammation and edema in the bronchia induce differentiation of bronchial epithelial cells into goblet cells, which results in the overproduction of secretory material. This results in coughs that produce sputum. In chronic obstructive pulmonary diseases, narrowed bronchia and damaged lungs cannot be restored to the original state. Furthermore, there are about 220,000 and 1,400,00 patients with chronic obstructive pulmonary diseases in Japan and the United States, respectively, and the diseases are the fourth leading cause of death in both countries. Thus, chronic obstructive pulmonary diseases are quite serious.

There is a report suggesting the correlation between chronic obstructive pulmonary diseases and IL-13 (Zheng T. et al, J Clin. Invest.; 106, 1081-1093, 2000). According to this report, transgenic mice in which respiratory epithelial cells were allowed to express IL-13, developed pulmonary emphysema, inflammation, and goblet cell hyperplasia.

### SUMMARY OF THE INVENTION

As described above, in bronchial asthma or chronic obstructive pulmonary diseases, changes in respiratory epithelial cells are crucial factors constituting the disease states. One of the morbid changes of respiratory epithelial cells is the differentiation into goblet cells. An objective of the present invention is to identify genes associated with the differentiation into goblet cells. Another objective of the present invention is to provide diagnostic markers for bronchial asthma and drug discovery targets.

Drugs suppressing the differentiation into goblet cells in respiratory epithelial tissues were developed only recently. This is a new approach in drug discovery. Once the mechanism underlying the differentiation into goblet cells is elucidated, it may be possible to establish a basic treatment for bronchial asthma. Furthermore, agents that affect the process of goblet cell differentiation are predicted to be useful in the treatment of diseases involving inflammation and overproduction of mucus, such as chronic obstructive pulmonary diseases, cystic fibrosis, chronic sinusitis, bronchiectasis, diffuse panbronchiolitis, as well as asthma.

A culture method (called the "air interface (AI) method") for differentiating human respiratory epithelial cells into goblet cells in the presence of IL-13 has been established by researchers of the Department of Geriatric and Respiratory Medicine, Tohoku University School of Medicine, Japan, who are collaborators in the present invention. Using this method, the present inventors predicted that goblet cell differentiation-associated genes can be identified by elucidating which gene expression varies in respiratory epithelial cells when stimulated by IL-13.

Conventionally, bronchial epithelial cells played a vital role in studies concerning the transport of water and electrolytes in humans and other animals. Moreover, particularly in humans, these cells have been significant in clarifying disease states of respiratory tract infections in cystic fibrosis and in establishing therapeutic methods. Over the past two decades, methods for culturing (*in vitro*) respiratory epithelial cells obtained from protease-treated trachea tissues have been improved by improving culture media and using growth-promoting substances. In addition, the AI method has been established, in which cilia and secretory granules can be produced *in vitro* by culturing cells under conditions similar to the environment around respiratory epithelial cells *in vivo.* In the AI method, the culture medium facing the mucous membrane side (apical side) of the cells is removed exposing cells to air while water and nutrients are supplied from the chorionic membrane side (basolateral side) (Van Scott MR., Exp Lung Res, 11: 75-94, 1986, Widdicombe JH., Am J Physiol, 258:L13-L18, 1990, Kim KC, J Biol Chem, 260: 4021-4027, 1985, Adler KB, Am J Respir Cell Mol Biol, 2:145-154, 1990).

Human bronchial epithelial cells cultured in the presence of human IL-13 using the air interface method were reported to express TGF-α (Booth BW, Adler KB, Bonner JC, Tournier F, Martin LD. Interleukin-13 induces proliferation of human airway epithelial cells in vitro via a mechanism mediated by transforming growth factor-α. Am J Respir Cell Mol Biol . 2001 Dec; 25(6): 739-743). In addition, the ion transport ability of human bronchial epithelial cells has been evaluated in a previous report, in which cells were cultured by the air interface method in the presence of IL-13 (Danahay H, Am J Physiol Lung Cell Mol Physiol, 282:L226-L236, 2002). However, these reports make no reference to goblet cell differentiation, and have not conducted any exhaustive gene expression analyses.

Furthermore, bronchial epithelial cells of guinea pigs has been reported to differentiate into goblet cells when cultured in the presence of human IL-13 for 14 days using the air-liquid interface method (Kondo, M., Tamaoki, J., Takeyama, K., Nakata, J. and Nagai, A. Interleukin-13 induces goblet cell differentiation in a primary cell culture from Guinea pig tracheal epithelium. Am J Respir Cell Mol Biol 27,536-541, 2002). However, there are no reports on exhaustive analyses of genes expressed in human bronchial epithelial cells cultured by the method described above.

On the other hand, the present applicants have identified eight types of allergy-associated genes whose expression levels decrease upon IL-4 or IL-13 stimulation in several lots of primary human respiratory epithelial cell cultures (Unexamined Published Japanese Patent Application No. (JP-A) 2002-191398). The applicants have also identified six types of allergy-associated genes whose expression levels greatly increase in several lots under the same conditions as described above (WO 02/052006 A1). The gene expression analyses in these two previous patent applications were carried out using a conventional culture method which induces no goblet cell differentiation.

Using oligonucleotide microarrays (GeneChip®, Affymetrix, Inc.) and air interface method, the present inventors compared the expression profiles of genes expressed in respiratory epithelial cells stimulated with IL-13 for goblet cell differentiation, with those of cells not stimulated with IL-13. The inventors selected genes whose expression levels increased by two folds or more or decreased by half or more of the initial levels as a result of the differentiation, and determined the expression levels of the genes. Then, the inventors confirmed the variation of the expression level of marker genes selected from the group described below in (a) or (b).

Furthermore, with respect to the mouse homologs of the human genes selected by the method described above, the inventors detected variations in the expression levels in respiratory hypersensitivity model mice. As a result, the variation pattern of expression levels of the mouse homologs coincided well with that of human genes.

The nucleotide sequences of the respective marker genes listed in (a) and (b) are known. The functions of the proteins encoded by each marker gene are described in the references listed in the "References" section in Tables 3-19 (increased) and Tables 20-36 (decreased) below. The nucleotide sequences of the mouse homologs of the marker genes of the present invention are also known. The functions of the proteins encoded by the mouse homologues of the respective marker genes are described in the references listed in the "References" section in Tables 40-62 (increased) and Tables 63-83 (decreased) below.

Among these groups of genes, some genes have been reported to be directly related to bronchial asthma. However, most of the genes have not been shown to be associated with an allergic disease. Furthermore, even for genes that are reported to be associated with bronchial asthma, there are no reports that focus on the aspect of combinations with other co-expressing genes whose expression levels vary at the same timing that the asthma-related genes do.

A close relationship between bronchial asthma symptoms and the marker genes of the present invention is suggested by the finding that the expression levels of marker genes vary in the differentiation process of respiratory epithelial cells into goblet cells. The relationship between the allergic response of the respiratory epithelium and the marker genes of the present invention was verified by the fact that the variation pattern of the expression levels of mouse homologs in the respiratory hypersensitivity mouse model is consistent with that in humans. Based on the findings described above, the present inventors revealed that tests for bronchial asthma or chronic obstructive pulmonary disease and screenings for therapeutic agents can be achieved by using as a marker the expression level of each marker gene or the activity of the protein encoded by each marker gene.

Specifically, the present invention relates to the following methods of testing for bronchial asthma or chronic obstructive pulmonary disease and the following methods of screening for candidate compounds for treating bronchial asthma or chronic obstructive pulmonary disease:
[1] a method of testing for bronchial asthma or chronic obstructive pulmonary disease, which comprises the steps of:
   (1) determining the expression level of a marker gene in a biological sample from a subject;
   (2) comparing the expression level determined in step (1) with the expression level of the marker gene in a biological sample from a healthy subject; and
   (3) judging the subject to have bronchial asthma or chronic obstructive pulmonary disease when the result of the comparison in step (2) indicates that (i) the expression level of the marker gene in the subject is higher than that in the control when the marker gene is a gene according to (a) or (ii) the expression level of the marker gene in the subject is lower than that in the control when said marker gene is a gene according to (b);
   wherein the marker gene is any one selected from the group according to (a) or (b) :
   (a) a group of genes whose expression levels increase when respiratory epithelial cells are stimulated with interleukin-13, and comprise any one of the nucleotide sequences of SEQ ID NOs: 25 to 310;
   (b) a group of genes whose expression levels decrease when respiratory epithelial cells are stimulated with interleukin-13 and comprise any one of the nucleotide sequences of SEQ ID NOs: 311 to 547;
[2] the testing method according to [1], wherein the biological sample is a respiratory epithelial cell;
[3] the testing method according to [1], wherein the gene expression level is measured by PCR analysis of the cDNA;
[4] the testing method according to [1], wherein the gene expression level is measured by detecting the protein encoded by the marker gene;
[5] a reagent for testing for bronchial asthma or chronic obstructive pulmonary disease, wherein the reagent comprises a polynucleotide comprising the nucleotide sequence of a marker gene, or an oligonucleotide having at least 15 nucleotides and comprising a nucleotide sequence complementary to the complementary strand of the nucleotide sequence of the marker gene, and wherein, the marker gene is any one selected from the group according to (a) or (b) in [1];
[6] a reagent for testing for bronchial asthma or chronic obstructive pulmonary disease, wherein the reagent comprises an antibody that recognizes a protein encoded by a marker gene, and wherein the marker gene is any one selected from the group according to (a) or (b) in [1];
[7] a method of screening for a therapeutic agent for bronchial asthma or chronic obstructive pulmonary disease, wherein the marker gene is any one selected from the group according to (a) or (b) in [1], and wherein the method comprises the steps of:
   (1) contacting a candidate compound with a cell expressing the marker gene;
   (2) measuring the expression level of said gene; and
   (3) selecting a compound that decreases the expression level of a marker gene belonging to group (a) or increases the expression level of a marker gene belonging to group (b), as compared to that in a control with which the compound has not been contacted;
[8] the method according to [7], wherein the cell is a respiratory epithelial cell or a goblet cell;
[9] the method according to [8], which comprises the step of culturing the respiratory epithelial cells under conditions in which culture medium is removed from the apical side of said cells and the culture medium is supplied from the basolateral side of the cells;
[10] a kit for screening for a candidate compound for a therapeutic agent to treat bronchial asthma or chronic obstructive pulmonary disease, wherein the kit comprises (i) a polynucleotide comprising the nucleotide sequence of a marker gene, or an oligonucleotide having at least 15 nucleotides and comprising a nucleotide sequence that is complementary to the complementary strand of the polynucleotide, and (ii) a cell expressing the marker gene, and wherein the marker gene is any one selected from the group according to (a) or (b) in [1];
[11] a kit for screening for a candidate compound for a therapeutic agent to treat bronchial asthma or chronic obstructive pulmonary disease, wherein the kit comprises (i) an antibody that recognizes a protein encoded by a marker gene, and (ii) a cell expressing the marker gene, wherein the marker gene is selected from the group according to (a) or (b) in [1];
[12] the kit according to [10] or [11], which further comprises a cell-supporting material to culture respiratory epithelial cells under conditions in which the culture medium is supplied from the basolateral side of the cells;
[13] the kit according to [12], which further comprises respiratory epithelial cells;
[14] an animal model for bronchial asthma or chronic obstructive pulmonary disease, wherein the animal is a transgenic nonhuman vertebrate wherein the expression level of a marker gene, or a gene functionally equivalent to the marker gene, has been increased in the respiratory tissue, wherein the marker gene is any one selected from the group according to (a) in [1] or the following (A):
   (A) a group of genes whose expression levels increase in the lung of an animal model for bronchial hypersensitivity induced by an exposure to the ovalbumin antigen, wherein the genes comprise any one of the nucleotide sequences of SEQ ID NOs: 954 to 1174;
[15] the animal model according to [14], wherein the nonhuman vertebrate is a mouse;
[16] an animal model for bronchial asthma or chronic obstructive pulmonary disease, wherein the animal is a transgenic nonhuman vertebrate wherein the expression level of a marker gene, or a gene functionally equivalent to the marker gene, has been decreased in the respiratory tissue, wherein the marker gene is any one selected from the group according to (b) in [1] or the following (B):
   (B) a group of genes whose expression levels decrease in the lung of an animal model for bronchial hypersensitivity induced by an exposure to the ovalbumin antigen, wherein the genes comprise any one of the nucleotide sequences of SEQ ID NOs: 1376 to 1515;
[17] the animal model according to [16], wherein the nonhuman vertebrate is a mouse;
[18] a method for producing an animal model for bronchial asthma or chronic obstructive pulmonary disease, which comprises the step of administering to a mouse any one of (i) to (iv):
   (i) a polynucleotide comprising the nucleotide sequence constituting any one of the genes selected from the gene group according to (A) in [14];
   (ii) a protein encoded by a polynucleotide comprising the nucleotide sequence constituting any one of the genes selected from the gene group according to [A] in [14];
   (iii) an antisense nucleic acid of a polynucleotide comprising the nucleotide sequence constituting any one of the genes selected from the gene group according to (B) in [16], a ribozyme, or a polynucleotide that suppresses the expression of a gene through an RNAi (RNA interference) effect; and,
   (iv) an antibody that binds to a protein encoded by a polynucleotide comprising the nucleotide sequence constituting any one of the genes selected from the gene group according to (B) in [16], or a fragment comprising an antigen-binding region thereof;
[19] an inducer that induces bronchial asthma in a mouse, wherein said inducer comprises as an active ingredient any one of (i) to (iv) in [18];
[20] a method of screening for a therapeutic agent for bronchial asthma or chronic obstructive pulmonary disease comprising the steps of:
   (1) administering a candidate compound to an animal subject,
   (2) assaying the expression level of the marker gene in a biological sample obtained from the animal subject, and
   (3) selecting a compound that decreases the expression level of a marker gene belonging to group (a) or (A) , or a compound that increases the expression level of a marker gene belonging to group (b) or (B) , as compared to that in a control with which the candidate compound has not been contacted,
   wherein the marker gene is any one selected from the group consisting of (a) or (b) in [1], (A) in [14], and (B) in [16], or a gene functionally equivalent to said marker gene;
[21] a method of screening for a therapeutic agent for bronchial asthma or chronic obstructive pulmonary disease comprising the steps of:
   (1) contacting a candidate compound with a cell into which a vector has been introduced, wherein the vector comprises a transcriptional regulatory region of a marker gene and a reporter gene that is expressed under the control of the transcriptional regulatory region,
   (2) measuring the activity of the reporter gene, and
   (3) selecting a compound that decreases the expression level of the reporter gene when the marker gene belongs to group (a), or a compound that increases the expression level of the reporter gene when the marker gene belongs to group (b) , as compared to that in a control with which the candidate compound has not been contacted,
   wherein the marker gene is any one selected from the group according to (a) or (b)in [1], or a gene functionally equivalent to the marker gene;
[22] a method of screening for a therapeutic agent for bronchial asthma or chronic obstructive pulmonary disease comprising the steps of:
   (1) contacting a candidate compound with a protein encoded by a marker gene,
   (2) measuring the activity of the protein, and
   (3) selecting a compound that decreases the activity when the marker gene belongs to group (a) , or a compound that increases the activity when the marker gene belongs to the group (b) , as compared to that in a control where the candidate compound has not been contacted,
   wherein the marker gene is any one selected from the group according to (a) or (b)in [1], or a gene functionally equivalent to the marker gene;
[23] a therapeutic agent for bronchial asthma or chronic obstructive pulmonary disease, which comprises as an active ingredient a compound obtainable by any one of the screening methods according to [7], [20], [21], and [22];
[24] a therapeutic agent for bronchial asthma or chronic obstructive pulmonary disease, which comprises as an active ingredient a marker gene or an antisense nucleic acid corresponding to a portion of the marker gene, a ribozyme, or a polynucleotide that suppresses the expression of the gene through an RNAi effect, wherein the marker gene is any one selected from the group according to (a) in [1] ;
[25] a therapeutic agent for bronchial asthma or chronic obstructive pulmonary disease, which comprises as an active ingredient an antibody recognizing a protein encoded by a marker gene, wherein the marker gene is any one selected from the group according to (a) in [1];
[26] a therapeutic agent for bronchial asthma or chronic obstructive pulmonary disease, which comprises as an active ingredient a marker gene, or a protein encoded by a marker gene, wherein the marker gene is any one selected from the group according to (b) in [1]; and
[27] a DNA chip for testing for bronchial asthma or a chronic obstructive pulmonary disease, on which a probe has been immobilized to assay a marker gene, and wherein the marker gene comprises at least a single type of gene selected from group (a) and (b) in [1].

The present invention also relates to a method for treating bronchial asthma or a chronic obstructive pulmonary disease, which comprises the step of administering a compound obtainable by any one of the screening methods according to [7], [20], [21], and [22]. The present invention further relates to the use of a compound obtainable by any one of the screening methods according to [7], [20], [21], and [22] in producing pharmaceutical compositions to treat bronchial asthma or chronic obstructive pulmonary diseases.

In addition, the present invention relates to a method for treating bronchial asthma or chronic obstructive pulmonary disease, wherein the method comprises administering (i) or (ii) described below. Alternatively, the present invention relates to the use of (i) or (ii) described below, in producing pharmaceutical compositions for treating bronchial asthma or chronic obstructive pulmonary disease:
(i) a gene according to (a) described above or an antisense nucleic acid corresponding to a portion of the gene, a ribozyme, or a polynucleotide that suppresses the expression of the gene through an RNAi effect; and
(ii) an antibody recognizing a protein encoded by a gene according to (a) described above.
   Furthermore, the present invention relates to a method for treating bronchial asthma or a chronic obstructive pulmonary disease, which comprises administering (iii) or (iv) described below. Alternatively, the present invention relates to the use of (iii) or (iv) described below, in producing pharmaceutical compositions to treat bronchial asthma or chronic obstructive pulmonary diseases:
(iii) a gene according to (b) described above; and
(iv) a protein encoded by a gene according to (b) described above.

### BRIEF DESCRIPTION OF THE DRAWINGS

Fig. 1 is a schematic diagram of the air interface (AI) method.
Fig. 2 is a schematic diagram showing the differences in the culture procedure between the air interface (AI) method and the immersed feeding (IMM) method.
Fig. 3 is a graph showing variations in the expression level of the pendrin gene during goblet cell differentiation when cultured by the AI method or the IMM method. The expression level (copy number/ng RNA) is indicated in the vertical axis, and the culture conditions and duration (in days) are indicated in the horizontal axis.
Fig. 4 is a graph showing the expression levels of the pendrin (PDS) gene in the lung of the mouse asthma model. The expression level (copy number/ng RNA) is indicated in the vertical axis, and the conditions used to treat mice and the number of individuals in each treated group are indicated in the horizontal axis.
   naive: untreated group; S-sal: OVA antigen-sensitized, physiological saline-inhaled group; S-OVA: OVA antigen-sensitized, OVA antigen-inhaled group; Pred: OVA antigen-sensitized, OVA antigen-inhaled, Prednisolone-treated group
Fig. 5 shows micrographs (x 400) to determine the localization of the PDS mRNA in the lung tissues of the mouse asthma model using in situ hybridization.
Fig. 6 shows micrographs (x 400) of the lung tissues of the mouse asthma model. The tissues were subjected to hematoxylin-eosin (HE) staining, periodic acid-Schiff (PAS) staining, or Alcian Blue staining.

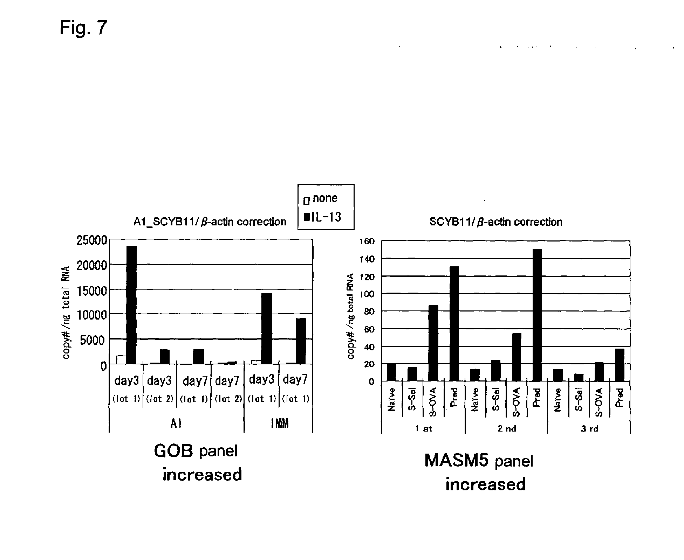

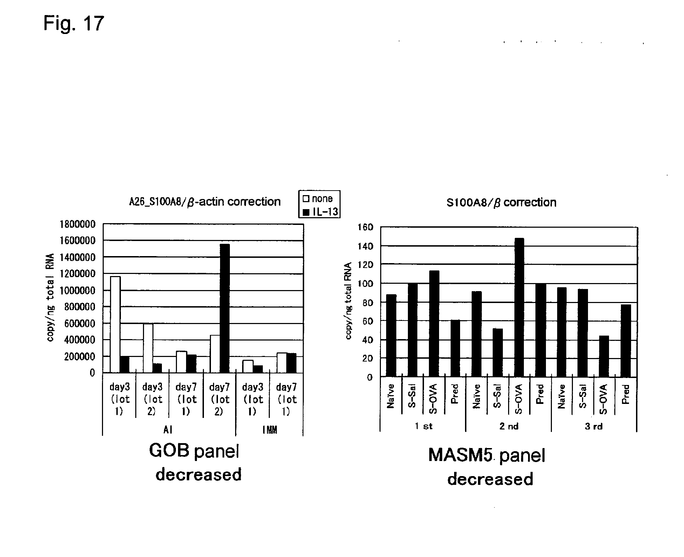

Figs 7-31 show the results of quantitative PCR assay analyses of genes whose expression levels varied in both humans and mice. The assays were carried out with ABI 7700 using cDNA of differentiated human goblet cells (human goblet cell differentiation model) or cDNA of the mouse OVA antigen-exposed bronchial hypersensitivity model. The vertical axis indicates the copy number of mRNA (copy number/ng total RNA). In the left panel, the horizontal axis indicates the culture conditions (AI method or IMM method) and duration (in days). In the right panel, the horizontal axis indicates the conditions used to treat mice and the number of antigen inhalation before collecting lung tissues.
   naive: untreated group; S-sal: OVA antigen-sensitized, physiological saline-inhaled group;
   S-OVA: OVA antigen-sensitized, OVA antigen-inhaled group; Pred: OVA antigen-sensitized, OVA antigen-inhaled, Prednisolone-treated group
Fig. 7 shows the assay result for the gene SCYB11. Likewise, the following Figures show the assay results for the respective genes. The symbols for the genes shown in the respective Figures are listed below.

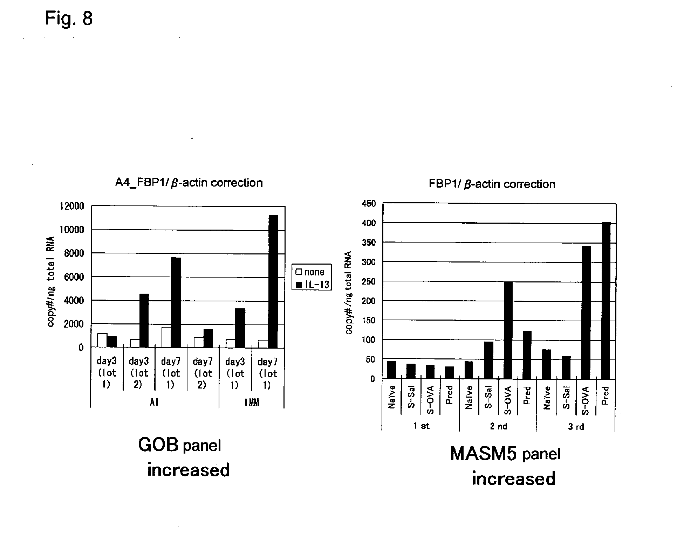

Fig. 8: FBP1

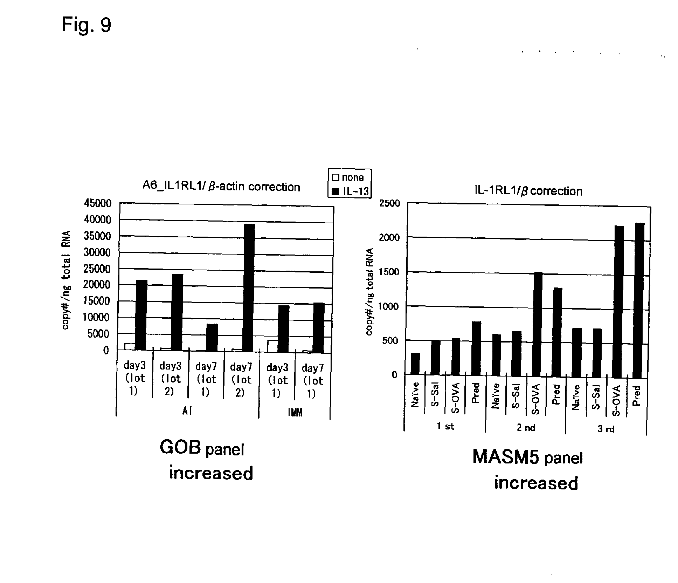

Fig. 9: IL1RL1

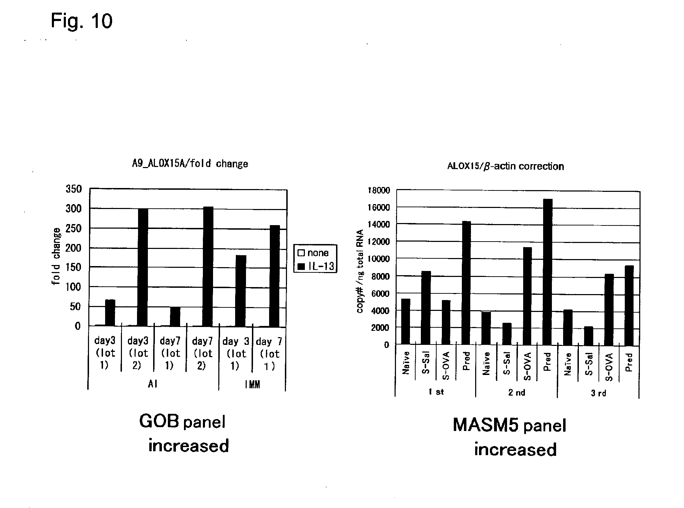

Fig. 10: ALOX15

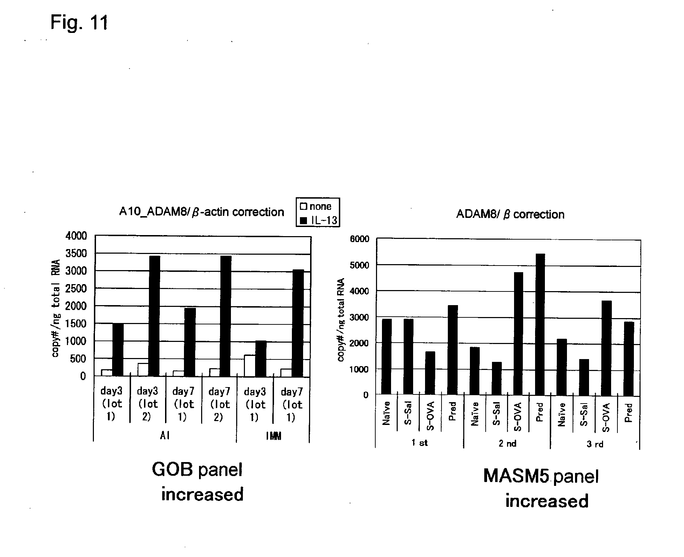

Fig. 11: ADAM8

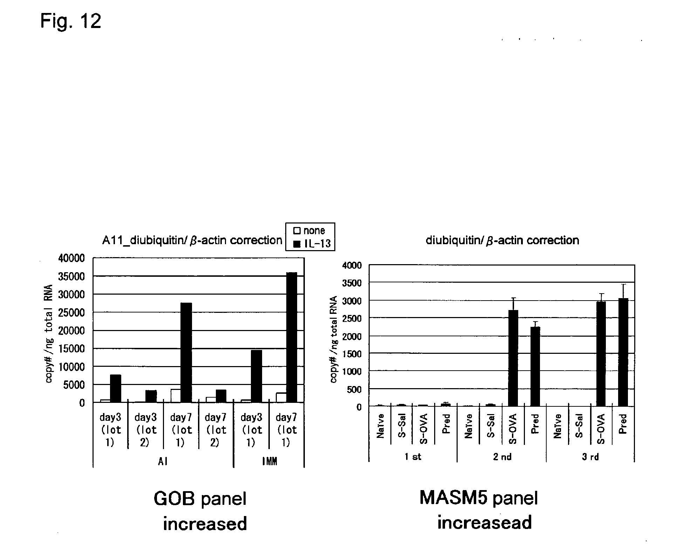

Fig. 12: diubiquitin

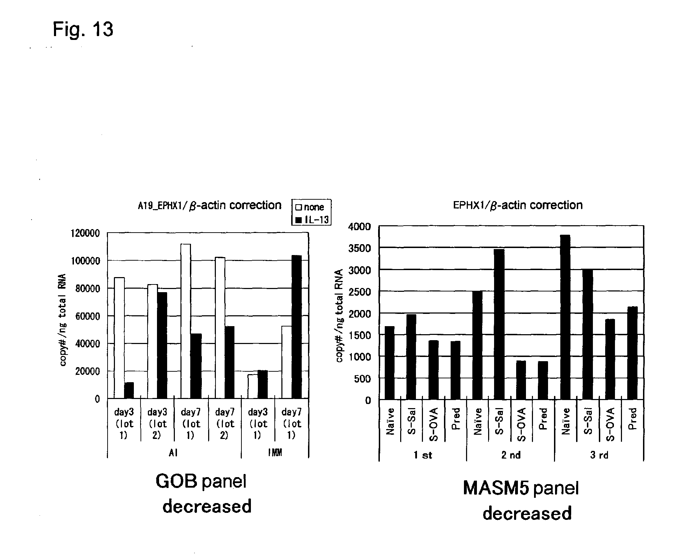

Fig. 13: EPHX1

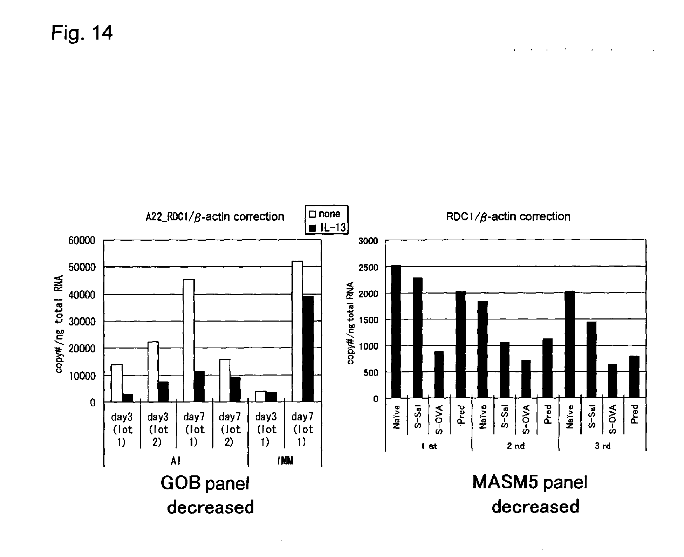

Fig. 14: RDC1

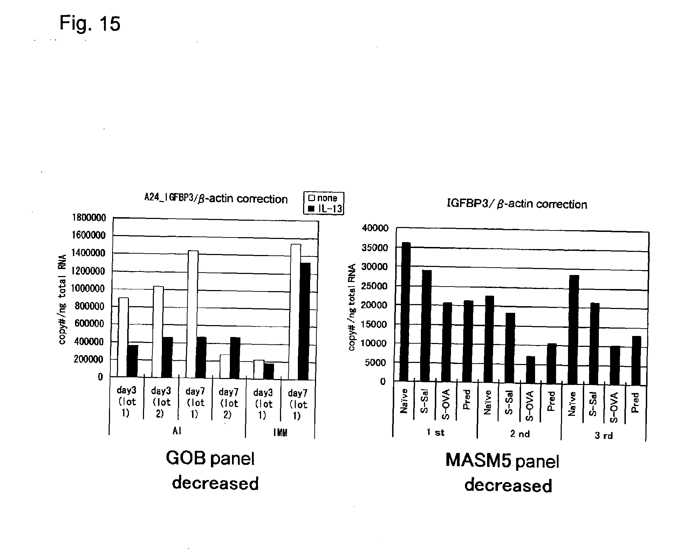

Fig. 15: IGFBP3

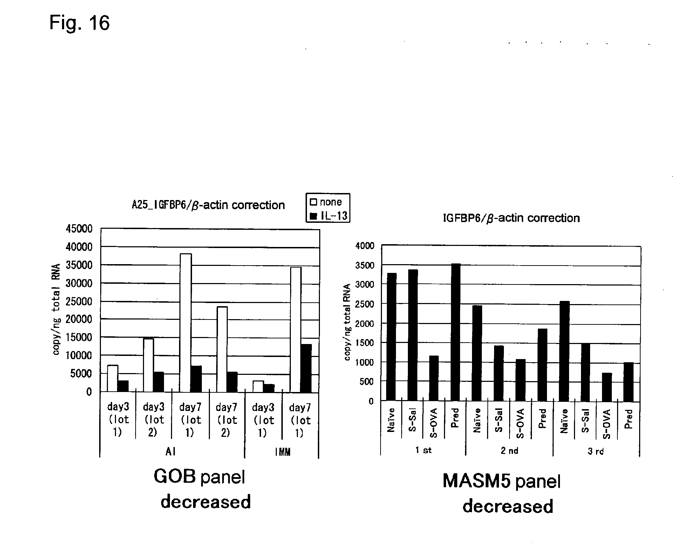

Fig. 16: IGFBP6
Fig. 17: S100A8

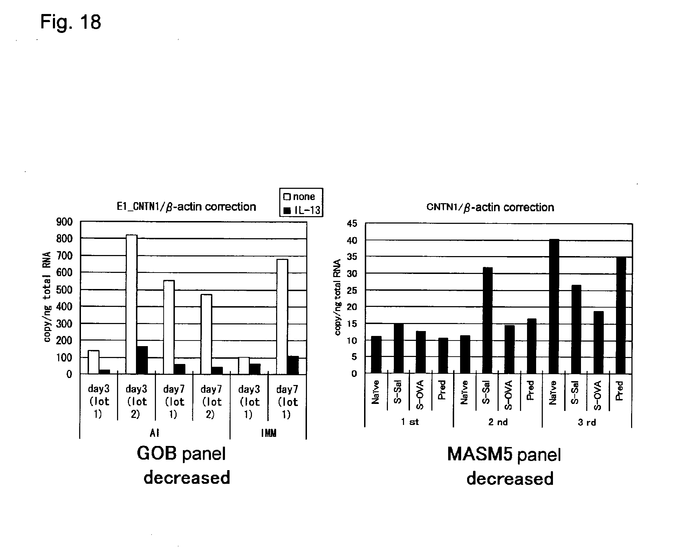

Fig. 18: CNTN1

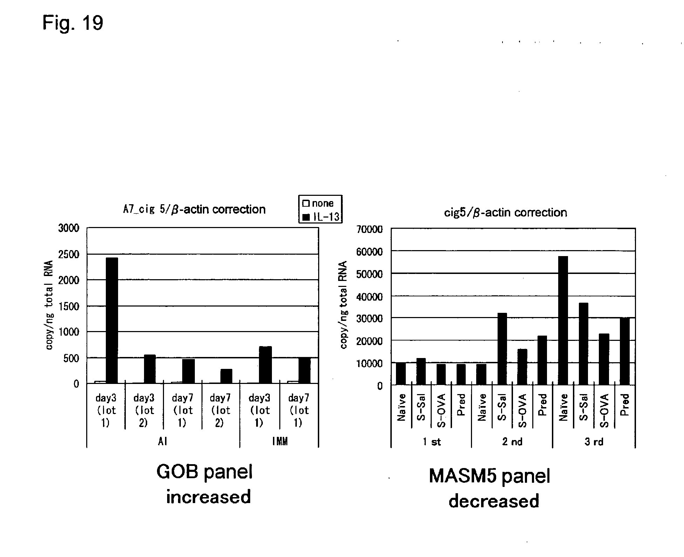

Fig. 19: cig5

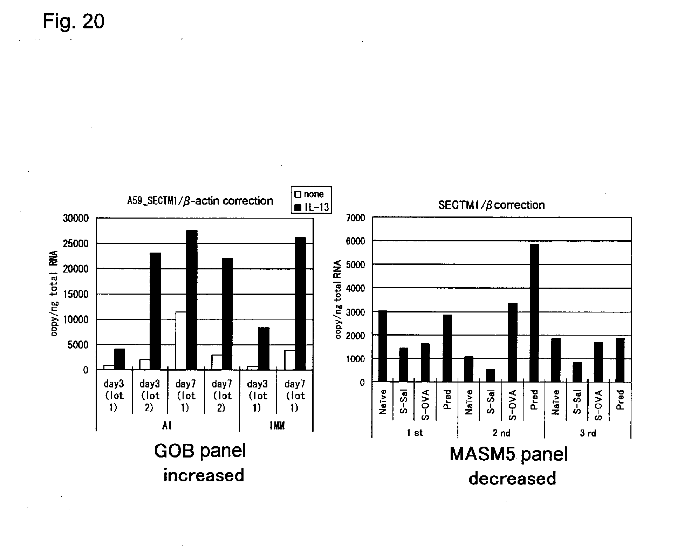

Fig. 20: SECTM1

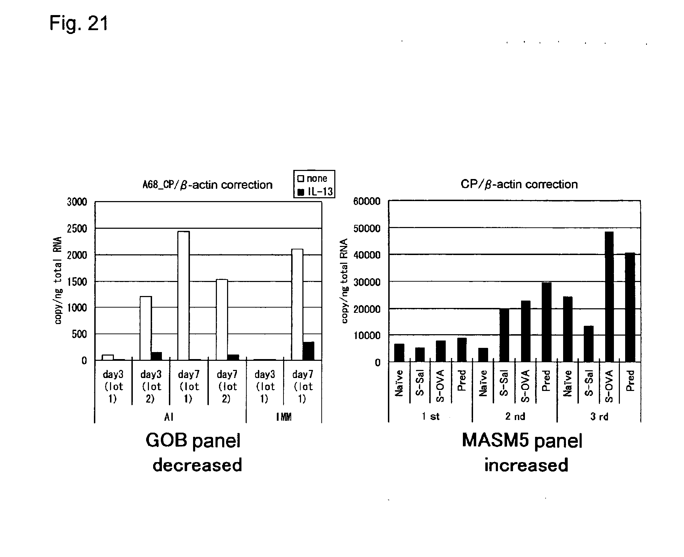

Fig. 21: CP

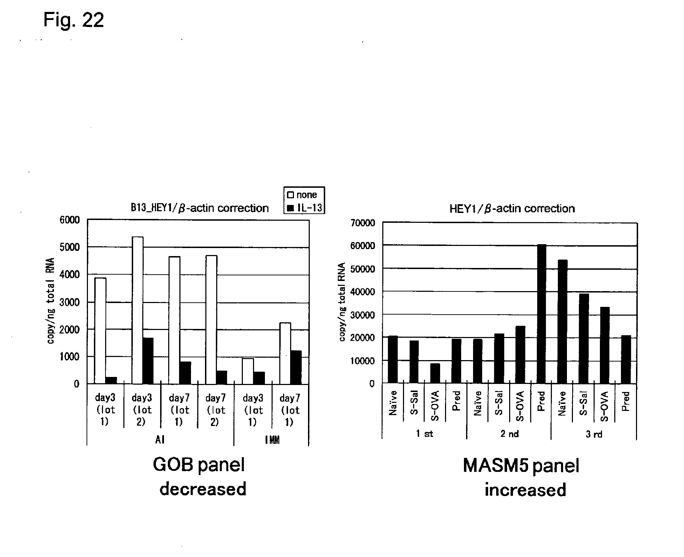

Fig. 22: HEY1

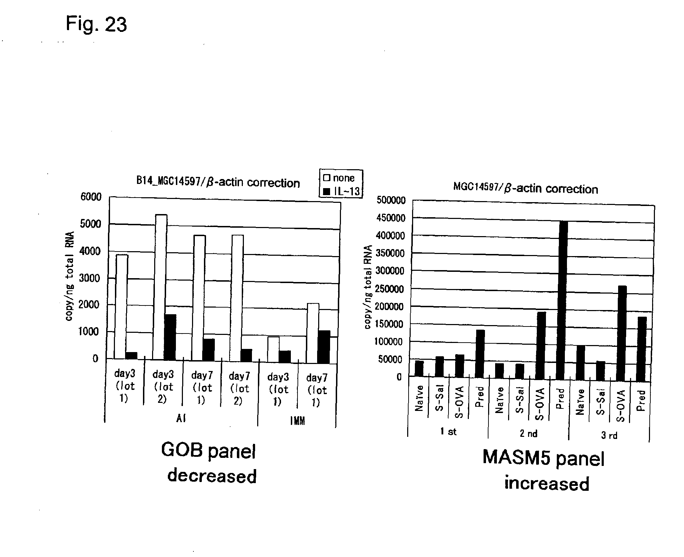

Fig. 23: MGC14597

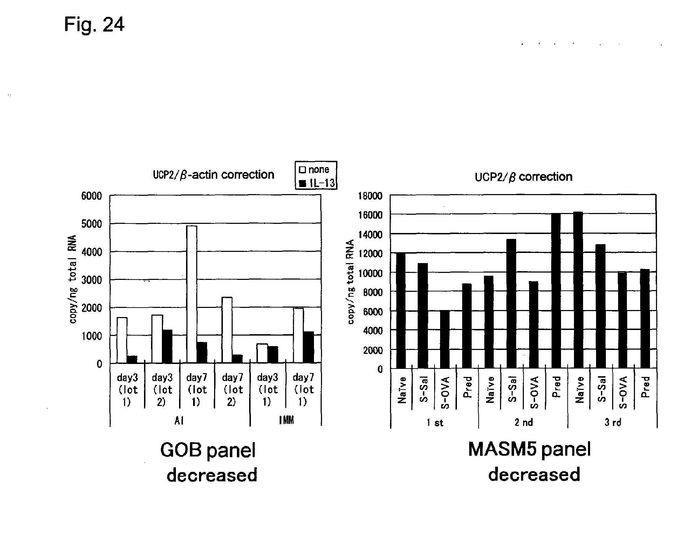

Fig. 24: UCP2

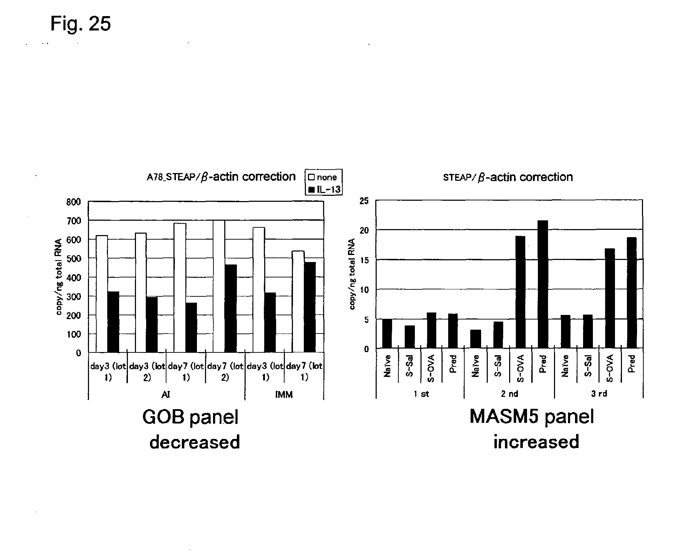

Fig. 25: STEAP

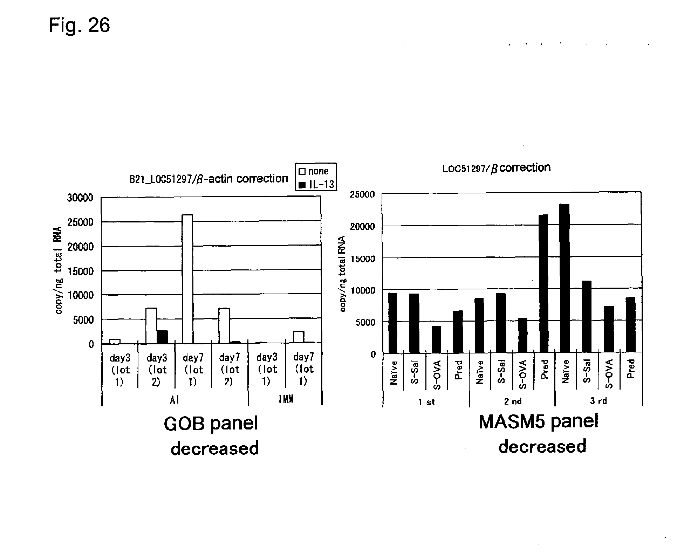

Fig. 26: LOC51297

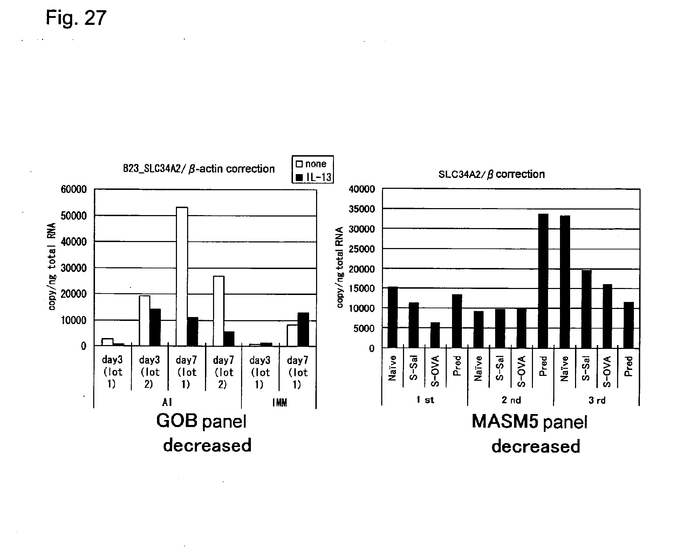

Fig. 27: SLC34A2

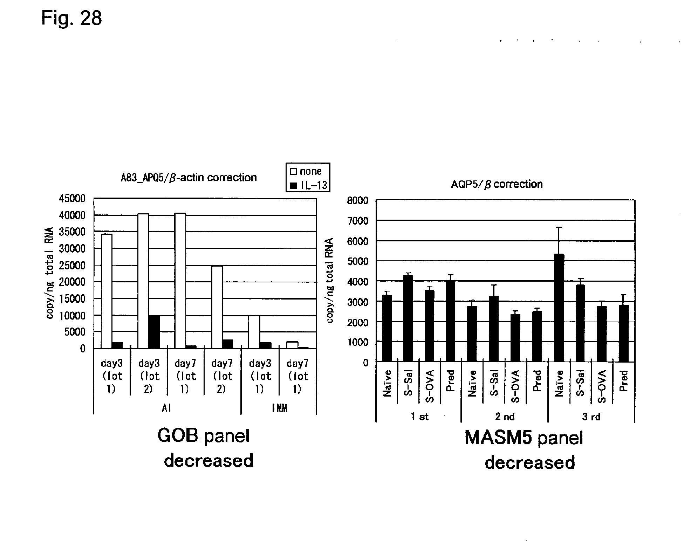

Fig. 28: AQP5

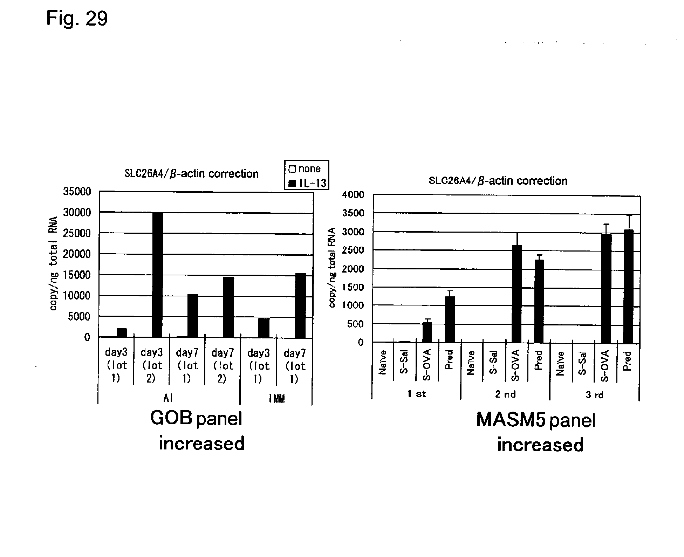

Fig. 29: SLC26A4

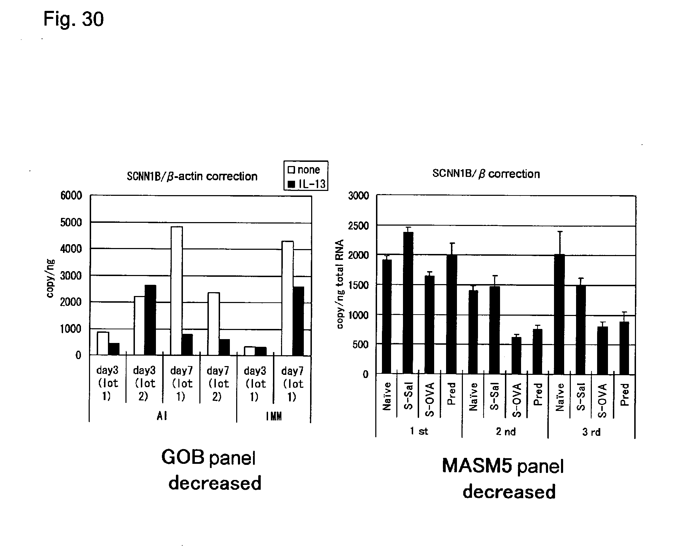

Fig. 30: SCNN1B

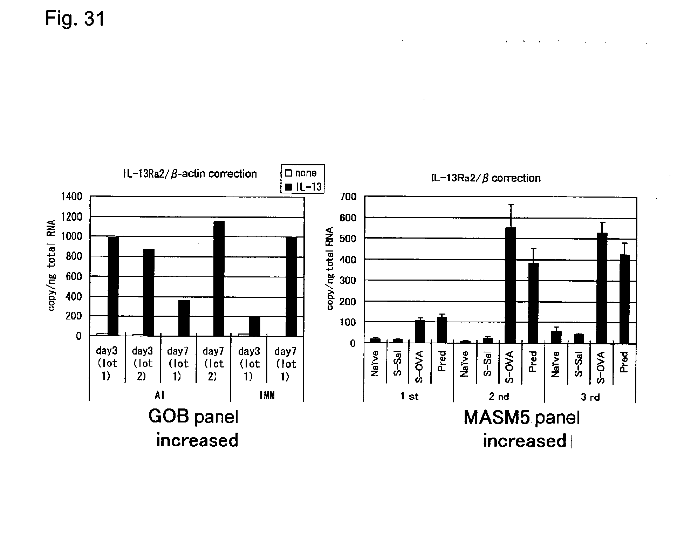

Fig. 31: IL-13Ra2

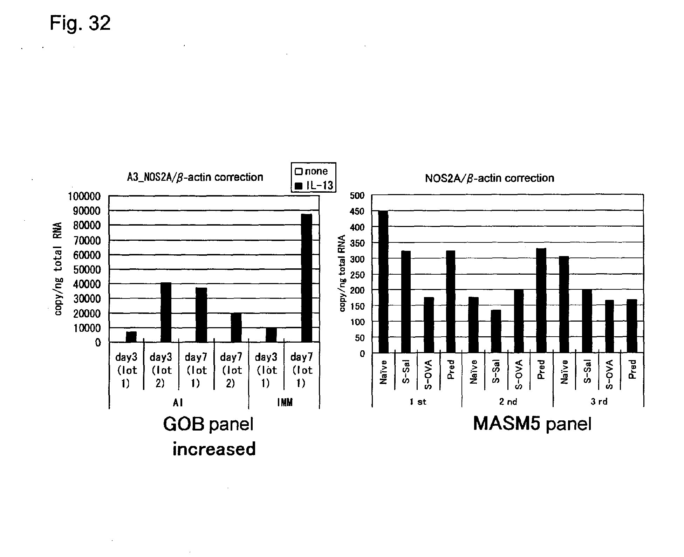

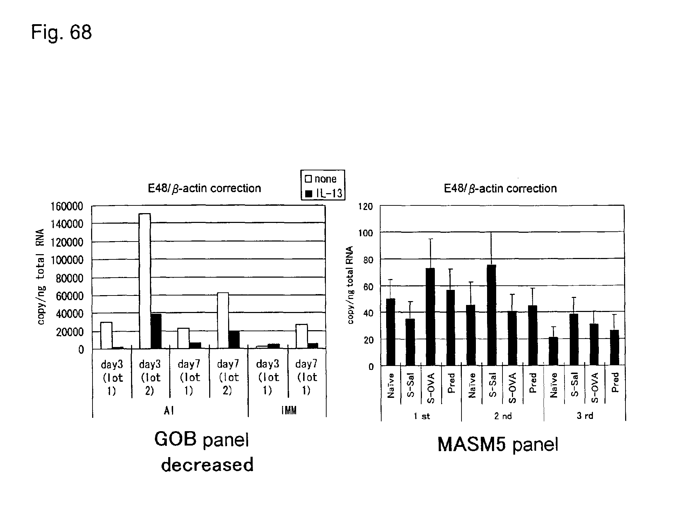

Figs 32-69 show the results of quantitative PCR assays for genes whose expression levels varied in humans. The assays were carried out with ABI 7700 using cDNA of differentiated human goblet cells (human goblet cell differentiation model) or cDNA of the mouse OVA antigen-exposed bronchial hypersensitivity model. The vertical axis indicates the copy number of mRNA (copy number/ng total RNA). In the left panel, the horizontal axis indicates the culture conditions (the AI method or the IMM method) and duration (in days). In the right panel, the horizontal axis indicates the conditions used to treat mice and the number of antigen inhalation before collecting lung tissues.
   naive: untreated group; S-sal: OVA antigen-sensitized, physiological saline-inhaled group;
   S-OVA: OVA antigen-sensitized, OVA antigen-inhaled group; Pred: OVA antigen-sensitized, OVA antigen-inhaled, Prednisolone-treated group
Figs 32-69 (varies in human)
Fig. 32 shows the assay result for the gene NOS2A. Likewise, the following figures show the assay results for the respective genes. The symbols for the genes shown in the respective figures are listed below.

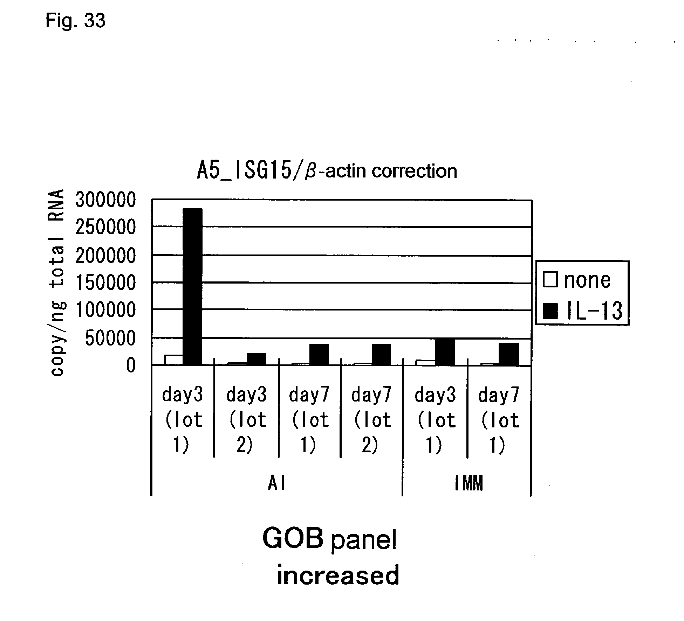

Fig. 33: ISG15 (only the result for the cDNA of human goblet cell differentiation model)

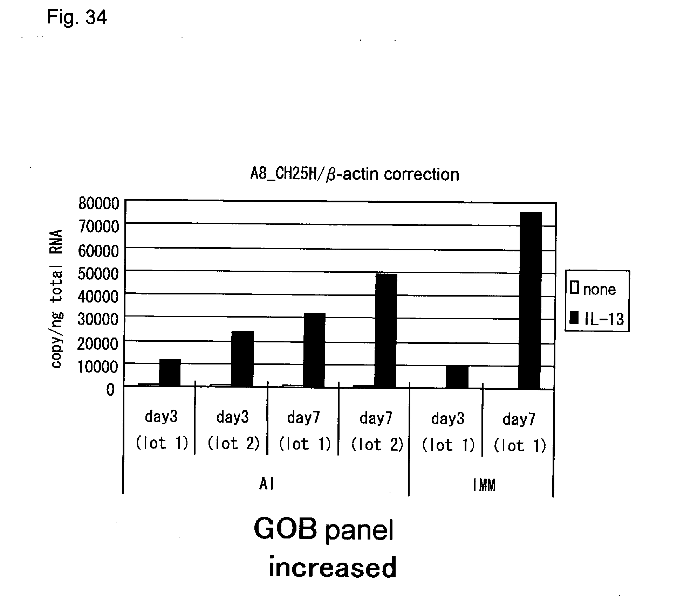

Fig. 34: CH25H (only the result for the cDNA of human goblet cell differentiation model)

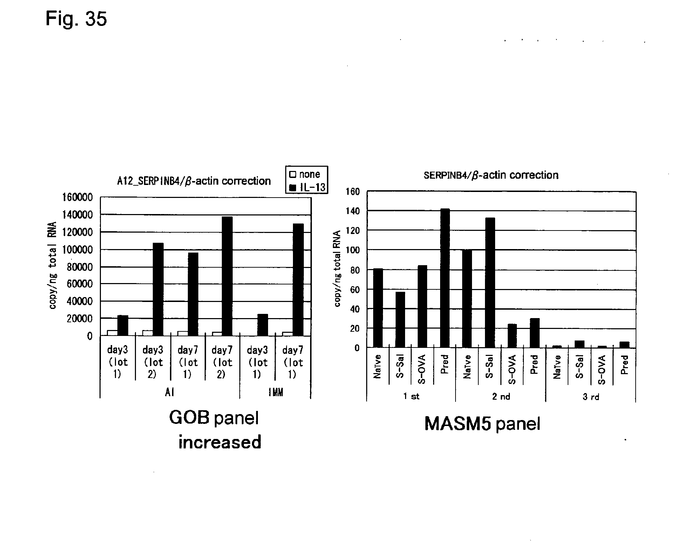

Fig. 35: SERPINB4

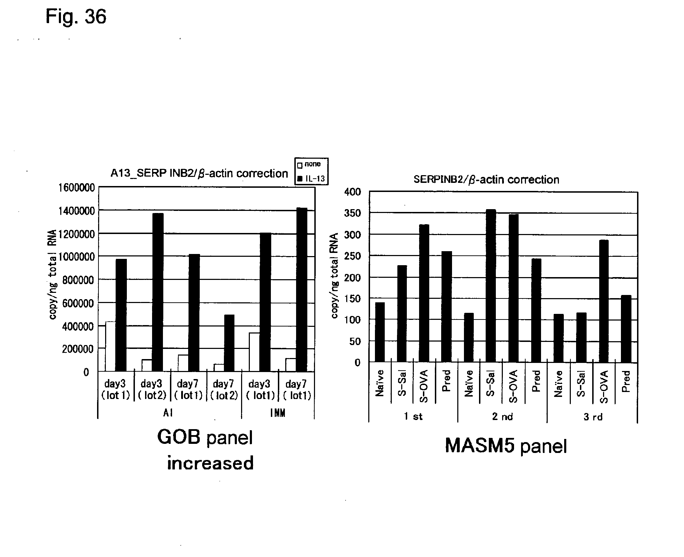

Fig. 36: SERPINB2

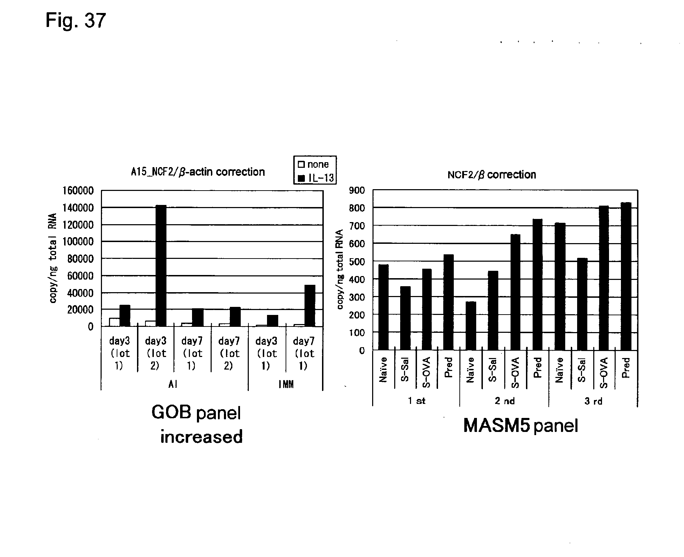

Fig. 37: NCF2

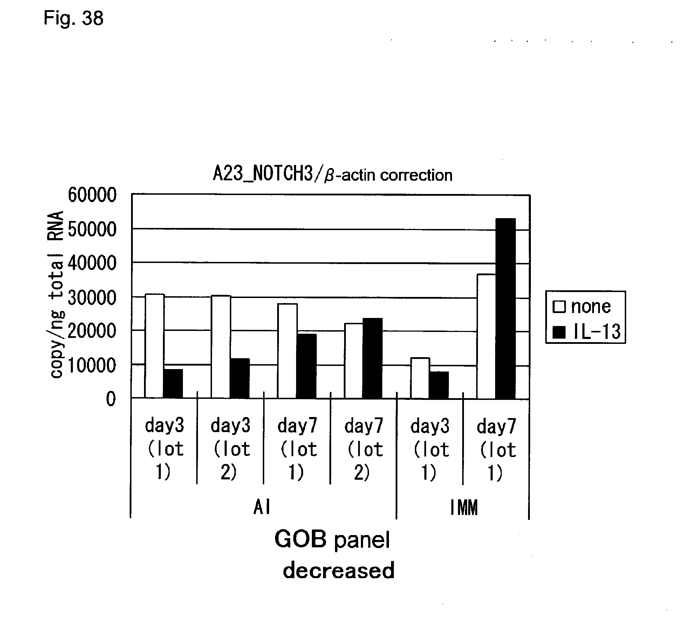

Fig. 38: NOTCH3 (only the result for the cDNA of human goblet cell differentiation model)

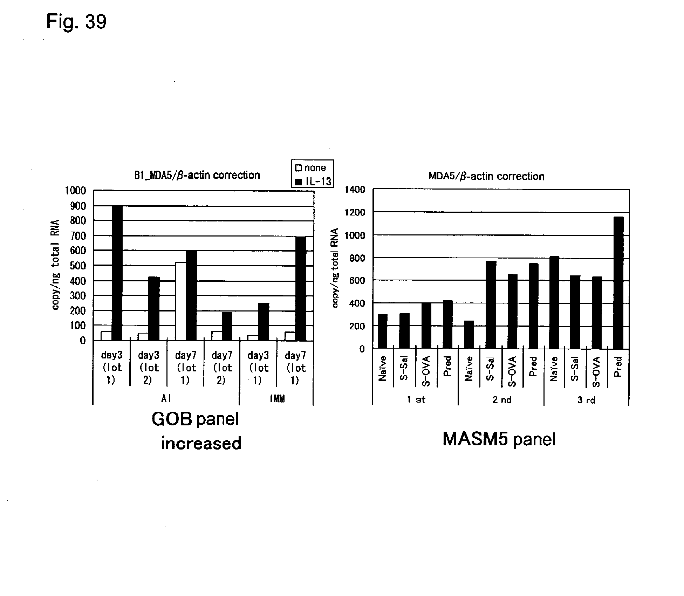

Fig. 39: MDA5

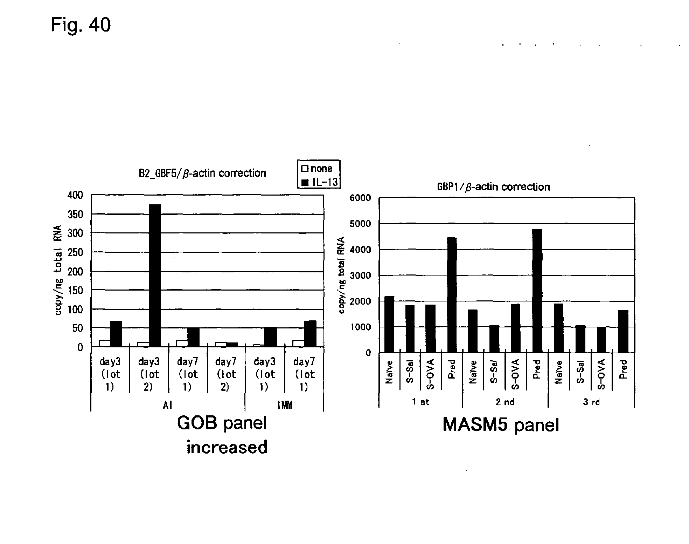

Fig. 40: GBF5

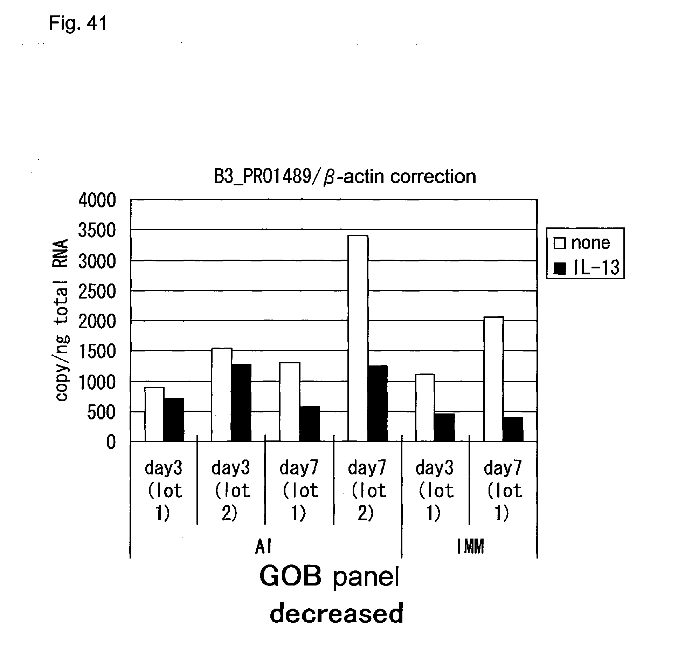

Fig. 41: PRO1489 (only the result for the cDNA of human goblet cell differentiation model)

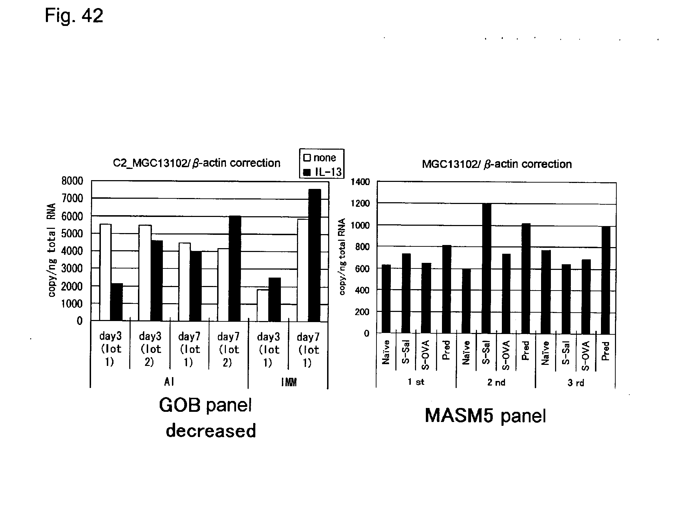

Fig. 42: MGC13102

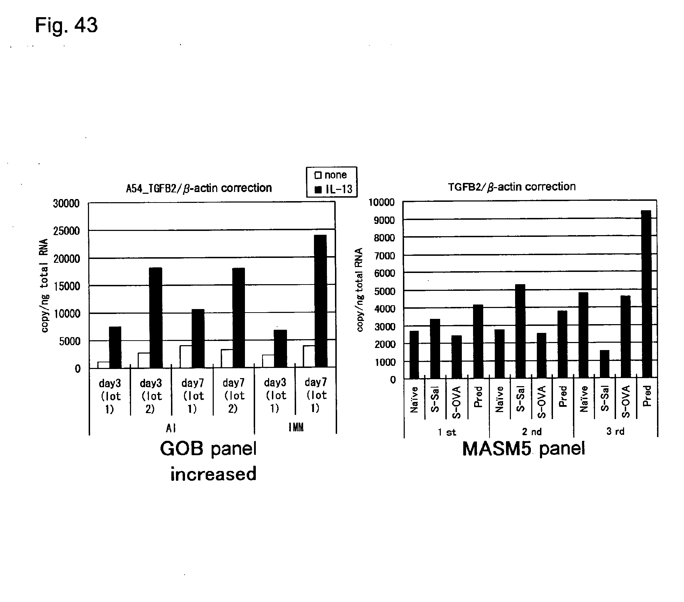

Fig. 43: TGFB2

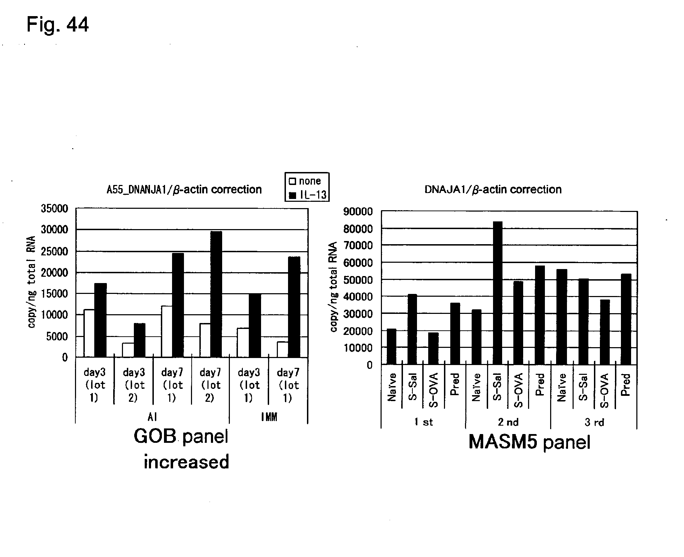

Fig. 44: DNAJA1

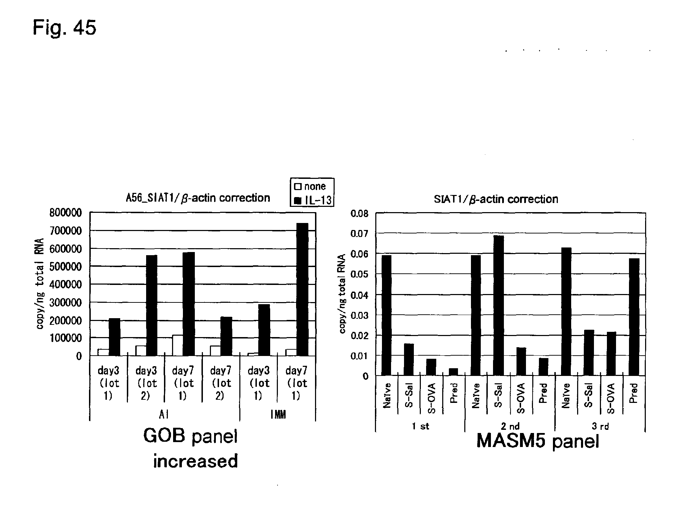

Fig. 45: SIAT1

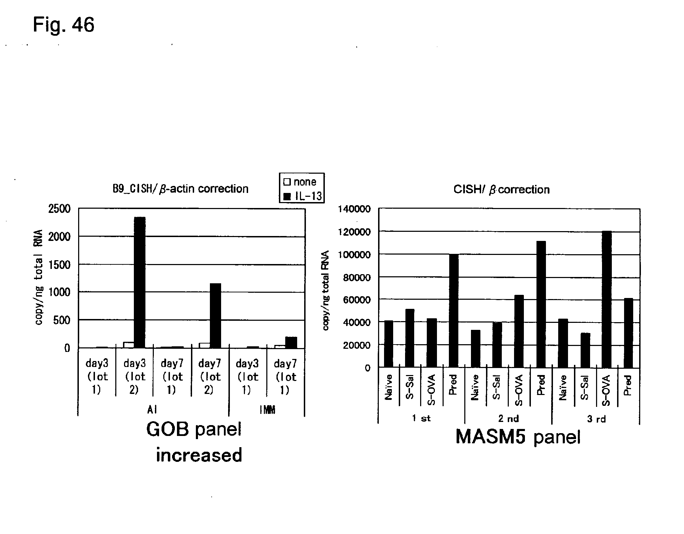

Fig. 46: CISH

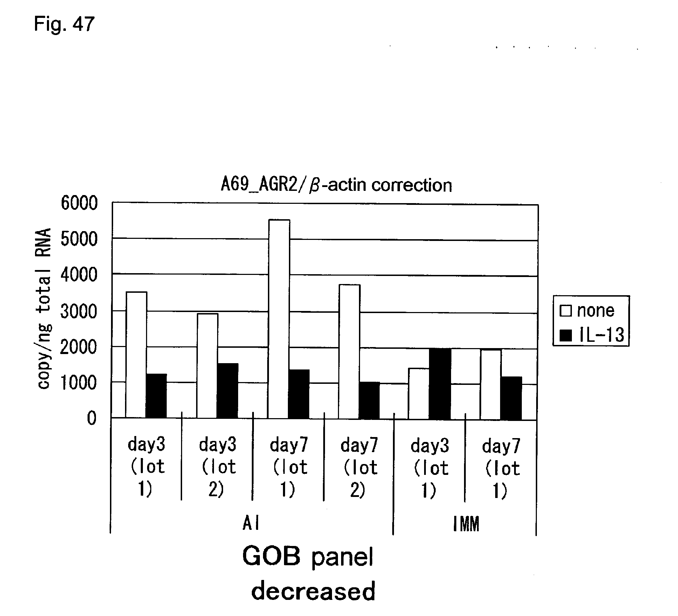

Fig. 47: AGR2 (only the result for the cDNA of human goblet cell differentiation model)

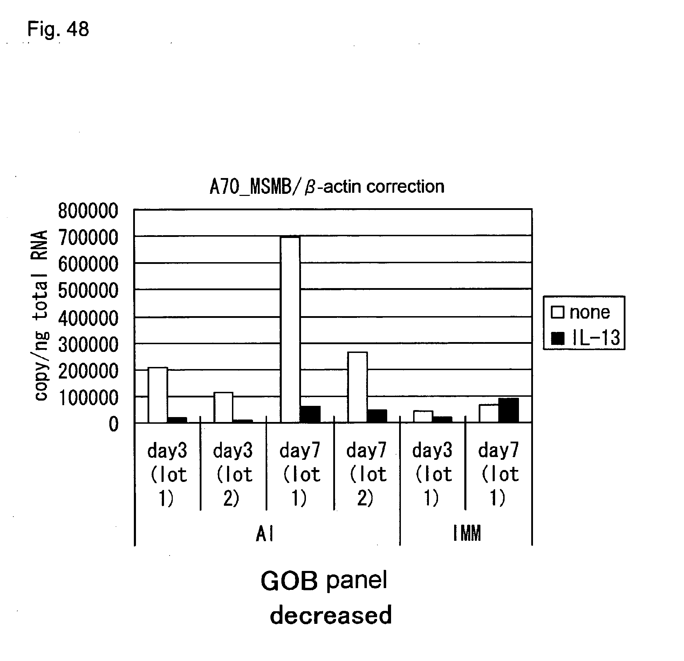

Fig. 48: MSMB (only the result for the cDNA of human goblet cell differentiation model)

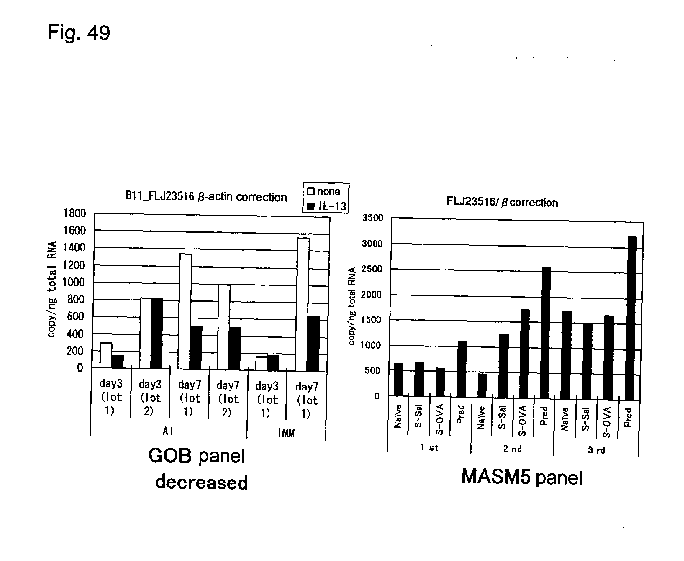

Fig. 49: FLJ23516

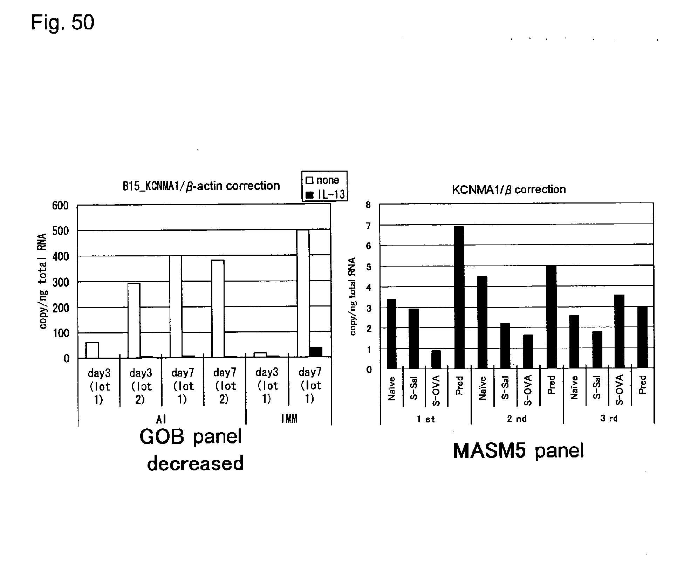

Fig. 50: KCNMA1

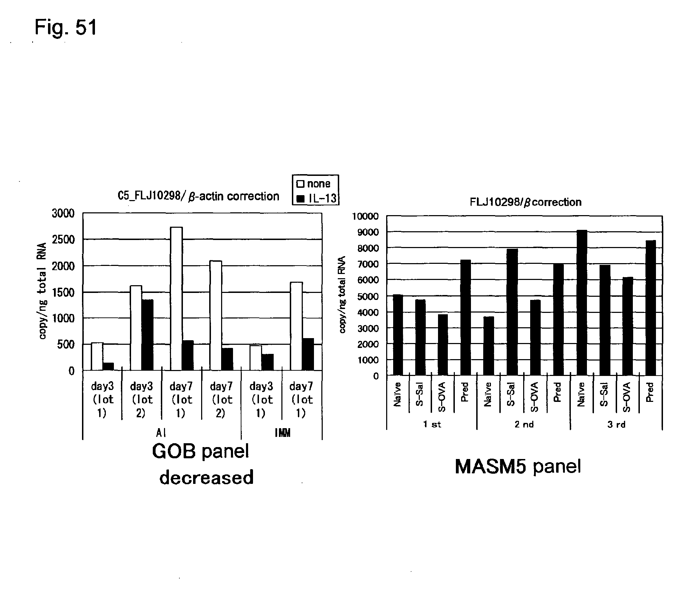

Fig. 51: FLJ10298

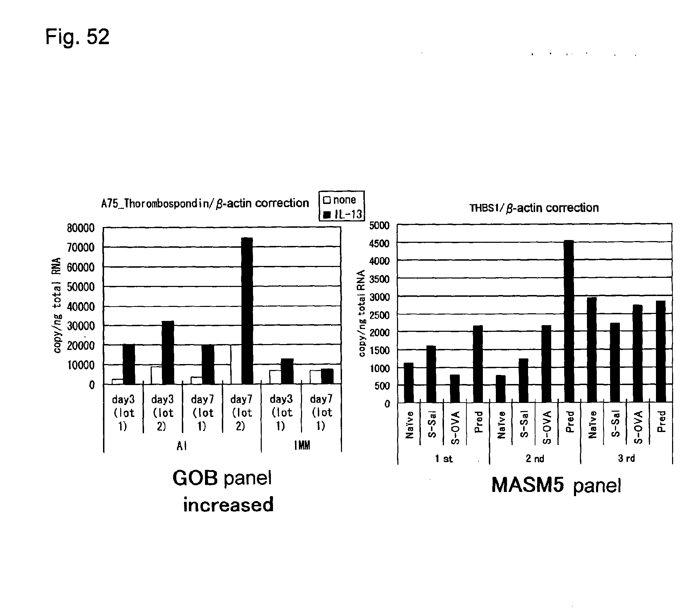

Fig. 52: THBS1

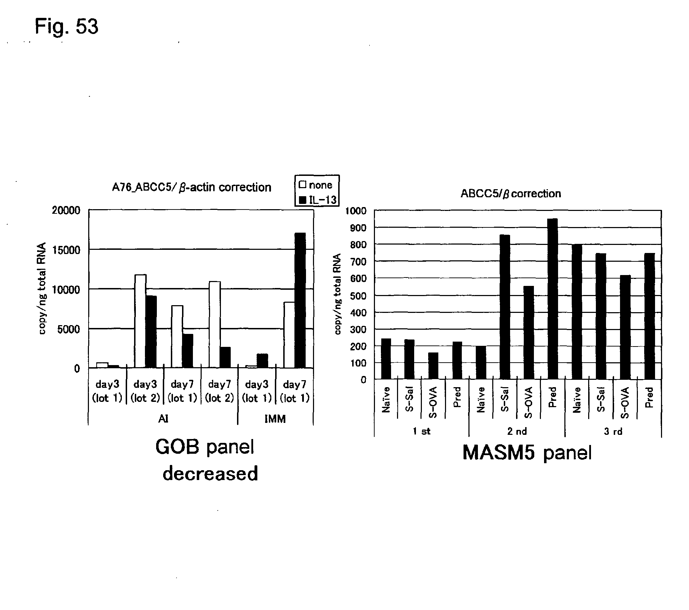

Fig. 53: ABCC5

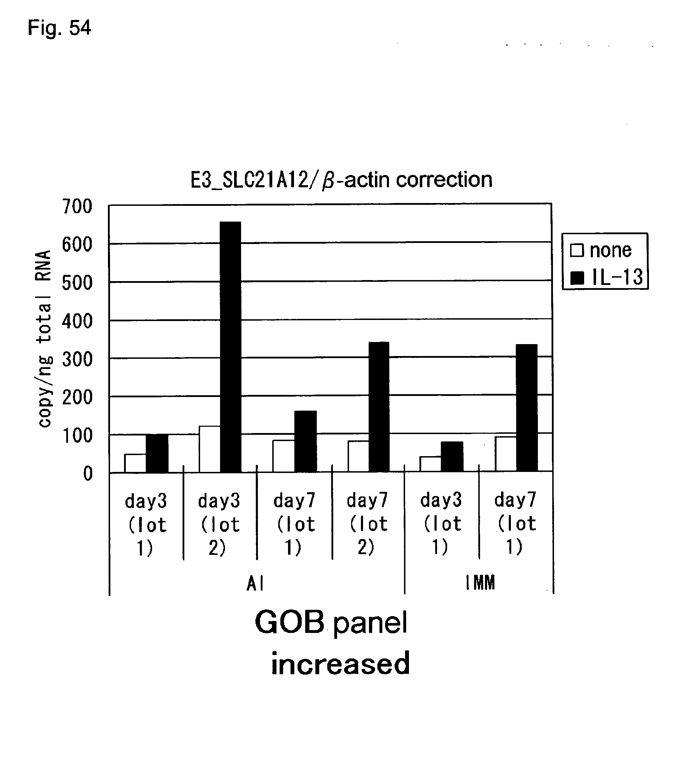

Fig. 54: SLC21A12 (only the result for the cDNA of human goblet cell differentiation model)

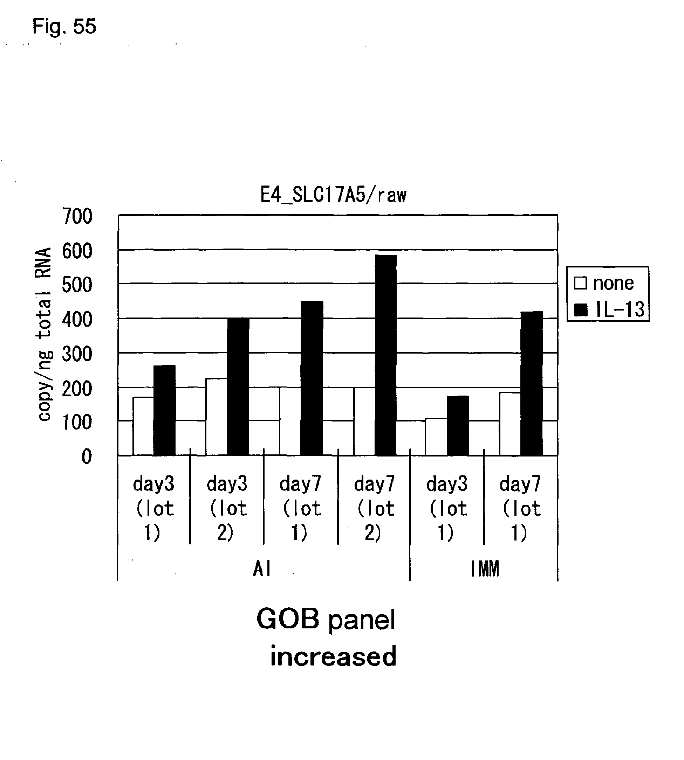

Fig. 55: SLC17A5 (only the result for the cDNA of human goblet cell differentiation model)

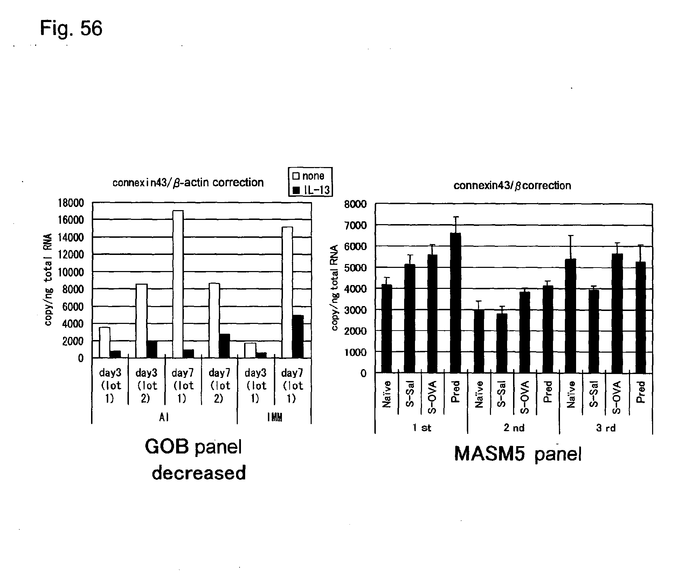

Fig. 56: connexin43

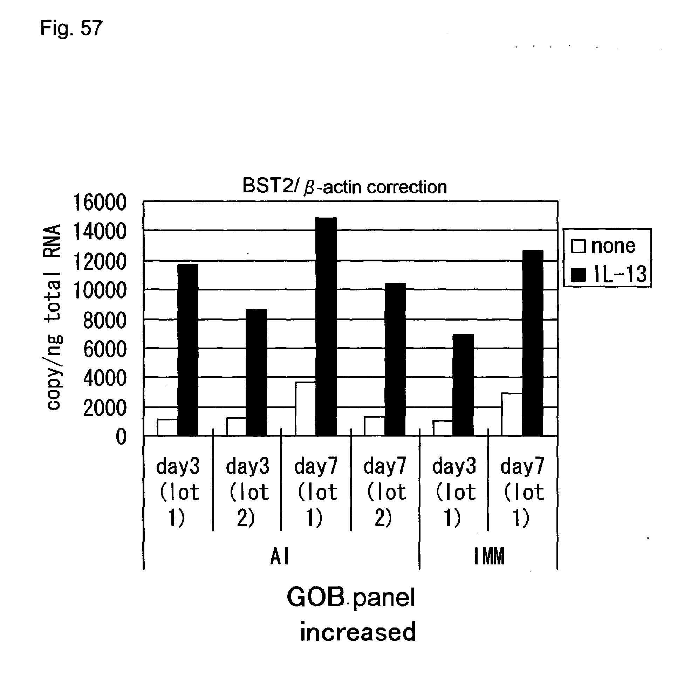

Fig. 57: BST2 (only the result for the cDNA of human goblet cell differentiation model)

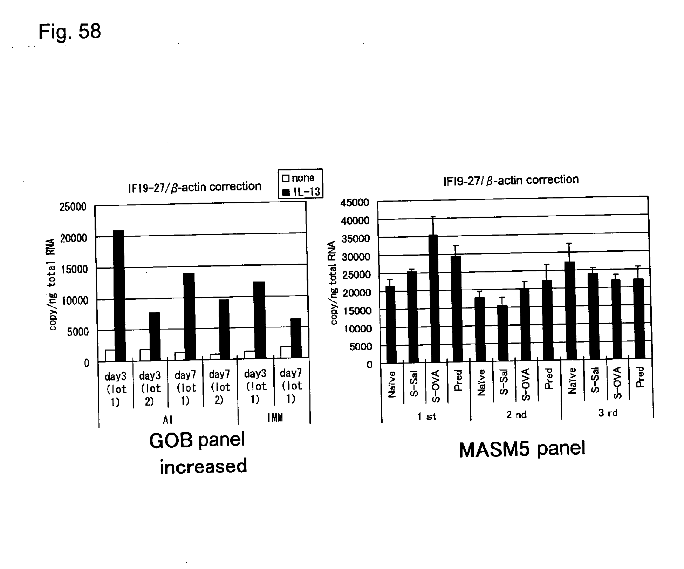

Fig. 58: IFI9-27

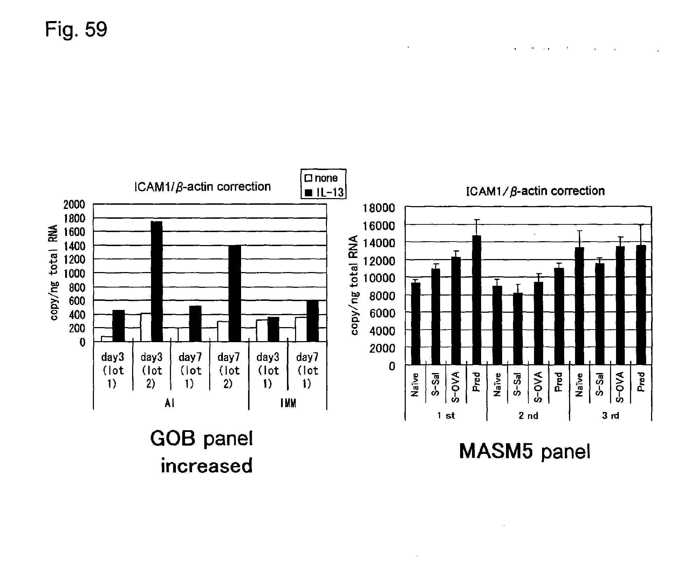

Fig. 59: ICAM1

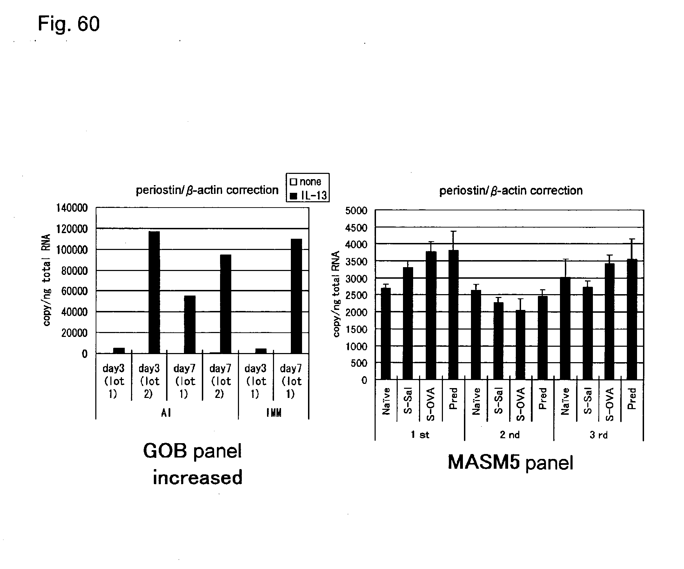

Fig. 60: periostin

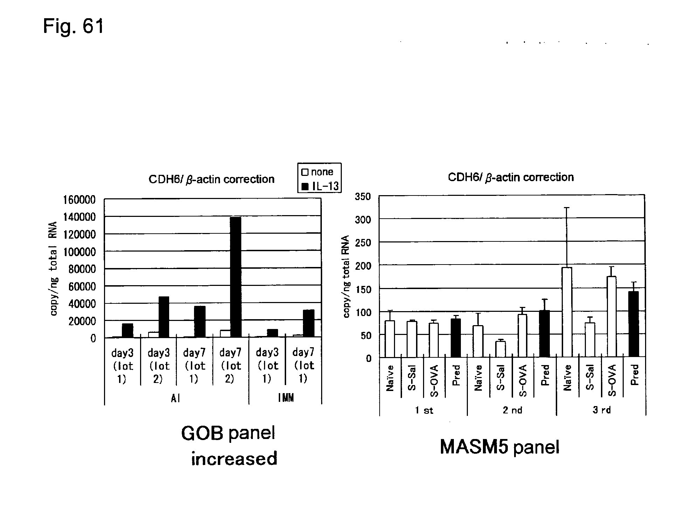

Fig. 61: CDH-6

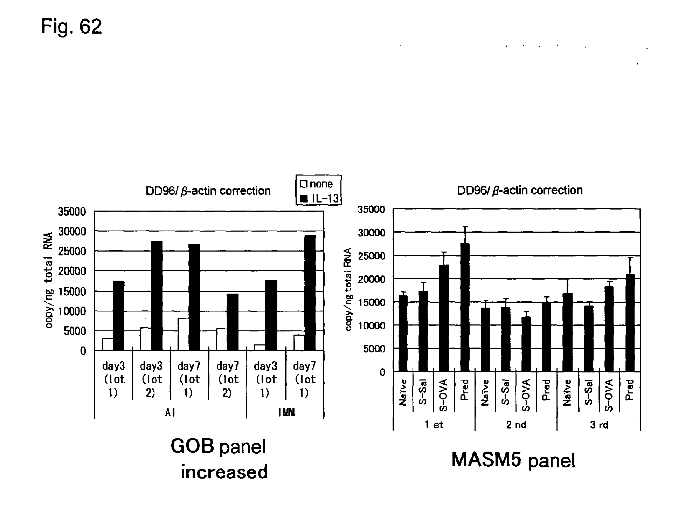

Fig. 62: DD96

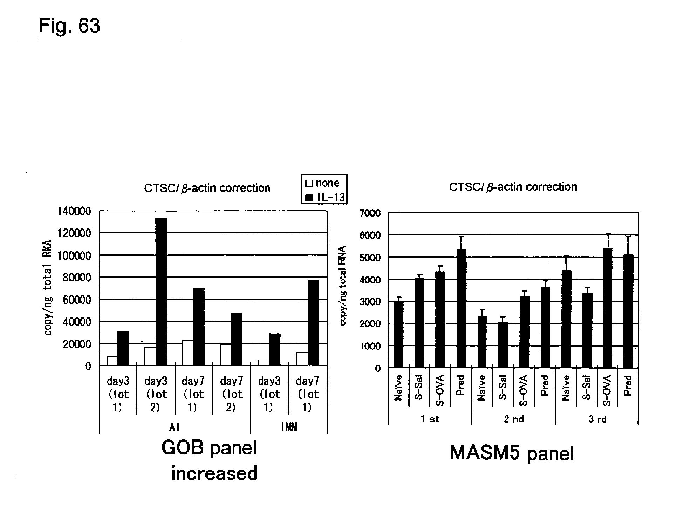

Fig. 63: CTSC

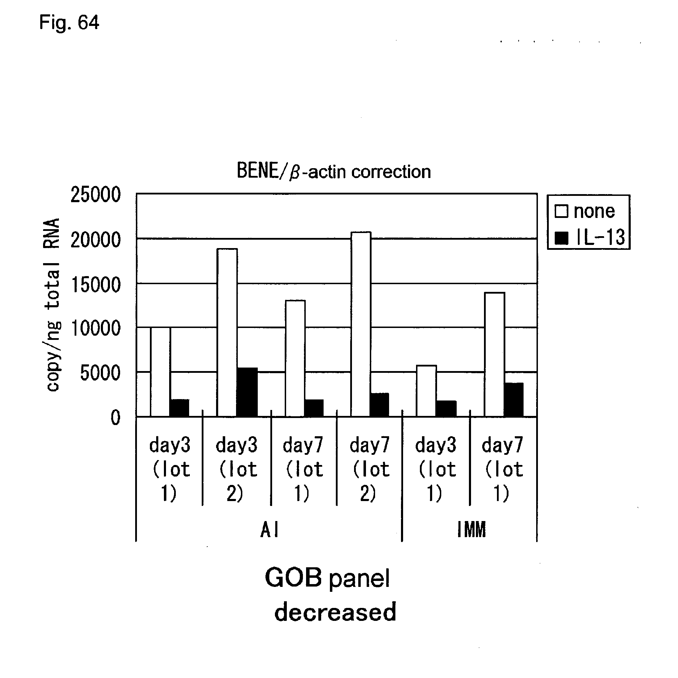

Fig. 64: BENE (only the result for the cDNA of human goblet cell differentiation model)

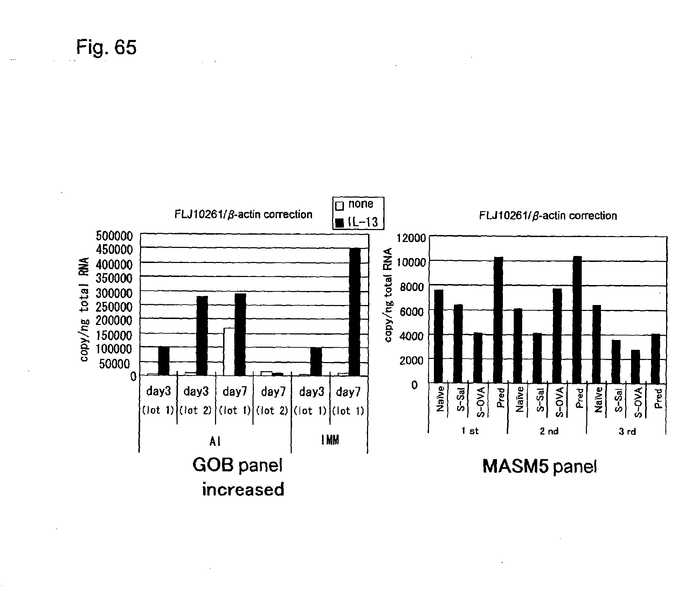

Fig. 65: FLJ10261

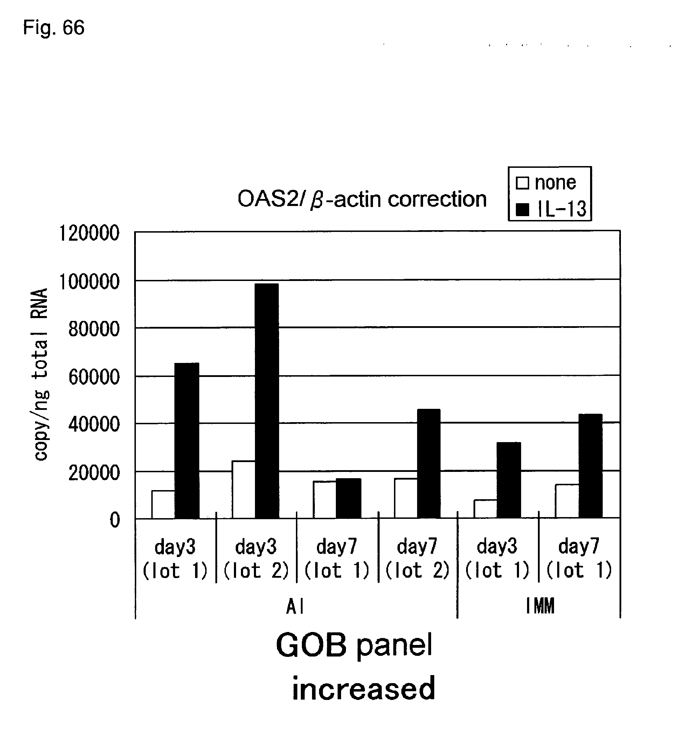

Fig. 66: OAS2 (only the result for the cDNA of human goblet cell differentiation model)

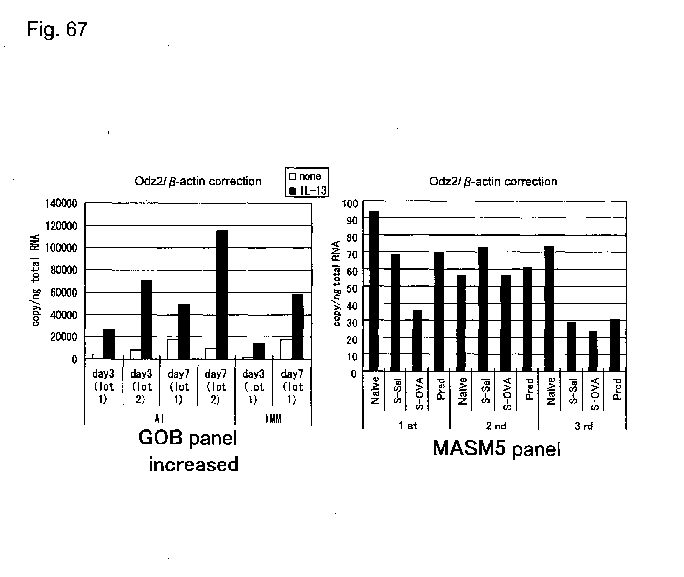

Fig. 67: Odz2
Fig. 68: E48

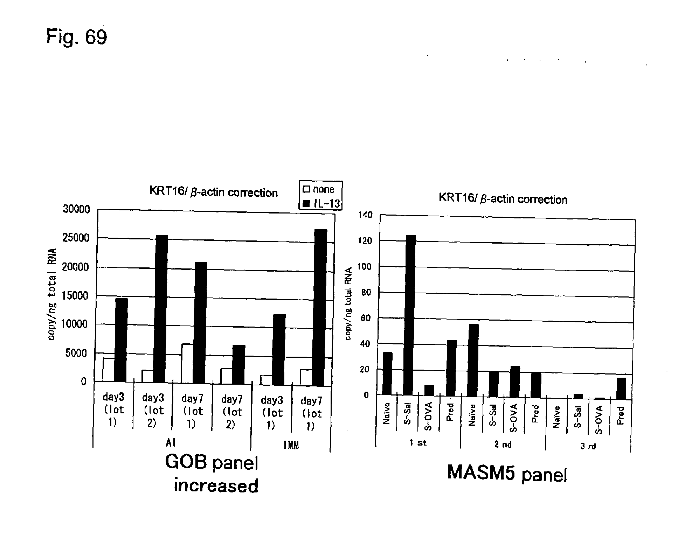

Fig. 69: KRT16

### DETAILED DESCRIPTION OF THE INVENTION

In the present invention, the term "allergic disease" is a general term used for a disease in which an allergic reaction is involved. More specifically, for a disease to be considered allergic, the allergen must be identified, a strong correlation between exposure to the allergen and the onset of a pathological change must be demonstrated, and it should have been proven that an immunological mechanism is behind the pathological change. Herein, the term "immunological mechanism" means that leukocytes show an immune response to allergen stimulation. Examples of allergens are dust mite antigens, pollen antigens, etc.

Representative allergic diseases are bronchial asthma, allergic rhinitis, pollinosis, insect allergy, etc. Allergic diathesis is a genetic factor that is inherited from allergic parents to children. Familial allergic diseases are also called atopic diseases, and their causative factor that can be inherited is atopic diathesis.

Bronchial asthma is characterized by respiratory tract inflammation and varying degrees of airflow obstruction, and shows paroxysmal cough, wheezing, and difficulty in breathing. The degree of airflow obstruction ranges from mild to life-threatening obstructions. Such airway obstructions can be reversed at least in part either through natural healing or by treatment. Various types of cells infiltrating into the respiratory tract, such as eosinophils , T cells (Th2) , and mast cells, are involved in the inflammation and the damaging of the mucosal epithelium of the respiratory tract. The reversibility of airway obstruction tends to decrease in adult patients affected by the disease for a long time. In such cases, "remodelings" such as thickening of the basement membrane under the respiratory epithelium is often seen. In sensitive patients, respiratory remodeling accompanies bronchial hypersensitivity.

Herein, a gene that can be used as a marker for bronchial asthma is referred to as "marker gene". A protein comprising an amino acid sequence encoded by a marker gene is referred to as a "marker protein". Unless otherwise stated, the term "marker gene" is used as a terminology that refers to one or more arbitrary gene(s) selected from the genes according to (a) or (b):
(a) a group of genes whose expression levels increase when respiratory epithelial cells are stimulated with interleukin-13, and comprise any one of the nucleotide sequences of SEQ ID NOs: 25 to 310;
(b) a group of genes whose expression levels decrease when a respiratory epithelial cell is stimulated with interleukin-13 and comprise any one of the nucleotide sequences of SEQ ID NOs: 311 to 547;

The nucleotide sequences of the marker genes of the present invention or portions of the genes are known in the art. Some of the amino acid sequences encoded by the nucleotide sequences of the marker genes of the present invention have already been identified. The GenBank accession numbers for obtaining the data of partial nucleotide sequences of the marker genes, together with names of the marker genes, are listed below. In addition, the amino acid sequences of the marker proteins are shown in Tables 84-113.

When a partial nucleotide sequence of a marker gene has been identified, one skilled in the art can determine the full-length nucleotide sequence of the marker gene based on the information of the partial nucleotide sequence. Such a full-length nucleotide sequence can be obtained, for example, through *in-silico* cloning. Specifically, an EST nucleotide sequence constituting a portion of a marker gene (query sequence) is compared with massive amounts of expressed sequence tag (EST) information accumulated in public databases. Based on the comparison result, information of other ESTs that share a nucleotide sequence that coincides with the query sequence over a certain length is selected. The newly selected EST information is used as a new query sequence to gain other EST information, and this is repeated. A set of multiple ESTs sharing a partial nucleotide sequence can thus be obtained by this repetition. A set of ESTs is referred to as a "cluster". The nucleotide sequence of a gene of interest can be identified by assembling the nucleotide sequences of ESTs constituting a cluster into a single nucleotide sequence.

Furthermore, one skilled in the art can design PCR primers based on the nucleotide sequence determined through *in-silico* cloning. The presence of a gene comprising the determined nucleotide sequence can be verified by determining whether a gene fragment whose size is as expected is amplified by RT-PCR using such primers.

Alternatively, the result of *in-silico* cloning can be assessed by Northern blotting. Northern blotting is carried out using a probe designed based on the information of the determined nucleotide sequence. As a result, if a band that agrees with the above nucleotide sequence information is obtained, the presence of a gene comprising the determined nucleotide sequence can be verified.

A gene of interest can be isolated empirically, in addition to *in-silico* cloning. First, a cDNA clone that provided nucleotide sequence information deposited as an EST is obtained. Then, the entire nucleotide sequences of the cDNA in that clone are determined. As a result, it may be possible to determine the full-length sequence of the cDNA. At least it is possible to determine a longer nucleotide sequence. The length of the cDNA in the clone can be pre-determined empirically when the vector structure is known.

Even if the clone that provided nucleotide sequence information of an EST is unavailable, there is a method known in the art by which an unknown part of a nucleotide sequence of a gene can be obtained based on a partial nucleotide sequence of the gene. For example, in some cases, a longer nucleotide sequence can be identified by screening a cDNA library using an EST as a probe. When a cDNA library comprising many full-length cDNA is used in the screening, a full-length cDNA clone can be readily isolated. For example, a cDNA library synthesized by the oligo-capping method is known to contain many full-length cDNA.

Furthermore, there is a technique known in the art to synthesize an unknown portion of a gene, based on the information of a partial nucleotide sequence of the gene. For example, RACE is a representative technique for isolating a gene comprising an unknown nucleotide sequence. In RACE, an oligonucleotide linker is artificially ligated to one end of a cDNA. The oligonucleotide linker consists of a known nucleotide sequence. Thus, PCR primers can be designed based on the information of a portion whose nucleotide sequence is already known as an EST and the nucleotide sequence of the oligonucleotide linker. The nucleotide sequence of the unknown region can be synthesized specifically by PCR using the primers designed as described above.

The method of testing for allergic diseases of the present invention comprises measuring the expression level of each marker gene in a biological sample from a subject and comparing the level with that of the marker gene in a control biological sample. When the marker gene is one of the genes according to (a) described above and the expression level is higher than that in the control, the subject is judged to be affected with bronchial asthma or a chronic obstructive pulmonary disease. Alternatively, when the marker gene is one of the genes according to (b) described above and the expression level is lower than that in the control, the subject is judged to be affected with bronchial asthma or a chronic obstructive pulmonary disease. In the present invention, a respiratory epithelial cell which has not been stimulated with IL-13, can be used as a control. Preferably, the control respiratory epithelial cell has been cultured by the AI method.

The standard value for the control may be pre-determined by measuring the expression level of the marker gene in the control, in order to compare the expression levels. Typically, for example, the standard value is determined based on the expression level of the above-mentioned marker gene in the control. For example, the permissible range is taken as ±2S.D. based on the standard value. A technique for determining the permissible range and the standard value based on a measured value for the marker gene is known in the art. Once the standard value is determined, the testing method of the present invention may be performed by measuring only the expression level in a biological sample from a subject and comparing the value with the determined standard value for the control.

When the marker gene is one of the genes according to (a) described above and the expression level in a subject is higher than the permissible range in comparison to that in the control, the subject is judged to be affected with bronchial asthma or a chronic obstructive pulmonary disease. Likewise, when the marker gene is one of the genes according to (b) described above and the expression level in a subject is lower than the permissible range in comparison to that in the control, the subject is judged to be affected with bronchial asthma or a chronic obstructive pulmonary disease. When the expression level of the marker gene falls within the permissible range, the subject is unlikely to be affected with bronchial asthma or a chronic obstructive pulmonary disease.

In this invention, expression levels of marker genes include transcription of the marker genes to mRNA, and translation into proteins. Therefore, the method of testing for bronchial asthma or a chronic obstructive pulmonary disease of this invention is performed based on a comparison of the intensity of expression of mRNA corresponding to the marker genes, or the expression level of proteins encoded by the marker genes.

The measurement of the expression levels of marker genes in the testing for bronchial asthma or a chronic obstructive pulmonary disease of this invention can be carried out according to known gene analysis methods. Specifically, one can use, for example, a hybridization technique using nucleic acids that hybridize to these genes as probes, or a gene amplification technique using DNA that hybridize to the marker genes of this invention as primers.

The probes or primers used for the testing of this invention can be designed based on the nucleotide sequences of the marker genes. The nucleotide sequences of the marker genes and a portion of amino acid sequences encoded by the genes are known. The GenBank accession numbers for the known nucleotide sequences of the respective marker genes of the present invention are shown below in Tables 3-19 (genes showing increased expression) and Tables 20-36 (genes showing decreased expression). When a gene has a number beginning with NM in the column of RefSeq in Tables, the full-length nucleotide sequence of the gene is known in the art. When a gene does not have a number beginning with NM in the column of RefSeq, a partial nucleotide sequence can be obtained based on the GenBank Accession number of the gene. As described above, the full-length nucleotide sequence of a gene can be obtained based on the information of a known partial nucleotide sequence. In addition, with respect to some of the marker genes of the present invention, the nucleotide sequences and the amino acid sequences encoded by them are shown in the Tables.

Genes of higher animals generally accompany polymorphism in a high frequency. There are also many molecules that produce isoforms comprising mutually different amino acid sequences during the splicing process. Any gene associated with bronchial asthma or a chronic obstructive pulmonary disease that has an activity similar to that of a marker gene is included in the marker genes of the present invention, even if it has nucleotide sequence differences due to polymorphism or being an isoform.

Herein, the marker genes include homologs of other species in addition to humans. Thus, unless otherwise specified, the expression "marker gene in a species other than human" refers to a homolog of the marker gene unique to the species or a foreign marker gene which has been introduced into an individual.

As used herein, the expression "homolog of a human marker gene" refers to a gene derived from a species other than a human, which can hybridize to the human marker gene as a probe under stringent conditions. Stringent conditions typically mean hybridization in 4x SSC at 65°C followed by washing with 0.1x SSC at 65°C for 1 hour. Temperature conditions for hybridization and washing that greatly influence stringency can be adjusted according to the melting temperature (Tm). Tm varies with the ratio of constitutive nucleotides in the hybridizing base pairs, and the composition of the hybridization solution (concentrations of salts, formamide, and sodium dodecyl sulfate). Therefore, considering these conditions, one skilled in the art can select an appropriate condition to produce an equal stringency experimentally or empirically.

An example of a homolog of the marker genes of the present invention, which is derived from another species, is the mouse homolog. Using the mouse model of bronchial hypersensitivity, the present inventors confirmed that the mouse genes according to (A) or (B) exhibit variation patterns of expression levels similar to that of human marker genes. This finding supports the fact that there is a close relationship between the human marker genes identified in the present invention and the allergic responses of tissues in the respiratory tract. This finding also supports the fact that homologs of various species can be used as marker genes of the present invention.

A polynucleotide comprising the nucleotide sequence of a marker gene or a nucleotide sequence that is complementary to the complementary strand of the nucleotide sequence of a marker gene and has at least 15 nucleotides , can be used as a primer or probe. Herein, the expression "complementary strand" means one strand of a double stranded DNA with respect to the other strand and which is composed of A: T (U for RNA) and G:C base pairs. In addition, "complementary" means not only those that are completely complementary to a region of at least 15 continuous nucleotides, but also those that have a nucleotide sequence homology of at least 70%, preferably at least 80%, more preferably 90%, and even more preferably 95% or higher. The degree of homology between nucleotide sequences can be determined by an algorithm, BLAST, etc.

Such polynucleotides are useful as a probe to detect a marker gene, or as a primer to amplify a marker gene. When used as a primer, the polynucleotide comprises usually 15 bp to 100 bp, preferably 15 bp to 35 bp of nucleotides. When used as a probe, a DNA comprises the whole nucleotide sequence of the marker gene (or the complementary strand thereof) , or a partial sequence thereof that has at least 15-bp nucleotides. When used as a primer, the 3' region must be complementary to the marker gene, while the 5' region can be linked to a restriction enzyme-recognition sequence or a tag.

"Polynucleotides" in the present invention may be either DNA or RNA. These polynucleotides may be either synthetic or naturally-occurring. Also, DNA used as a probe for hybridization is usually labeled. Examples of labeling methods are those as described below. Herein, the term "oligonucleotide" means a polynucleotide with a relatively low degree of polymerization. Oligonucleotides are included in polynucleotides. The labeling methods are as follows:
· nick translation labeling using DNA polymerase I;
· end labeling using polynucleotide kinase;
· fill-in end labeling using Klenow fragment (Berger, SL, Kimmel, AR. (1987) Guide to Molecular Cloning Techniques, Method in Enzymology, Academic Press; Hames, BD, Higgins, SJ. (1985) Genes Probes: A Practical Approach. IRL Press; Sambrook, J., Fritsch, EF, Maniatis, T. (1989) Molecular Cloning: a Laboratory Manual, 2nd Edn. Cold Spring Harbor Laboratory Press);
· transcription labeling using RNA polymerase (Melton, DA, Krieg, PA, Rebagkiati, MR, Maniatis, T, Zinn, K, Green, MR. (1984) Nucleic Acid Res., 12, 7035-7056); and
· non-isotopic labeling of DNA by incorporating modified nucleotides (Kricka, LJ. (1992) Non-isotopic DNA Probing Techniques. Academic Press).

Tests for bronchial asthma or a chronic obstructive pulmonary disease using hybridization techniques, can be performed using, for example, Northern hybridization, dot blot hybridization, or the DNA microarray technique. Furthermore, gene amplification techniques, such as the RT-PCR method may be used. By using the PCR amplification monitoring method during the gene amplification step in RT-PCR, one can achieve a more quantitative analysis of the expression of a marker gene of the present invention.

In the PCR gene amplification monitoring method, the detection target (DNA or reverse transcript of RNA) is hybridized to probes that are labeled with a fluorescent dye and a quencher which absorbs the fluorescence. When the PCR proceeds and Taq polymerase degrades the probe with its 5'-3' exonuclease activity, the fluorescent dye and the quencher draw away from each other and the fluorescence is detected. The fluorescence is detected in real time. By simultaneously measuring a standard sample in which the copy number of a target is known, it is possible to determine the copy number of the target in the subject sample with the cycle number where PCR amplification is linear (Holland, P. M. et al., 1991, Proc. Natl. Acad. Sci. USA 88: 7276-7280; Livak, K. J. et al. , 1995, PCR Methods and Applications 4(6): 357-362; Heid, C. A. et al., 1996, Genome Research 6: 986-994; Gibson, E. M. U. et al., 1996, Genome Research 6: 995-1001). For the PCR amplification monitoring method, for example, ABI PRISM7700 (Applied Biosystems) may be used.

The method of testing for bronchial asthma or a chronic obstructive pulmonary disease of the present invention can be also carried out by detecting a protein encoded by a marker gene. Hereinafter, a protein encoded by a marker gene is described as a "marker protein". For such test methods, for example, the Western blotting method, the immunoprecipitation method, and the ELISA method may be employed using an antibody that binds to each marker protein.

Antibodies used in the detection that bind to the marker protein may be produced by techniques known to those skilled in the art. Antibodies used in the present invention may be polyclonal or monoclonal (Milstein, C. et al., 1983, Nature 305 (5934): 537-40). For example, a polyclonal antibody against a marker protein may be produced by collecting blood from mammals sensitized with the antigen, and separating the serum from this blood using known methods. As a polyclonal antibody, serum containing a polyclonal antibody may be used. If necessary, a fraction containing the polyclonal antibody can be further isolated from this serum. Also, a monoclonal antibody may be obtained by isolating immune cells from mammals sensitized with the antigen, fusing these cells with myeloma cells and such, cloning the resulting hybridomas, and then collecting the antibody from the hybridoma culture.

In order to detect a marker protein, such an antibody may be appropriately labeled. Alternatively, instead of labeling the antibody, a substance that specifically binds to the antibody, for example, protein A or protein G, may be labeled to detect the marker protein indirectly. More specifically, such a detection method includes the ELISA method.

A protein or a partial peptide thereof used as an antigen may be obtained, for example, by inserting a marker gene or a portion thereof into an expression vector, introducing the construct into an appropriate host cell to produce a transformant, culturing the transformant to express the recombinant protein, and purifying the expressed recombinant protein from the culture or the culture supernatant. Alternatively, the amino acid sequence encoded by a gene or an oligopeptide comprising a portion of the amino acid sequence encoded by a full-length cDNA are chemically synthesized to be used as an immunogen.

Furthermore, in the present invention, a test for an allergic disease can be performed using as an index not only the expression level of a marker gene but also the activity of a marker protein in a biological sample. Activity of a marker protein means the biological activity intrinsic to the protein. Typical methods for measuring the activity of each protein are described below.

### [Protease]

A protease sample is electrophoresed under a non-reducing condition in an SDS polyacrylamide gel co-polymerized with a substrate such as gelatin. After electrophoresis, the gel is allowed to stand still in an appropriate buffer at 37°C for 16 hours. The gel is stained with Coomassie Brilliant Blue R250 after 16 hours. The protease activity can be assessed by verifying that the electrophoretic position corresponding to the protease is not stained on the gel, i.e., gelatin at that position has been hydrolyzed.
Chen, J. M. et al., J. Biol. Chem. 266, 5113-5121 (1991)

### [Protease inhibitor]

A protease inhibitor is electrophoresed under a non-reducing condition in an SDS polyacrylamide gel co-polymerized with a protease substrate such as gelatin. After electrophoresis, the gel is allowed to stand still in an appropriate buffer containing a protease at 37°C for 16 hours. After 16 hours, the gel is stained with Coomassie Brilliant Blue R250. The activity of the protease inhibitor can be assessed by verifying that the electrophoretic position corresponding to the protease inhibitor is not stained on the gel, i.e., gelatin has not been hydrolyzed at that position.
Greene J. et al., J. Biol. Chem. 271, 30375-30380 (1996)

### [Transcription factor]

A transcription factor is incubated at room temperature with a double-stranded oligo DNA, which has been labeled with ³²p or such and contains a target sequence of the transcription factor. The incubation allows the transcription factor to bind to the oligo DNA. After incubation, the sample is electrophoresed in a native polyacrylamide gel without SDS. The mobility of the labeled oligo DNA is determined using the radioactivity of ³²P or such as an index. When the transcription factor has the activity of binding to the oligo DNA, the mobility of the labeled oligo DNA decreases and thus the band shifts to a higher-molecular-weight position. The binding specificity for the target sequence can be assessed by verifying that an excess amount of non-labeled double-stranded oligo DNA inhibits the binding between the transcription factor and the labeled oligo DNA.

In addition, the ability to activate transcription by a transcription factor can be estimated by a procedure which comprises the steps of: co-introducing into cells of a cell line such as HeLa or HEK293, an expression vector comprising a reporter gene such as chloramphenicol acetyltransferase (CAT) downstream of a target sequence and another expression vector comprising the transcription factor gene downstream of a promoter from human cytomegalovirus (CMV) , and after 48 hours, preparing a cell lysate and determining the expression level of CAT in the lysate.
Zhao F. et al., J. Biol. Chem. 276, 40755-40760 (2001)

### [Kinase]

A kinase is added to a buffer (20 mM HEPES, pH7.5, 10 mM MgCl₂, 2 mM MnCl₂, 2 mM dithiothreitol, and 25 µM ATP) containing myelin basic protein as a substrate, and then [γ-³²P]ATP is added thereto. The resulting mixture is incubated at 37°C for 10 minutes. After 10 minutes, Laemmli buffer is added to stop the reaction, and the reaction solution is subjected to SDS polyacrylamide gel electrophoresis. After electrophoresis, the gel is dried and the radioactivity of the phosphorylated myelin basic protein is detected on X-ray film.
Park SY. et al., J. Biol. Chem. 275, 19768-19777 (2000)

### [Phosphatase]

A phosphatase is added to a buffer (25 mM MES (pH 5.5) , 1.6 mM dithiothreitol, and 10 mM pNPP) containing p-nitrophenyl phosphate (pNPP) as a substrate. The resulting mixture is incubated at 37°C for 30 minutes. After 30 minutes, 1N NaOH is added to stop the reaction, and the absorbance at 405 nm, a result of pNpp hydrolysis, is measured.
Aoyama K. et al., J. Biol. Chem. 276, 27575-27583 (2001)

### [Chemokine and chemokine receptor]

Cells overexpressing a chemokine receptor are suspended in Hank's balanced salt solution containing the calcium-sensitive fluorescent dye fura-2. The cells are stimulated with the chemokine. An increase in the intracellular calcium level that resulted from the chemokine stimulation is measured with a fluorescence detector such as LS50B (Perkin Elmer).
Zhou N. et al., J. Biol. Chem. 276, 42826-42833 (2001)

### [Cytokine and cytokine receptor]

Cells expressing a cytokine receptor are stimulated with a cytokine. The resulting cell proliferation is assessed by thymidine uptake.

Alternatively, it is possible to assess the cytokine-mediated activation of a transcription factor downstream of the cytokine receptor based on the expression of a reporter gene such as luciferase.
Piek E. et al., J. Biol. Chem. 276, 19945-19953 (2001)

### [Ion channel]

An ion channel-containing cell membrane is attached to the open end, the area of which is a few µm², of a glass pipette. The ion channel activity can be determined by the patch-clamp method which comprises measuring the electric current passing through the channel when a potential difference is generated between the inside and outside of the pipette.
Hamill, O. P. et al., Pfluegers Arch. 391, 85-100 (1981)

### [Cell adhesion molecule]

Cells expressing an adhesion molecule on the cell surface are incubated in a plate coated with the ligand of the molecule. The number of cells adhering to the plate is determined.
Fujiwara H. et al., J. Biol. Chem. 276, 17550-17558 (2001)

### [Extracellular matrix protein]

A suspension of cells expressing a receptor of an extracellular matrix protein such as integrin, is added to a plate coated with an extracellular matrix protein. The plate is incubated at 37°C for 1 hour. After incubation, the cells are fixed and a DNA-binding fluorescent dye such as Hoechst 33342, is added thereto. After the reaction, the fluorescence intensity is determined using a fluorometer. The number of adhered cells quantified based on the fluorescence intensity is used to assess the activity of the extracellular matrix protein.
Miyazaki K. et al., Proc. Natl. Acad. Sci. U. S. A. 90, 11767 (1993)

Normally, a biological material collected from a subject is used as a sample in the testing method of the present invention. A preferred biological sample is blood. Blood samples include whole blood, and plasma and serum prepared from whole blood. The biological sample of the present invention includes sputum, secretions from the nasal mucous membrane, bronchoalveolar lavage fluid, exfoliated airway epithelial cells, in addition to blood. Methods for collecting biological samples are known in the art.

When the biological sample is cells such as respiratory tract epithelial cells, samples for immunological measurements of the aforementioned proteins can be made by preparing a lysate. Alternatively, samples for measuring mRNA corresponding to the aforementioned genes can be prepared by extracting mRNA from this lysate. A commercially available kit is useful when extracting a lysate or mRNA from a biological sample. Alternatively, biological samples in the liquid form such as blood, nasal mucous secretions, and bronchoalveolar lavage fluids can be made into samples for measurement of proteins and genes by diluting with a buffer and such, as necessary.

A lysate prepared from an above-mentioned biological sample can be used as a sample in immunological assays for marker proteins. Alternatively, mRNA extracted from the lysate can be used as a sample in assays for mRNA corresponding to marker genes. A commercially available kit can be used to prepare a lysate or to extract mRNA from a biological sample. When a marker protein is secreted into blood, the expression level of the encoding gene can be compared by determining the amount of the protein of interest in a sample of a subject's body fluid such as blood or serum. The sample can be diluted with a buffer or such, as required, to be used in the method of the present invention.

When mRNA is measured, the measured value of the expression levels of marker genes in the present invention can be corrected by known methods. As a result of correction, variations in gene expression levels in cells can be compared. Based on the measured values of the expression levels of genes that do not show great variations in each cell in the above biological samples (for example, housekeeping genes), the correction of the measured values is done by correcting the measured values of the expression levels of marker genes in this invention. Genes whose expression level does not greatly vary include β-actin and GAPDH.

Furthermore, the present invention provides reagents for the testing methods of the present invention. Specifically, the present invention relates to a reagent for testing bronchial asthma or a chronic obstructive pulmonary disease, which comprise a polynucleotide comprising the nucleotide sequence of a marker gene, or an oligonucleotide having at least 15 nucleotides and comprising a nucleotide sequence complementary to the complementary strand of the nucleotide sequence of the marker gene. The present invention also relates to a reagent for testing bronchial asthma or a chronic obstructive pulmonary disease, which comprises an antibody recognizing a marker protein.

The oligonucleotide or antibody constituting the reagents of the present invention can be pre-labeled with an appropriate labeling substance depending on the assay. Alternatively, the oligonucleotide or antibody constituting the reagents of the present invention can be pre-immobilized on an appropriate support depending on the assay. Furthermore, the reagents of the present invention can be prepared as test kits in combination with an additive necessary for the testing and storage, in addition to the oligonucleotide or antibody described above. Exemplary additives constituting such a kit are listed below. If required, these may be added in advance. A preservative may also be added to each.

A buffer for diluting the reagent or biological sample;
positive control;
negative control;
substrate to be used for detecting a label;
reaction vessel; and
instruction manual describing assay protocols.

The expression level of a marker gene of the present invention has been confirmed to change in respiratory epithelial cells upon IL-13 stimulation in comparison to that in non-stimulated respiratory epithelial cells. Thus, bronchial asthma or a chronic obstructive pulmonary disease can be tested using as an index the expression level of a marker gene.

Tests for bronchial asthma or a chronic obstructive pulmonary disease according to the present invention include, for example, the following. Even if a patient is not diagnosed as being affected with bronchial asthma or a chronic obstructive pulmonary disease in a routine test in spite of symptoms suggesting these diseases, whether or not such a patient is suffering from bronchial asthma or a chronic obstructive pulmonary disease can be easily determined by performing a test according to the present invention. More specifically, when the marker gene is one of the genes according to (a) mentioned above, an increase in the expression level of the marker gene in a patient whose symptoms suggest bronchial asthma or chronic obstructive pulmonary disease, implies that the symptoms are caused by bronchial asthma or a chronic obstructive pulmonary disease. Alternatively, when the marker gene is one of the genes according to (b) mentioned above, likewise, a decrease in the expression level of a marker gene in a patient whose symptoms suggest bronchial asthma or a chronic obstructive pulmonary disease, implies that the symptoms are caused by bronchial asthma or a chronic obstructive pulmonary disease.

In addition, the present invention facilitates tests to determine whether bronchial asthma or a chronic obstructive pulmonary disease is improving in a patient. In other words, the present invention can be used to judge the therapeutic effect on bronchial asthma or a chronic obstructive pulmonary disease. Furthermore, when the marker gene is one of the genes according to (a), an increase in the expression level of the marker gene in a patient, who has been diagnosed as being affected by bronchial asthma or a chronic obstructive pulmonary disease, implies that the disease has progressed more. Alternatively, when the marker gene is one of the genes according to (b) , likewise a decrease in the expression level of the marker gene in a patient, who has been diagnosed as being affected by bronchial asthma or a chronic obstructive pulmonary disease, implies that the disease has progressed more.

Furthermore, the severity of bronchial asthma or a chronic obstructive pulmonary disease may also be determined based on the difference in expression levels. In other words, when the marker gene is one of the genes according to (a), the degree of increase in the expression level of the marker gene is correlated with the severity of bronchial asthma or chronic obstructive pulmonary disease. Alternatively, when the marker gene is one of the genes according to (b) , the degree of decrease in the expression level of the marker gene is correlated with the severity of bronchial asthma or chronic obstructive pulmonary disease.

The present invention also relates to animal models for bronchial asthma or chronic obstructive pulmonary disease, comprising a nonhuman transgenic animal in which the expression level of a marker gene according to (a) or a gene functionally equivalent to the marker gene has been elevated in the respiratory epithelium.

The present invention revealed that stimulation with IL-13 increased the expression level of a marker gene according to (a) in respiratory epithelial cells. Thus, an animal in which the expression level of a marker gene according to (a) or a gene functionally equivalent to the marker gene in respiratory epithelial cells has been artificially increased, can be used as an animal model for bronchial asthma or chronic obstructive pulmonary diseases.

The present invention also relates to an animal model for bronchial asthma or chronic obstructive pulmonary disease, which is a nonhuman transgenic animal in which the expression level of a marker gene according to (b) , or a gene functionally equivalent to the marker gene, has been decreased in respiratory epithelial cells.

The present invention revealed that stimulation with IL-13 decreased the expression level of a marker gene according to (b) in respiratory epithelial cells. Thus, an animal in which the expression level of a marker gene according to (b) or a gene functionally equivalent to the marker gene in respiratory epithelial cells has been artificially decreased can be used as an animal model for bronchial asthma or chronic obstructive pulmonary disease.

A "functionally equivalent gene" as used in this invention is a gene that encodes a protein having an activity similar to a known activity of a protein encoded by the marker gene. A representative example of a functionally equivalent gene includes a counterpart of a marker gene of a subject animal, which is intrinsic to the animal.

For example, genes according to group (A) and group (B) described above are functionally equivalent mouse genes. The genes according to group (A) and group (B) described above are used as preferred marker genes in performing the screenings according to the present invention using mice.

In addition, the present invention identified the mouse counterpart genes of the marker genes according to (a) and (b). Such counterpart genes are shown in (A) and (B) , respectively. These counterparts are genes whose expression levels in respiratory epithelial cells showed a twofold or more difference between the mouse model for bronchial asthma and normal mice. Thus, an animal model for bronchial asthma can be created by controlling the expression level of a counterpart gene or administering a counterpart gene. Namely, the present invention relates to a method for creating an animal model for bronchial asthma or a chronic obstructive pulmonary disease by controlling the expression level of a gene selected from the group of genes according to (A) or (B). Alternatively, the present invention relates to a method for creating an animal model for bronchial asthma or a chronic obstructive pulmonary disease by administering the protein encoded by a gene selected from the group of genes according to (A) or (B) , or administering an antibody against the protein.

First, similarly to the group of genes according to (a), the group of genes according to (A) can induce bronchial asthma or a chronic obstructive pulmonary disease by the increase in their expression levels. Alternatively, an animal model for bronchial asthma or chronic obstructive pulmonary disease can be created by introducing a gene selected from such groups of genes, or by administering a protein encoded by such a gene. Such counterpart genes or proteins are preferably introduced/administered to mice, because they derive from mice.

In addition, similarly to the group of genes according to (b), the group of genes according to (B) can induce bronchial asthma or chronic obstructive pulmonary disease by the suppression of their expression levels. Alternatively, bronchial asthma or chronic obstructive pulmonary disease can be induced by suppressing the expression of a gene selected from such groups of genes or the activity of a protein encoded by such a gene. An antisense nucleic acid, a ribozyme, or an RNAi can be used to suppress the expression. The activity of a protein can be controlled effectively by administering a substance that inhibits the activity, such as an antibody. Namely, in an animal inherently having a gene selected from the group of genes according to (B) , i.e. , mice, bronchial asthma or chronic obstructive pulmonary disease is induced by administering such a substance.

The animal model for bronchial asthma or chronic obstructive pulmonary disease is useful for detecting physiological changes due to bronchial asthma or chronic obstructive pulmonary disease. Furthermore, the use of the animal model for bronchial asthma or chronic obstructive pulmonary disease to reveal additional functions of marker genes and evaluate drugs whose targets are the marker genes, also have a great significance.

In addition, the animal model for bronchial asthma or chronic obstructive pulmonary disease of the present invention can be used to elucidate the mechanism underlying bronchial asthma or chronic obstructive pulmonary disease and also to test the safety of compounds obtained by screening. For example, when an animal model for bronchial asthma or chronic obstructive pulmonary disease according to the present invention develops the symptoms of asthma or chronic obstructive pulmonary disease, or when a measured value involved in a certain allergic disease alters in the animal, a screening system can be constructed to explore compounds having activity to alleviate the disease.

As used herein, the expression "an increase in the expression level" refers to any one of the following: where a marker gene introduced as a foreign gene is expressed artificially; where the transcription of a marker gene intrinsic to the subject animal and the translation thereof into the protein are enhanced; or where the hydrolysis of the protein, which is the translation product, is suppressed.

As used herein, the expression "a decrease in the expression level" refers to either the state in which the transcription of a marker gene of the subject animal and the translation thereof into the protein are inhibited, or the state in which the hydrolysis of the protein, which is the translation product, is enhanced. The expression level of a gene can be determined, for example, by a difference in signal intensity on a DNA chip as shown below in the Example. Furthermore, the activity of the translation product -the protein- can be determined by comparing with that in the normal state.

Representative transgenic animals include: animals to which a marker gene has been introduced and expressed artificially; marker gene knockout animals; and knock-in animals in which another gene has been substituted for a marker gene. A transgenic animal, into which an antisense nucleic acid of a marker gene, a ribozyme, a polynucleotide having an RNAi effect, or a DNA functioning as a decoy nucleic acid or such has been introduced, can be used as the transgenic animal of the present invention. Such transgenic animals also include, for example, animals in which the activity of a marker protein has been enhanced or suppressed by introducing a mutation(s) into the coding region of the gene, or the amino acid sequence has been modified to become resistant or susceptible to hydrolysis. Mutations in an amino acid sequence include substitutions, deletions, insertions, and additions. In addition, the expression itself of a marker gene of the present invention can be controlled by introducing a mutation (s) into the transcriptional regulatory region of the gene.

An amino acid substitution is preferably a "conservative amino acid substitution" -a mutation of an amino acid into a different amino acid that conserves the properties of the amino acid side-chain-. A "conservative amino acid substitution" is a replacement of one amino acid residue belonging to one of the following groups having a chemically similar side chain with another amino acid in the same group. Groups of amino acid residues having similar side chains have been defined in the art. These groups include amino acids with basic side chains (e.g., lysine, arginine, histidine) , acidic side chains (e.g., aspartic acid, glutamic acid), uncharged polar side chains (e.g., glycine, asparagine, glutamine, serine, threonine, tyrosine, cysteine), nonpolar side chains (e.g. , alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The number of amino acids that are mutated is not particularly restricted, as long as the activity is maintained. Normally, it is within 50 amino acids, preferably within 30 amino acids, more preferably within 10 amino acids, and even more preferably within 3 amino acids. The site of mutation may be any site, as long as the activity is maintained.

Methods for obtaining transgenic animals by targeting a particular gene are known. That is, a transgenic animal can be obtained by any of the following methods: mixing a gene and ovum and treating with calcium phosphate; introducing a gene directly into the nucleus of an oocyte in a pronuclei with a micropipette under a phase contrast microscope (microinjection method, US Patent No. 4873191) ; or using embryonic stem cells (ES cells). Furthermore, a method for infecting ovum with a gene-inserted retroviral vector, the sperm vector technique for transducing a gene into ovum via sperm, or such, have also been developed. The sperm vector technique is a gene recombination technique for introducing a foreign gene by fertilizing ovum with sperm after a foreign gene has been incorporated into sperm by adhesion or the electroporation method, etc. (M. Lavitrano, et al., Cell, 57, 717, 1989).

When a promoter whose transcription activity is controlled by a substance such as an appropriate drug is used in the expression vector, the expression level of a foreign marker gene can be regulated by administering the substance to the transgenic animal.

Transgenic animals used as the animal model for bronchial asthma or chronic obstructive pulmonary disease of the present invention can be produced using all vertebrates except humans. More specifically, transgenic animals having various transgenes or modified gene expression levels are being produced using vertebrates such as mice, rats, rabbits, miniature pigs, goats, sheep, monkeys, dogs, cats, or cattle.

In addition, the present invention relates to screening methods for candidate compounds for therapeutic agents to treat bronchial asthma or chronic obstructive pulmonary disease. According to the present invention, a marker gene is selected from the group according to the above (a) or (b). When the gene is selected from the group according to (a) , the expression level is significantly elevated in respiratory epithelial cells stimulated with IL-13 in comparison with unstimulated respiratory epithelial cells. When the gene is selected from the group according to (b) , the expression level is significantly decreased in respiratory epithelial cells stimulated with IL-13 in comparison with unstimulated respiratory epithelial cells.

Thus, when the marker gene belongs to group (a), a therapeutic agent for bronchial asthma or chronic obstructive pulmonary disease can be obtained by selecting a compound capable of decreasing the expression level of the marker gene. On the other hand, when the marker gene belongs to group (b) , a therapeutic agent for bronchial asthma or chronic obstructive pulmonary disease can be obtained by selecting a compound capable of increasing the expression level of the marker gene.

As used herein, the expression "a compound that increases the expression level of a gene" refers to a compound that promotes any one of the steps of gene transcription, gene translation, or expression of a protein activity. On the other hand, the expression "a compound that decreases the expression level of a gene", as used herein, refers to a compound that inhibits any one of these steps.

A method of screening for a therapeutic agent for an allergic disease of this invention can be carried out either *in vivo* or *in vitro.* This screening method can be performed, for example, according to the steps as described below:
(1) administering a candidate compound to an animal subject;
(2) measuring the expression level of a marker gene in a biological sample from the animal subject;
(3) selecting a compound that decreases the expression level of a marker gene belonging to group (a) , or a compound that increases the expression level of a marker gene belonging to group (b) , as compared to that in a control with which the candidate compound has not been contacted;

In the screening methods of the present invention, a gene functionally equivalent to any one of the genes selected from the group according to (a) or (b) described above, can be used as a marker gene. A representative example of a functionally equivalent gene includes a counterpart marker gene of a subject animal, which is intrinsic to the animal.

An animal used in the screening method of the present invention includes, for example, an animal model for bronchial asthma known in the art. For example, the animal model for ovalbumin (hereinafter abbreviated as "OVA") antigen-exposed bronchial hypersensitivity has been reported as an animal model for bronchial asthma. Bronchial hypersensitivity can be induced as follows: 50 µg OVA and 1 mg aluminum hydroxide as an adjuvant are injected into the peritoneal cavity of Balb/c mice (male, seven-week old), and after 10 days, the mice are sensitized with OVA by the same procedure. Then, after 10 days, 1% OVA is given to the mice by inhalation using Ultra-nebulizer model UN701 (Azwell, Inc.) for 30 minutes every four days three times in total. The enhanced bronchial hypersensitivity is monitored by detecting respiratory constriction caused by acetylcholine (6.25-2000 mg/kg) using a respirator (model 131, New England Medical Instruments Inc.) 24 hours after the final antigen inhalation (Nagai H. et al, Int Arch Allergy Immunol; 108: 189-195, 1995).

Furthermore, an animal model for chronic obstructive pulmonary disease is also known in the art. The animal model can be created using mice, rats, rabbits, miniature pigs, dogs, horses, etc. For example, an animal model for chronic obstructive pulmonary disease, which develops symptoms such as pulmonary emphysema, can be created by giving erastase to a New Zealand white rabbit three times by inhalation (Brenner M. et al., Chest, 121, 201-209, 2002). The screening according to the present invention can be practiced by administering a candidate compound to such an animal model and then monitoring variations in the expression level of a marker gene of the present invention.

A screening method using an animal model typically comprises monitoring the expression level of a marker gene that is inherently contained in the animal model. Thus, for example, the expression level of the mouse homolog of a marker gene is measured when the screening method uses a mouse model. Mouse genes according to (A) are genes whose expression levels are elevated in respiratory tissues of an OVA antigen-exposed bronchial hypersensitivity mouse model. On the other hand, mouse genes according to (B) are genes whose expression levels are decreased in respiratory tissue of the same mouse model. These mouse homolog genes can be used as marker genes in the screening methods of the present invention.

In addition to mouse homologs, one skilled in the art can identify similar homologs of various animal species based on the disclosure of the present invention. For example, various genes (or proteins) exhibiting a high homology to the nucleotide sequence or the amino acid sequence of a human marker gene or a mouse homolog can be identified by using homology searches. Alternatively, such homologs derived from other species can be isolated by hybridization to the marker gene.

However, with respect to screening methods comprising an animal model to which a human gene has been introduced, not only animal homologs but also human genes may be measured as marker genes.

Thus, the influence of a candidate compound for a pharmaceutical agent on the expression level of a marker gene can be assessed by contacting an animal subject with the candidate compound and monitoring the effect of the compound on the expression level of the marker gene in a biological sample derived from the animal subject. The variation in the expression level of the marker gene in a biological sample derived from the animal subject can be monitored using the same technique as used in the testing method of the present invention described above. Furthermore, based on the evaluation, a candidate compound for a pharmaceutical agent can be selected by screening. A compound that decreases the expression level is selected as a candidate compound for a pharmaceutical agent, when the marker gene is any one of the genes according to group (a); a compound that increases the expression level is selected as a candidate compound for a pharmaceutical agent, when the marker gene is any one of the genes according to group (b).

More specifically, a screening according to the present invention can be achieved by collecting respiratory epithelial cells as a sample from an animal subject, and comparing the expression level of a marker gene between the sample and a control with which the candidate compound has not been contacted. Methods for collecting and preparing respiratory epithelial cells are known in the art.

An animal subject may be stimulated with an allergen or IL-13 in a screening method of the present invention using an animal subj ect. The screening can be conducted by administering the candidate compound before or after the stimulation, or simultaneously, and comparing the expression level of a marker gene with that in a control. As a result, an effect of the candidate compound on the expression of a marker gene that responds to such stimulation can be evaluated. A compound having an activity to regulate the response of a marker gene to a stimulation with an allergen or IL-13 can be obtained through the screening.

These screening methods enable the selection of drugs involved in the expression of marker genes in various ways. More specifically, for example, drug candidate compounds having the following actions can be found:

When a marker gene belongs to group (a):
- suppression of a signal transduction pathway to induce the expression of the marker gene;
- suppression of the transcription activity of the marker gene; and
- inhibition of the stabilization of the transcription product of the marker gene or promotion of the decomposition thereof, etc;

When a marker gene belongs to group (b):
- activation of a signal transduction pathway to induce the expression of a marker gene;
- promotion of the transcription activity of the marker gene; and
- stabilization of the transcription product of the marker gene or inhibition of the decomposition thereof, etc;

Furthermore, methods of *in vitro* screening include, for example, a method that comprises contacting cells expressing a marker gene with a candidate compound and selecting a compound that decreases the expression level of a gene when the gene belongs to group (a), or alternatively selecting a compound that increases the expression level of a gene when the gene belongs to group (b). The screening can be conducted, for example, according to a method comprising the steps of:
(1) contacting a candidate compound with a cell expressing the marker gene;
(2) measuring the expression level of said gene; and
(3) selecting a compound that decreases the expression level of a marker gene belonging to group (a) or increases the expression level of a marker gene belonging to group (b), as compared to that in a control with which the compound has not been contacted;

In the present invention, cells expressing a marker gene can be obtained by inserting the marker gene to an appropriate expression vector, and introducing said vector into a suitable host cell. Any vector and host cell may be used as long as it is able to express a marker gene of this invention. Examples of host cells in the host-vector system are *Escherichia coli,* yeast, insect cells, animal cells, and such, and vectors that can be used for respective host cells can be appropriately selected.

Vectors may be introduced into hosts by a biological, physical, or chemical method, or such. Examples of biological methods are methods using viral vectors, methods using specific receptors, and cell-fusion methods (HVJ (Sendai virus) method, polyethylene glycol (PEG) method, electric cell fusion method, microcell-mediated chromosome transfer). Examples of physical methods are the microinjection method, electroporation method, and the method using the gene particle gun (gene gun). Examples of chemical methods are the calcium phosphate precipitation method, liposome method, DEAE-dextran method, protoplast method, erythrocyte ghost method, erythrocyte membrane ghost method, and microcapsule method.

In a screening method of the present invention, cells constituting respiratory tissues, such as epithelial cells and goblet cells can be used as cells expressing a marker gene. More specifically, epithelial cells, goblet cells, endothelial cells, smooth muscle cells, fibroblast cells, mucosal cells, and so on can be used.

Cells constituting respiratory tissues include a cell line established from the respiratory epithelium. Such a cell line can be used preferably in practicing a screening method of the present invention, because homogeneous cells can be prepared on a large scale and the cells can be cultured by a simple method. Such a respiratory epithelial cell line can be established, for example, by the following procedure. Namely, cells are collected from the lung, trachea, or mucous membrane by protease treatment or such. In some cases, cells can be immortalized and established as cell lines through infection of a virus such as Hepatitis B virus (HBV). A previously established cell line can be used in a screening according to the present invention. Cell lines from the respiratory epithelium, which can be used in the present invention, are listed below. The corresponding accession numbers in the ATCC cell bank are shown within parentheses.
Human lung cancer cell A549 (ATCC No. CCL-185)
   SHP-77 (ATCC No. CRL-2195)
Human bronchial epithelial cell BEAS-2B (ATCC No. CRL-9609)
   HBE4-E6/E7 (ATCC No. CRL-2078)
   NL20 (ATCC No. CRL-2503)
   NCI-H727 (ATCC No. CRL-5815)
   MeT-5A (ATCC No. CRL-9444)
   BBM (ATCC No. CRL-9482)
   BZR (ATCC No. CRL-9483)
Human mucosal endothelial cell NCI-H292 (ATCC No. CRL-1848)

A screening method of the present invention can be practiced by contacting a candidate compound with cells of a respiratory epithelial cell line described above and measuring the expression level of a marker gene within the cells. Based on the assay result, a compound that decreases the expression level of the gene is selected when the marker gene belongs to group (a) , or a compound that increases the expression level of the gene is selected when the marker gene belongs to group (b), in comparison with a control with which the candidate compound has not been contacted.

When used in a screening method of the present invention, respiratory epithelial cells can be cultured by using a method known in the art. It is preferable to use the AI method described above to culture respiratory epithelial cells. As used herein, the term the "AI method" refers to a culture method in which respiratory epithelial cells are in contact with air on the apical side and the culture medium is supplied from the basolateral membrane side. The term "air" in the AI method refers to air containing 5% CO₂ gas, which is typically used in culturing mammalian cells. In the AI method, the air is used after being sterilized with a filter.

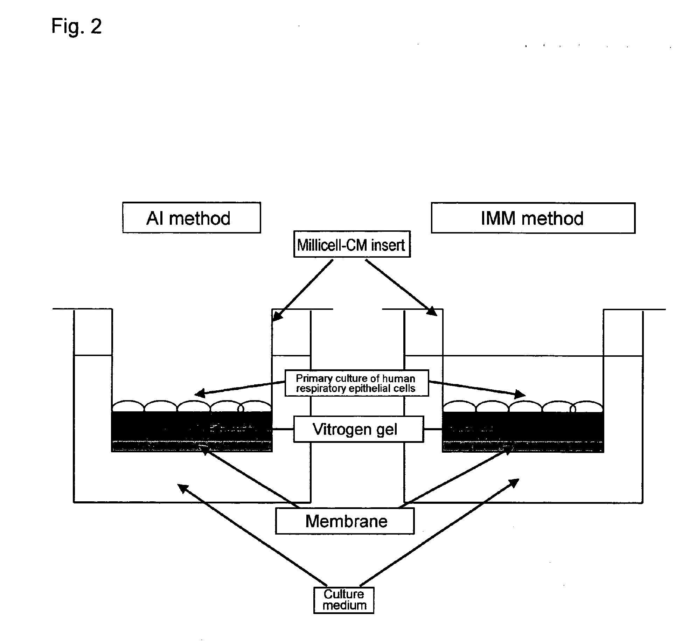

Animal cells are typically cultured in a culture medium under a constant concentration of CO₂. However, in the AI method, respiratory epithelial cells are cultured in contact with air. The difference between the AI method and the IMM method, which is a conventional culture method for respiratory epithelial cells, is schematically illustrated in Fig. 2.

When cultured by the AI method, respiratory epithelial cells differentiate into goblet cells upon IL-13 stimulation. Thus, the possibility of selecting a compound having an effect on the process of goblet cell differentiation can be increased by pre-culturing respiratory epithelial cells using the AI method. In a screening method of the present invention, respiratory epithelial cells can be treated with IL-13. Specifically, respiratory epithelial cells may be treated with IL-13 before or after contacting a candidate compound with the respiratory epithelial cells, or simultaneously.

When cultured by the AI method, respiratory epithelial cells differentiate into goblet cells upon IL-13 stimulation. Thus, an influence of a candidate compound on the expression level of a marker gene that is expressed in the process of goblet cell differentiation can be determined by monitoring as an index, the effect of the candidate compound on respiratory epithelial cells stimulated with IL-13.

The culture method for respiratory epithelial cells according to the AI method is known in the art. For example, respiratory epithelial cells can be cultured by the AI method based on disclosures in the reports indicated below.
Yamaya M.; Kokyu Vol. 12 No. 10, pp. 1238-1243 (1993);
Yamaya et al., Am. J. Physiol. 262 (Lung Cell Mol. Physiol. 6): L713-L724 (1992)

More specifically, first, tissues of the respiratory epithelium are collected from a living body, and a suspension of respiratory epithelial cells is prepared by protease treatment. A respiratory epithelial cell line may also be used. Respiratory epithelial cells from any mammalian species including humans can be used for the screening methods of the present invention. The resulting respiratory epithelial cells are cultured on a support. A preferred cell density of respiratory epithelial cells on the support falls within about 10⁴-10⁸ cells/cm², preferably within about 10⁶ cells/cm². Excess cells flowing out of the support are removed and the remaining is further cultured.

A material that can hold respiratory epithelial cells and supply components of the culture medium to the cells from the bottom of the cell layer, is used as a support. For example, a filter with pores whose size is too small for cells to pass through is preferably used as a support in the AI method. The filter used as a support may be coated with a material having affinity for the cells. Such materials include, for example, collagen gel. In the Examples, a commercially available filter (Millipore; Millicell-HA) coated with Vitrogen gel (CELTRIX; Vitrogen was used after gelation) is used in the AI method. The filter is attached to the bottom of an appropriate cuvette. When a suspension of respiratory epithelial cells is added to the cuvette, a cell layer is formed on the filter. Then, the culture according to the AI method can be done by floating the collagen gel-coated cuvette in a well filled with a medium.

A typical culture medium for respiratory epithelial cells may be used in the culture according to the present invention. Specifically, such a medium includes a culture medium comprising a 1:1 mixture of Dulbecco's MEM and Ham F12, which contains 2% Ultroser G, and the following antibiotics: penicillin, streptomycin, gentamycin, and amphotericin B.

Thus, the culture according to the AI method can be practiced by adhering cells to the above-mentioned filter, continuing culture in a state in which the filter side contacts the medium and the cell side contacts air. A test compound or IL-13 can be contacted with respiratory epithelial cells by adding it to the medium. In the AI method, IL-13 is added to the medium typically at the concentration of 5-100 ng/mL, preferably of 30-80 ng/mL, for example, of 50 ng/mL in order to stimulate respiratory epithelial cells. It is preferable to use IL-13 derived from the same species from which the respiratory epithelial cells are derived.

In the screening method of this invention, expression levels of marker genes can be compared not only based on the expression levels of proteins encoded by the genes, but also based on the corresponding mRNAs detected. For performing the comparison of expression levels using mRNA, the process for preparing an mRNA sample as described above is carried out in place of the process for preparing a protein sample. Detection of mRNA and protein can be performed by known methods as described above.

Furthermore, based on the disclosure of this invention, it is possible to obtain a transcriptional regulatory region for a marker gene of this invention and construct a reporter assay system. A reporter assay system is a system for screening for a transcriptional regulatory factor that acts on a transcriptional regulatory region using as an index the expression level of a reporter gene localized downstream of the transcriptional regulatory region.

Specifically, the present invention relates to a method of screening for therapeutic agents for bronchial asthma or chronic obstructive pulmonary disease, in which a marker gene is any one selected from the group according to (a) or (b) , or a gene functionally equivalent to the marker gene, which method comprises the steps of:
(1) contacting a candidate compound with a cell into which a vector containing a transcriptional regulatory region of a marker gene and a reporter gene under the control of the transcriptional regulatory region have been introduced;
(2) measuring the activity of said reporter gene; and
(3) selecting a compound that decreases the expression level of said reporter gene when the marker gene belongs to group (a), or a compound that increases the expression level of said reporter gene when the marker gene belongs to group (b), as compared to that in a control with which the candidate compound has not been contacted;

Examples of transcription regulatory regions are promoters, enhancers, and furthermore, CAAT box and TATA box, which are normally seen in the promoter region.

Also, as reporter genes, CAT (chloramphenicol acetyltransferase) gene, luciferase gene, growth hormone genes, and such may be used.

Alternatively, a transcription regulatory region of each marker gene of this invention can be obtained as follows. That is, first, a screening is performed by a method that uses PCR or hybridization based on the nucleotide sequences of marker gene cDNA disclosed in this invention, and a genomic DNA clone containing the cDNA sequence is obtained from a human genome DNA library such as the BAC library or YAC library. Based on the obtained genomic DNA sequence, the transcription regulatory region of a cDNA disclosed in this invention is estimated, and the transcription regulatory region is obtained. A reporter construct is constructed by cloning the obtained transcription regulatory region so that it is positioned upstream of the reporter gene. The obtained reporter construct is transfected into a cultured cell strain and is made into a transformant for screening. A candidate compound is contacted with this transformant. The screening of this invention can be performed by selecting a compound capable of decreasing the expression level of a marker gene when the gene belongs to group (a) ; or selecting a compound capable of increasing the expression level of a marker gene when the marker gene belongs to group (b) .

A screening method based on the activity of a marker gene can be used as an *in vitro* screening method of the present invention. Specifically, the present invention relates to a method of screening for a therapeutic agent for bronchial asthma or chronic obstructive pulmonary disease, in which the marker gene is any one selected from the group according to (a) or (b) , or a gene functionally equivalent to the marker gene, which method comprises the steps of:
(1) contacting a candidate compound with the protein encoded by a marker gene;
(2) measuring the activity of said protein; and
(3) selecting a compound that decreases said activity when the marker gene belongs to group (a), or a compound that increases said activity when the marker gene belongs to group (b), as compared to that in a control with which the candidate compound has not been contacted.

A compound having the activity of inhibiting the activity of a marker protein of the present invention can be selected through screening using the activity as an index, when the marker gene belongs to group (a). Such a compound that can be obtained as described above suppresses the activity of the respective marker gene belonging to group (a). Thus, the compound can control bronchial asthma or chronic obstructive pulmonary disease by inhibiting the marker protein whose expression has been induced in respiratory epithelial cells.

A compound having the activity of enhancing the activity of a marker protein can be selected through screening using the activity as an index, when the marker gene belongs to group (b). Such a compound that can be obtained as described above enhances the activity of the respective marker gene belonging to group (b). Thus, the compound can control bronchial asthma or chronic obstructive pulmonary disease by activating the marker protein whose expression has been inhibited in respiratory epithelial cells.

In addition to compound preparations synthesized by existing chemical methods, such as steroid derivatives and compound preparations synthesized by combinatorial chemistry, candidate test compounds used in such screenings include, mixtures of multiple compounds such as extracts from animal or plant tissues, or microbial cultures, and their purified preparations.

A polynucleotide, antibody, cell strain, or model animal necessary for various screening methods according to this invention can be combined in advance into a kit. A substrate compound used for the detection of a marker, a medium and vessel for cell culturing, positive and negative standard samples, and furthermore, a manual describing how to use the kit, may also be packaged in the kit. For example, such a kit may have a combination of a filter or a filter-attached cuvette to be used in the culture of respiratory epithelial cells according to the AI method, a culture well in which the cuvette is installed and the culture is maintained, a culture medium, and such.

A compound selected by a screening method of the present invention can be used as a therapeutic agent for bronchial asthma or chronic obstructive pulmonary disease. An antisense nucleic acid or a ribozyme capable of suppressing the expression level of a marker gene according to (a), or a polynucleotide that suppresses the expression of the gene through an RNAi effect can also be used as a therapeutic agent for bronchial asthma or chronic obstructive pulmonary disease.

Furthermore, an antibody recognizing a peptide comprising the amino acid sequence of a protein encoded by any one of the genes according to (a) can also be used as a therapeutic agent for bronchial asthma or chronic obstructive pulmonary disease. Each marker gene according to (a) is a gene whose expression level is increased in respiratory epithelial cells stimulated with IL-13. Thus, a therapeutic effect on bronchial asthma or chronic obstructive pulmonary disease can be achieved by suppressing the expression of the genes or the function of proteins encoded by the genes.

In addition, any marker gene according to (b) and the protein encoded by the gene can be used as a therapeutic agent for bronchial asthma or chronic obstructive pulmonary disease.

A therapeutic agent for an allergic disease according to this invention can be formulated by including a compound selected by a screening method of the present invention as an active ingredient, and mixing it with a physiologically acceptable carrier, excipient, diluent, or such. The therapeutic agent can be administered orally or parenterally to ameliorate the allergy symptoms.

Oral drugs can take any dosage form selected from the group of granules, powders, tablets, capsules, solutions, emulsions, suspensions, etc. Injections can include subcutaneous injections, intramuscular injections, or intraperitoneal injections.

Furthermore, when the compound to be administered comprises a protein, a therapeutic effect can be achieved by introducing a gene encoding the protein into the living body using gene therapy techniques. Techniques for treating diseases by introducing a gene encoding a therapeutically effective protein into the living body and expressing it therein are known.

Alternatively, an antisense nucleic acid, a ribozyme, or a polynucleotide that suppresses the expression of a corresponding gene by an RNAi effect can be incorporated downstream of an appropriate promoter sequence to be administered as an expression vector of an antisense RNA, a ribozyme, or an RNA having the RNAi effect. When this expression vector is introduced into mononuclear cells of an allergy patient, the therapeutic effect on the allergy can be achieved by reducing the expression level of the gene by expressing a corresponding antisense nucleic acid, ribozyme, or polynucleotide that suppresses the expression of a corresponding gene by an RNAi effect. *In vivo* or *ex vivo* methods are known for introducing the expression vector into mononuclear cells.

The expression "antisense RNA" refers to an RNA comprising a nucleotide sequence complementary to the sense sequence of a gene. When an antisense RNA is used to suppress gene expression, such an RNA typically comprises a nucleotide sequence of 15 or more consecutive nucleotides, for example, 20 or more consecutive nucleotides, or 30 or more consecutive nucleotides. For example, an antisense nucleic acid capable of hybridizing to a region comprising an initiation codon is thought to be highly effective in suppressing the expression of the corresponding gene.

The term "ribozyme" refers to an RNA that has the catalytic activity of digesting RNA in a nucleotide sequence-specific manner. There are two types of ribozymes: hammerhead ribozymes and hairpin ribozymes. Both ribozymes are composed of a nucleotide sequence portion complementary to the region to be digested and a nucleotide sequence portion that maintains the structure required for the catalytic activity. The nucleotide sequence complementary to the region to be digested can be arbitrary. Therefore, when the nucleotide sequence of this region is set to be complementary to the nucleotide sequence of a target gene, a ribozyme can be designed to control the expression of a marker gene.

The expression "RNAi (RNA interference) effect" refers to the phenomenon where a double-stranded RNA comprising a nucleotide sequence identical to that of an mRNA strongly suppresses the expression of the mRNA. Thus, such a double-stranded RNA comprising a nucleotide sequence identical to that of the mRNA of a marker gene can be used to suppress the expression of the marker gene. A double-stranded RNA comprising a nucleotide sequence having at least 20 or more consecutive nucleotides is preferably used to exert an RNAi effect. The double strand may be composed of separate strands or a stem-and-loop structure of a single RNA chain.

With respect to an antisense nucleic acid, a ribozyme, or a polynucleotide exerting the RNAi effect, a complementary nucleotide sequence and an identical nucleotide sequence are not limited to a perfectly complementary nucleotide sequence and a perfectly identical nucleotide sequence, respectively. When having a high sequence complementarity or identity, the RNAs exhibit the activity of suppressing expression. When having typically 70% or higher, preferably 80% or higher, more preferably, 90% or higher, still more preferably 95% or higher, for example, 98% or higher identity to a nucleotide sequence or a nucleotide sequence complementary to a nucleotide sequence, an RNA can be deemed to have a high identity or complementarity.

Although the dosage may vary depending on the age, sex, body weight, and symptoms of a patient, and also treatment effects, method for administration, treatment duration, type of active ingredient contained in the drug composition, or such, it can be usually administered in the range of 0.1 mg to 500 mg, preferably 0.5 mg to 20 mg per dose for an adult. However, since the dosage varies according to various conditions, an amount less than the above-described dosage may be sufficient in some cases, whereas in others, a dosage exceeding the above-described range may be required.

The present invention also provides a DNA chip for diagnosing bronchial asthma or chronic obstructive pulmonary disease, on which a probe has been immobilized. The probe is used to detect a marker gene that is at least a single gene selected from group (a) or group (b). There is no limitation on the type of the marker gene. The more the marker gene number, the more are the markers that can be used for the diagnosis. In general, the accuracy of diagnosis is high if more markers are used. When multiple marker genes are detected, it is advantageous to select genes having different properties. Genes that are assumed to be different with respect to the mechanism of expression level variation or and the function of the encoded proteins may be defined as "genes having different properties".

Exemplary combinations of marker genes are shown below. These combinations can enhance the accuracy of allergy testing.

### [Two or more genes selected from the group consisting of marker genes for proteases and protease inhibitors]

Proteases and protease inhibitors can serve as markers for the balance between tissue disruption and construction. Specifically, a chip for testing allergic bronchial asthma or chronic obstructive pulmonary disease can be prepared by accumulating probes for detecting genes selected from genes belonging to the protease group and protease inhibitor group among the marker genes of the present invention. Marker genes belonging to each group are listed at the end of this specification.

### [Two or more genes selected from the group consisting of marker genes for cytokines, cytokine receptors, chemokines, chemokine receptors, CD antigens, antibodies, and antibody receptors]

Any combination of the genes listed above contains a pair of substances that are mutually related as a ligand-and-receptor. An immune response may be viewed as a result of the interaction between these substances. Accordingly, the immunological state of respiratory epithelial tissues may be determined by using these marker genes in combination. A pair of molecules in a ligand-and-receptor relationship may be selected as marker genes. Alternatively, one of the molecules in the pair may be selected as a marker gene when only that molecule has been shown to be a marker gene of the present invention.

### [Two or more genes selected from the group consisting of marker genes for cytokines, extracellular matrix proteins, cytoskeletal proteins, cell adhesion molecules, and transcription factors]

Extracellular matrix proteins include collagen. Cytoskeletal proteins include keratin, small proline-rich protein and involucrin. Cell adhesion molecules include cadherin and desmocollin. Transcription factors include jun, fos, and myc. The degree of the differentiation of respiratory epithelial tissues or remodeling (repair) of inflammatory lesions can be assessed by monitoring the expression levels of marker genes.

### [Two or more genes selected from marker genes encoding enzymes]

Once a gene is selected from marker genes encoding enzymes, then it is possible to know which metabolic processes occur in respiratory epithelial cells. For example, the metabolism of lipid mediators and lipid molecules participating in the barrier function of the respiratory epithelium can be determined based on the expression levels of lipid-metabolizing enzymes. Such lipid-metabolizing enzymes include, for example, phospholipase A2, cyclooxygenase-2, prostaglandin D2 synthase, and fatty acid desaturases 1 and 2.

Alternatively, a chip for testing for bronchial asthma or chronic obstructive pulmonary disease, which contains densely immobilized probes capable of detecting genes selected from those constituting groups (a) and (b) , is effective in order to achieve a more accurate diagnosis. The selected genes are a combination of any multiple genes. Specifically, typically 10 or more, for example, 30 or more, preferably 50 or more, more preferably 60 or more, still more preferably 80 or more, or 100 or more genes can be selected from group (a). Likewise, typically 10 or more, for example, 30 or more, preferably 50 or more, more preferably 60 or more, still more preferably 80 or more, or 100 or more genes can be selected from group (b). Much more genes, for example, 150 or more, preferably 180 or more, more preferably 200 or more genes may be selected from each of the groups (a) and (b).

The present invention provides marker genes belonging to groups (a) and (b) described below for bronchial asthma or chronic obstructive pulmonary disease:
(a) group of genes whose expression levels are increased in respiratory epithelial cells upon stimulation with IL-13; and
(b) group of genes whose expression levels are decreased in respiratory epithelial cells upon stimulation with IL-13.

The use of the expression level of each gene as a marker makes it possible to establish a method of testing for bronchial asthma or chronic obstructive pulmonary disease; create animal models for bronchial asthma or chronic obstructive pulmonary disease; and screen for candidate compounds for therapeutic agents for treating the diseases. All marker genes of the present invention are genes whose expression levels vary upon stimulation with IL-13 in respiratory epithelial cells cultured by the AI method. The AI method enables the culture of respiratory epithelial cells under conditions similar to the original conditions in the body. Thus, there is a high possibility that the expression levels of marker genes found throughout the present invention are indeed altered upon stimulation with IL-13 in tissues of the respiratory tract. As described herein in Examples, the expression levels of the marker genes of the present invention are indeed increased in the mouse asthma model. Thus, all the marker genes of the present invention can be used as markers for bronchial asthma or chronic obstructive pulmonary disease, and as targets in treating bronchial asthma or chronic obstructive pulmonary disease.

The variation in the expression level of each marker gene of the present invention correlates to the disease state. Thus, bronchial asthma or chronic obstructive pulmonary disease can be treated by controlling the expression levels of the marker genes and the activities of the proteins encoded by the marker genes. For example, when the expression level of a gene of interest is increased in respiratory epithelial cells accompanied by the differentiation of the cells into goblet cells, the expression of the gene or the activity of the encoded protein is inhibited in a therapeutic strategy for treating bronchial asthma or chronic obstructive pulmonary disease. In contrast, when the expression level of a gene of interest is decreased in respiratory epithelial cells, the expression of the gene or the activity of the encoded protein is enhanced in a therapeutic strategy for treating bronchial asthma or chronic obstructive pulmonary disease. Furthermore, the marker genes can be used as novel clinical diagnostic markers to monitor bronchial asthma or chronic obstructive pulmonary disease in the treatment of the diseases.

The expression level of each marker gene provided by this invention can be easily determined, regardless of the type of allergen. Therefore, the overall pathology of an allergic reaction can be understood.

Additionally, the methods of testing for bronchial asthma or chronic obstructive pulmonary disease of this invention have low invasiveness towards patients since analysis of expression levels can be carried out using a biological sample. Furthermore, gene expression analysis has enabled highly sensitive measurements using small amounts of samples. Year after year in gene analysis technology, high throughput methods are being improved and costs are being decreased. Therefore, in the near future, the methods of testing for bronchial asthma or chronic obstructive pulmonary disease of this invention are expected to become important bedside diagnostic methods (methods that can be performed outside labs). In this sense, diagnostic value of the marker genes of this invention is high.

Furthermore, the present invention reveals that the expression level of pendrin in respiratory epithelial cells is increased upon IL-13 stimulation and that the PDS gene encoding pendrin is one of genes participating in the differentiation of respiratory epithelium cells into goblet cells. The expression level of pendrin is also increased in the lung of the asthma model mouse, and thus the present invention shows that the PDS gene encoding pendrin is closely associated with bronchial asthma or chronic obstructive pulmonary disease. The development of drugs for suppressing goblet cell differentiation did not start until recently. Thus, the present invention provides a new approach in drug discovery. In addition, the present invention reveals genes participating in goblet cell differentiation, enabling a more fundamental therapy that uses the genes. Furthermore, agents that control the expression level of genes participating in goblet cell differentiation or the activity of proteins participating in goblet cell differentiation can be used in the treatment of diseases characterized by inflammation and overproduction of mucus, such as chronic obstructive pulmonary disease, cystic fibrosis, chronic sinusitis, bronchiectasis, and diffuse panbronchiolitis, as well as asthma.

Any patents, published patent applications, and any prior art references cited herein are incorporated by reference. Hereinafter, the present invention is described more specifically based on Examples, but it is not to be construed as being limited thereto.

### EXAMPLE 1

### The air interface (AI) method and the immersed feeding (IMM) method

### 1. The air interface method:

Approval for this study was obtained from the Ethical Committee of the Faculty of Medicine, The Tohoku University, Japan. Tracheal tissues derived from anatomical specimens were stretched on plates. The epithelia were removed and allowed to stand still in phosphate buffer containing protease (0.05%) at 4°C overnight. The following day, a culture medium containing fetal calf serum was added to the samples to neutralize enzyme activity, and respiratory epithelial cells were isolated by shaking the samples.

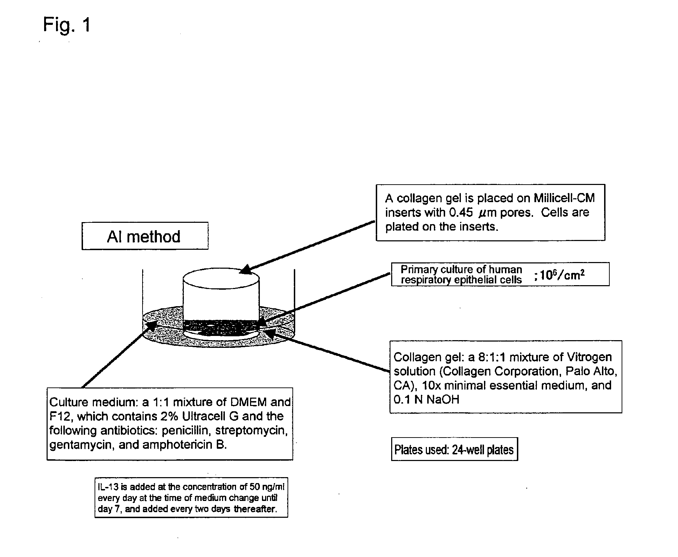

After the cell count was determined, cells were plated at the cell density of 10⁶ cells/cm² on a filter membrane with 0.45-µm pores, being attached to the bottom of a Millicell-HA Culture Plate Insert (Millipore Corp.). At the time of plating, Vitrogen gel (Vitrogen from Celtrix Pharmaceuticals, Inc. was used after gelation) was placed on the filter membrane as a growth-supporting material, and the epithelial cells were placed thereon. The Millicell inserts were placed in a 24-well plate (Falcon) containing a culture medium, which was a 1: 1 mixture of Dulbecco's MEM and Ham F12 containing 2% Ultroser G and the antibiotics, penicillin, streptomycin, gentamycin, and amphotericin B. The cells were incubated overnight. Then, cells that had not adhered to the collagen gel were removed, and the remaining cells were cultured while the cell side was in contact with air (air interface) for approximately two weeks (See Fig. 1). The basic procedures of the AI method by which respiratory epithelial cells were cultured were the same as those described in the following reports:
Yamaya M; Kokyu, Vol. 12, No. 10, pp. 1238-1243 (1993); and
Yamaya et al., Am. J. Physiol. 262 (Lung Cell Mol. Physiol. 6): L713-L724, 1992.

### 2. The immersed feeding method (IMM method):

As basically done in the AI method, Vitrogen gel was placed on a filter membrane, and epithelial cells were placed thereon. The IMM method is different from the AI method in the point that the IMM method comprises adding a medium to cover the epithelial cells. Then, the filter membrane was placed in a 24-well plate (Falcon) containing the same medium as that used in the AI method. The cells were incubated for approximately two weeks (See Fig. 2). The basic procedures of the IMM method by which respiratory epithelial cells were cultured were the same as those described in the following reports:
Yamaya M; Kokyu, Vol. 12, No. 10, pp. 1238-1243 (1993); and
Yamaya et al., Am. J. Physiol. 262 (Lung Cell Mol. Physiol. 6): L713-L724, 1992.

### EXAMPLE 2

### Stimulation of bronchial epithelial cells with IL-13

In the AI method in Example 1, human IL-13 (Peprotech, Inc.) was added to the medium at the concentration of 50 ng/mL when changing the medium, every day for 7 days. After 7 days, human IL-13 was added to the medium when the medium was changed, every two days. After 14 days of incubation, cells were treated by PAS staining for acidic sugar chains and Alcian blue staining for basic sugar chains. The result showed that the cells had differentiated into goblet cells comprising a huge glycoprotein, mucin.

Human IL-13 was also added in the IMM method. However, goblet cell differentiation was not observed. The objective of this study is to screen genes associated with the differentiation of respiratory epithelial cells into goblet cells upon IL-13 stimulation by the AI method. Therefore, instead of completely differentiated day-14 cells, cells that were in the process of undergoing cell differentiation were harvested at day 3 and day 7. Furthermore, cells from two different lots were used in the culture. The culture conditions used are described below.

**Table 1**

| Lot 1 | | | |
|---|---|---|---|
| Culture method | Stimulation with IL-13 | Day 3 | Day 7 |
| AI | + | 1 | 5 |
| IMM | + | 2 | 6 |
| AI | - | 3 | 7 |
| IMM | - | 4 | 8 |

| Lot 2 | | | |
|---|---|---|---|
| Culture method | Stimulation with IL-13 | Day 3 | Day 7 |
| AI | + | 9 | 11 |
| AI | - | 10 | 12 |

### EXAMPLE 3

### Preparation of RNA for GeneChips

Respiratory epithelial cells treated by the procedure described above were lysed with ISOGEN (Nippon Gene Co. , Ltd.). RNA was isolated from the solution according to the protocol attached to ISOGEN. Chloroform was added to the solution. After the mixture was stirred and centrifuged, the aqueous layer was collected. Then, isopropanol was added to the aqueous solution. After stirring and centrifuging the solution, the precipitated total RNA was collected. Approximately 5 µg to 15 µg total RNAs were extracted from sample Nos. 1 to 12. The total RNAs were analyzed for gene expression using HG-U95A to HG-U95E from Affymetrix. The type A gene chip comprises about 12,000 probes designed based on the information on the nucleotide sequences of full-length cDNAs. Each of the type B, C, D, and E gene chips comprises about 50,000 probes designed based on the information on the nucleotide sequences of ESTs.

### EXAMPLE 4

### Synthesis of cRNA for GeneChips

Single stranded cDNA was prepared from 5 µg of total RNA by reverse transcription using Superscript II Reverse Transcriptase (Life Technologies) following the method of Expression Analysis Technical Manual by Affymetrix, and by using T7-(dT)₂₄ (Amersham Pharmacia) as a primer. The T7-(dT)₂₄ primer comprises a nucleotide sequence in which d (T)₂₄ is added to a T7 promoter nucleotide sequence, as shown below.

T7-(dT)24 primer (SEQ ID NO: 1)
5'-GGCCAGTGAATTGTAATACGACTCACTATAGGGAGGCGG-(dT)24-3'

Next, according to Expression Analysis Technical Manual, DNA ligase, DNA polymerase I, and RNase H were added to synthesize double stranded cDNA. After phenol-chloroform extraction of cDNA, the extract was passed through Phase Lock Gels, and was purified by ethanol precipitation.

Furthermore, using BioArray High Yield RNA Transcription Labeling Kit, biotin-labeled cRNA was synthesized. Approximately 20-50 µg of biotinated cRNA was synthesized from Sample Nos. 1 to 12. Using RNeasy Spin column (QIAGEN), cRNA was purified and then fragmented by heat treatment.

15 µg of this cRNA was added to a hybridization cocktail, according to the Expression Analysis Technical Manual. This was placed in an array and was hybridized for 16 hours at 45°C.

After the array was washed, streptavidin phycoerythrin was added for staining. After washing, a mixed antibody solution of normal goat IgG and biotinylated goat IgG was added to the array. Furthermore, in order to enhance fluorescence intensity, streptavidin phycoerythrin was added again for staining. After washing, this was set in a scanner and was analyzed by the GeneChip software Suite 4.0.

### EXAMPLE 5

### GeneChip analysis

Data analysis was performed using the GeneChip analysis software Suite 4.0. Average Intensity (1) and Background Average (2) were determined by Absolute Analysis, and four average values were obtained (AI method, no stimulation; AI method, IL-13 stimulation; IMM method, no stimulation; and IMM method, IL-13 stimulation) by subtracting (2) from (1). These four values were used as scale factors for comparison analysis.

First, absolute analysis was performed to analyze one chip data. Positives and negatives were determined by comparing the fluorescence intensity of perfect matches and mismatches of a probe set. Determination of the three categories of Absolute Calls, i.e., P (present) , A (absent), and M (marginal), were made by values of Pos Fraction, Log Avg, and Pos/Neg:
Pos Fraction; ratio of positive pairs.
Log Avg; average of the log of fluorescence intensity ratio between probe cells of perfect match and mismatch.
Pos/Neg; ratio of the number of positive pairs and negative pairs.

Additionally, Average Difference (Avg Diff), which is the average value of the difference in fluorescence intensities between perfect matching and mismatching probe cells, was calculated for each gene.

Next, Comparison Analysis was performed on two sets of data. For example, comparison was made between the AI method, no stimulation of day 3 and the AI method, IL-13 stimulation of day 3, and the difference in expression levels was ranked as follows. Determination of the 5 categories of difference calls, which are I, D, MI, MD, and NC, were made from values of Inc/Dec, Inc Ratio, Dpos-Dneg Ratio, and Log Avg Ratio Change.
Inc: Number of probe pairs that corresponded to IL-13 stimulation and no stimulation and that were judged to have increased expression levels when stimulated by IL-13.
Dec: Number of pairs judged to have decreased expression levels when stimulated by IL-13.
Inc/Dec: Ratio of the number of pairs judged to be Inc and number of pairs judged to be Dec.
Inc Ratio: Number of pairs judged to be Inc/number of pairs actually used.
Dpos/Dneg Ratio: Ratio between the number of Neg Change subtracted from that of Pos Change, and the number of pairs actually used.
Pos Change: Difference between the number of positive pairs in Absolute Analysis of IL-13 stimulation, and the number of positive pairs in Absolute Analysis of no stimulation.
Neg Change: Difference between the number of negative pairs in Absolute Analysis of IL-13 stimulation, and the number of negative pairs in Absolute Analysis of no stimulation.
Log Avg Ratio Change: Difference between Log Avg in Absolute Analysis of IL-13 stimulation and no stimulation.
Increased: I,
Decreased: D,
Marginally Increased: MI,
Marginally Decreased: MD, and
No Change: NC

1. A group of genes associated with goblet cell differentiation, which had been narrowed down from the genes on the gene chips of HG-U95A to HG-U95E (group (a)/ a group of genes whose expression levels were increased; and group (b)/ a group of genes whose expression levels were decreased)

The sequences and the number of genes in gene chips A to E, whose expression levels were found to increase by two folds or more or decrease by half or less upon IL-13 stimulation in both Lots 1 and 2 under the culture conditions of the AI method, are shown in each category in Table 2. The column labeled "Increased" contains the sequences and the numbers of genes whose expression levels increased upon IL-13 stimulation. The column labeled "Decreased" contains the sequences and the numbers of genes whose expression levels decreased upon IL-13 stimulation. The annotations on the genes selected using EST chips of B to E are described according to the database NetAffx (TM) of the June/2002 version provided by Affymetrix.

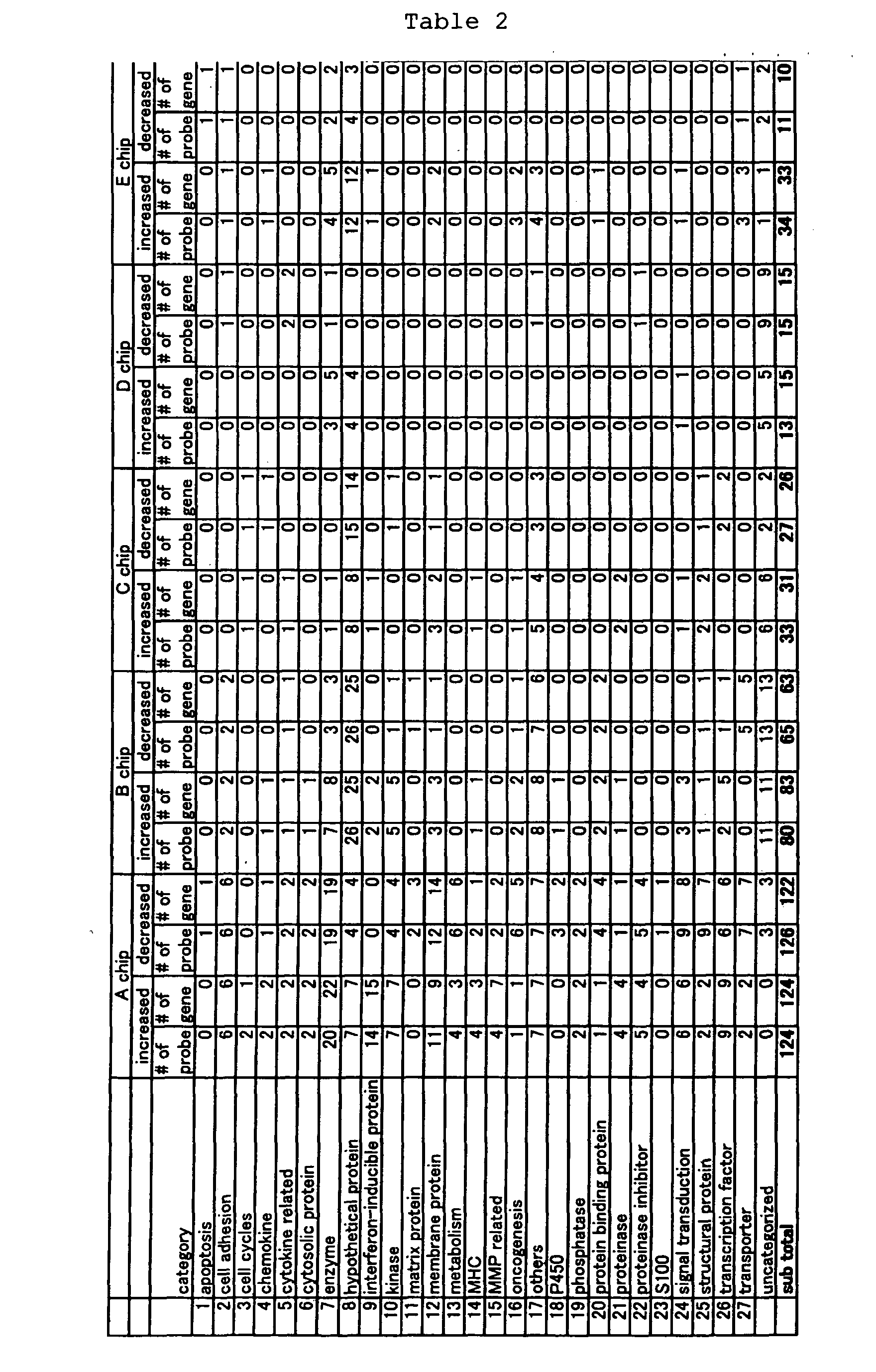

Tables 3 to 19 (a group of genes whose expression levels increased upon IL-13 stimulation) and Tables 20 to 36 (a group of genes whose expression levels decreased upon IL-13 stimulation) include lists of categorized genes on the chips of HG-U95A to HG-U95E . The Tables also include values of fold changes upon IL-13 stimulation in lot 1 and 2 when the AI method or the IMM method was used.

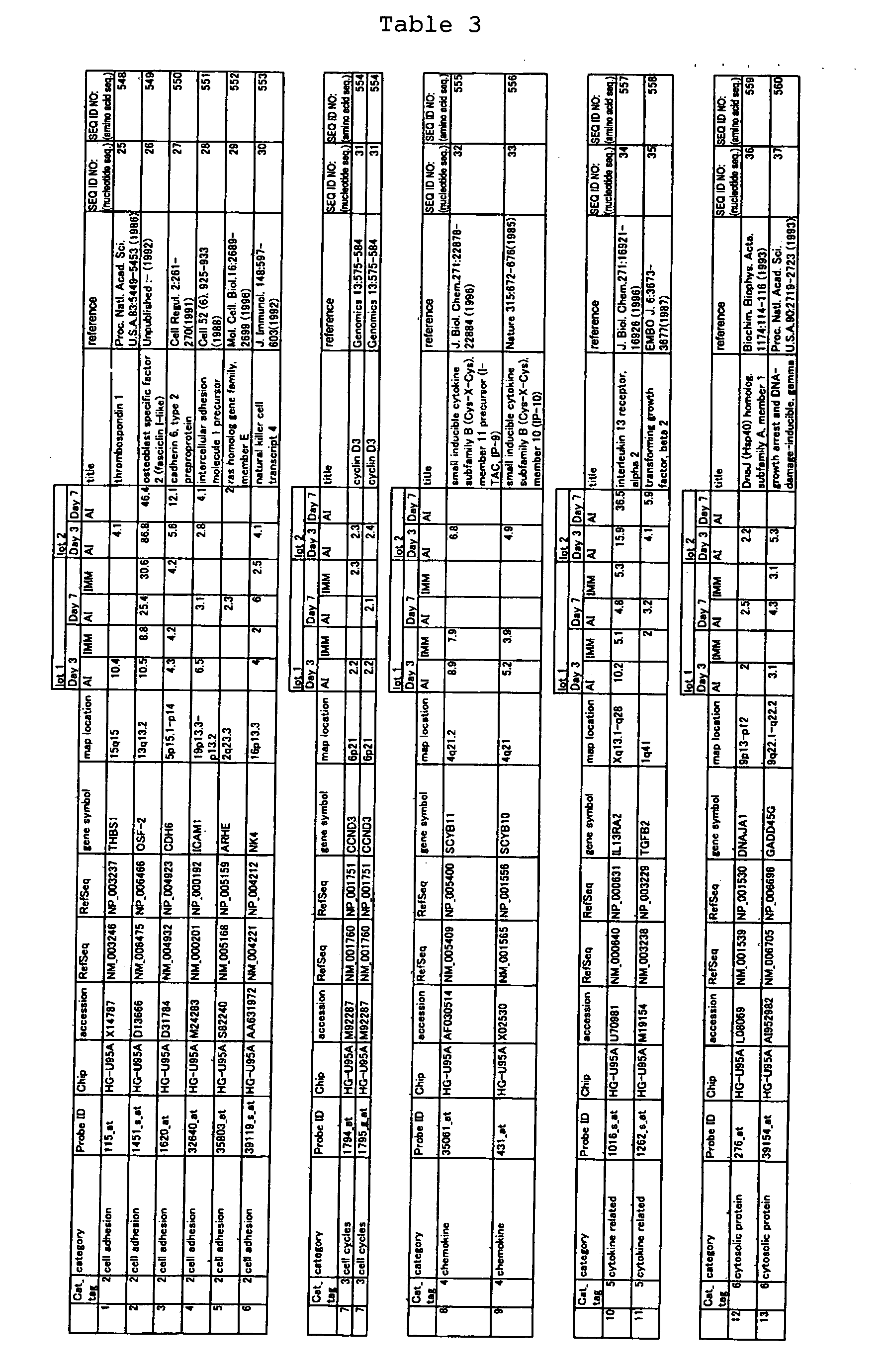

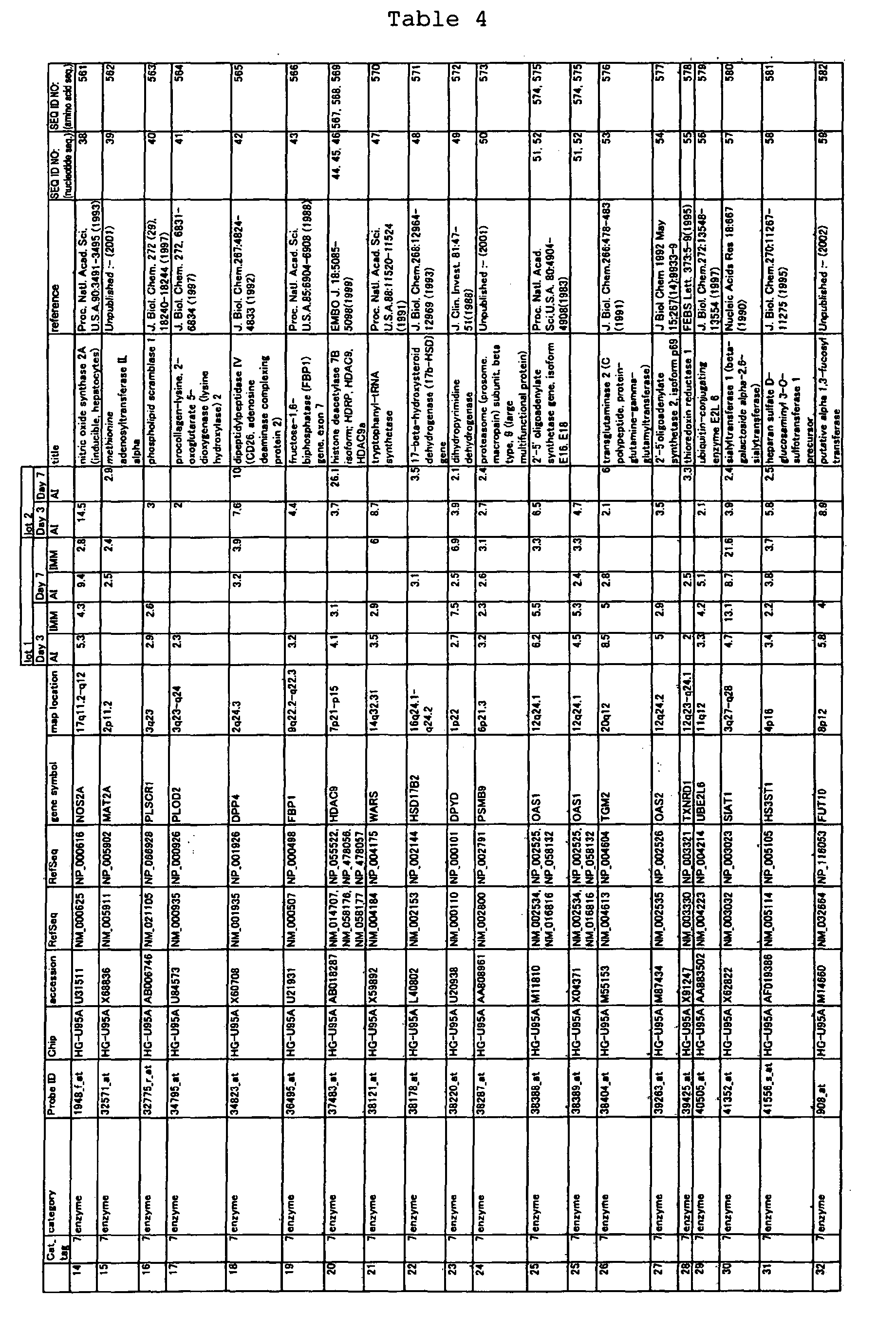

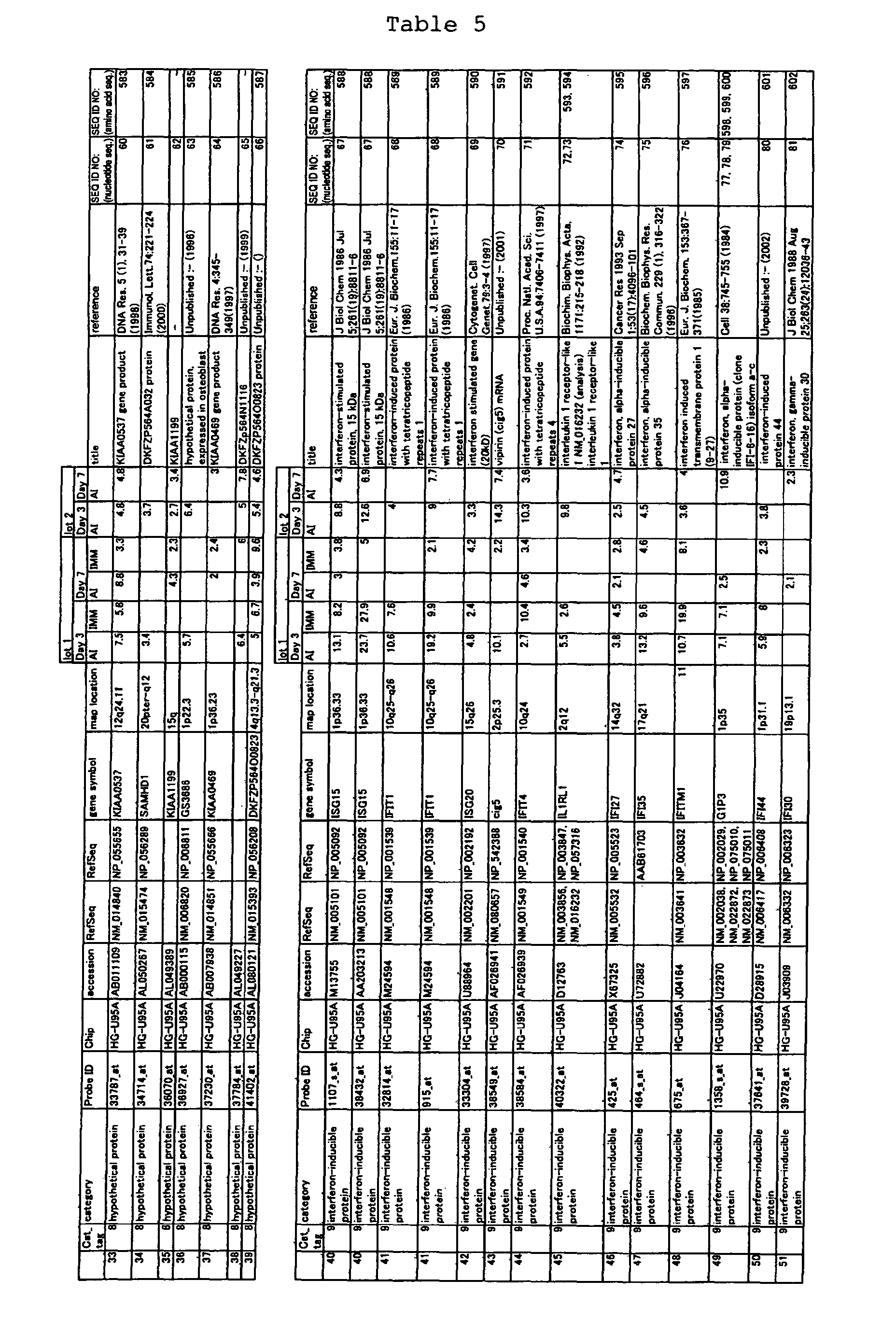

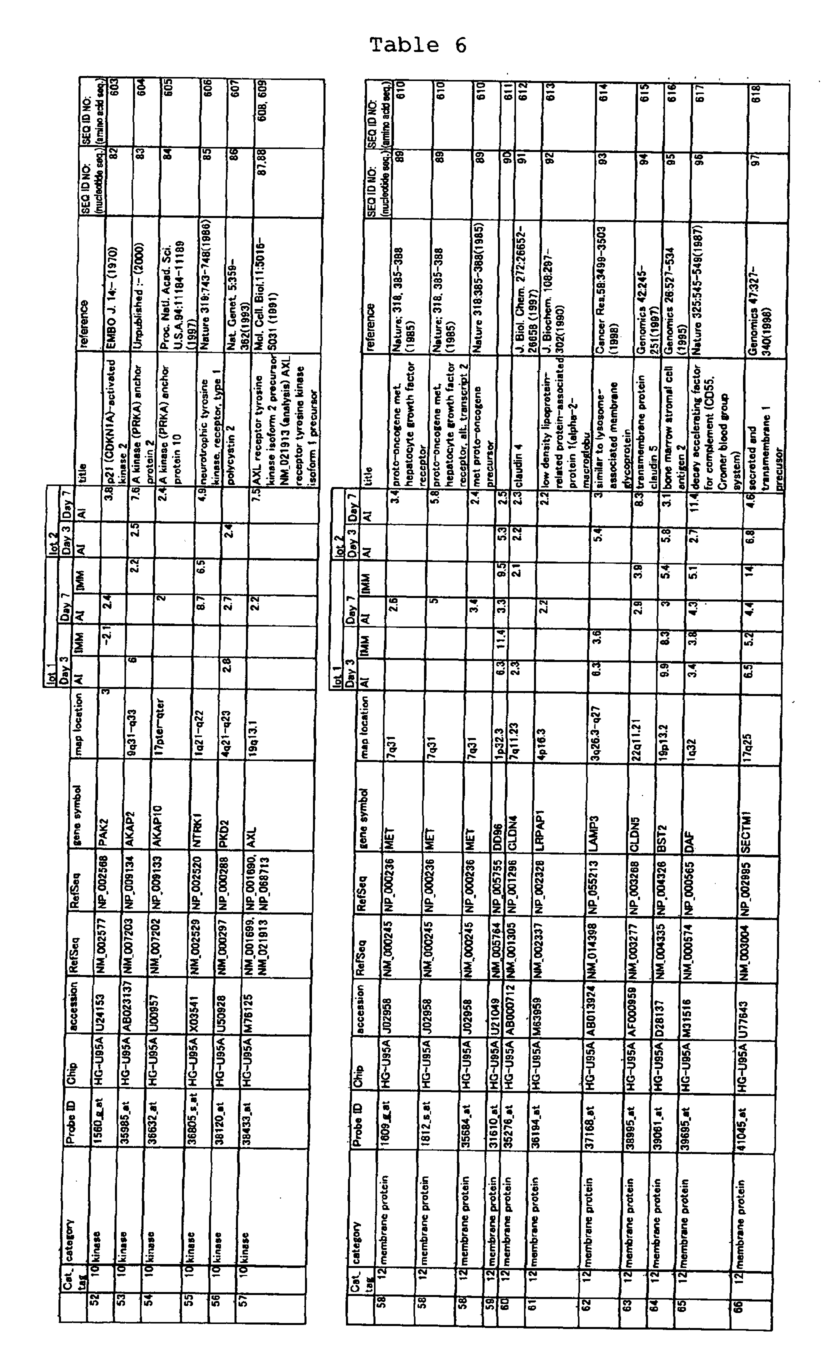

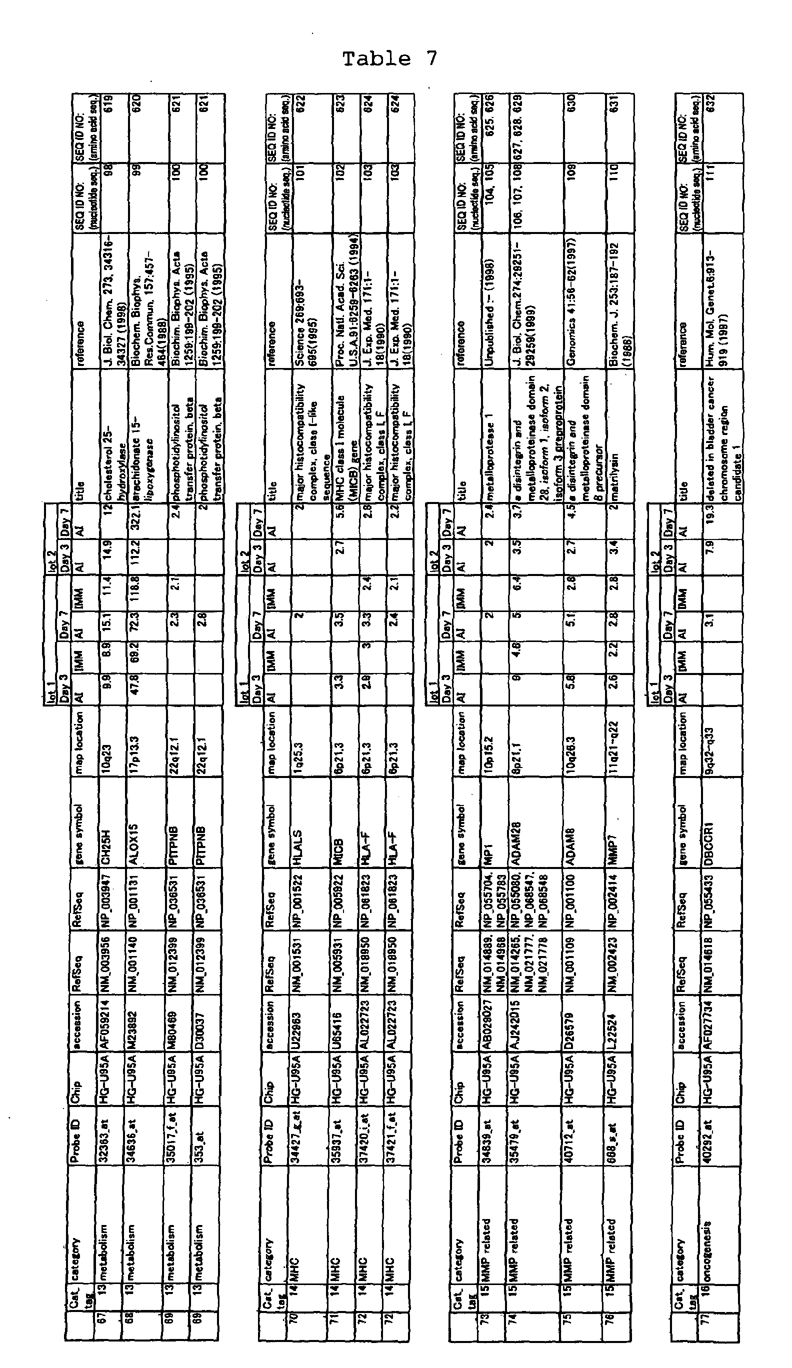

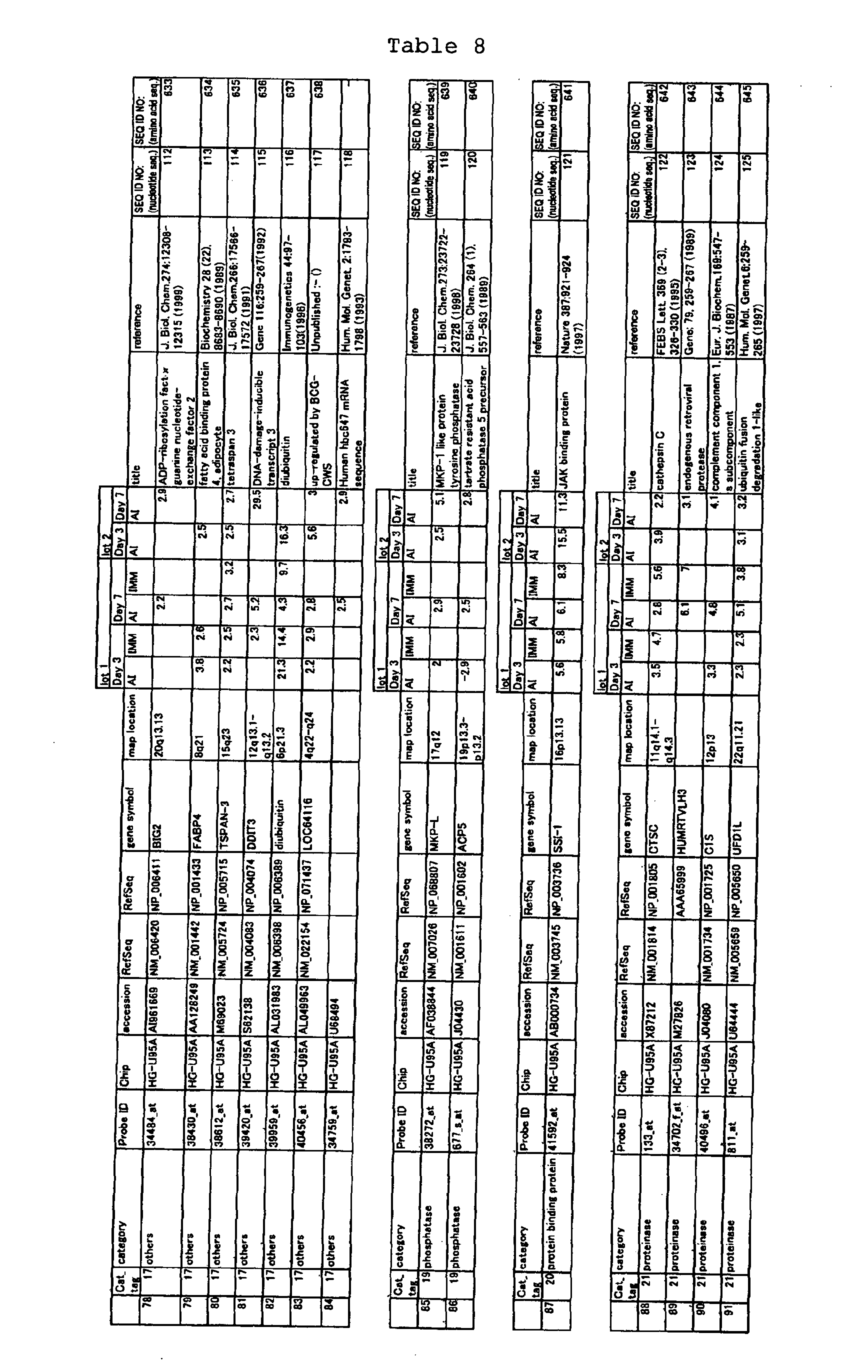

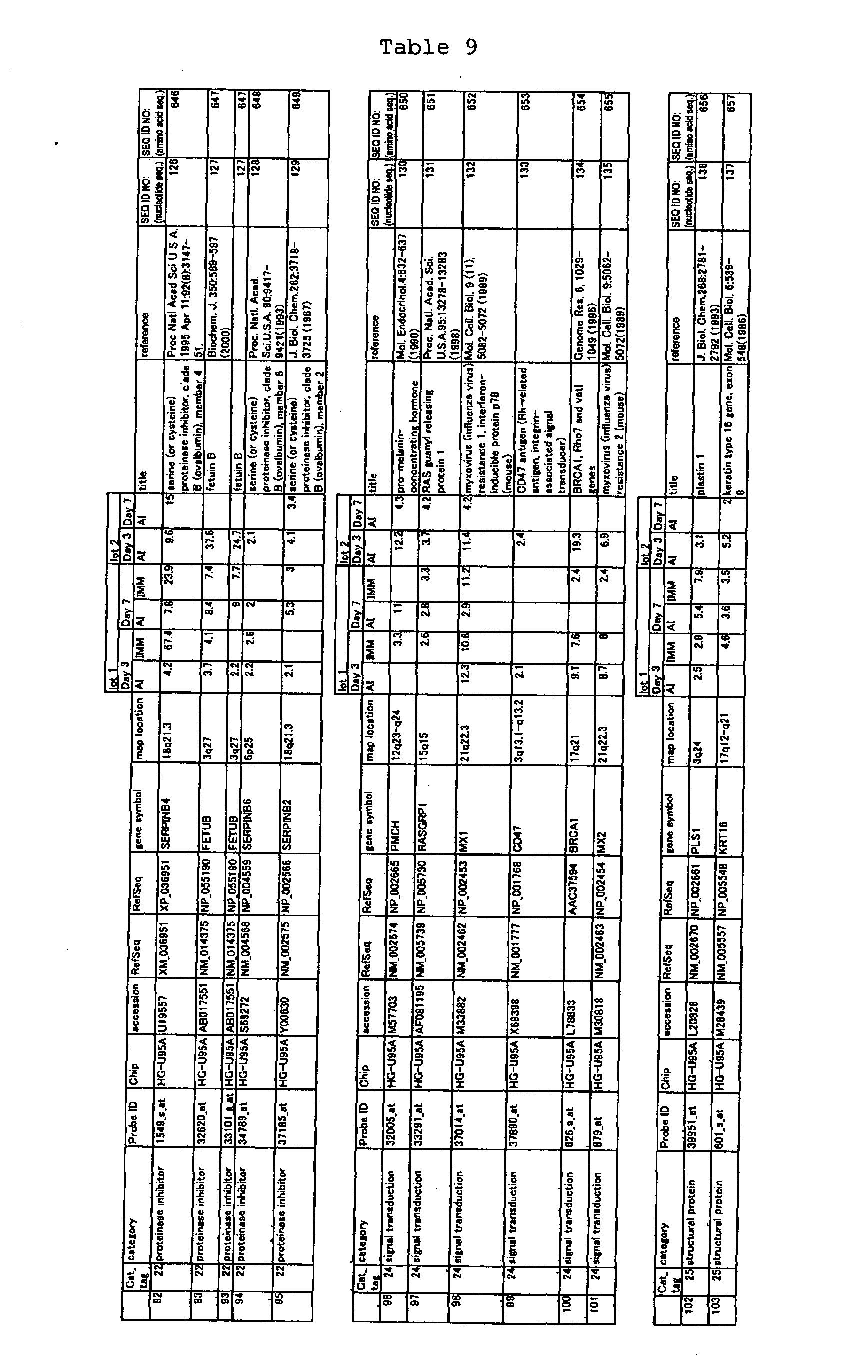

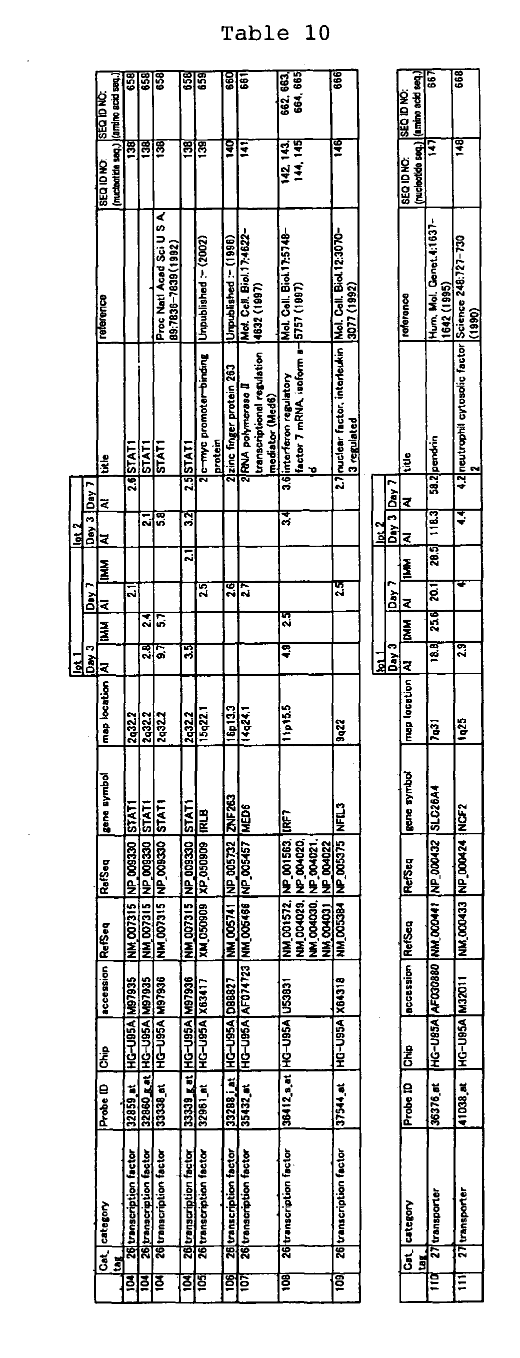

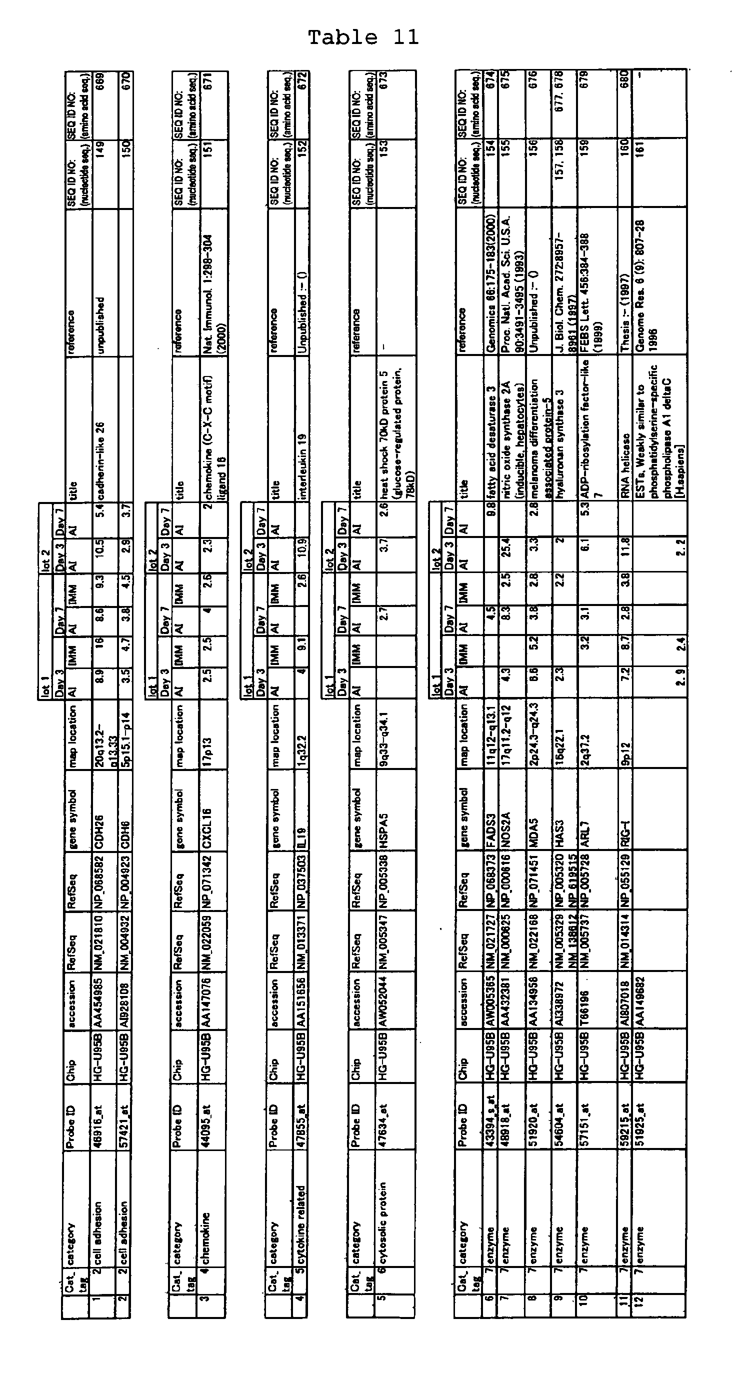

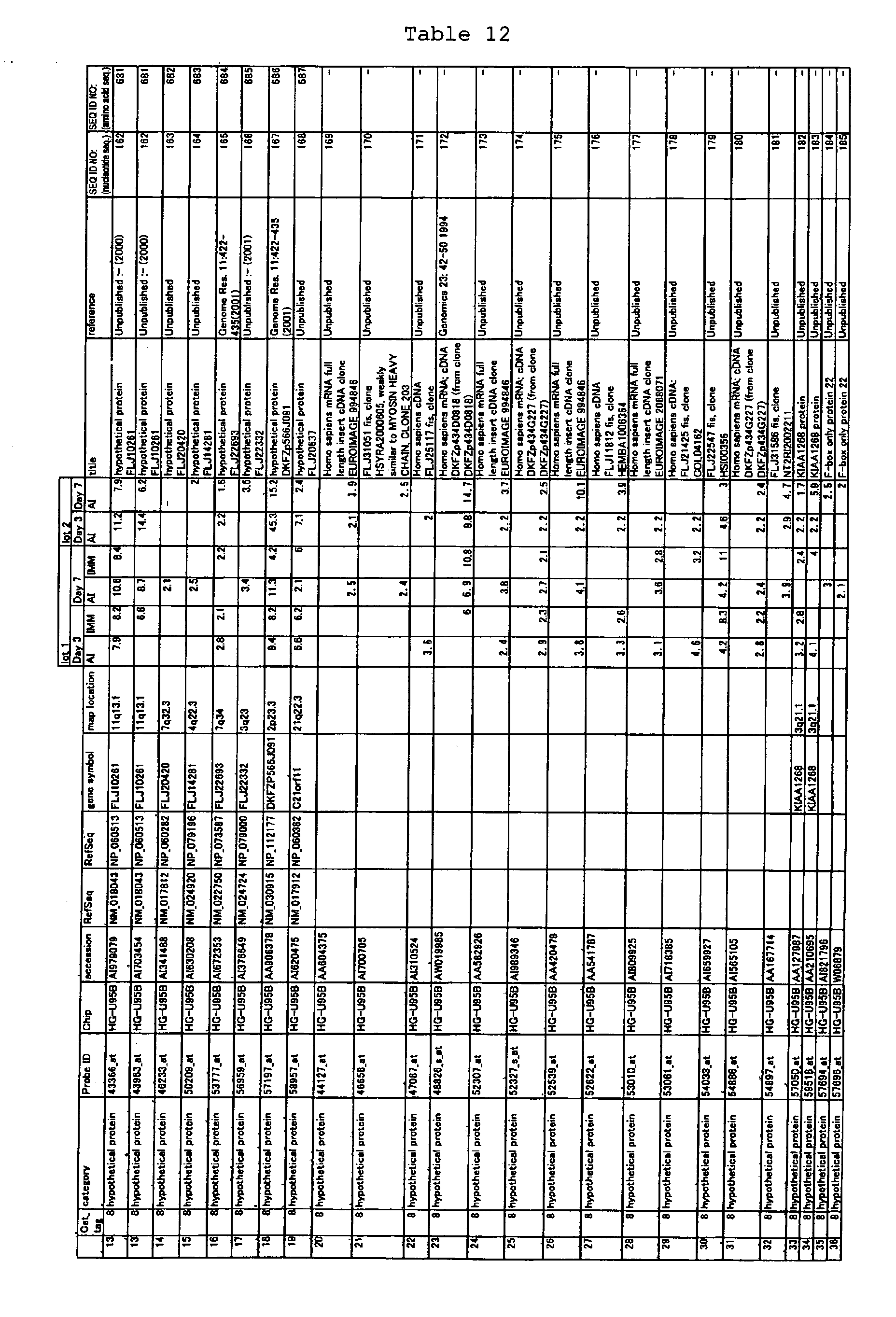

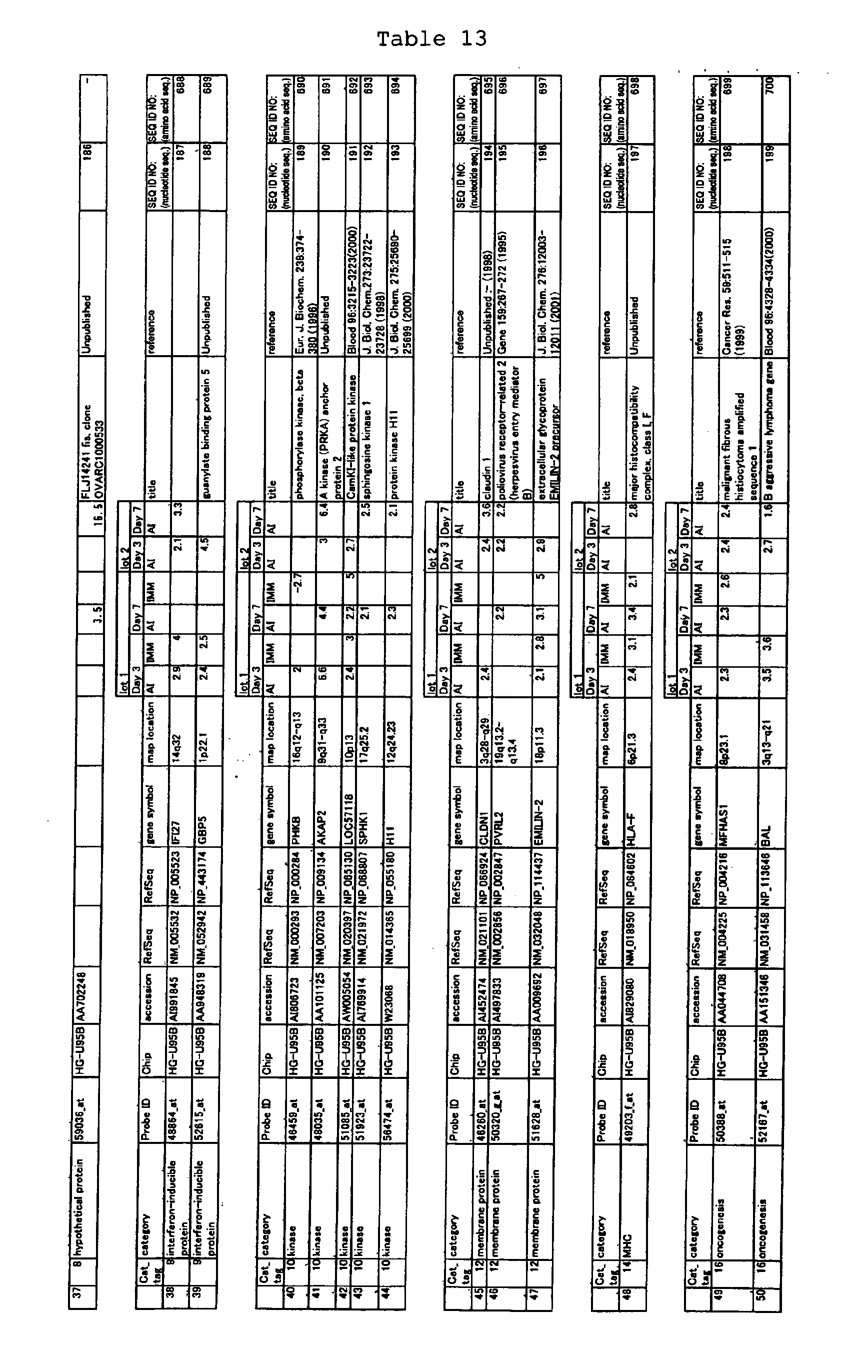

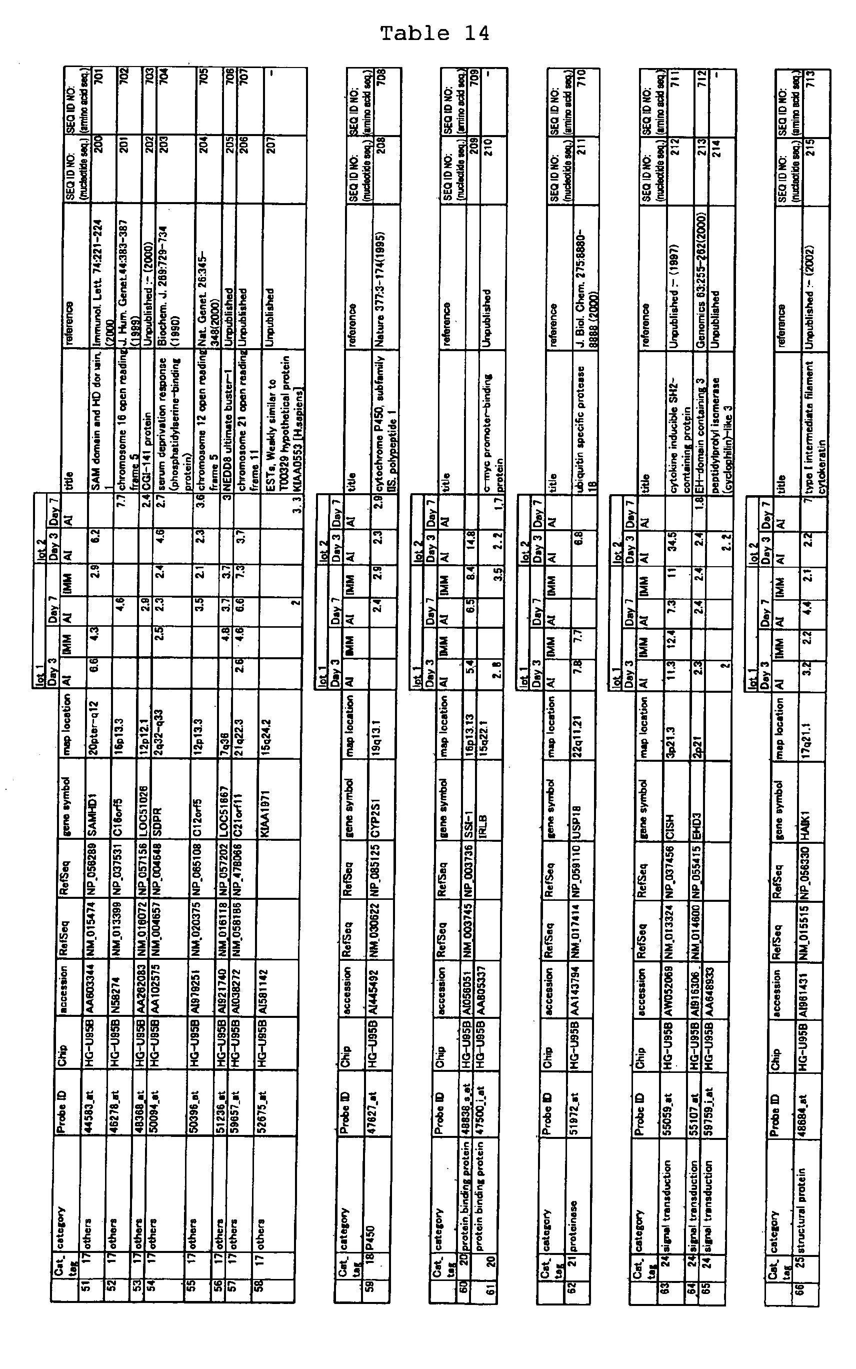

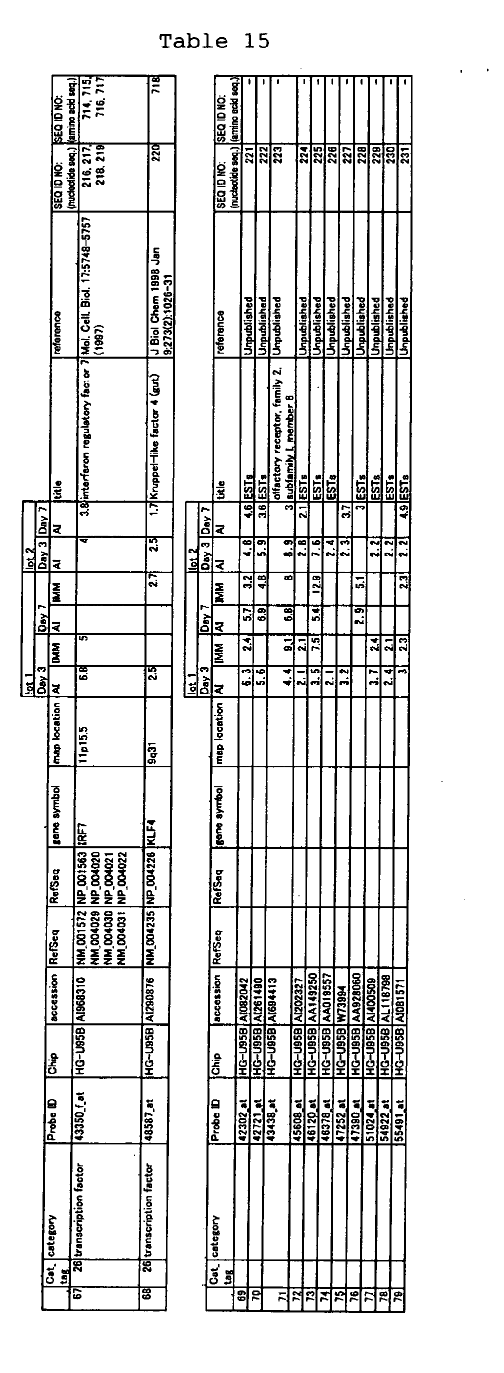

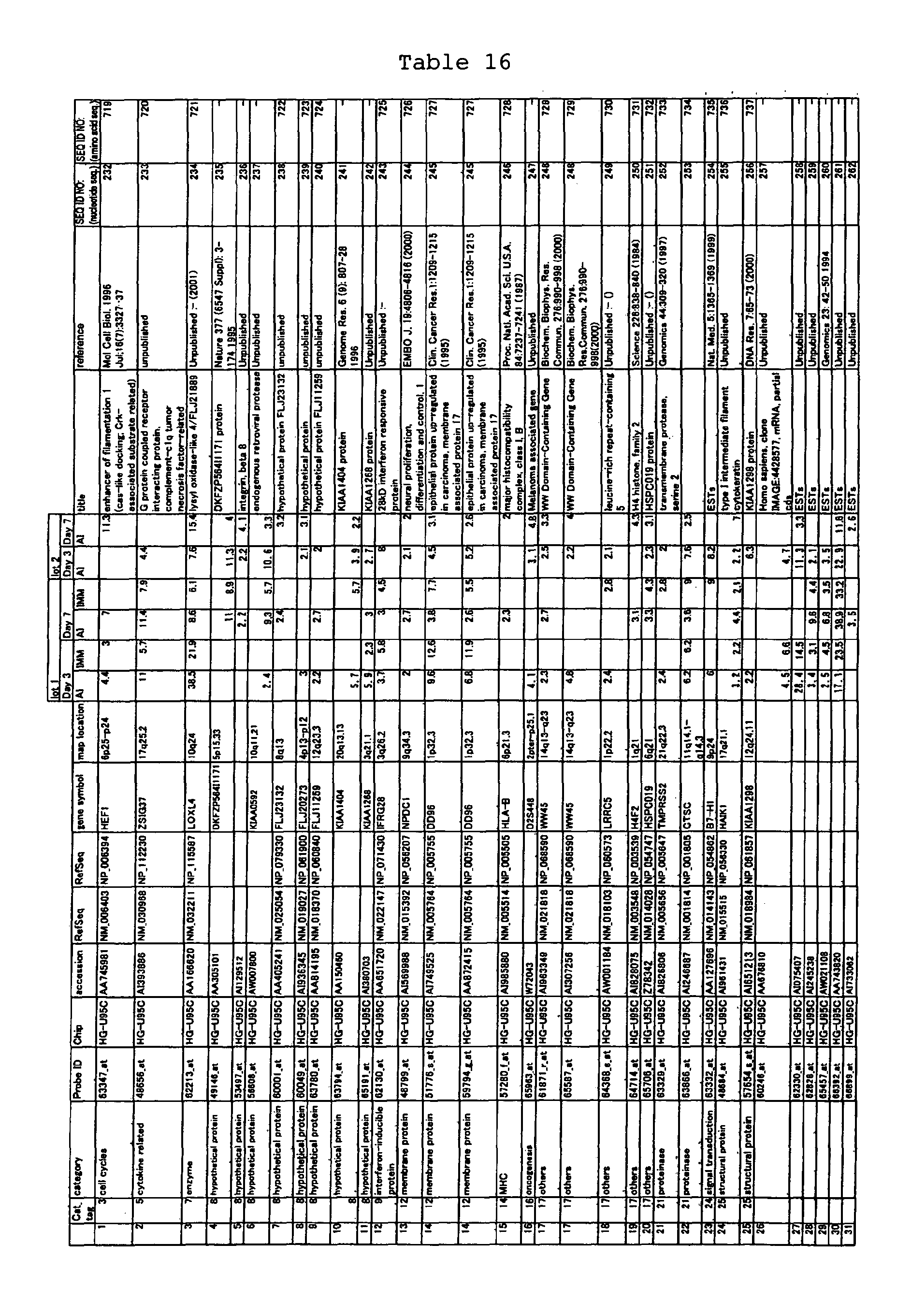

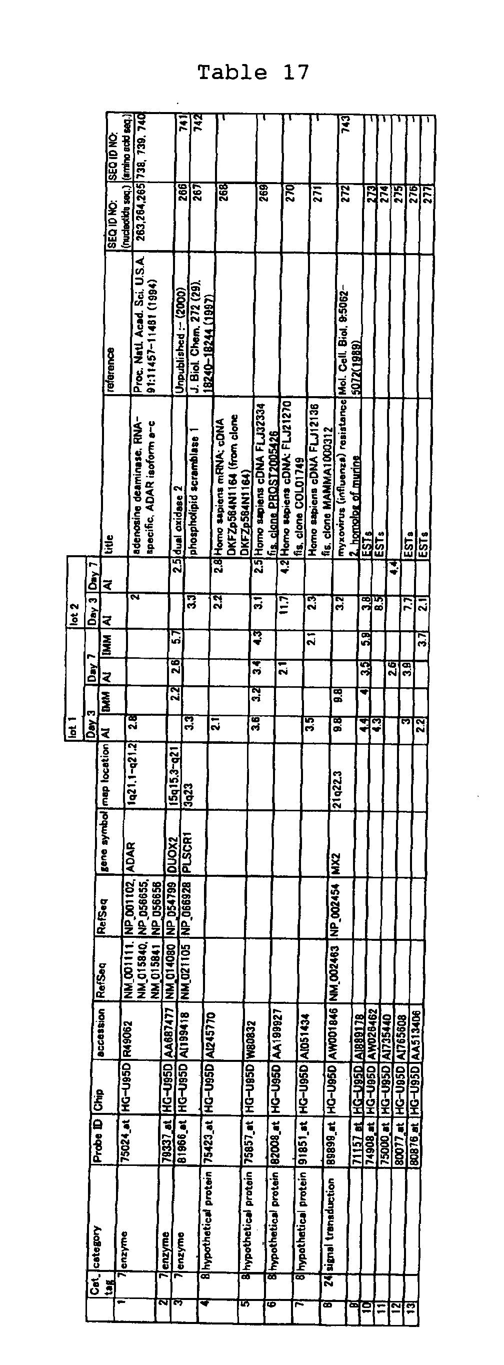

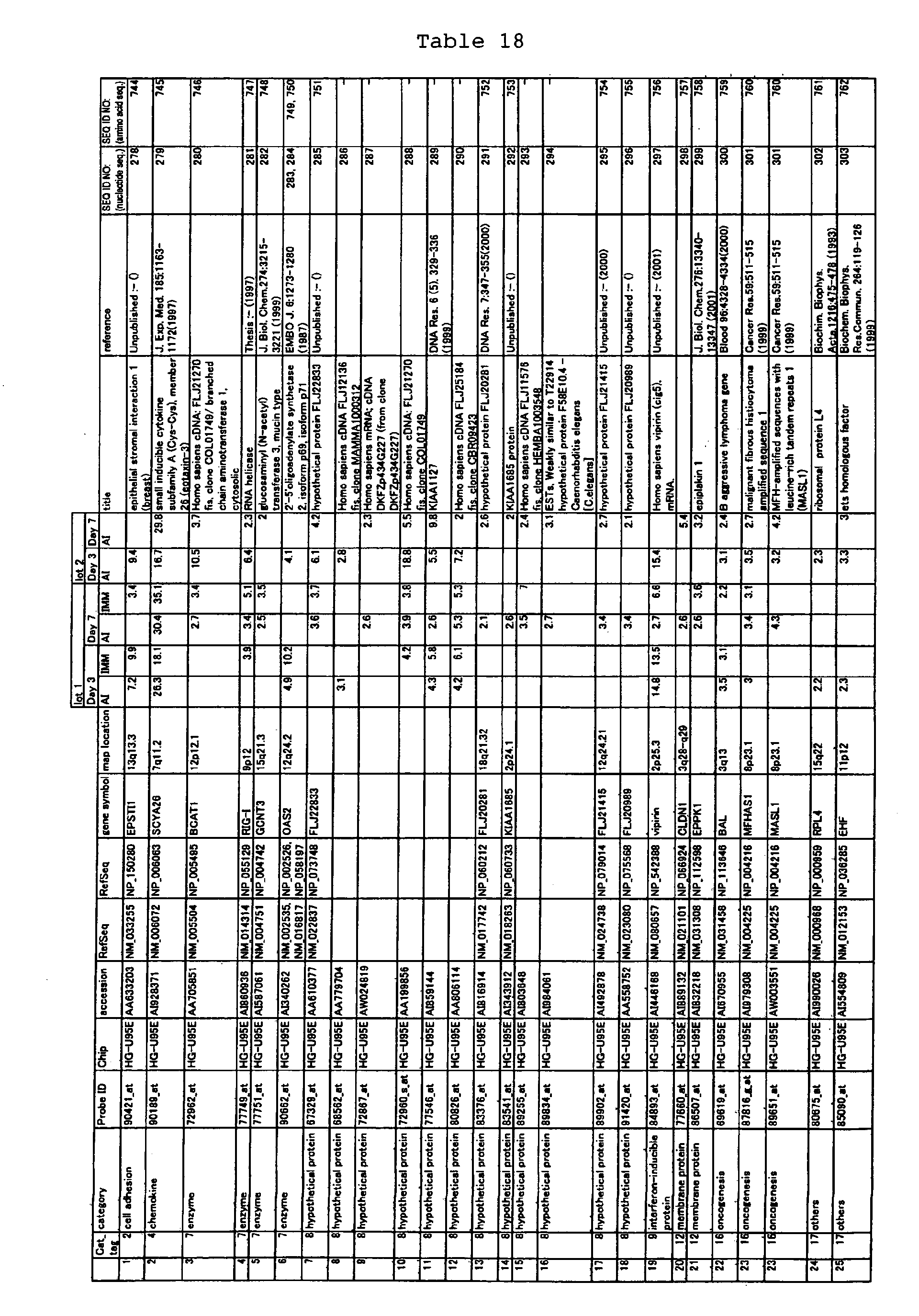

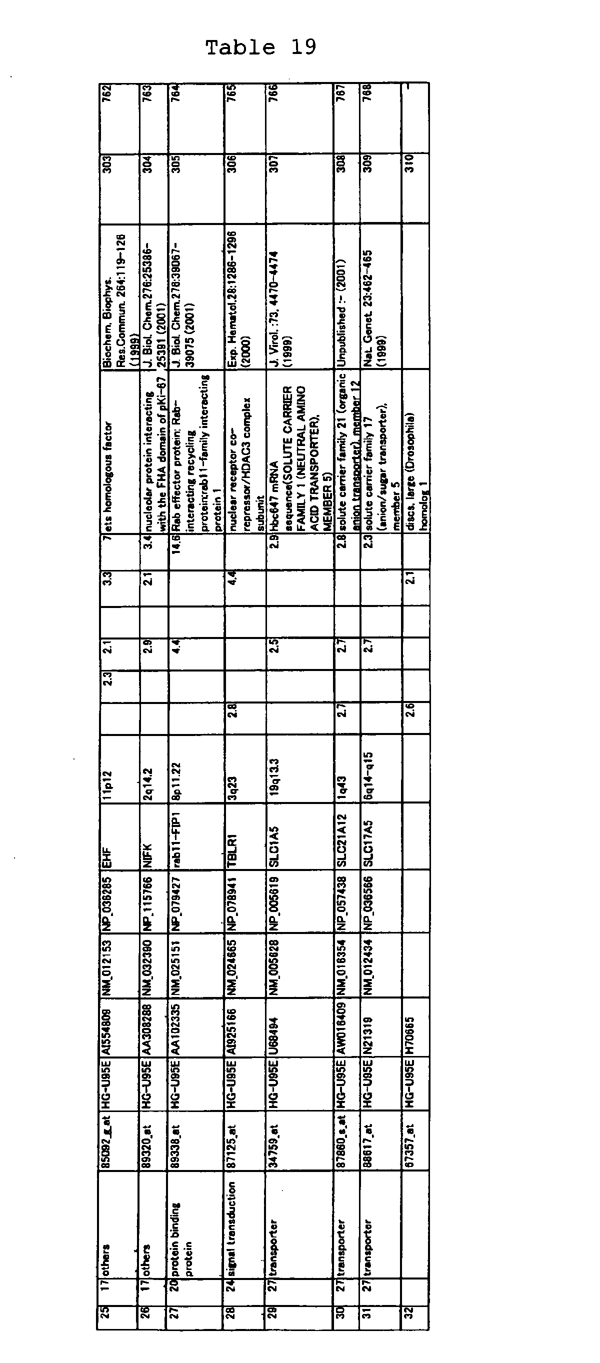

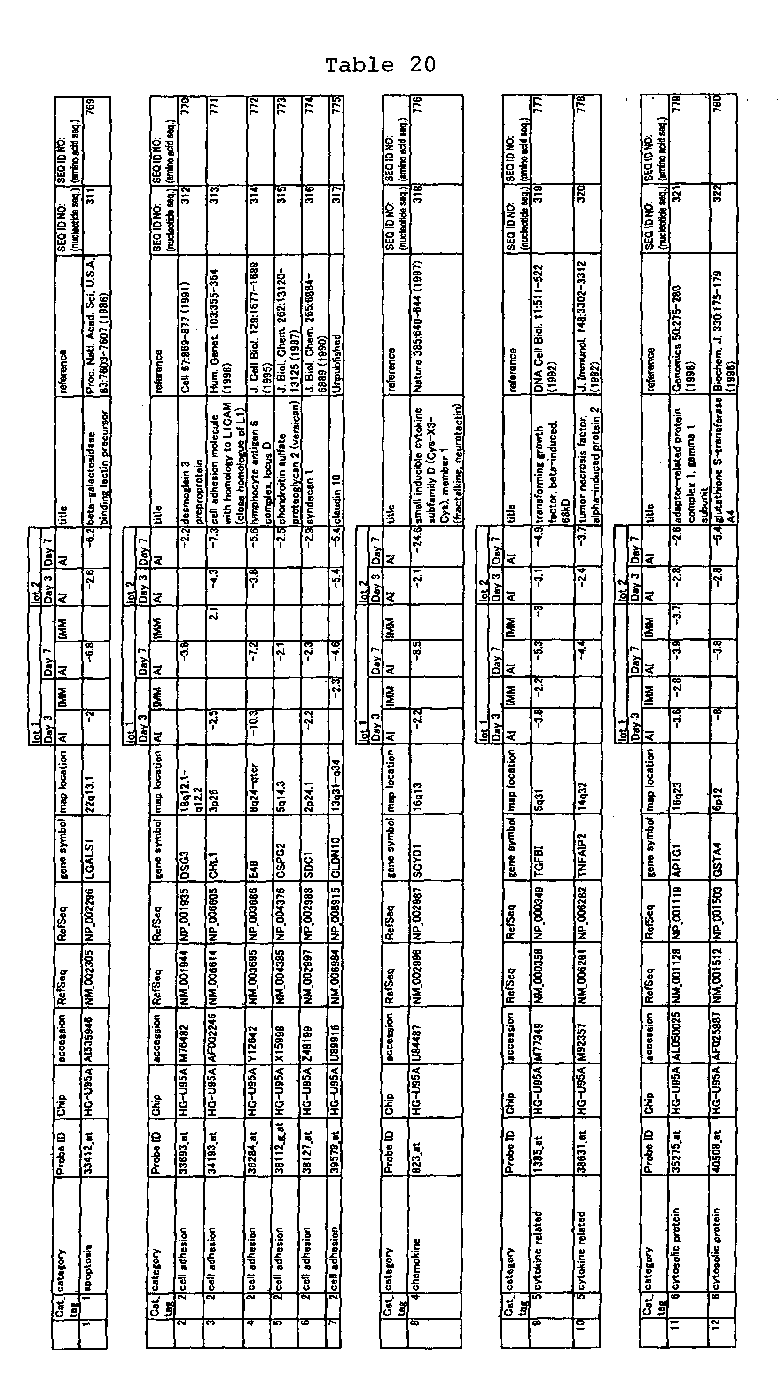

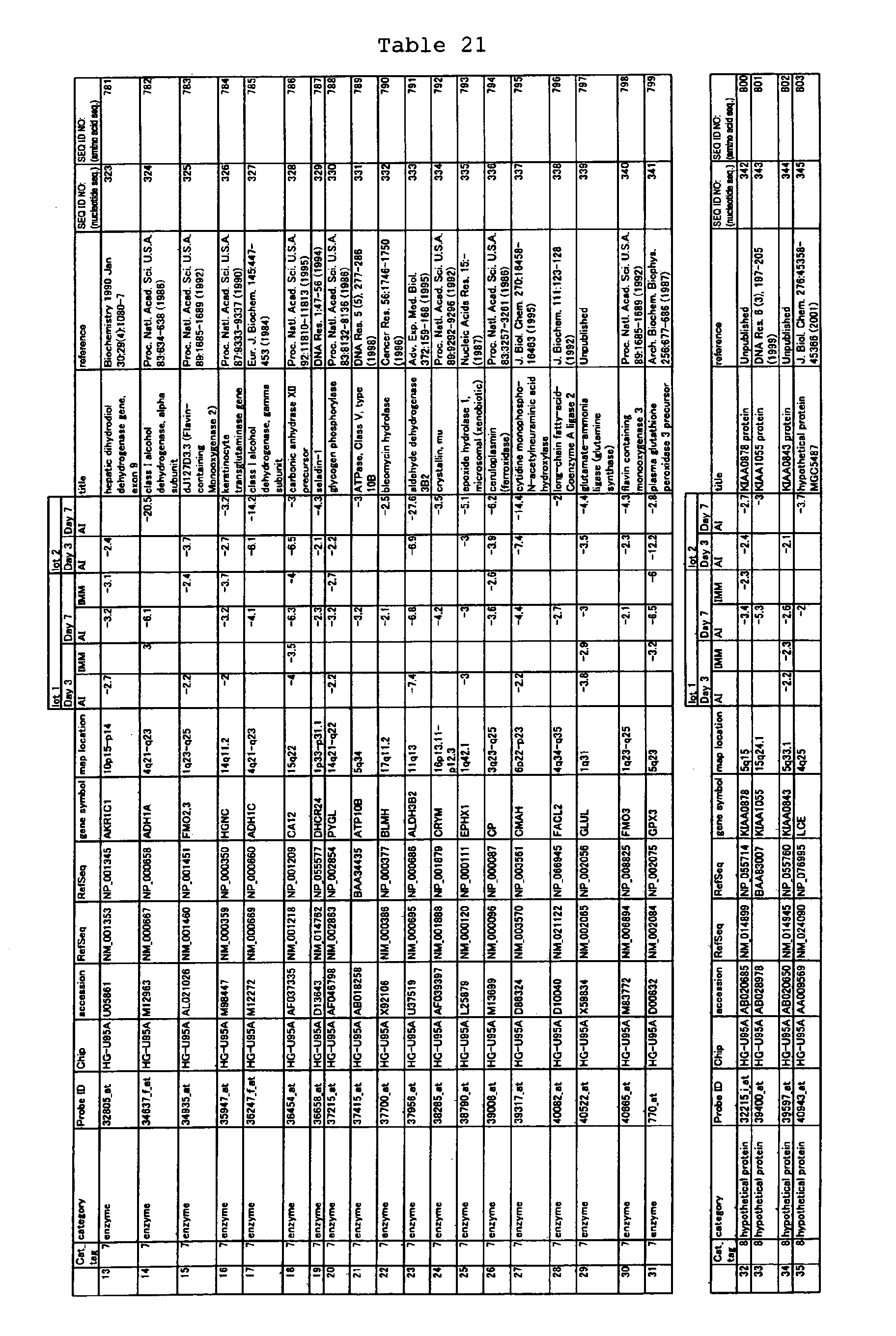

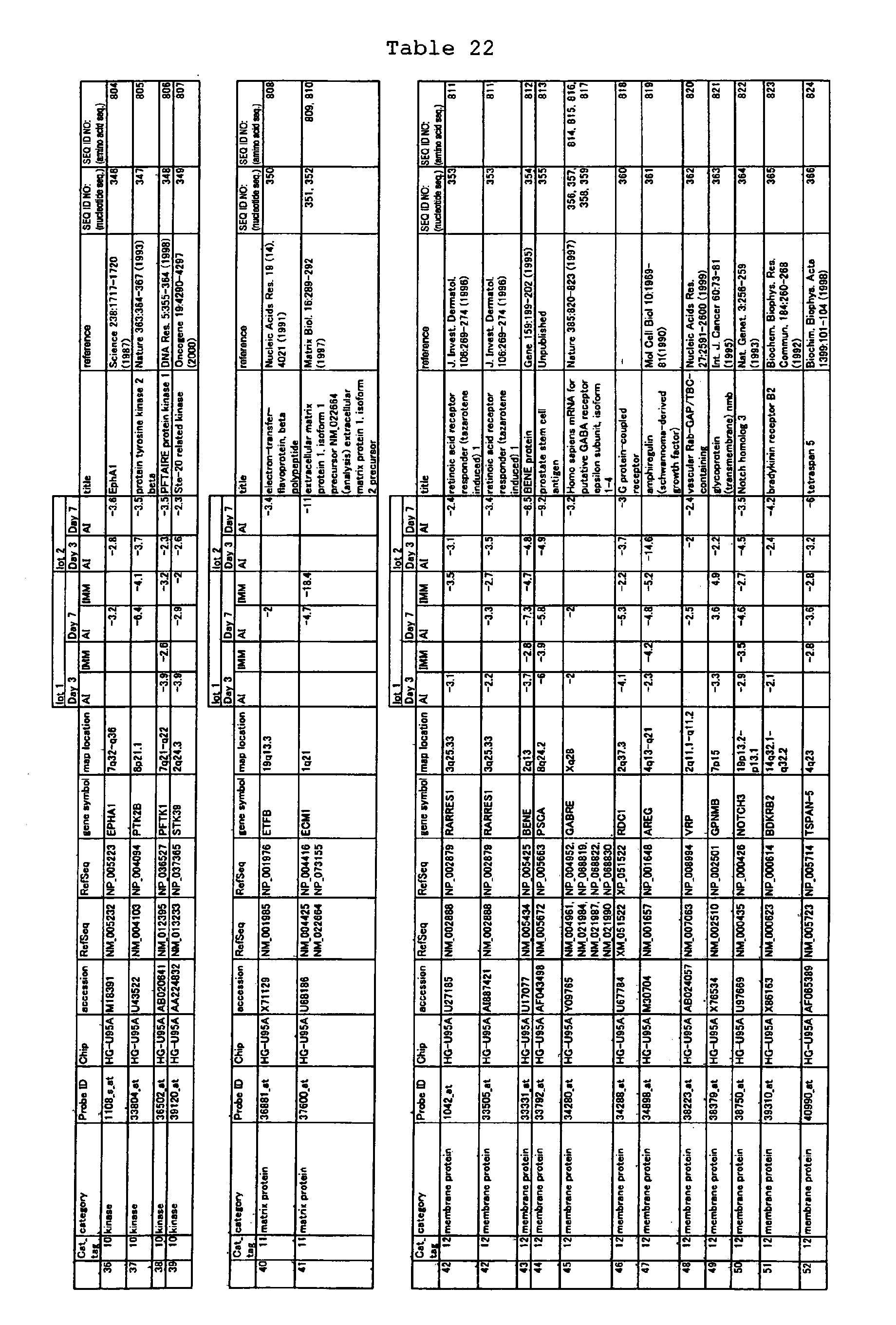

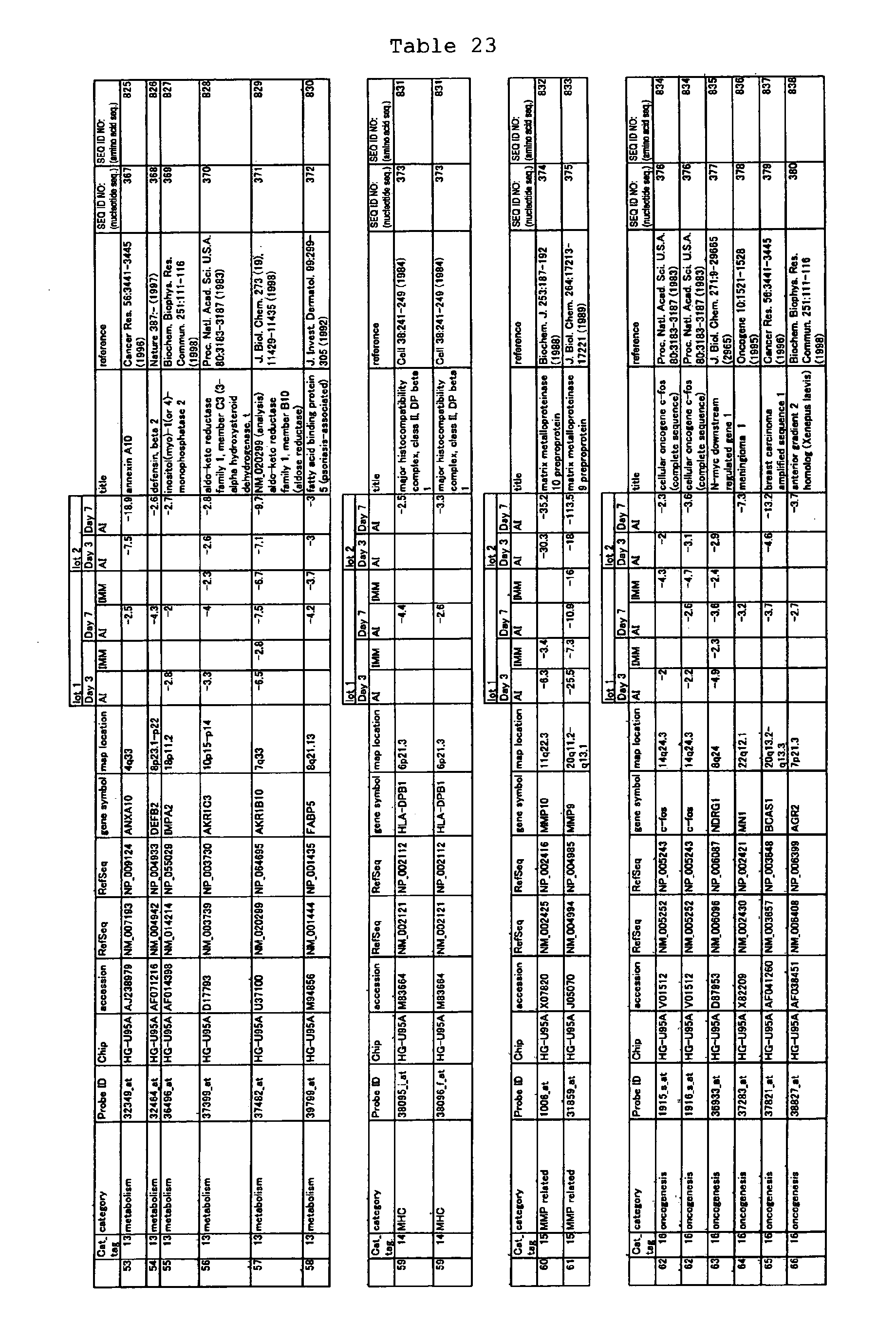

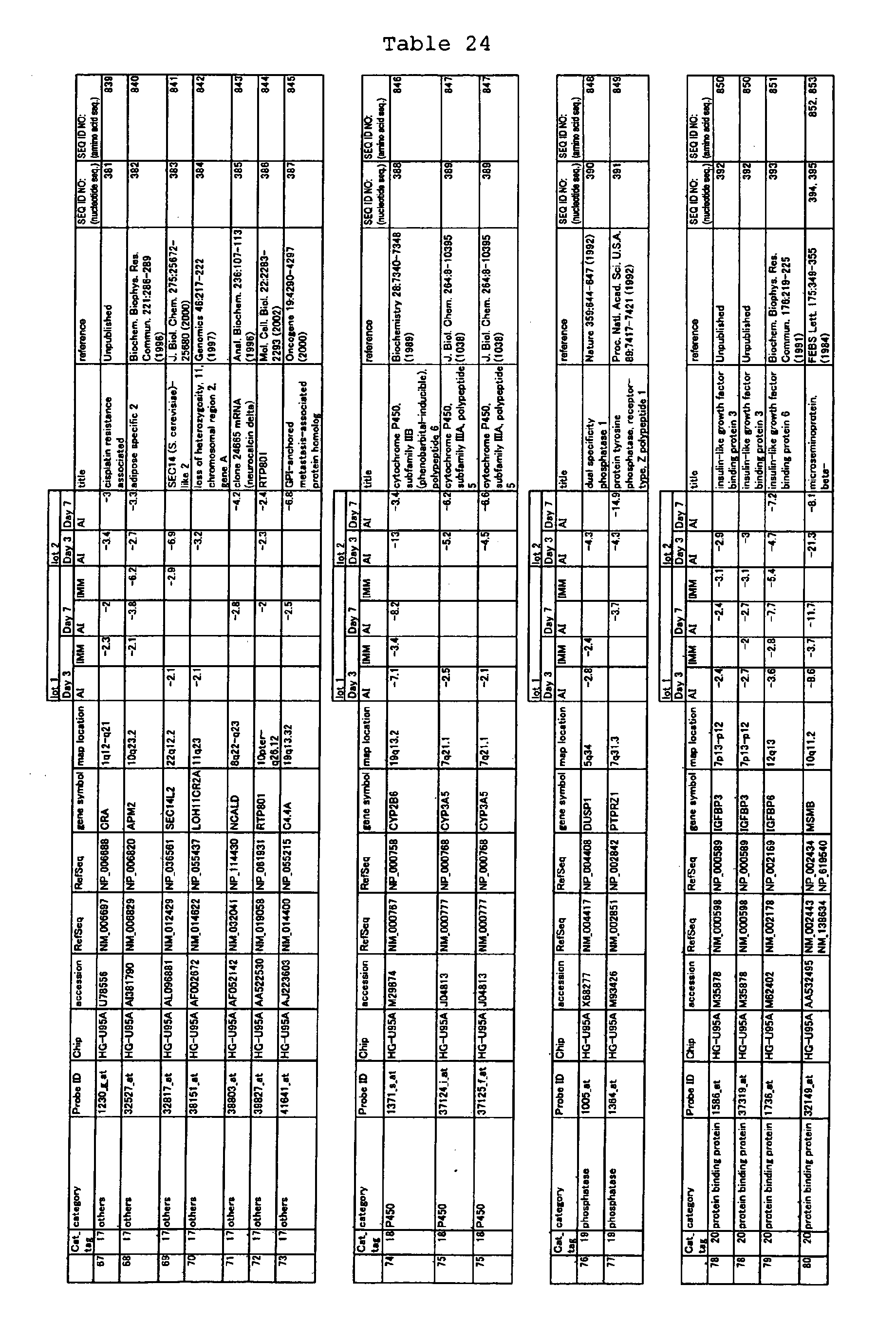

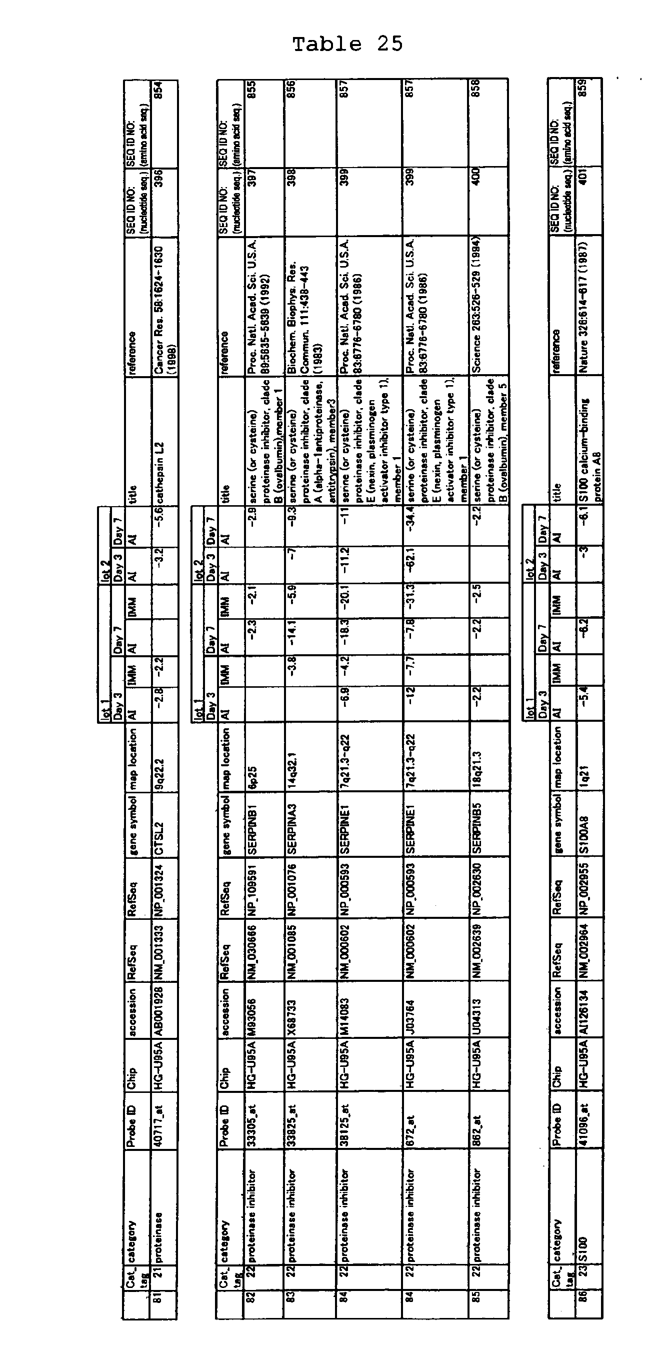

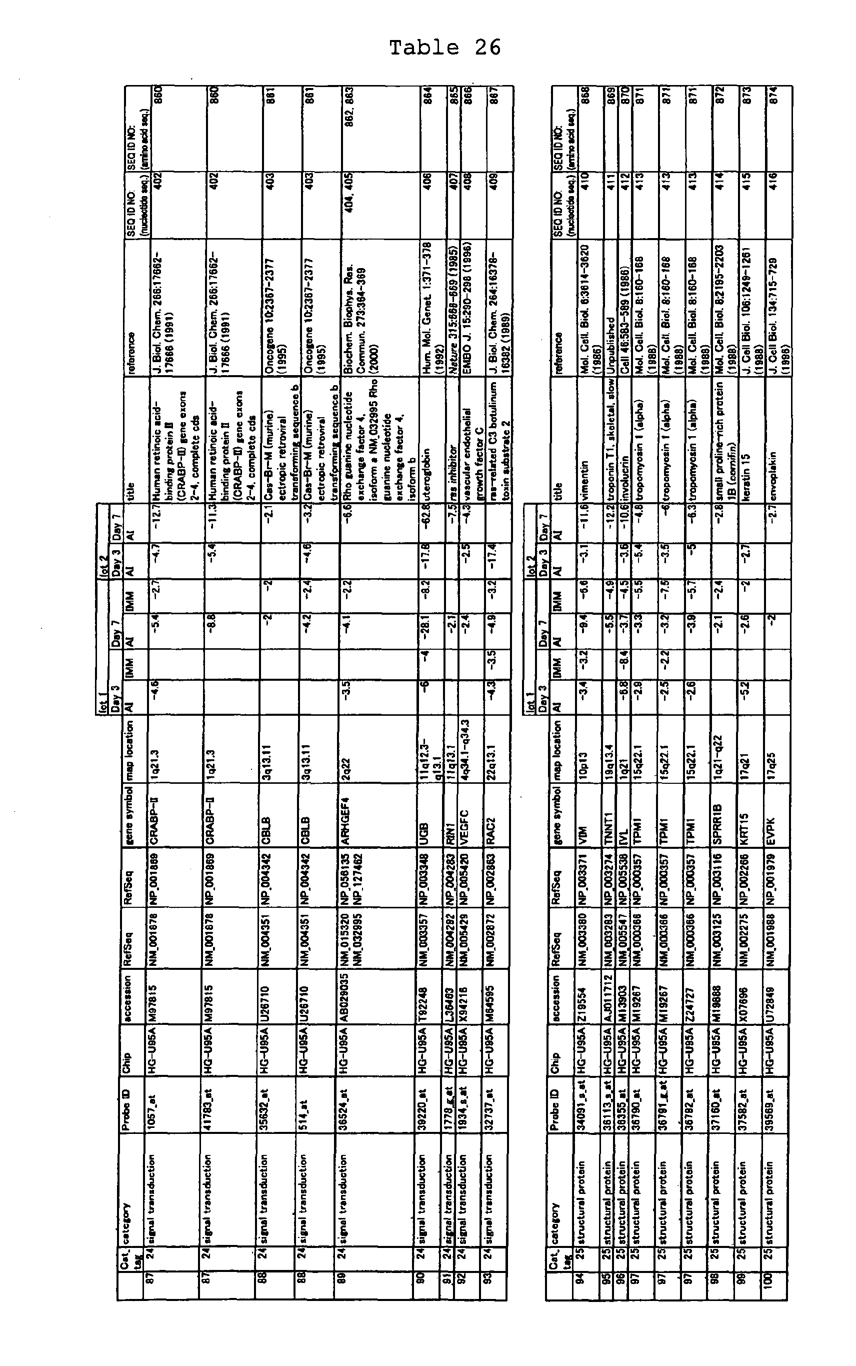

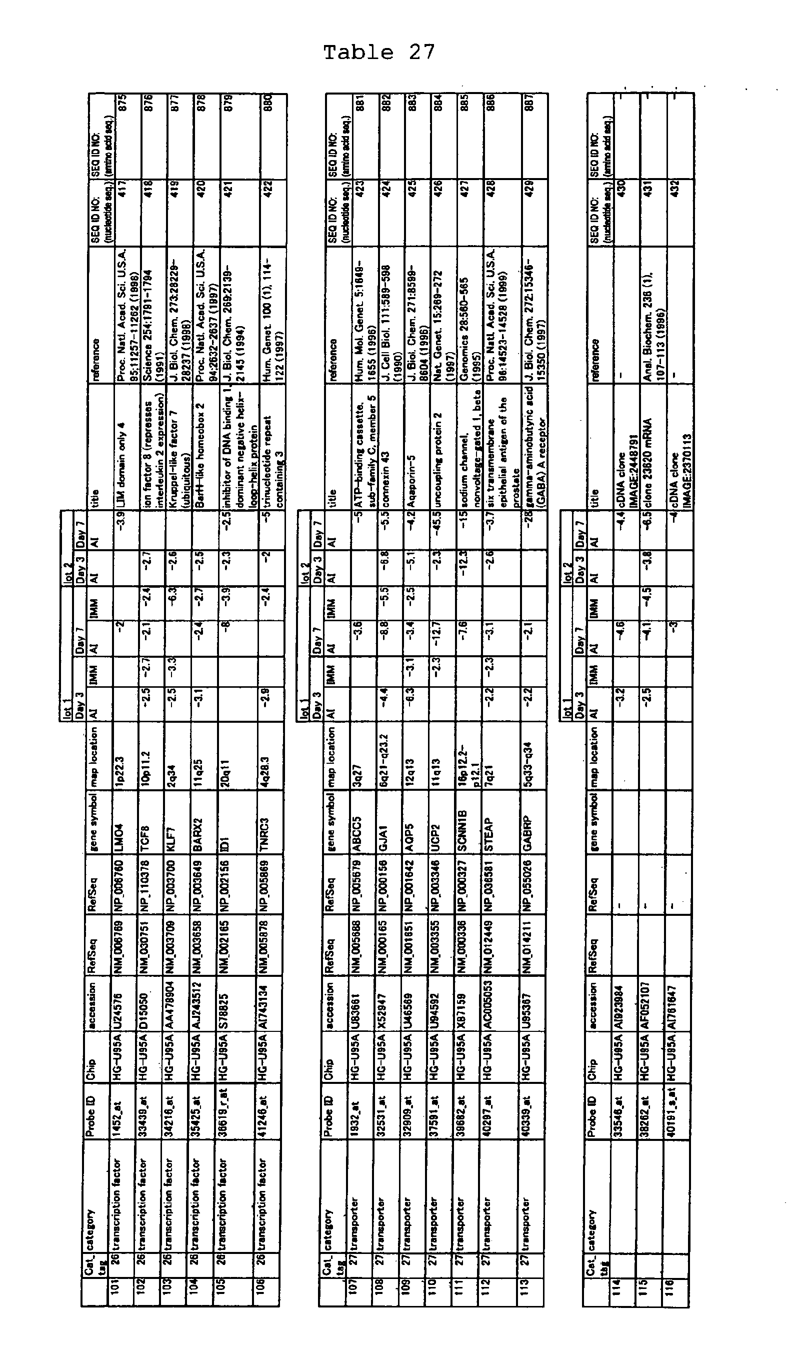

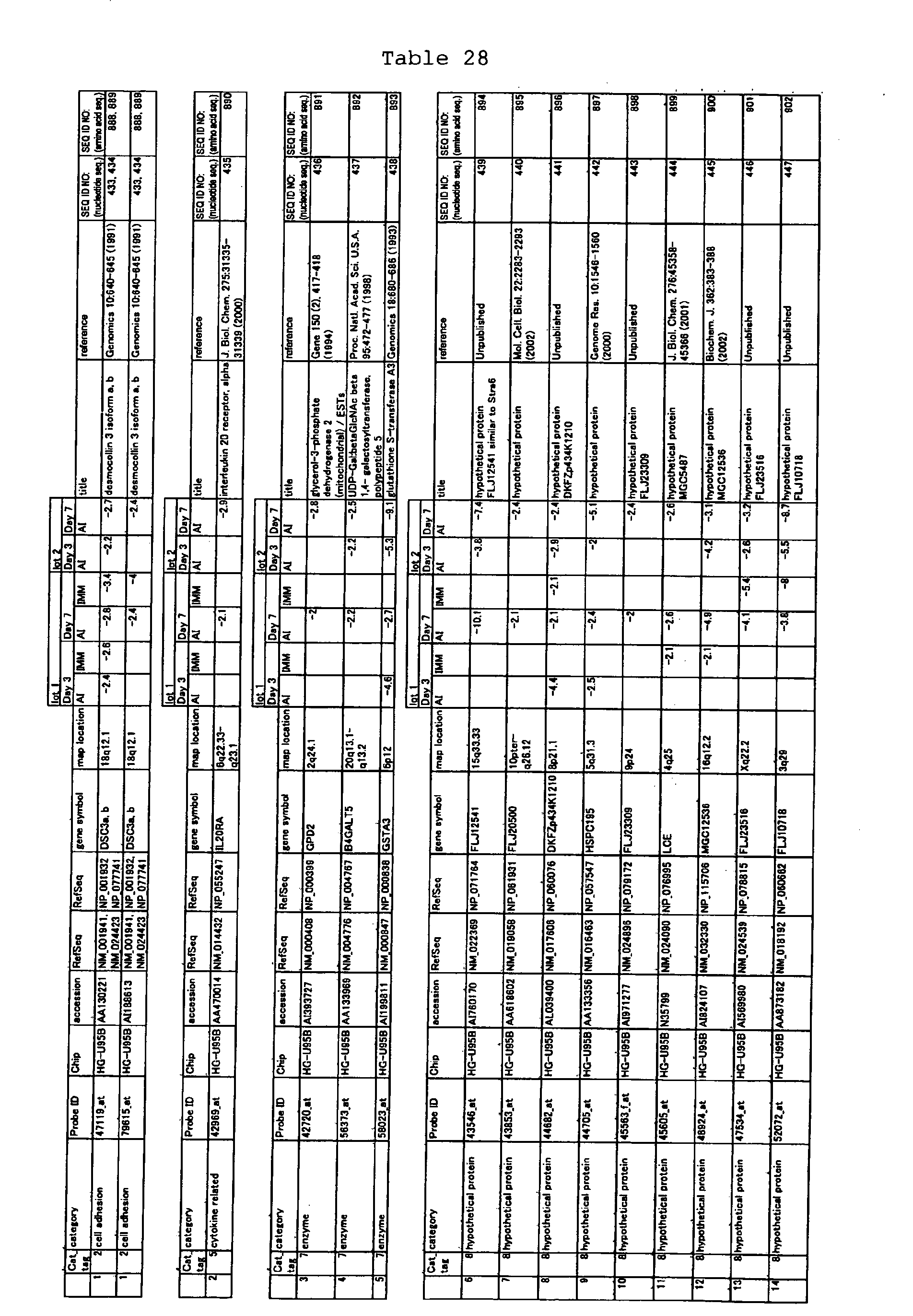

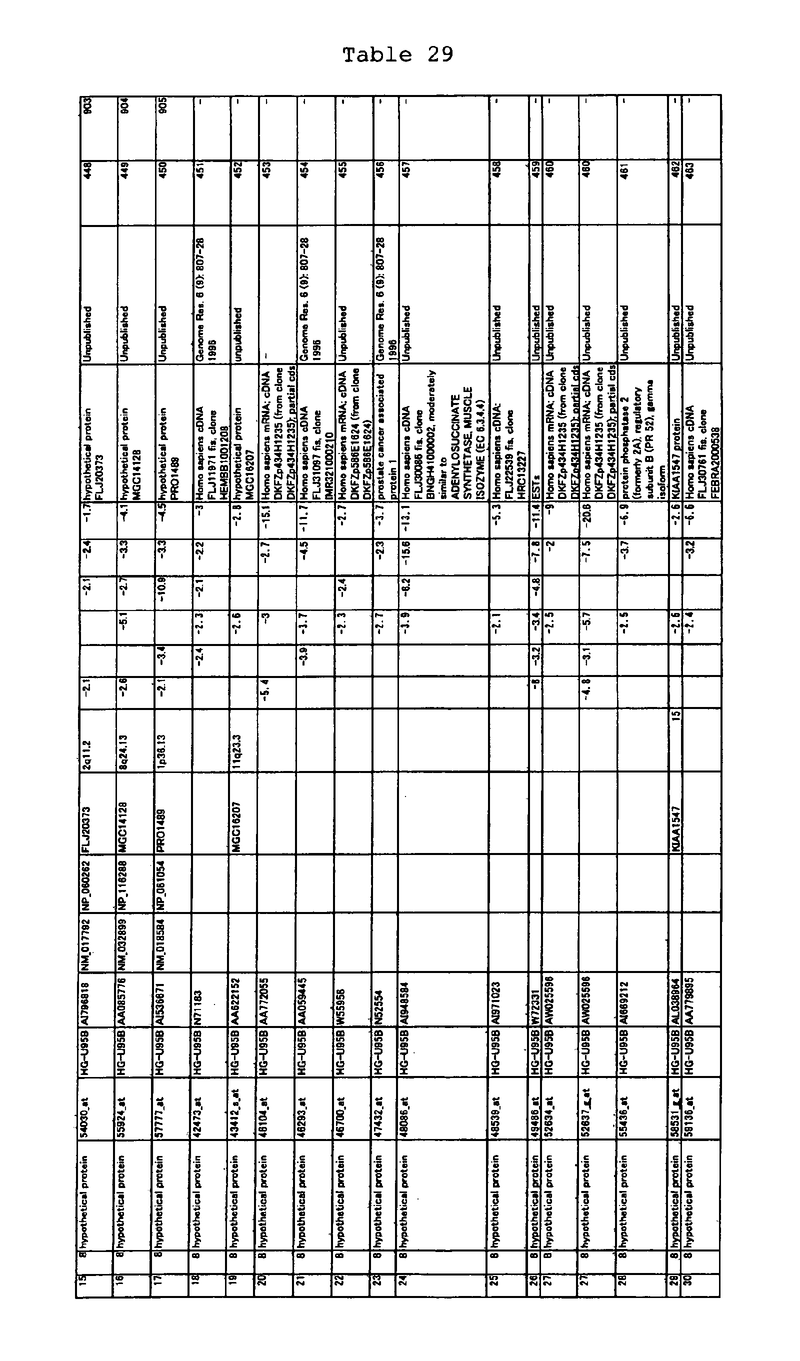

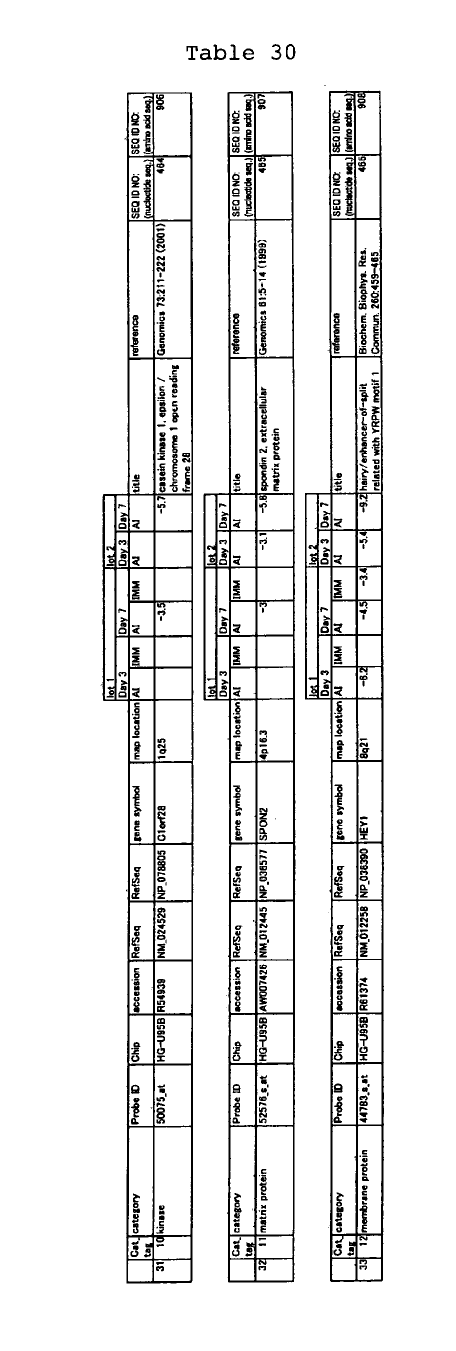

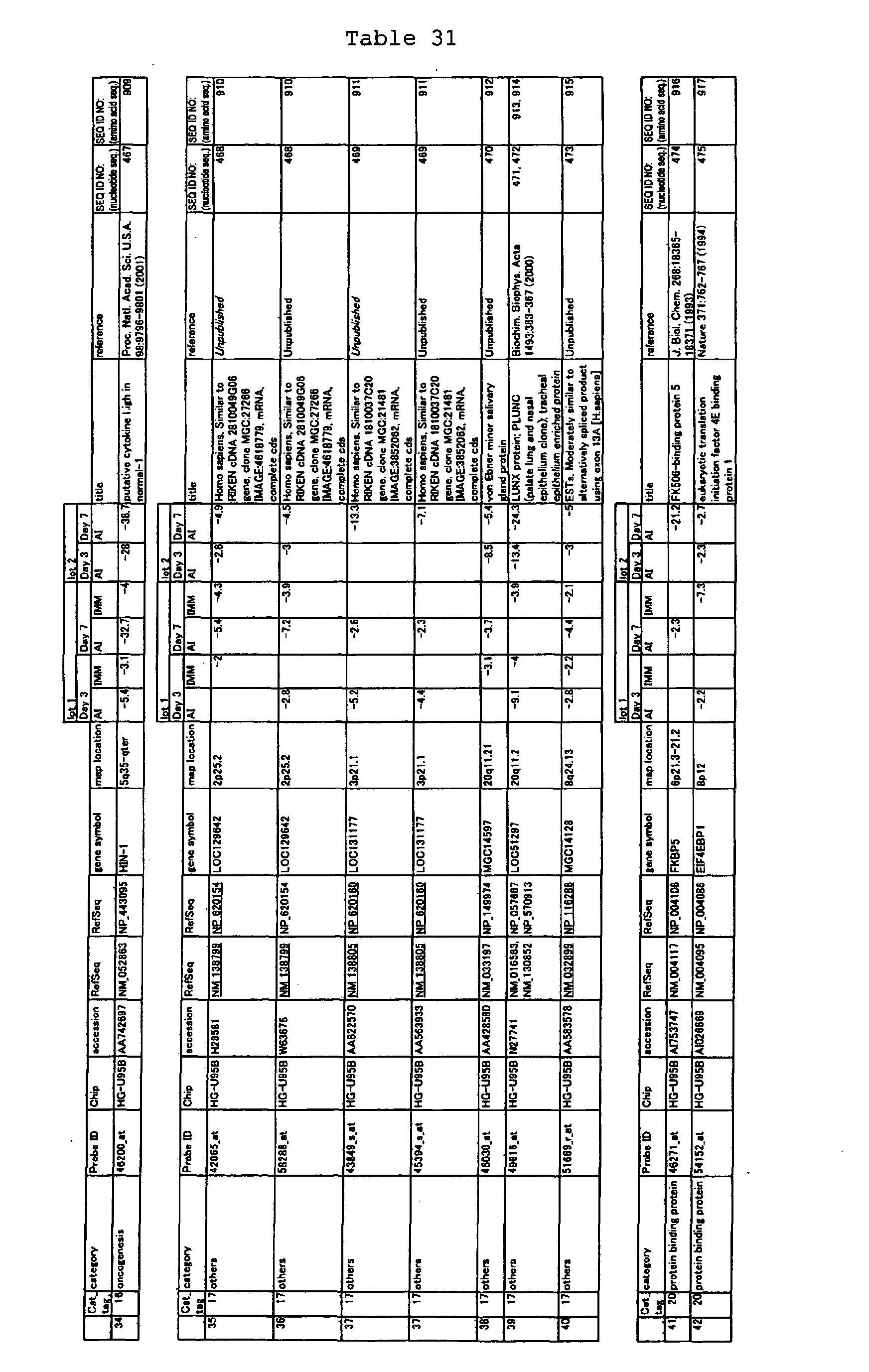

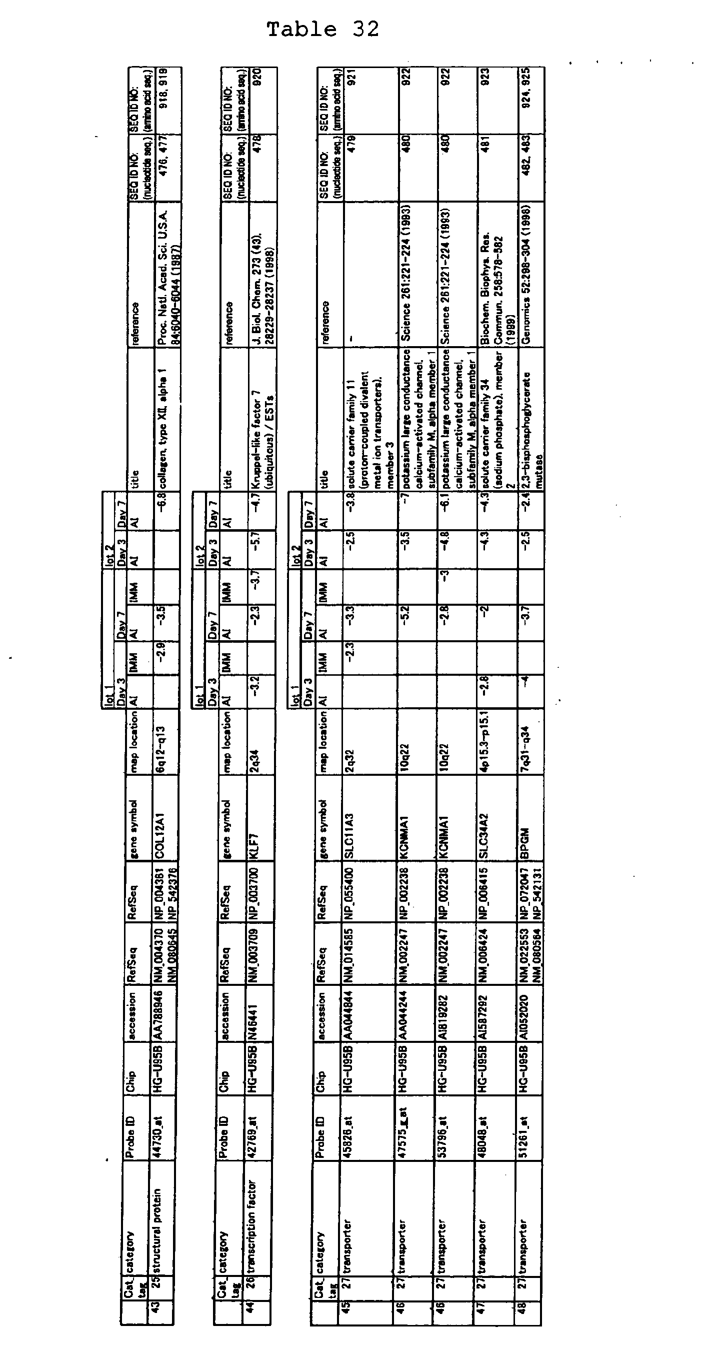

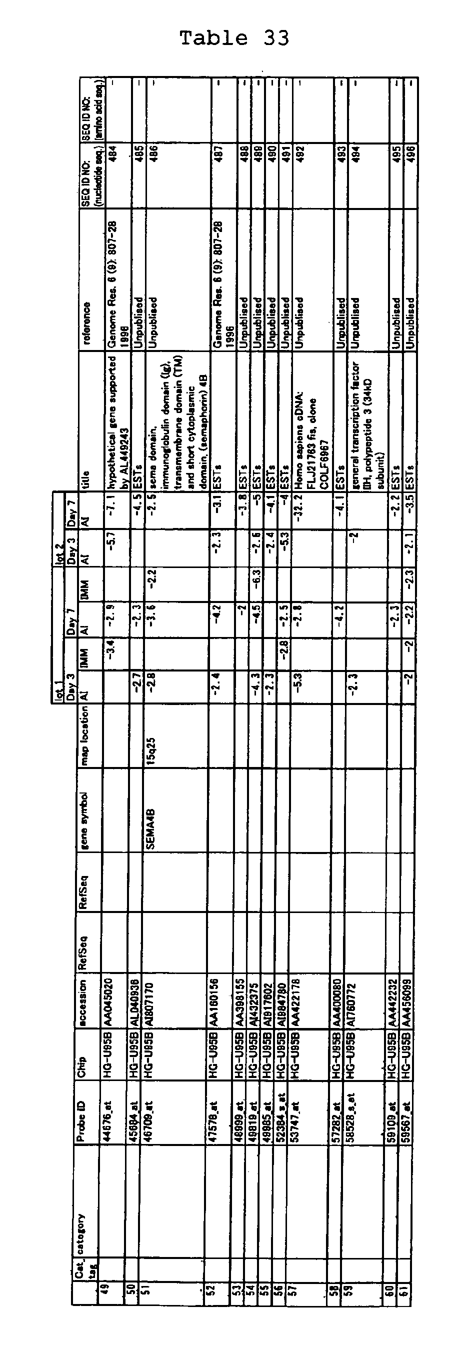

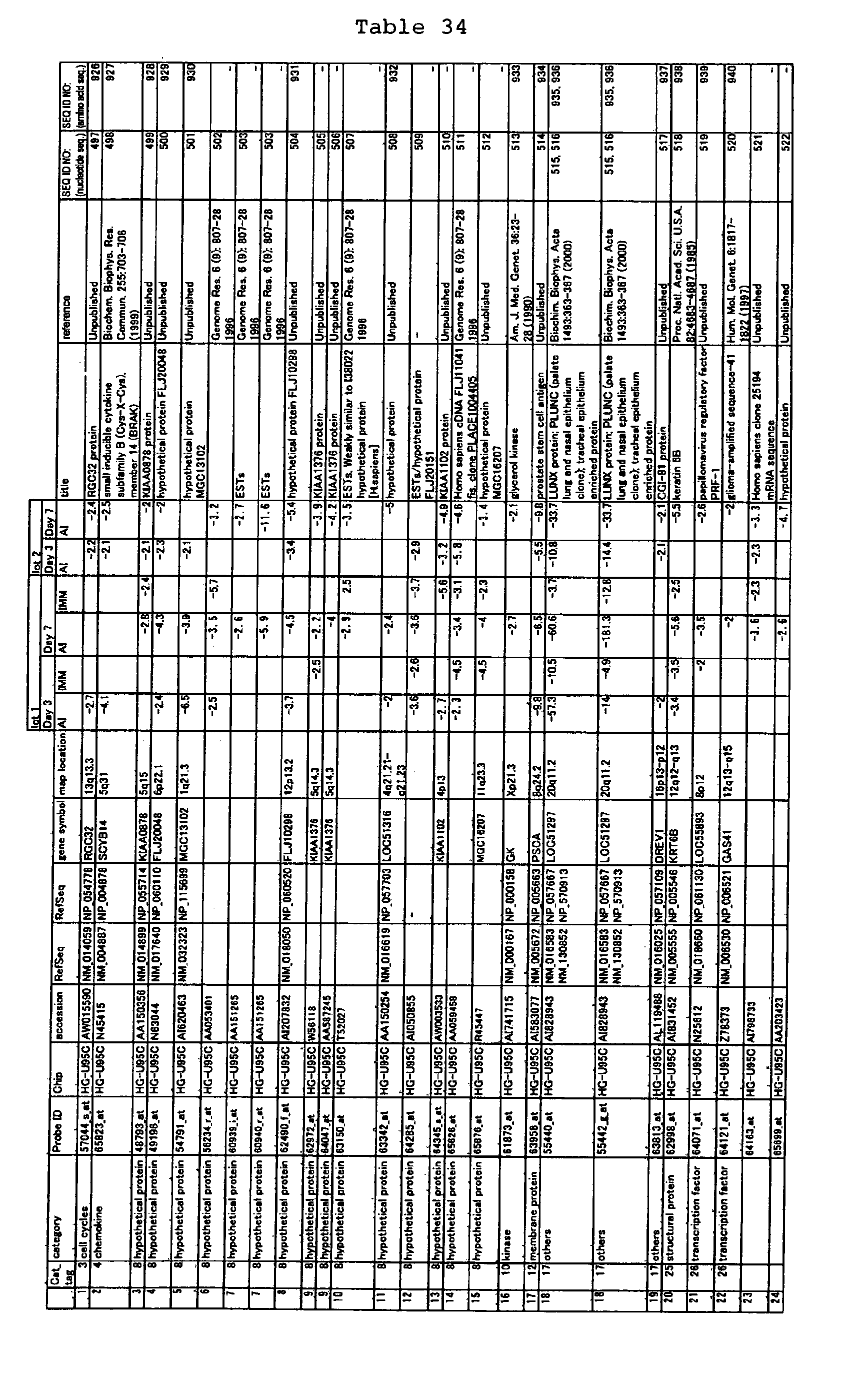

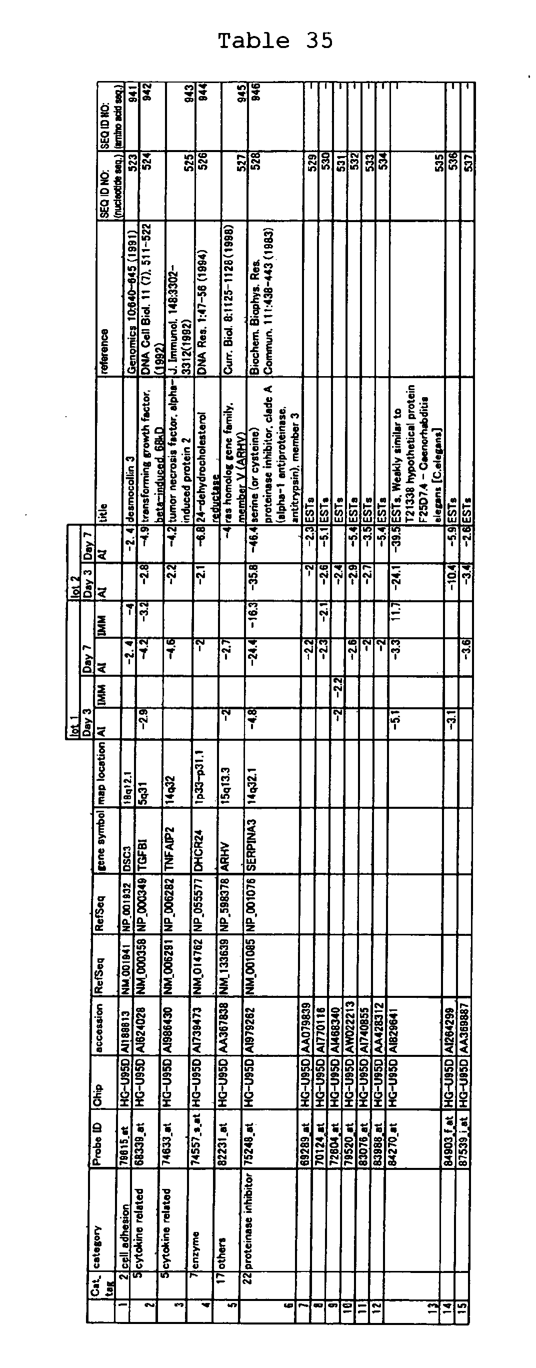

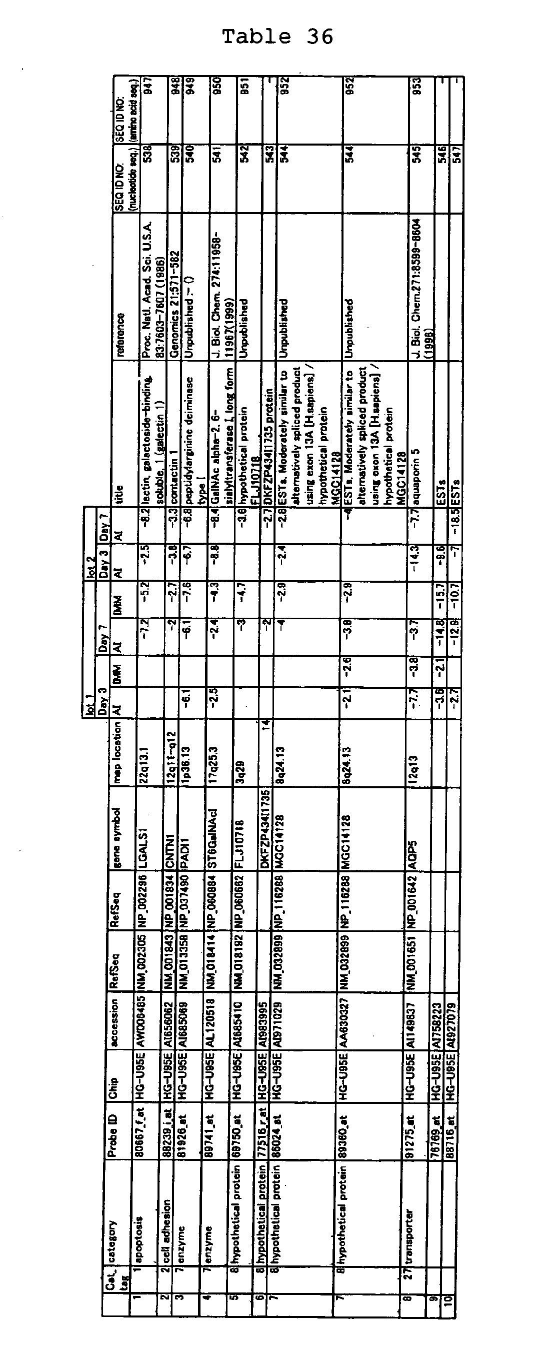

RefSeq gene sequences on the chips of HG-U95A to HG-U95E and the amino acid sequences thereof, and, if RefSeq genes are unavailable, EST sequences, are shown in the Sequence Listing.

### 2. Pendrin gene

Among the sequences whose expression levels change in response to IL-13 stimulation in both Lots 1 and 2 in the respiratory epithelial cells cultured by the AI method, the pendrin gene (RefSeq: NM_000441 and NM_000432; SEQ ID NOs: 2 and 3) was selected by the analysis described above, as a gene whose expression level was increased on day 3 and day 7 by a factor of ten or more. The Pendrin gene belongs to the category of transporters. In respiratory epithelial cells cultured with the IMM method, the expression level of the pendrin gene was also found to be increased by a factor of 20 or more in response to IL-13 stimulation on day 3 and day 7 in both Lots 1 and 2.

This gene is closely associated with allergies induced by IL-13 stimulation. The analysis result for the pendrin gene obtained using HG-U95A chip is shown in Table 37.

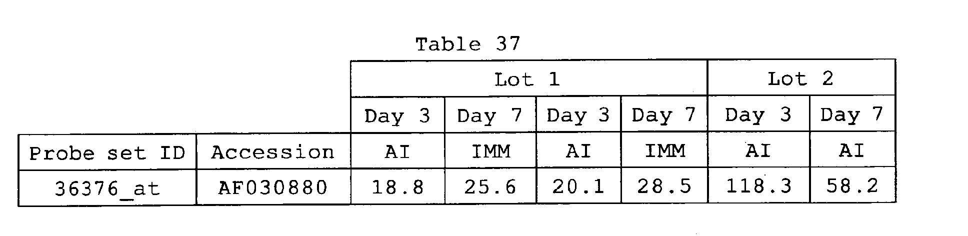

The PDS gene is a causative gene of the hereditary disease Pendred's syndrome, which is characterized by congenital deafness and goiters (Everett L. A. et al., Nat. Genet. 17: 411-22 (1997)). The gene was reported as a sulfuric acid transporter, because of the presence of a sulfuric acid transporter domain. However, after the report, the protein has been studied as a protein that transports other anions such as Cl⁻ and I⁻ (Scott D. A. et al . , Nat. Genet. 21(4): 440-3 (1999); Scott D.A. and Karniski L. P., Am. J. Physiol. 278: C207-11 (2000)). Pendrin is an 86-kDa transmembrane protein that consists of 780 amino acid residues and has a 12 transmembrane domain. In humans, the gene has been found to be expressed in the inner ear and thyroid gland at high levels, and in the kidney, endometrium, and placenta at lower levels (Rayaux I.E. et al., Endocrinology 141: 839-45 (2000) ; Bidart J. M. et al. , J. Clin. Endocrinol. Metab. 85: 2028-33 (2000)). On the other hand, in mice and rats, the gene is expressed in the kidney at a high level, and the expression is also detectable in the endometrium and placenta. The PDS gene encoding pendrin has been mapped on chromosome 7q31 , the location of the DFNB4 locus. The causative gene of congenital colon disorder, DRA (SLC26A3; down-regulated in colonic adenoma), has been mapped immediately downstream of the PDS gene in an inverse configuration.

The DRA gene encodes a sulfur transporter that is expressed at high levels in the colon and mucous membranes, and the transporter is structurally very similar to pendrin. Another gene exhibiting a high similarity to the PDS gene is DTDST (SLC26A2; diastrophic dysplasia) that is a causative gene of diastrophic dysplasia, which has been mapped on chromosome 5q32-q33.1. DTDST is also known to encode a protein functioning as a sulfur transporter. PDS gene knockout mice are deaf and are affected with vestibular function disorders. The inner ears are normal in 15-day olds or younger fetuses, but enlargement, sensory cell deformities , and otocranial deformities are developed after that (Everett L. A. et al., Hum. Mol. Genet. 10(2): 153-61 (2001) ) .

### EXAMPLE 6

### Determination of the expression levels of candidate genes in bronchial epithelial cells cultured by the AI method or the IMM method

Quantitative PCR assays were further performed with ABI 7700 using two batches of epithelial cells cultured respectively by the AI method and the IMM method described in Example 1 to quantitatively determine the expression level of the pendrin gene selected in Example 5. The primers and TaqMan probe used in the assays with ABI 7700 were designed based on the information on the sequence of the pendrin gene utilizing Primer Express (PE Biosystems). The 5' and 3' ends of the TaqMan probe were labeled with FAM (6-carboxy-fluorescein) and TAMRA (6-carboxy-N,N,N',N'-tetramethylrhodamine), respectively. The sequences of oligonucleotides of the forward primer (F), reverse primer (R) , and TaqMan probe (TP) for the pendrin gene are shown below. The GenBank accession number corresponding to the nucleotide sequence of each marker gene is shown in parenthesis after the name. Pendrin (AF030880)

F: TTTGCCTCCTGAACTTCCACC (SEQ ID NO: 4)

R: CCTACTGACACTGCAATAGCATAAGC (SEQ ID NO: 5)

TP: cttgttctcggagatgctggctgcat (SEQ ID NO: 6)

Total RNA extracted by the aforementioned method was treated with DNase (Nippon Gene). Then, cDNA, which was reverse transcribed using random hexamer (GIBCO BRL) as primer, was used as a template. For a standard curve to calculate the number of copies, a plasmid clone containing a nucleotide sequence region that is amplified by both primers was prepared for each of the genes, and this was diluted stepwise to be used as template for carrying out the reaction. The composition of reaction solution for monitoring PCR amplification is shown in Table 38.

**Table 38**

| Composition of reaction in ABI-PRISM 7700 (Amount per well) | |
|---|---|
| Sterilized distilled water | 23.75 (µL) |
| 10x TaqMan buffer A | 5 |
| 25mM MgCl₂ | 7 |
| dATP(10 mM) | 1.0 |
| dCTP(10 mM) | 1.0 |
| dGTP(10 mM) | 1.0 |
| dUTP (20 mM) | 1.0 |
| Forward Primer (10 µM) | 1.0 |
| Reverse Primer (10 µM) | 1.0 |
| TaqMan probe (2.0 µM) | 2.5 |
| AmpliTaq Gold (5 U/µL) | 0.25 |
| AmpErase UNG (1 U/µL) | 0.5 |
| Template solution | 5 |
| Total | 50 |

Additionally, to correct the differences of cDNA concentration in the sample, a similar quantitative analysis was performed for β-actin gene and glyceraldehyde-3-phosphate dehydrogenase (GAPDH) gene as internal standards for correction. By correcting based on the number of copies of these genes, the number of copies of the genes of interest was calculated.

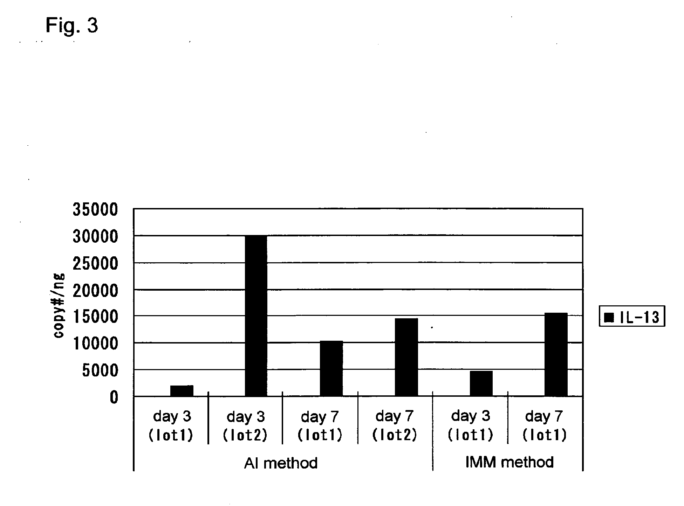

Primers and probes for measuring β-actin or GAPDH were designed from Primer Express (Applied Biosystems) based on the genetic information of each gene. The nucleotide sequences are as shown below. The β-actin-corrected expression levels (copy/5 ng RNA) for marker genes are shown in Figs. 3. 
β-actin forward primer (SEQ ID NO: 7)
TCA CCC ACA CTG TGC CCA TCT ACG A
 
β-actin reverse primer (SEQ ID NO: 8)
CAG CGG AAC CGC TCA TTG CCA ATG G
 
β-actin TaqMan probe (SEQ ID NO: 9)
(FAM)ATGCCCTCCCCCATGCCATCCTGCGT(TAMRA)-3'
 
GAPDH forward primer (SEQ ID NO: 10)
GAAGGTGAAGGTCGGAGT
 
GAPDH reverse primer (SEQ ID NO: 11)
GAAGATGGTGATGGGATTTC
 
GAPDH TaqMan probe (SEQ ID NO: 12)
(FAM)CAAGCTTCCCGTTCTCAGCC(TAMRA)-3'
 FAM: 6-carboxy-fluorescein
TAMRA: 6-carboxy-N,N,N',N'-tetramethylrhodamine

As a result of quantitative PCR, the expression level of the pendrin gene (selected in Example 5) in the respiratory tract epithelial cells was elevated by hundred folds or more as a result of IL-13 stimulation in respiratory tract epithelial cells when cultured according to the AI method or IMM method. Based on these results, it was presumed that the expression level of the marker gene was elevated in respiratory tract epithelial cells in response to IL-13.

The marker genes of this invention show common behavior among different lots of bronchial epithelial cells by IL-13 stimulation known to have a close relationship to allergic reactions. Therefore, the marker genes of this invention are thought to be important genes that regulate the progression of allergic reactions.

### EXAMPLE 7

### RNA recovery from the lung of OVA antigen-exposed bronchial hypersensitivity mouse model

The OVA antigen-exposed bronchial hypersensitivity model has been reported as a bronchial asthma model. 50 µg OVA and 1 mg aluminum hydroxide (an adjuvant) were injected into the peritoneal cavity of Balb/c mice (male, seven-week old), and after 10 days the mice was sensitized with OVA under the same conditions. Then, after 10 days, 1% OVA was given by inhalation using the Ultra-nebulizer model UN701 (Azwell(Co., Ltd.)) for 30 minutes every four days three times in total. Enhanced bronchial hypersensitivity was monitored by detecting the respiratory constriction caused by acetylcholine (6.25-2000 µg/kg) using an artificial respirator (model 131, New England Medical Instruments Inc.) 24 hours after the final antigen inhalation (Nagai H. et al, Int Arch Allergy Immunol; 108: 189-195, 1995) . Bronchial hypersensitivity can be induced by this treatment.

Variations in the expression level of the mouse pendrin gene were studied using RNA from the lungs of this model.

The test was conducted using the following four groups: OVA antigen-exposed bronchial hypersensitivity group (called the "S-OVA group"; N=7)); and three control groups: untreated group (called the "naive group";(N=6)); physiological saline-inhaled group to which the OVA antigen was given twice for immunization and physiological saline was given by inhalation (called the "S-Sal group"; (N=6)); and the Prednisolone-administered group, to which Prednisolone was given by inhalation 10 times in total from the day before antigen inhalation until the final antigen inhalation, and the development of bronchial hypersensitivity was suppressed by giving 5 mg/kg Prednisolone orally (called the "Pred-group"; (N=7)).

The left lungs were removed 24 hours after the antigen was inhaled three times, by which time, the symptoms of bronchial hypersensitivity can be seen. The lung tissues were dissolved in 2 ml of Isogen (Nippon Gene; Wako Pure Chemical Industries) and immediately crushed with the homogenizer DIAX100 (Heidolph). RNA was isolated from 1 ml of this solution according to the protocol attached to Isogen. Chloroform was added to the solution. After the mixture was stirred and centrifuged, the aqueous layer was recovered. Then, isopropanol was added. After the mixture was stirred and centrifuged, the precipitated total RNA was collected. Total RNAs (approximately 20-60 µg) were extracted from the samples of the four groups (N=26) described above.

### EXAMPLE 8

### Determination of the expression level of pendrin gene in the lung of OVA antigen-exposed bronchial hypersensitivity model

Quantitative PCR assay was performed with ABI 7700 using the lung RNAs described in Example 8 to quantitatively determine the expression level of the mouse pendrin gene (RefSeq: NM_011867, NM_035997, SEQ ID NO: 13/DNA, and SEQ ID NO: 14/amino acid sequence). The primers and TaqMan probe used in the assay with ABI 7700 were designed based on the information on the sequence of the pendrin gene utilizing Primer Express (Applied Bio Systems). The 5' and 3' ends of the TaqMan probe were labeled with FAM (6-carboxy-fluorescein) and TAMRA (6-carboxy-N,N,N',N'-tetramethylrhodamine), respectively. The sequences of oligonucleotides of the forward primer (F) , reverse primer (R) and TaqMan probe (TP) for the pendrin gene are shown below. The GenBank accession number corresponding to the nucleotide sequence of the mouse pendrin gene is shown in parenthesis after the name.

mouse pendrin (AF167411)
F: GGTTCTTGCCTCCTGTCCTG (SEQ ID NO: 15)
R: AATGGAAAAGGATGCAGCCA (SEQ ID NO: 16)

TP: catctgtgggcctgttttcggacatg (SEQ ID NO: 17)

Total RNA extracted by the aforementioned method was treated with DNase (Nippon Gene). Then, cDNA, which was reverse transcribed using random hexamer (GIBCO BRL) as primer, was used as a template. For a standard curve to calculate the number of copies, a plasmid clone comprising a nucleotide sequence region that is amplified by both primers was prepared for each of the genes, and this was diluted stepwise to be used as a template for carrying out the reaction. The composition of the reaction solution for monitoring PCR amplification is shown in Table 39.

**Table 39**

| Composition of the reaction solution in ABI-PRISM 7700 (Amount per well) | |
|---|---|
| Sterilized distilled water | 23.75 (µL) |
| 10x TaqMan buffer A | 5 |
| 25mM MgCl₂ | 7 |
| dATP(10 mM) | 1.0 |
| dCTP(10 mM) | 1.0 |
| dGTP(10 mM) | 1.0 |
| dUTP (20 mM) | 1.0 |
| Forward Primer (10 µM) | 1.0 |
| Reverse Primer (10 µM) | 1.0 |
| TaqMan probe (2.0 µM) | 2.5 |
| AmpliTaq Gold (5 U/µL) | 0.25 |
| AmpErase UNG (1 U/µL) | 0.5 |
| Template solution | 5 |
| Total | 50 |

Additionally, to correct the differences of cDNA concentration in the sample, a similar quantitative analysis was performed for mouse β-actin gene and mouse glyceraldehyde-3-phosphate dehydrogenase (GAPDH) gene as internal standards for correction. By correcting based on the number of copies of these genes, the number of copies of the genes of interest was calculated.

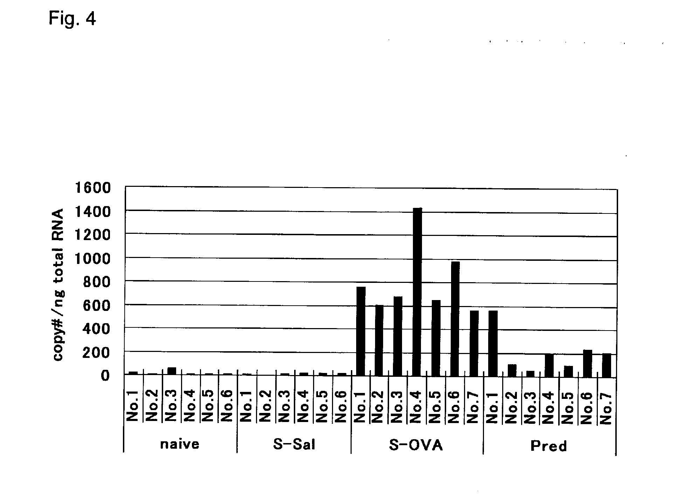

Primers and probes for measuring mouse β-actin or mouse GAPDH were designed from Primer Express (Applied Biosystems) based on the genetic information of each gene. The nucleotide sequences are as shown below. The mouse β-actin-corrected expression levels (copy/5 ng RNA) for each of the genes are shown in Fig. 4. 
mouse β-actin forward primer (SEQ ID NO: 18)
ACTATTGGCAACGAGCGGTTC
 mouse β-actin reverse primer (SEQ ID NO: 19) GGATGCCACAGGATTCCATACC 
mouse β-actin TaqMan probe (SEQ ID NO: 20)
(FAM) CCTGAGGCTCTTTTCCAGCCTTCCTTCT (TAMRA) -3'
 
mouse GAPDH forward primer (SEQ ID NO: 21)
GCACCACCAACTGCTTAGCC
 
mouse GAPDH reverse primer (SEQ ID NO: 22)
CTTTGGCATTGTGGAAGGGCTCATG
 
mouse GAPDH TaqMan probe (SEQ ID NO: 23)
(FAM) GATGCAGGGATGATGTTCTGG (TAMRA)-3'
 FAM: 6-carboxy-fluorescein
TAMRA: 6-carboxy-N,N,N',N'-tetramethylrhodamine

According to the result of quantitative PCR, the expression level in the lung of OVA antigen-exposed bronchial hypersensitivity mice was about 50 times higher than that in the lung of physiological saline-inhaled mice. This finding suggests that the pendrin gene may be an important gene that controls the progression of allergic reactions, particularly asthma because the gene is expressed at a higher level in the lung of OVA antigen-exposed bronchial hypersensitivity model mouse that mimics human asthma.

### EXAMPLE 9

### Determination of the localization of pendrin mRNA in the lung of OVA antigen-exposed bronchial hypersensitivity model by in situ hybridization (hereinafter referred to as "ISH")

After perfusion fixation with 10% buffered neutral formalin, the pulmonary tissues were collected from three mice each of the four groups (the untreated group; the physiological saline-inhaled group; the Prednisolone-administered group; and the OVA antigen-inhaled group) used in Example 9. The tissues were fixed with 10% buffered neutral formalin, and then embedded in paraffin to prepare tissue blocks.

All paraffin blocks from the mouse lung samples were sliced into 7 µm sections. Then, the sections were treated with hematoxylin for nuclear staining. Among the sections, sections exhibiting good tissue morphology were selected from a single individual each of the physiological saline-inhaled group and OVA antigen-inhaled group. The sections were tested by ISH. The nucleotide sequence of the ISH probe is shown in SEQ ID NO: 24.

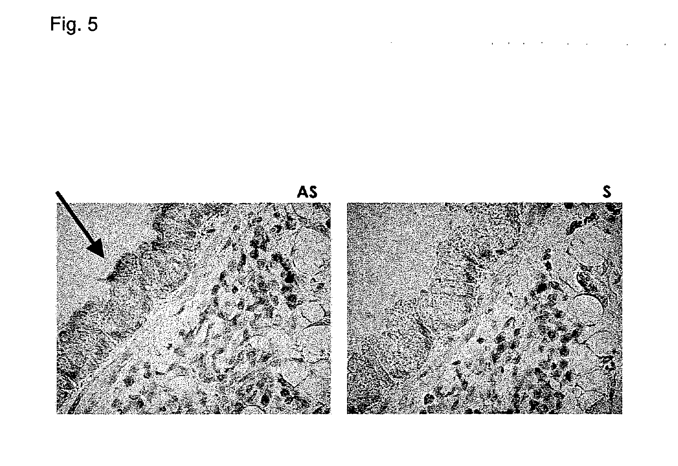

The paraffin sections of mouse lung tissues from the physiological-saline-inhalation group and the OVA-antigen-inhalation group were rehydrated by deparaffinization (washed with water after treatment with xylene, 100%, 90%, 80%, and 70% alcohol). Then, the sections were treated with the above probe. After the staining, the sections were treated for nuclear staining. The condition used for the ISH experiments is described below. The result of ISH is shown in Fig. 5.
Probe concentration: 250 ng/ml
hybridization temperature: 60°C
Duration of hybridization: 6 hours
Post-hybridization wash: 0.1x SSC/70°C /6 minutes/3 times
Coloring reagents: NBT/BCIP
Duration of color development: 7 hours

The ISH result showed that the mouse lung sections from the OVA antigen inhalation group gave a specific staining pattern with the antisense probe. Blue deposits were detectable in the bronchia, bronchiole and macrophages in the pulmonary alveoli. Blue deposits with similar intensity were also found on the epithelial cells of bronchial mucosa. The sense probe resulted in no deposits.

### EXAMPLE 10

### PAS staining and Alcian Blue staining of lung tissues of OVA antigen-exposed bronchial hypersensitivity model

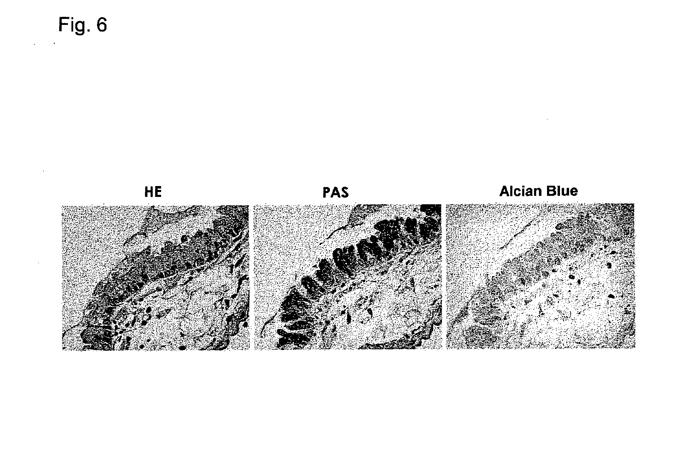

The localization of the huge glycoprotein mucin in the lung tissue of OVA antigen-exposed bronchial hypersensitivity model was confirmed by PAS staining for acidic sugar chains and Alcian Blue staining for basic sugar chains. The paraffin blocks of mouse lung tissues from the physiological-saline-inhalation group and the OVA-antigen-inhalation group used in Example 10 were sliced into 3-µm sections. After being rehydrated by deparaffinization (washed with water after treatment with xylene, 100%, 90%, 80% and 70% alcohol) , the sections were treated by PAS staining and Alcian Blue staining. The result obtained by the staining is shown in Fig. 6. The reaction conditions used are as follows:
PAS staining:
   1% periodate solution for 10 minutes
   washing with water for 5 minutes
   cold Schiff's reagent for 15 minutes
   sulfuric water for 2 minutes 3 times
   washing with water
Alcian Blue staining:
   3% acetic acid for 1 minute
   Alcian Blue staining solution (pH 2.5) for 30 minutes
   3% acetic acid; washing five times
   washing with water
   dehydration, clearing and mounting
   70% alcohol for 5 minutes
   80% alcohol for 5 minutes
   90% alcohol for 5 minutes
   100% alcohol for 5 minutes twice
   xylene for 5 minutes twice
   xylene type mounting agent; mounting with cover glasses

Both PAS staining and Alcian Blue staining resulted in positive reactions in the cytoplasmic granules in epithelial cells and goblet cells of bronchial mucosal membrane. This indicates that the epithelial cells and goblet cells of bronchial mucosal membrane contain mucin. According to the results obtained in Examples 12 and 13, the pendrin mRNA are localized in the epithelial cells and goblet cells of bronchial mucosal membrane.

### EXAMPLE 11

### Variations in the expression levels of marker genes in bronchial hypersensitivity model mouse

### 1. RNA recovery from the lung of OVA antigen-exposed bronchial hypersensitivity model mouse

As mentioned above, the OVA antigen-exposed bronchial hypersensitivity model using 7-week old male Balb/c mice has been reported to mimic human asthma. This mouse model is prepared as described in Example 7. In such mice, bronchial hypersensitivity is enhanced after the final antigen inhalation. Thus, symptoms quite similar to those of asthma can be induced in this model.

In this Example, RNAs were isolated from the lung and trachea 24 hours after the first, second or third exposure to OVA antigen, and cDNA and cRNA were synthesized from the RNAs. The respective samples were analyzed using a mouse GeneChip (MG-U74A-C), and the result obtained was compared to that from the human goblet cell differentiation model.

RNAs were isolated from the lung and trachea 24 hours after the first, second and third exposure to OVA antigen. The test was conducted using the following four groups: OVA antigen-inhaled bronchial hypersensitivity group (S-OVA); the three control groups: untreated group (naive) ; physiological saline-inhaled group in which OVA antigen was given twice for immunization and physiological saline was given by inhalation (S-Sal); and Prednisolone-treated group, in which Prednisolone was given by inhalation 10 times in total from the day before antigen inhalation until the final antigen inhalation, and the development of bronchial hypersensitivity was suppressed by giving 5 mg/kg Prednisolone orally (Pred).

The lung and trachea were resected 24 hours after the first, second and third exposure to OVA antigen. Each tissue was crushed with a homogenizer called Polytrone immediately after dissolving in Isogen (Nippon Gene; Wako Pure Chemical Industries). RNA was isolated from 1 ml of this solution according to the protocol attached to Isogen. Chloroform was added to the solution. After the mixture was stirred and centrifuged, the aqueous layer was recovered. Then, isopropanol was added to the aqueous solution obtained. After the mixture was stirred and centrifuged, the precipitated total RNA was collected. Total RNAs (approximately 20-60 µg) were extracted from the samples of the twelve groups described above.

### 2. Synthesis of cRNA for GeneChip

Biotinylated cRNA was synthesized by the same method as described in Example 4. About 20-50 µg biotinylated cRNAs were synthesized from the cDNAs obtained from the twelve groups described above. The cRNAs were purified using RNeasy Spin column (QIAGEN) , and then converted into fragments by heat treatment. A 15-µg aliquot of each cRNA was added to a Hybridization Cocktail according to the Expression Analysis Technical Manual. The cocktail is added to an array chip, followed by incubation for hybridization at 45°C for 16 hours. After hybridization, the chip was stained and analyzed by the same procedure as described in Example 4.

### 3. GeneChip analysis

Data analysis was performed using Suite 4.0, which is a GeneChip analysis software. Average Intensity (1) and Background Average (2) were determined by Absolute Analysis, and four average values obtained (naive group, S-Sal group, S-OVA group, and Pred group) by subtracting (2) from (1). These four values were used as scale factors for comparison analysis.

First, absolute analysis was performed to analyze one chip data. Positives and negatives were determined by comparing the fluorescence intensity of perfect match and mismatch of a probe set. Determination of the three categories of Absolute Calls, i.e., P (present) , A (absent) , and M (marginal) , were made by values of Pos Fraction, Log Avg, and Pos/Neg:
Pos Fraction; ratio of positive pairs.
Log Avg; average of the log of fluorescence intensity ratio between probe cells of perfect match and mismatch.
Pos/Neg; ratio of the number of positive pairs and negative pairs.

Additionally, Average Difference (Avg Diff), which is the average value of the difference in fluorescence intensities between perfect matching and mismatching probe cells, was calculated for each gene.

Next, Comparison Analysis was performed on two sets of data. For example, comparison was made between S-Sal group and S-OVA group, and the difference in expression levels was ranked as follows.

Determination of the 5 categories of difference calls, which are I, D, MI, MD, and NC, were made from values of Inc/Dec, Inc Ratio, Dpos-Dneg Ratio, and Log Avg Ratio Change.
Inc: Number of probe pairs that corresponded to S-Sal group and S-OVA group and that were judged to have increased expression levels in S-OVA group.
Dec: Number of pairs judged to have decreased expression levels in S-OVA group.
Inc/Dec: Ratio of the number of pairs judged to be Inc and number of pairs judged to be Dec.
Inc Ratio: Number of pairs judged to be Inc/number of pairs actually used.
Dpos/Dneg Ratio: Ratio between the number of Neg Change subtracted from that of Pos Change, and the number of pairs actually used.
Pos Change: Difference between the number of positive pairs in Absolute Analysis of S-Sal group, and the number of positive pairs in Absolute Analysis of S-OVA group.
Neg Change: Difference between the number of negative pairs in Absolute Analysis of S-Sal group, and the number of negative pairs in Absolute Analysis of S-OVA group.
Log Avg Ratio Change: Difference between Log Avg in Absolute Analysis of S-Sal group and S-OVA group.
Increased: I,
Decreased: D,
Marginally Increased: MI,
Marginally Decreased: MD, and
No Change: NC

### 4. Comparison of a group of genes associated with goblet cell differentiation, which was narrowed down using the chips of HG-U95A to HG-U95E, with a group of genes derived from the OVA antigen-exposed bronchial hypersensitivity model, which was narrowed down using the chips of MG-U74A, MG-U74B, and MG-U74C

NetAffx database (Affymetrix) was searched for the mouse counterparts of the genes narrowed down using HG-U95A to HG-U95E chips as described above. The Fold Change values are shown in Tables 40 to 83, which were obtained by further analyzing the counterpart genes contained in mouse GeneChip MG-U74A to MG-U74C comparatively between S-Sal group and S-OVA group using Suite4.0 (Affymetrix).

Based on the expression levels in the mouse asthma model, the genes categorized are shown in Tables 40 to 62 (mouse counterpart genes of the human genes whose expression levels were found to increase by IL-13 under the culture conditions according to the AI method) and Tables 63 to 83 (mouse counterpart genes of the human genes whose expression levels were found to be decreased by IL-13 under the culture condition according to the AI method).

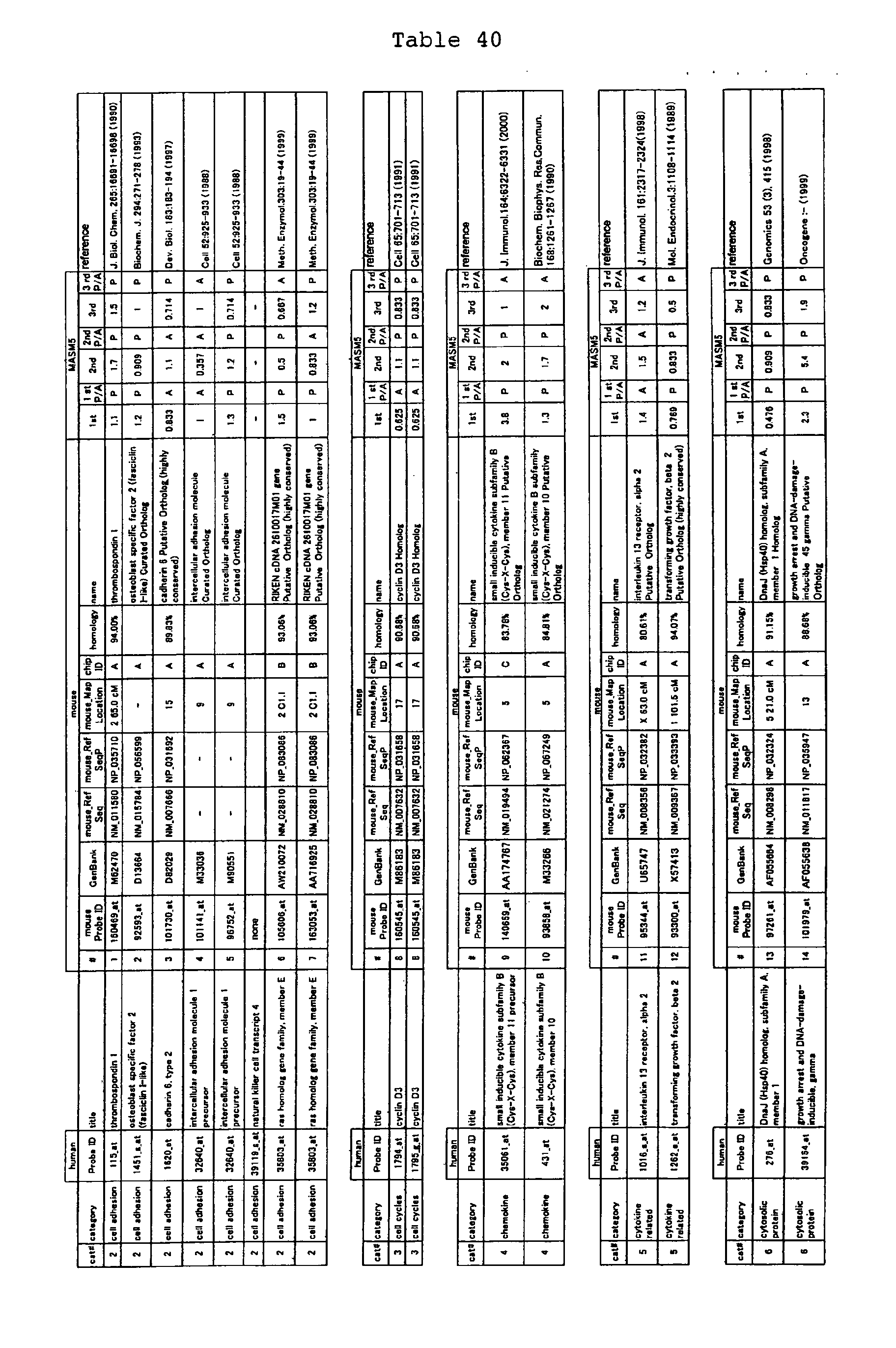

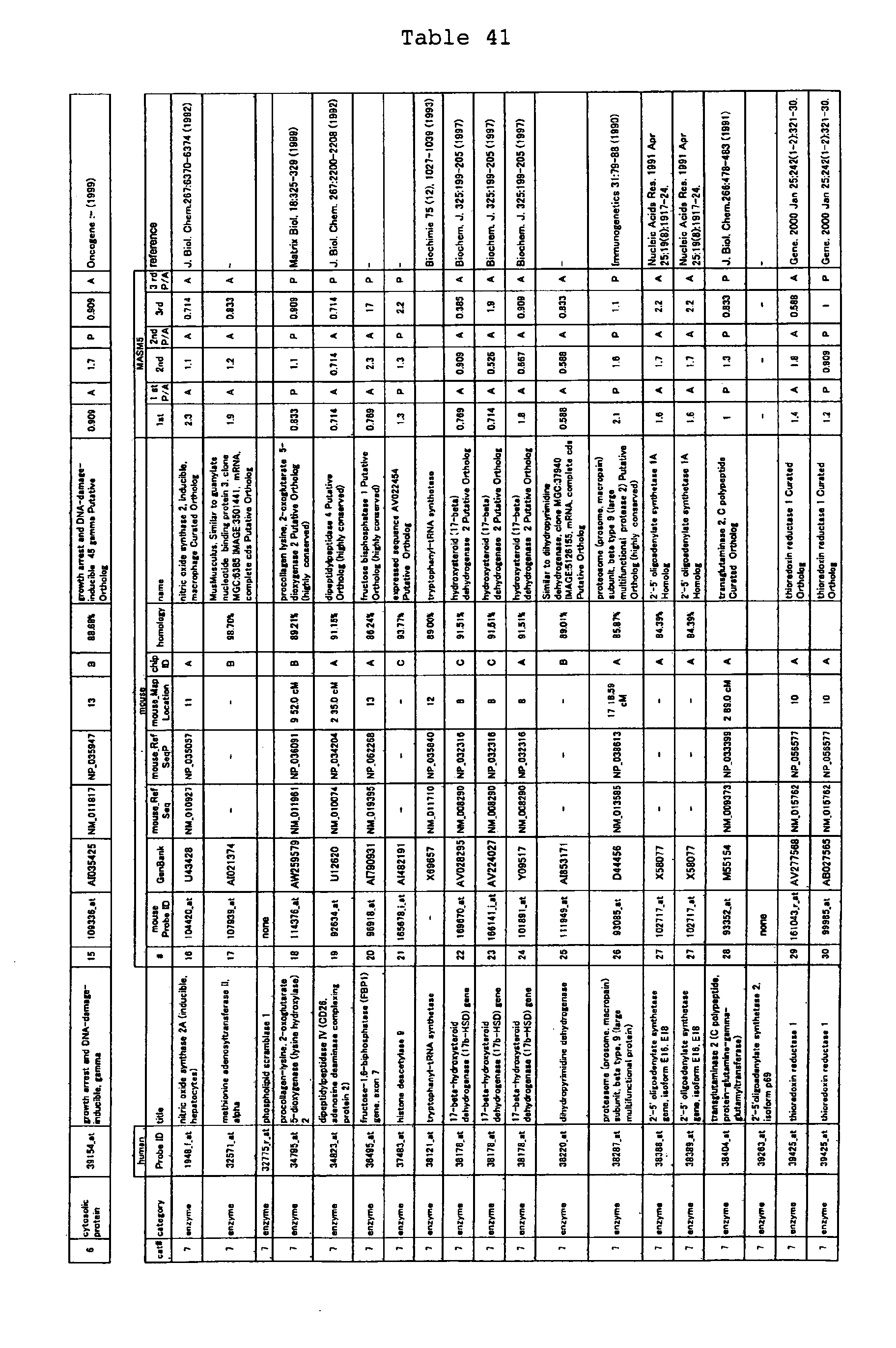

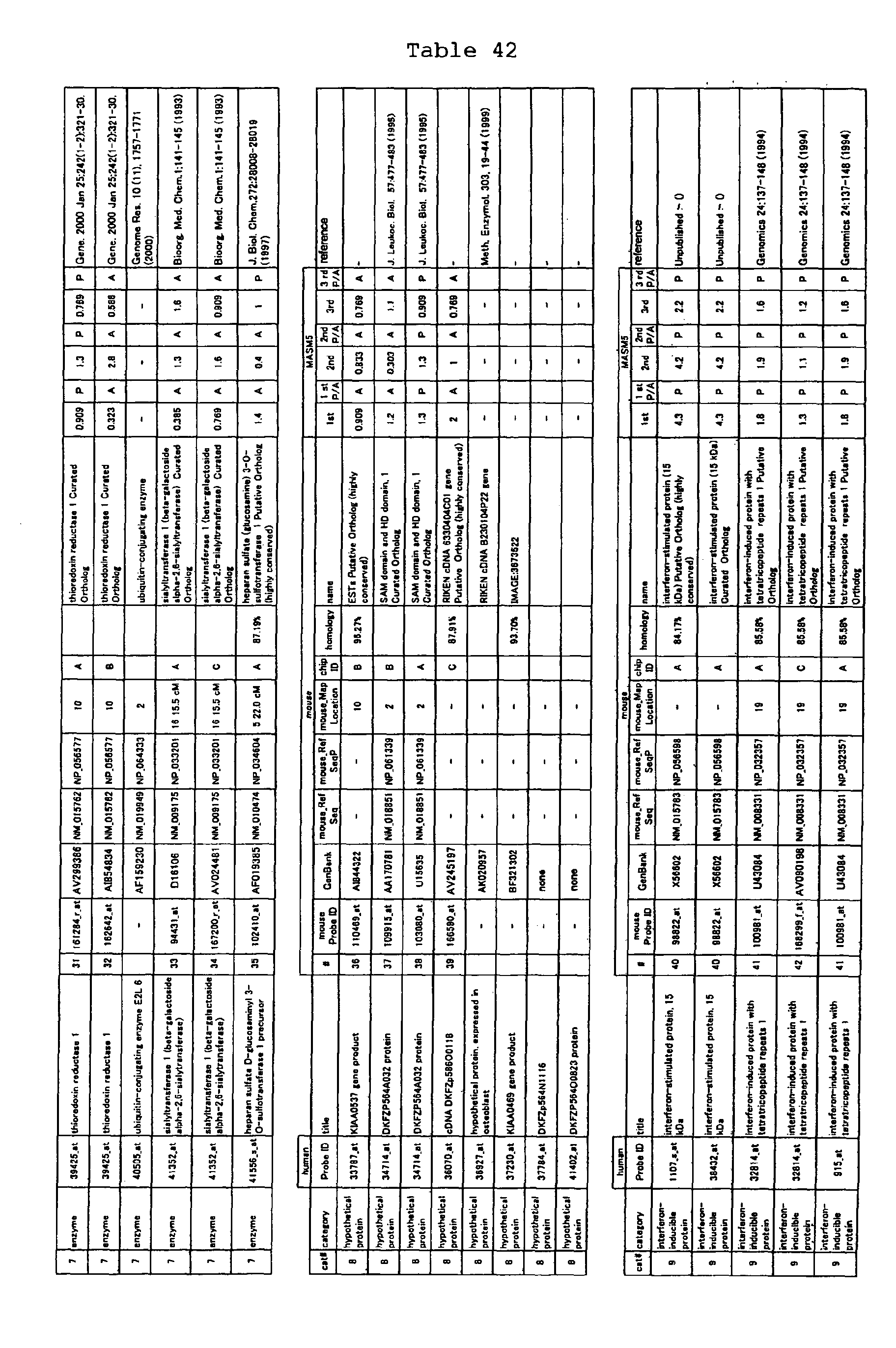

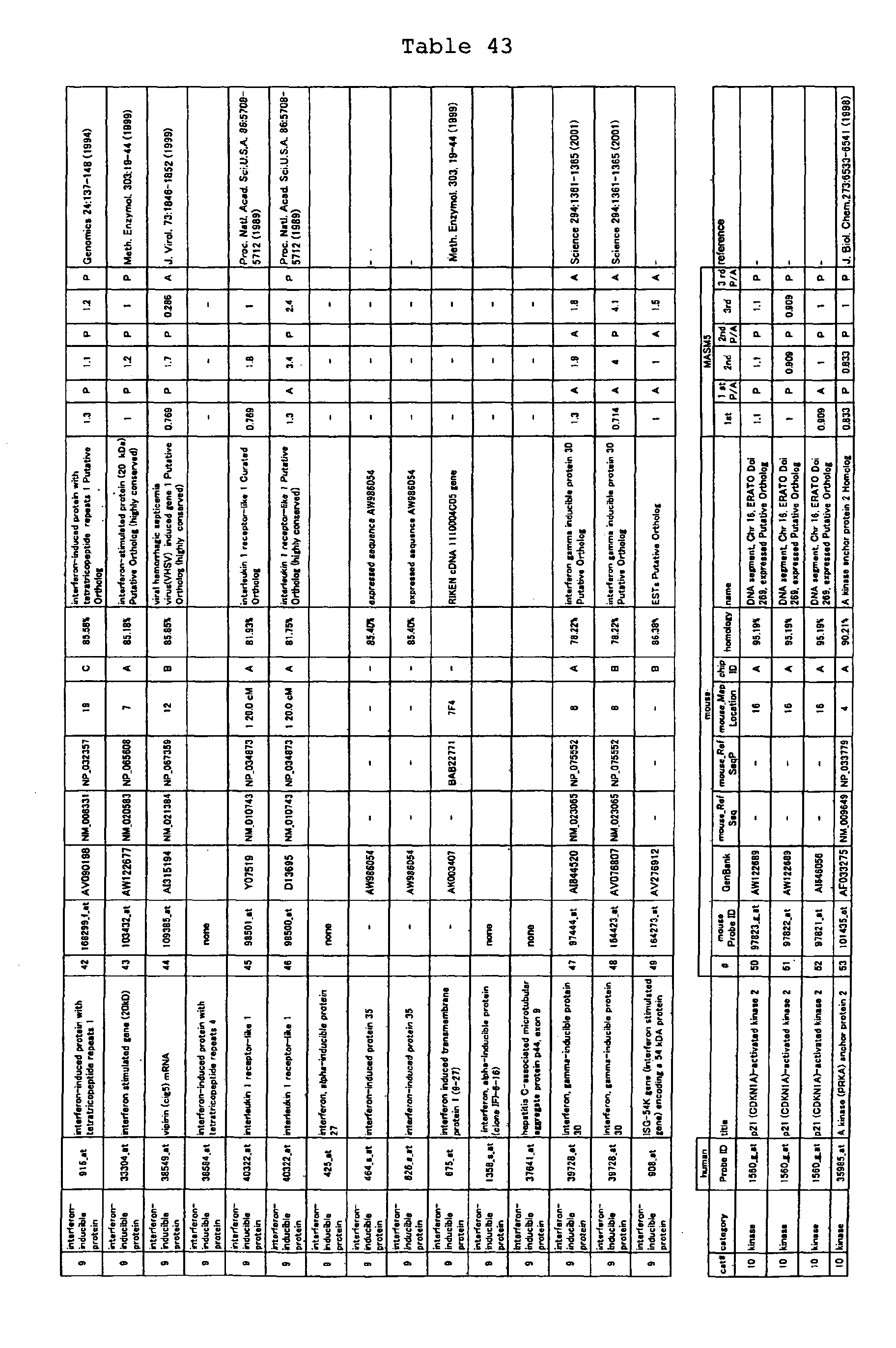

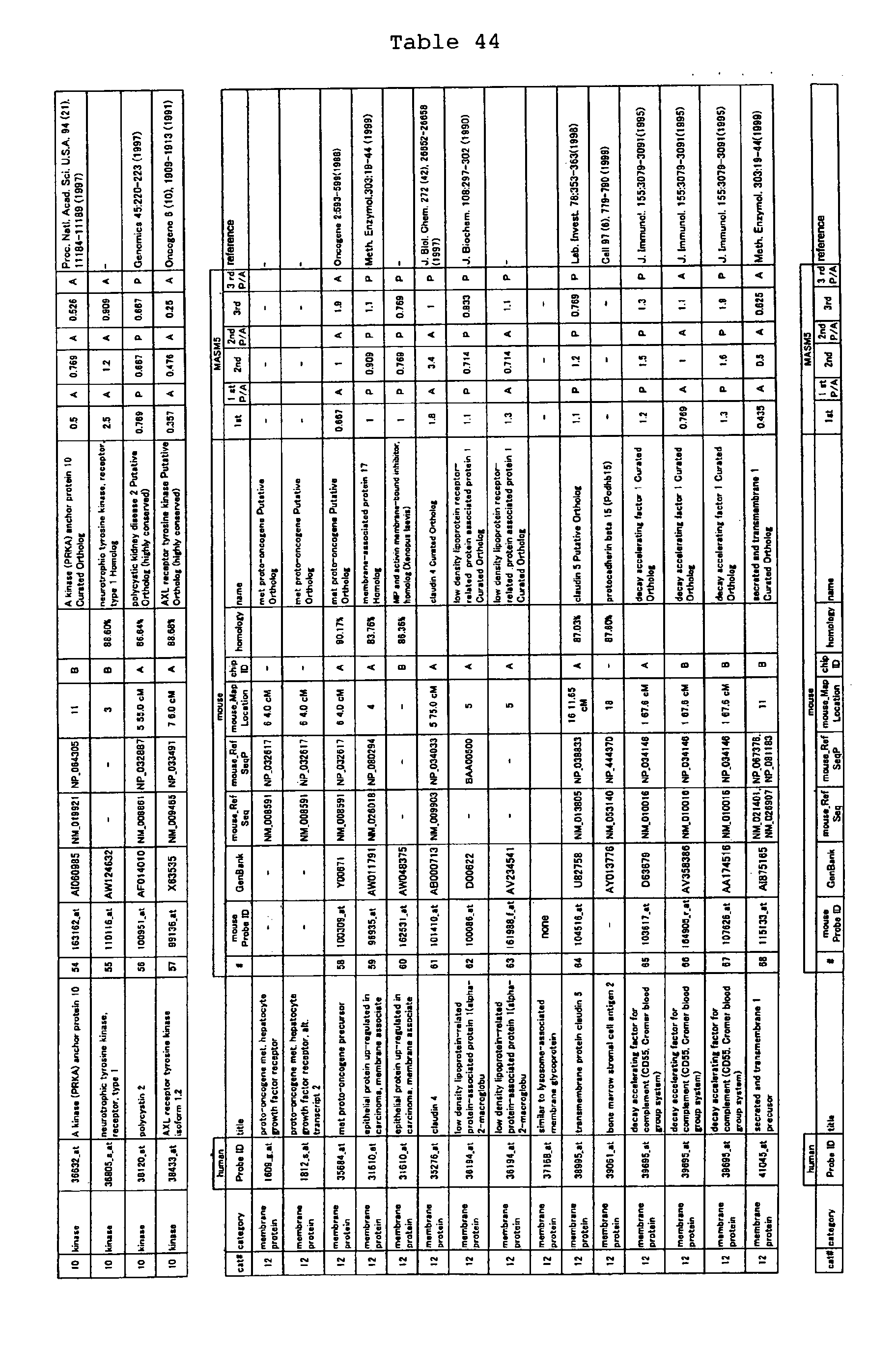

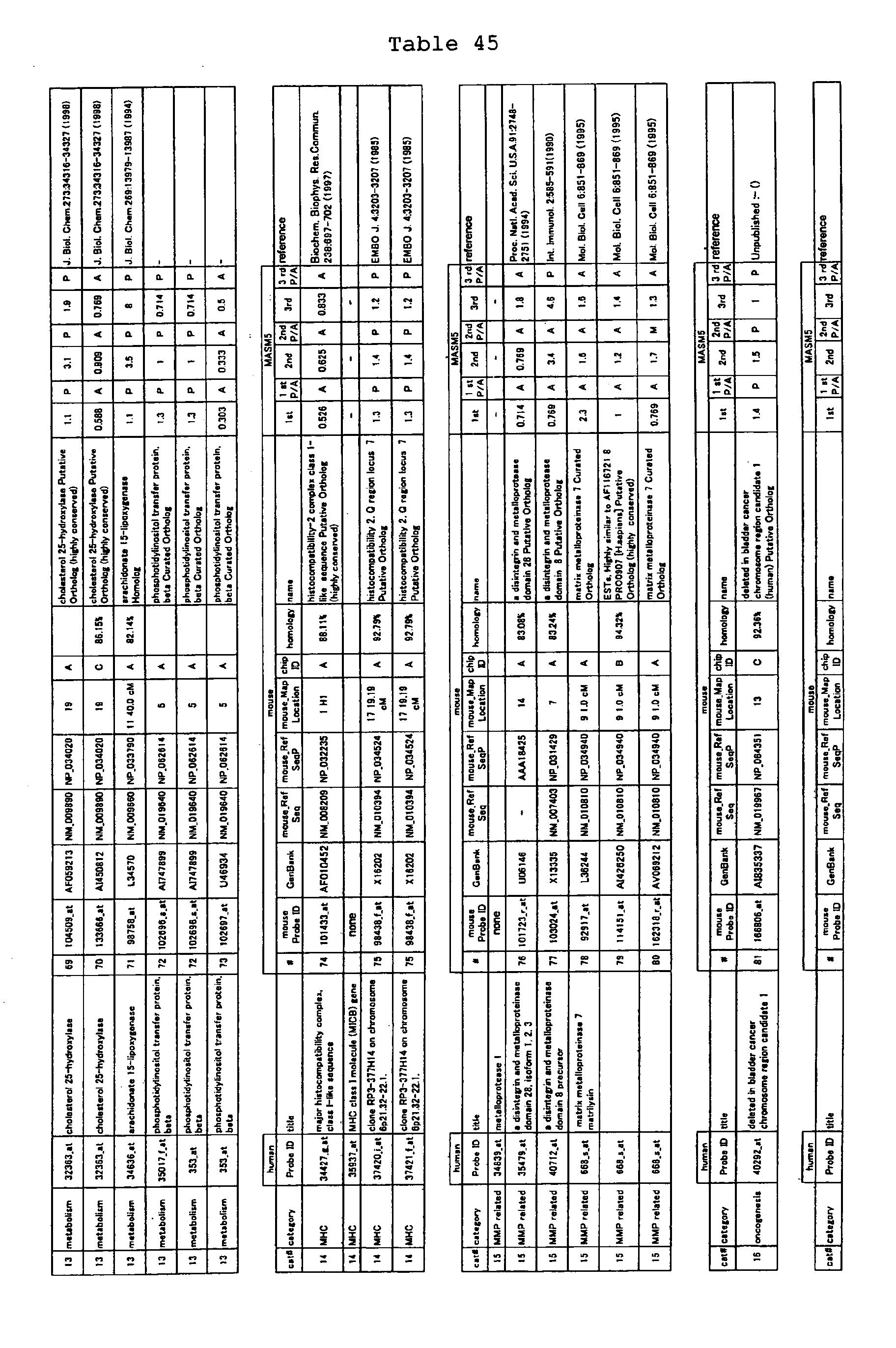

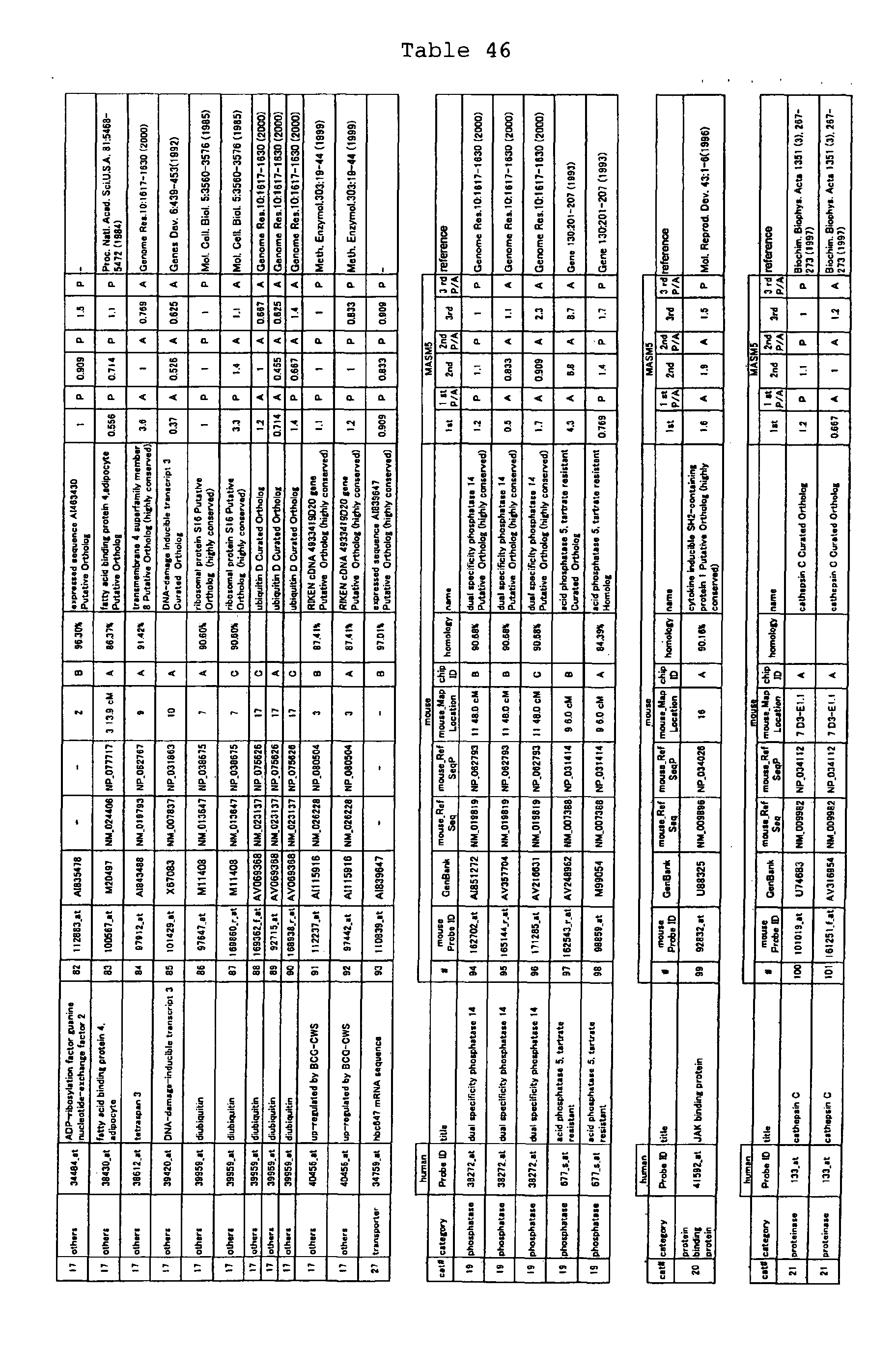

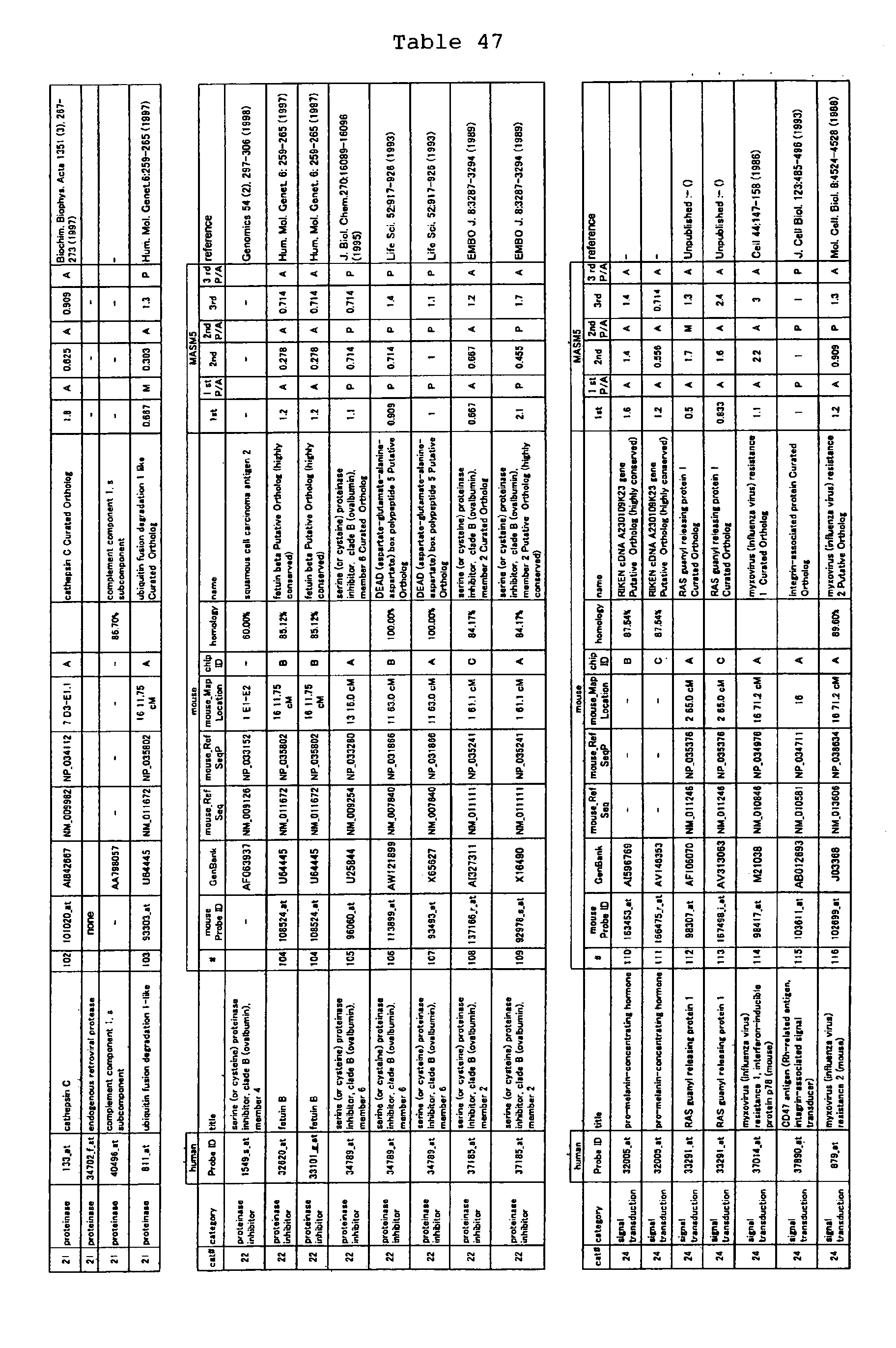

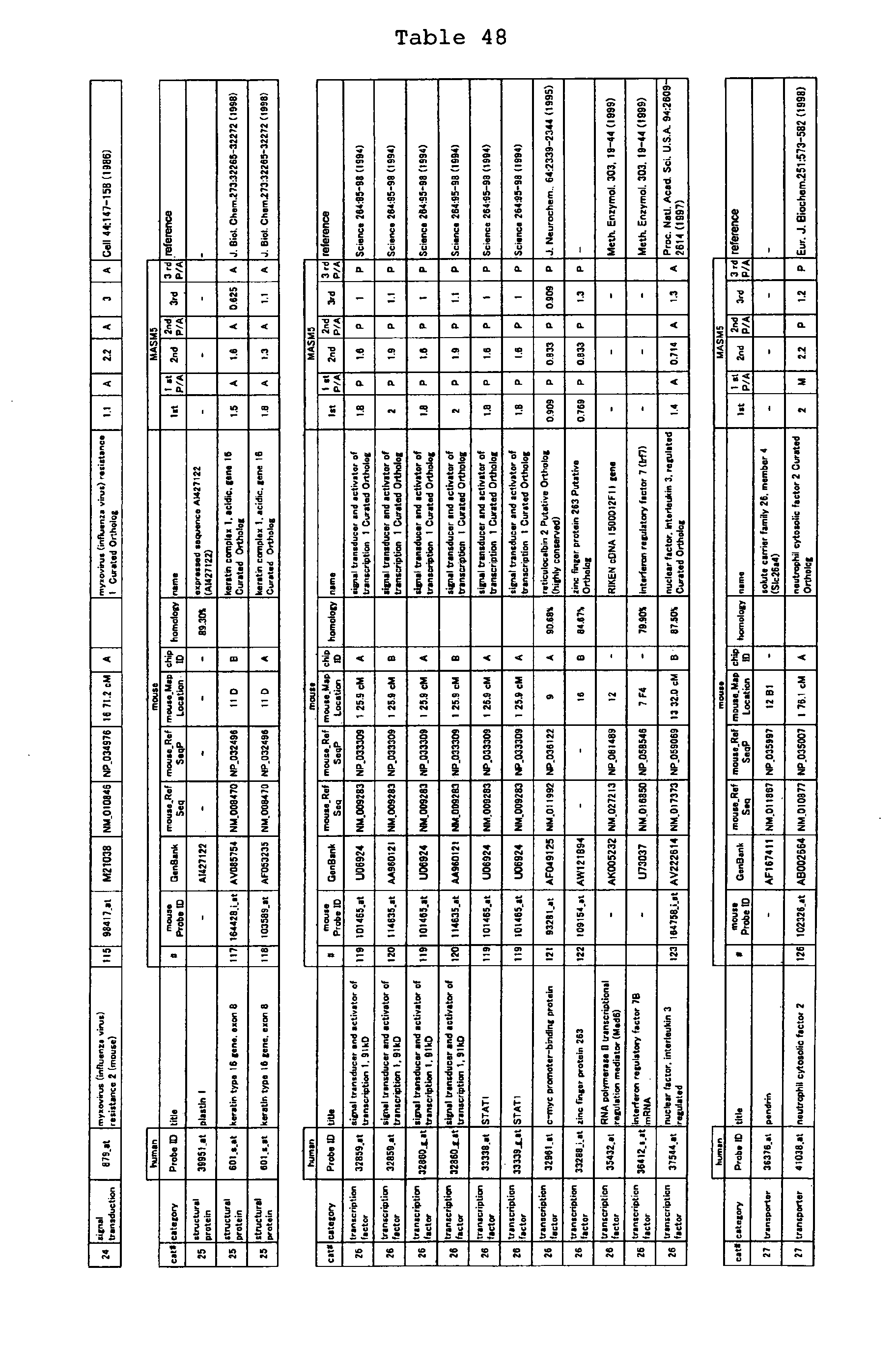

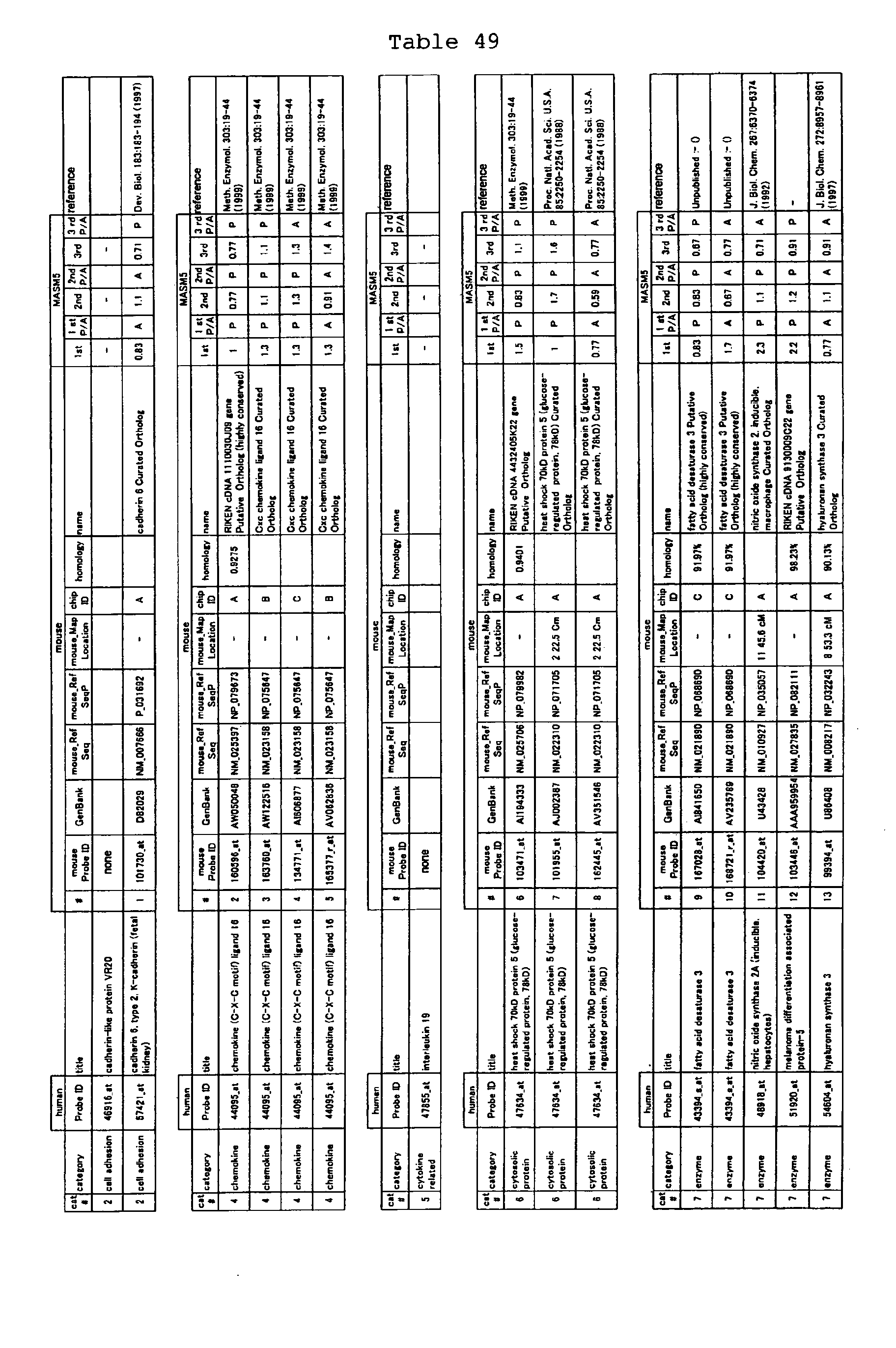

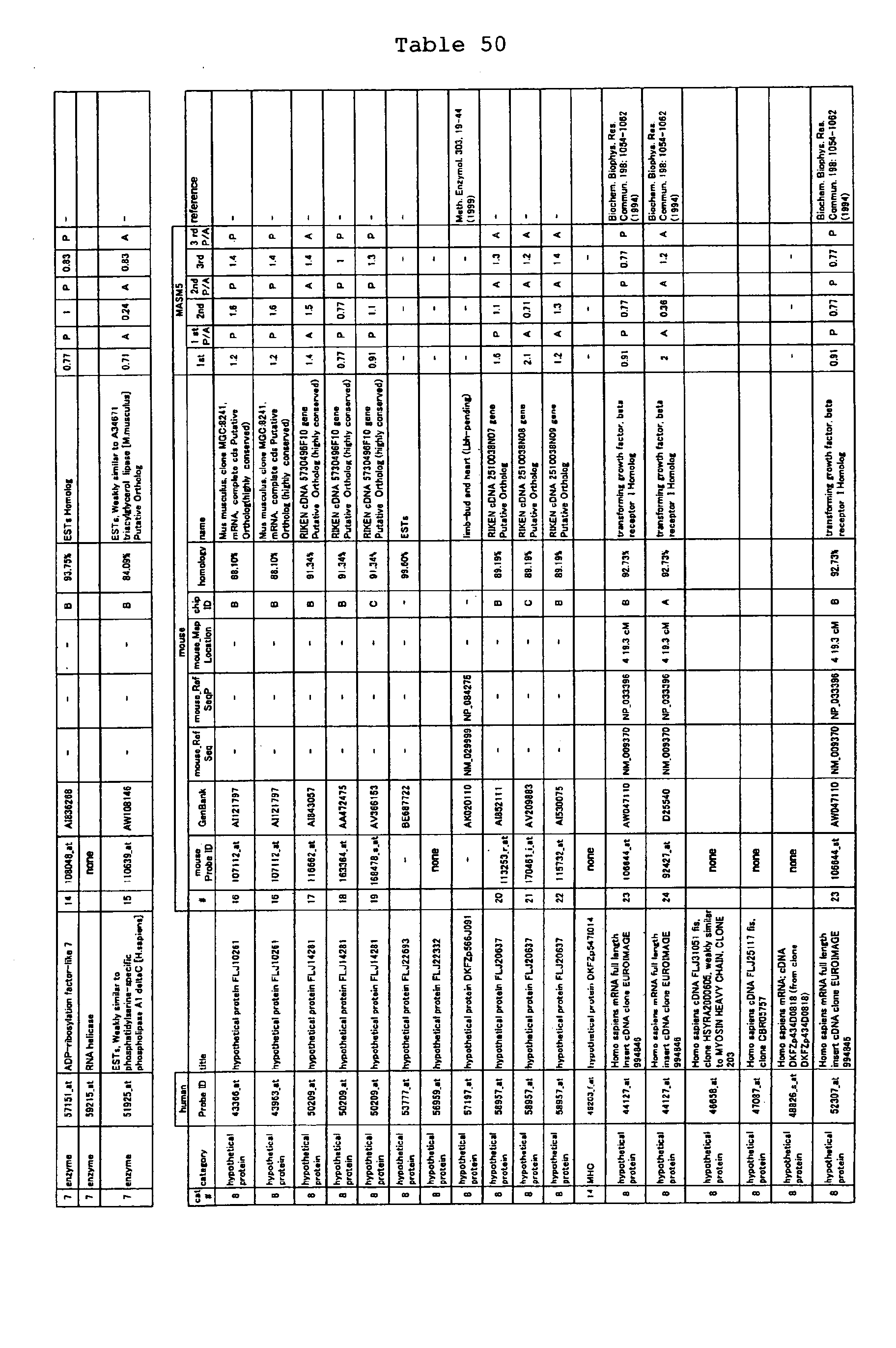

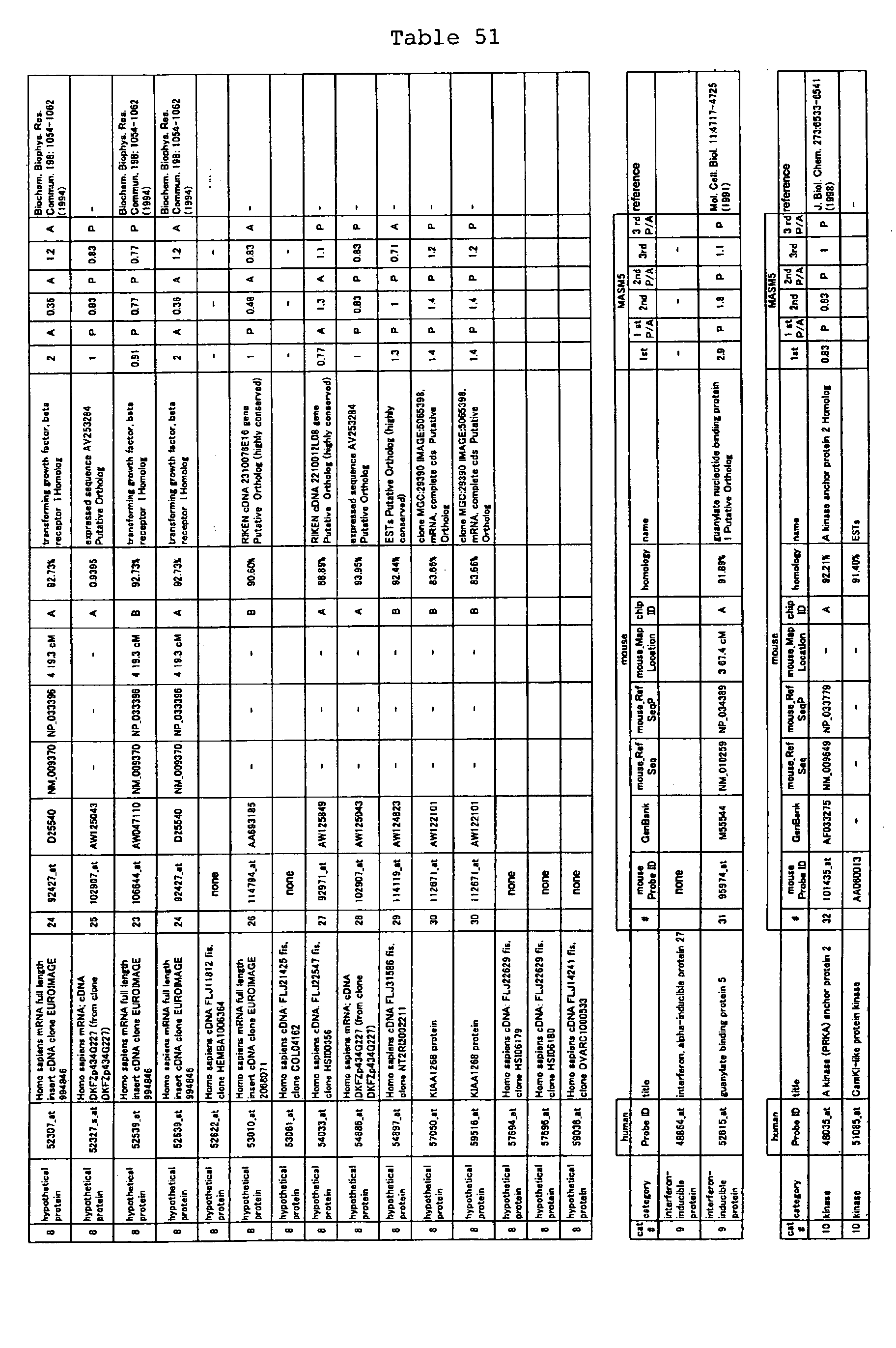

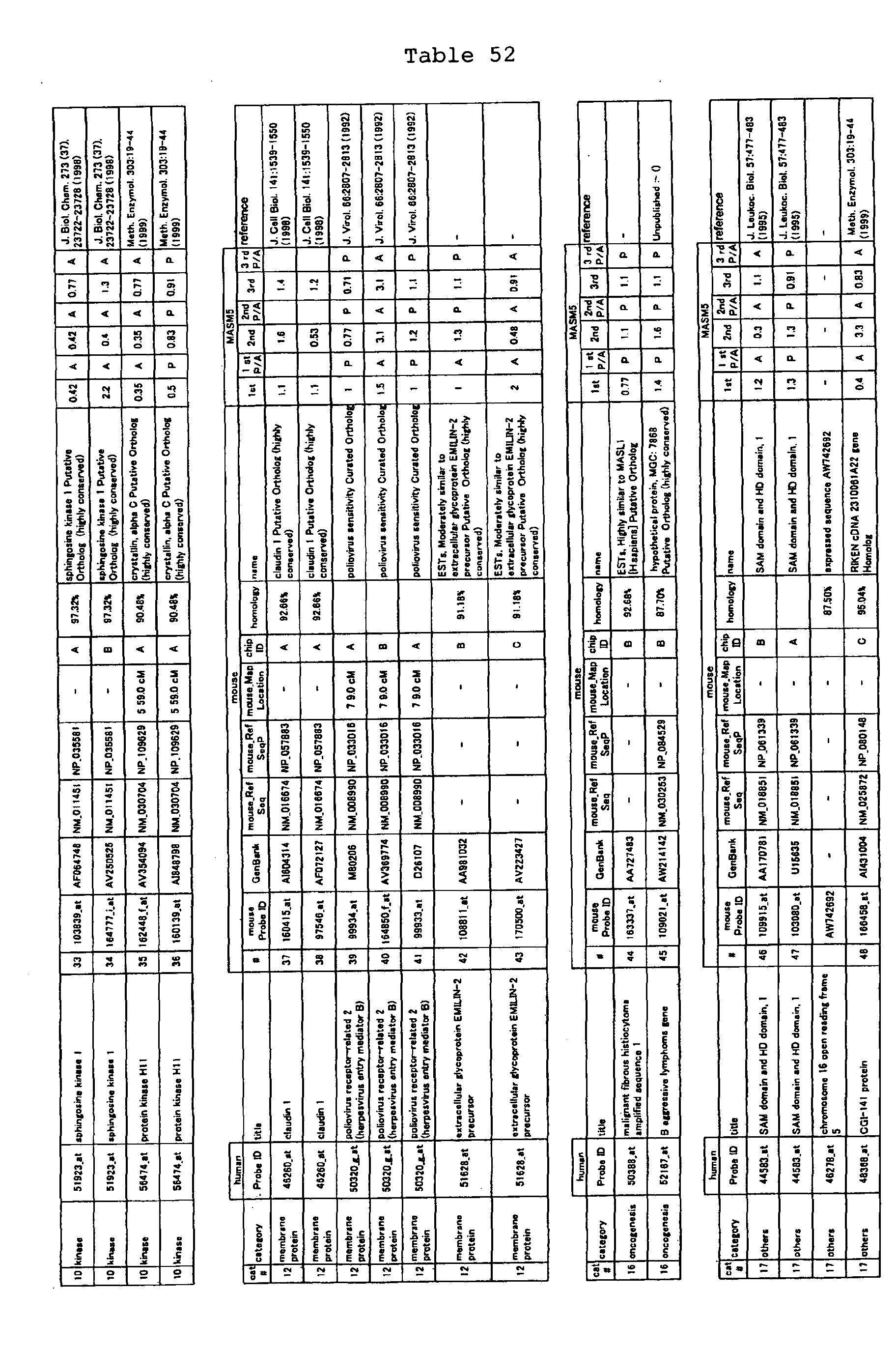

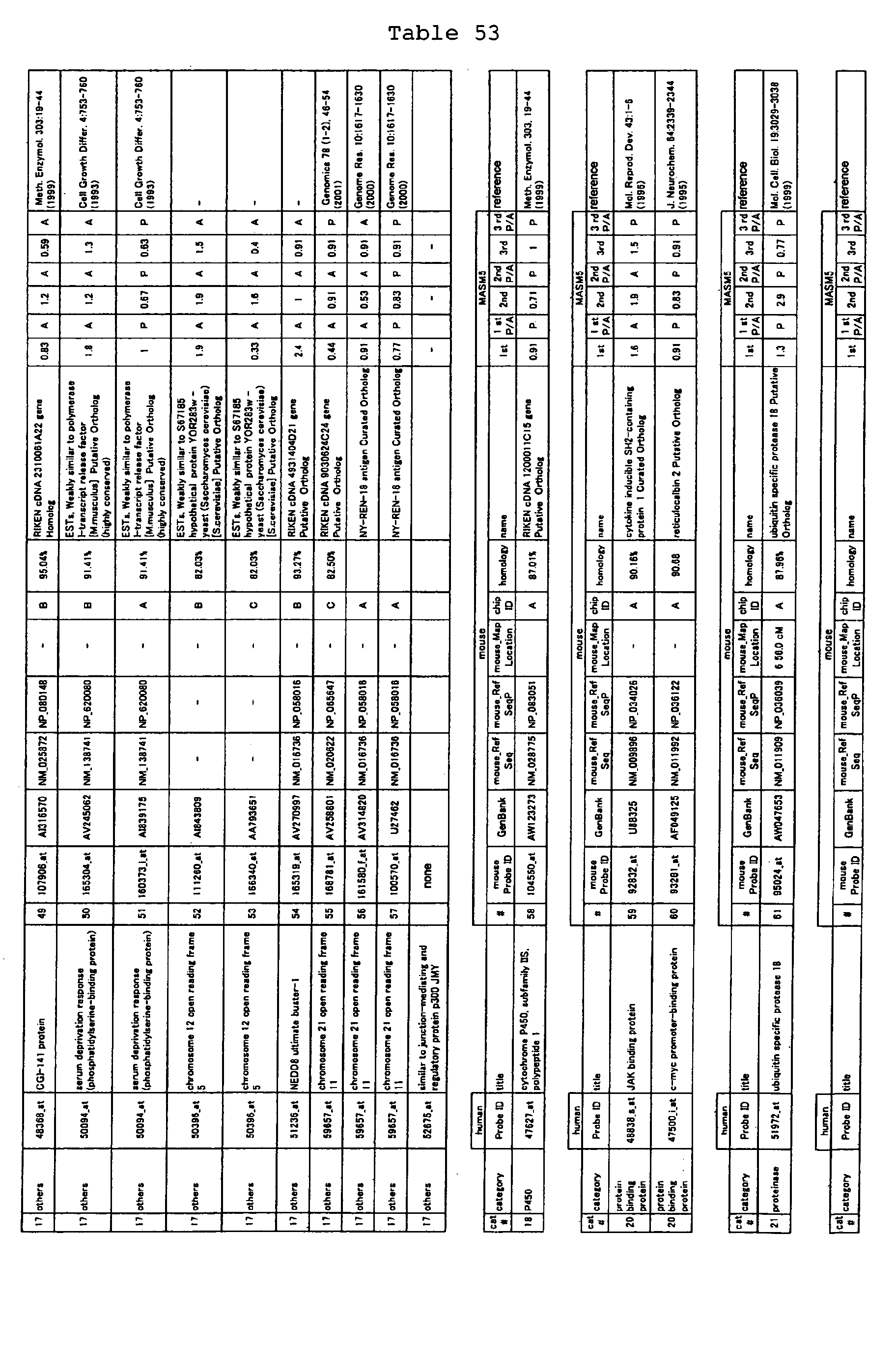

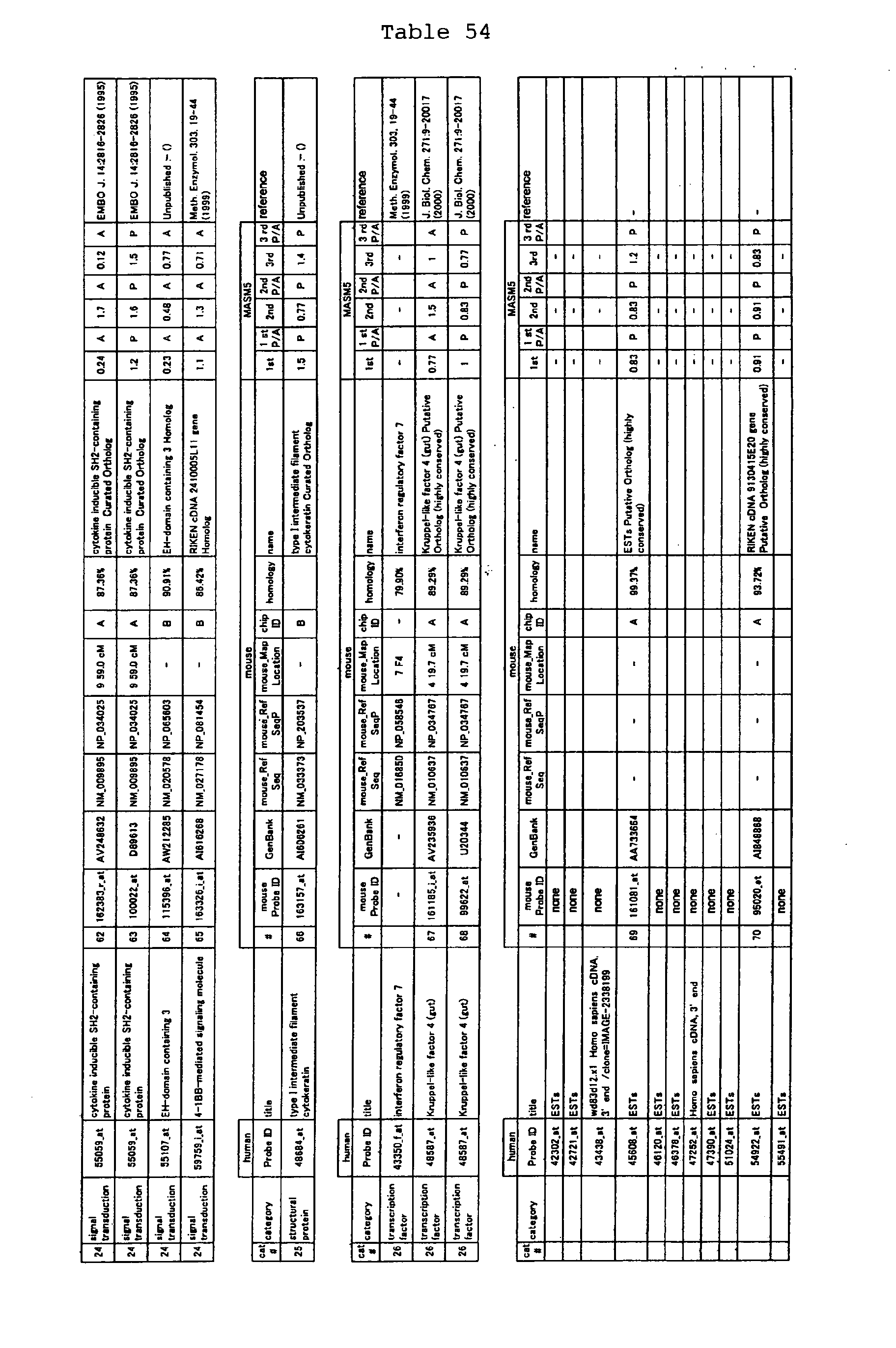

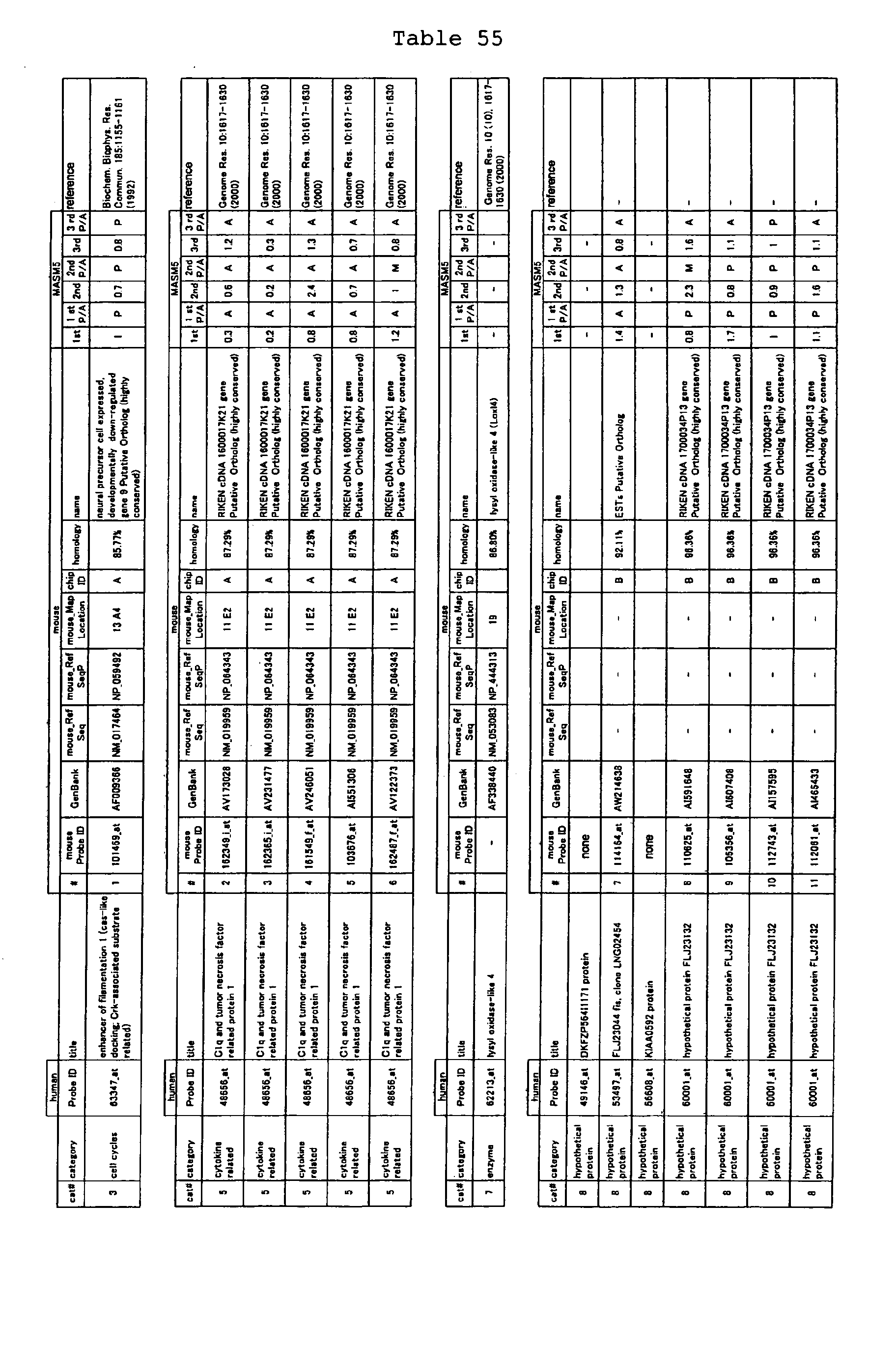

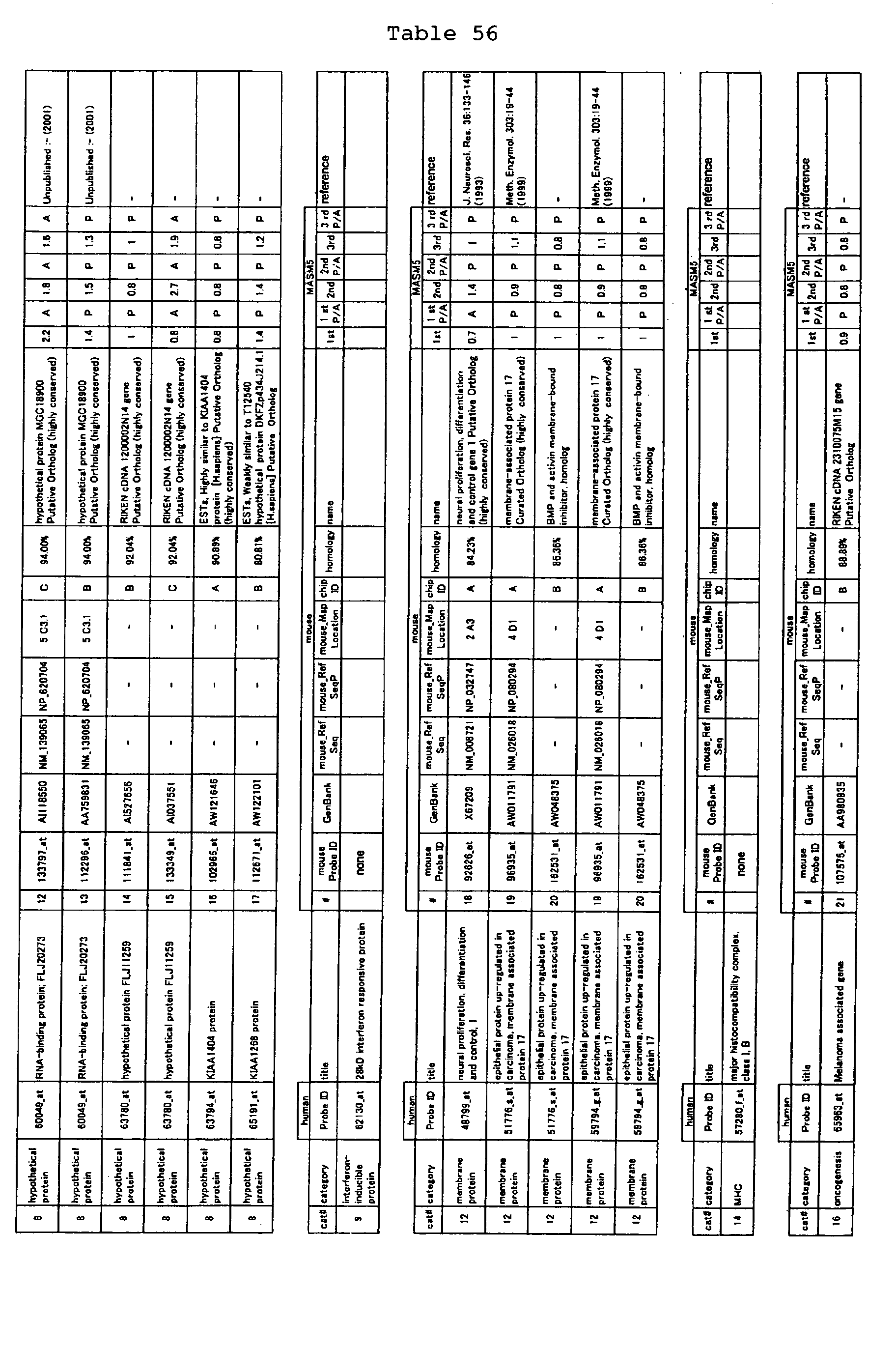

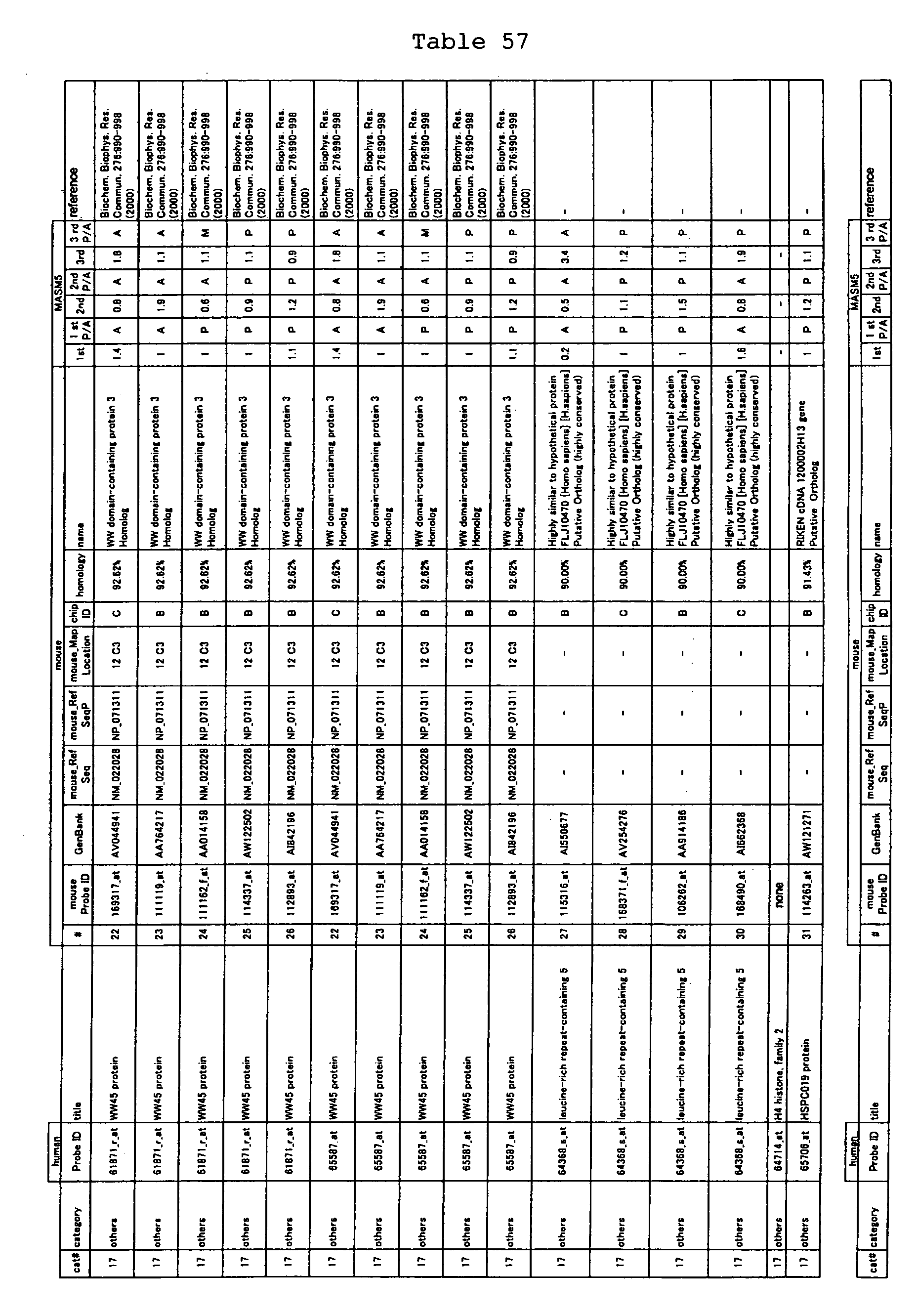

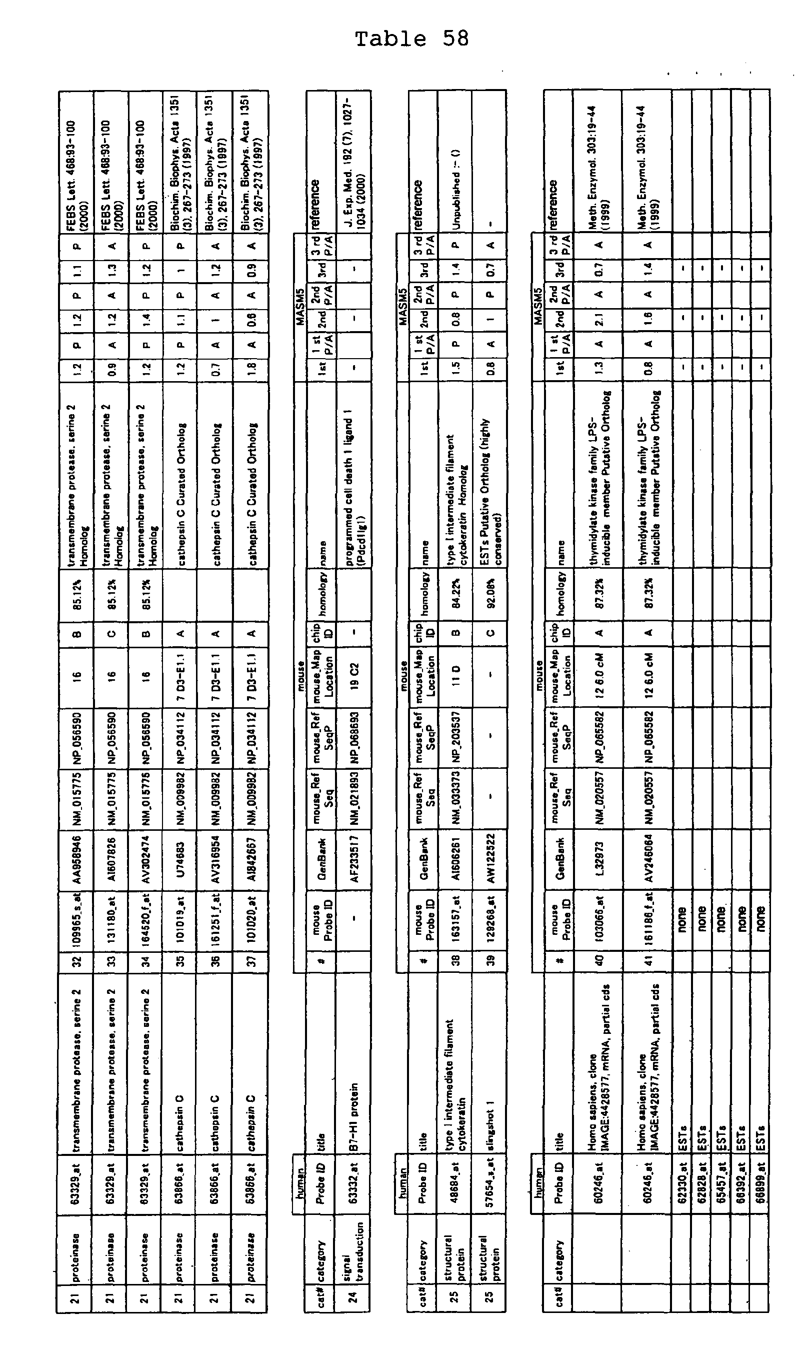

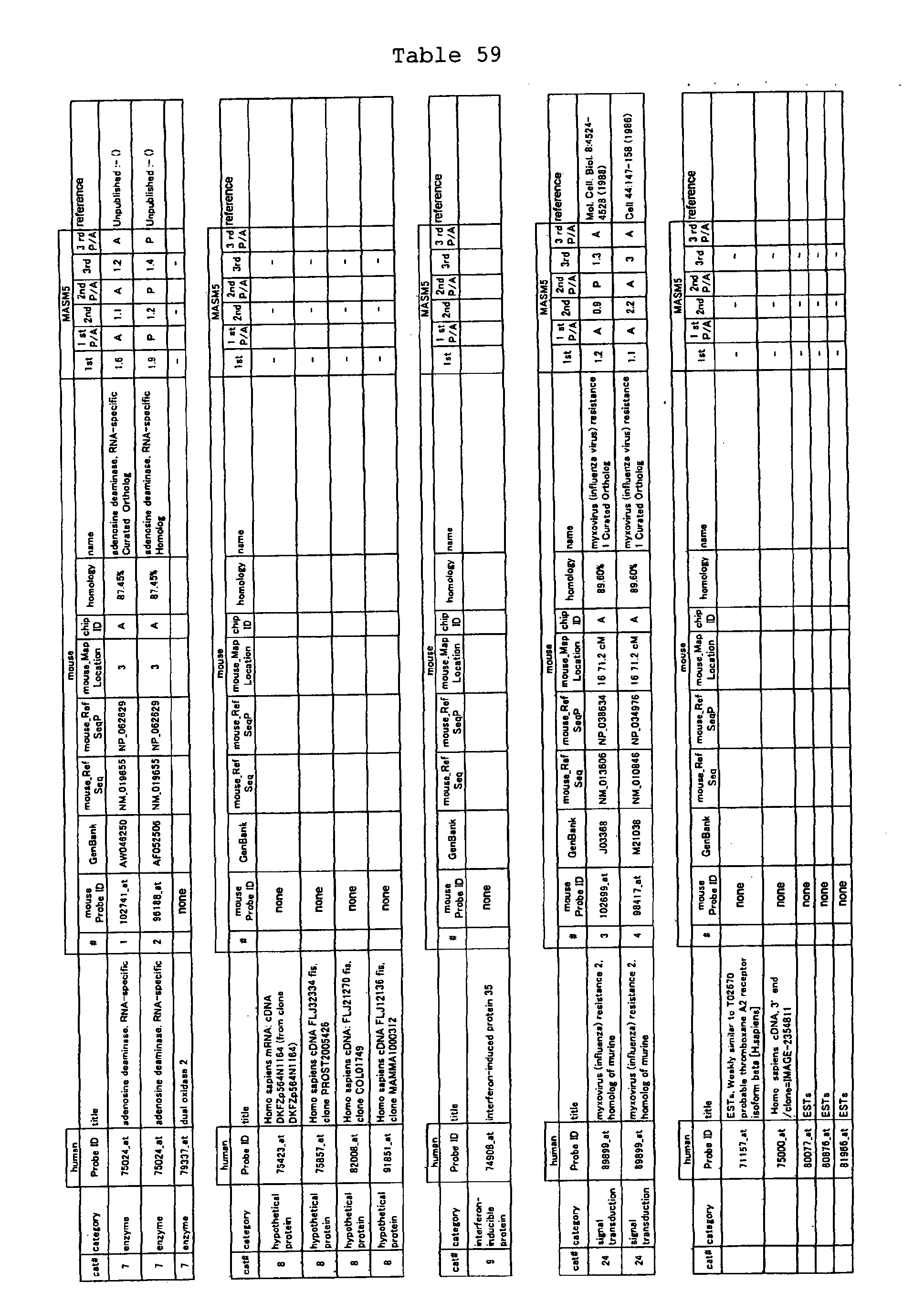

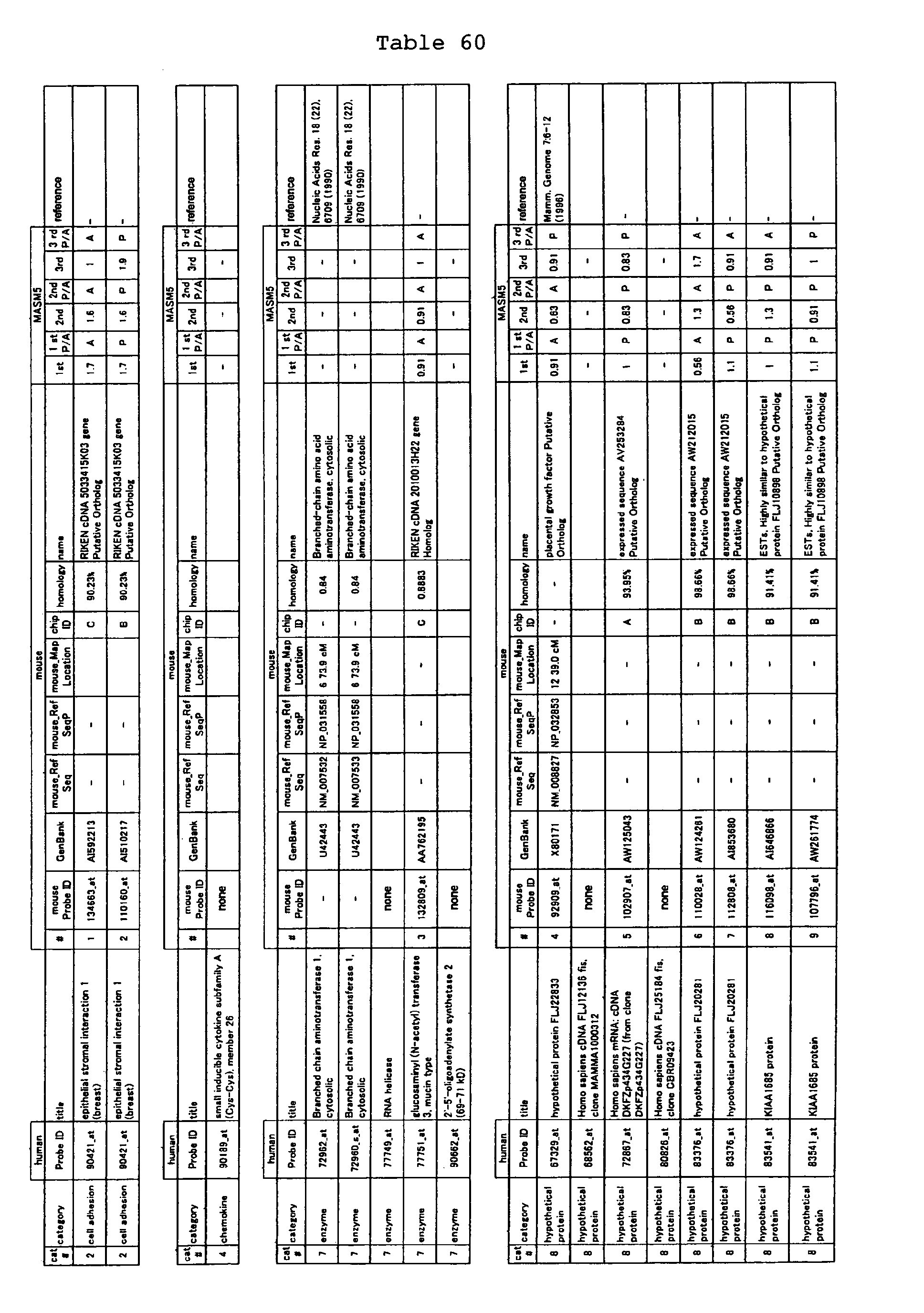

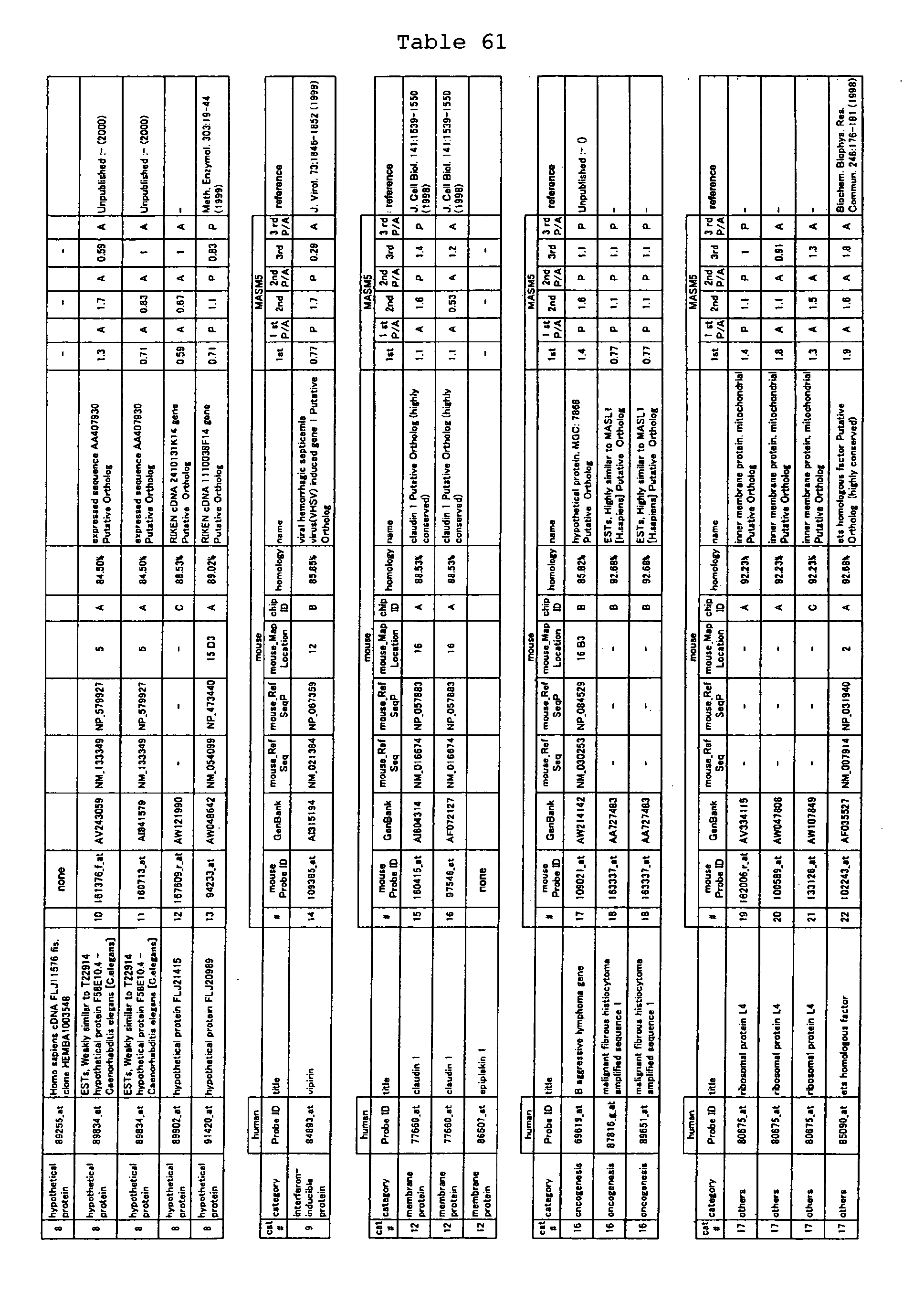

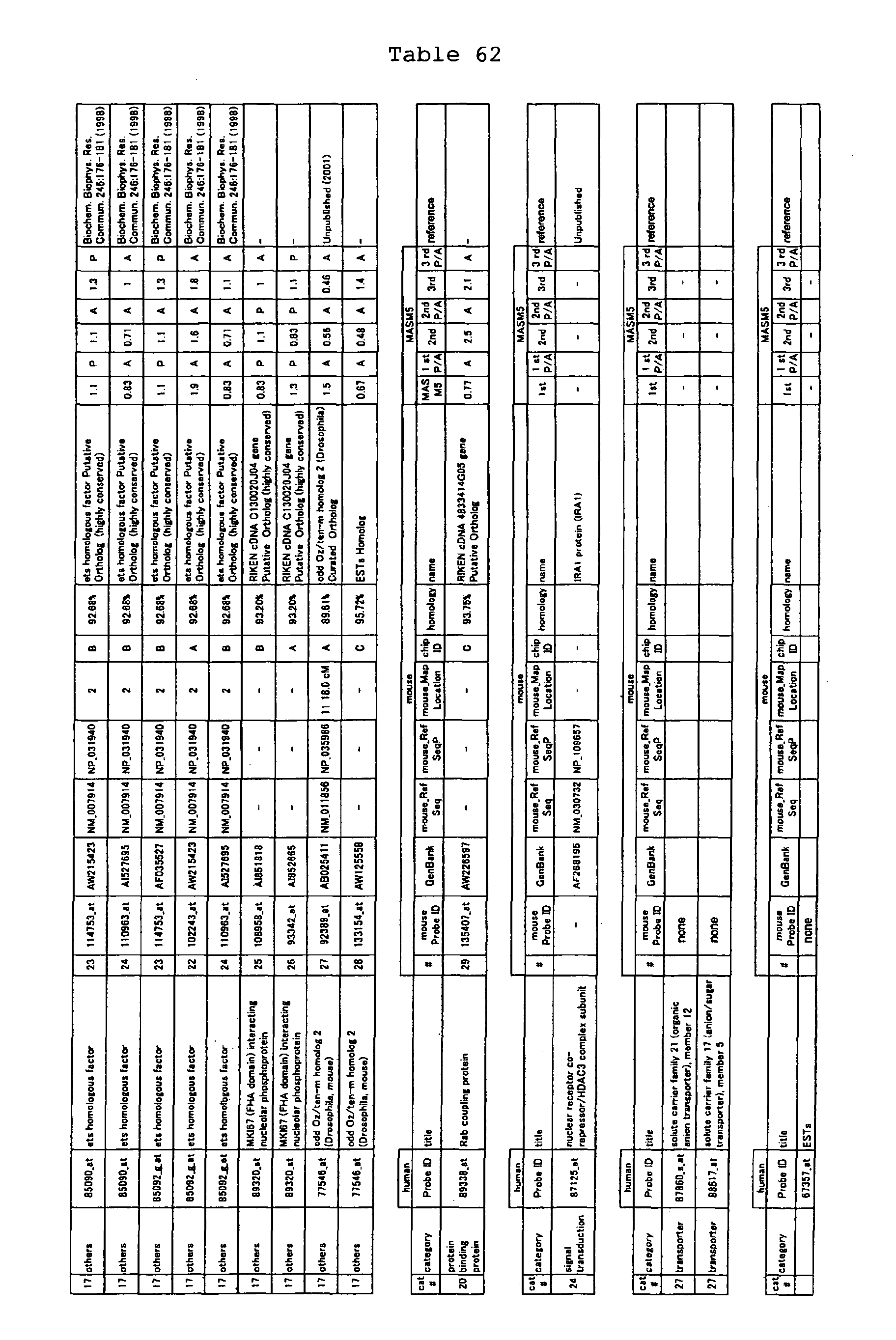

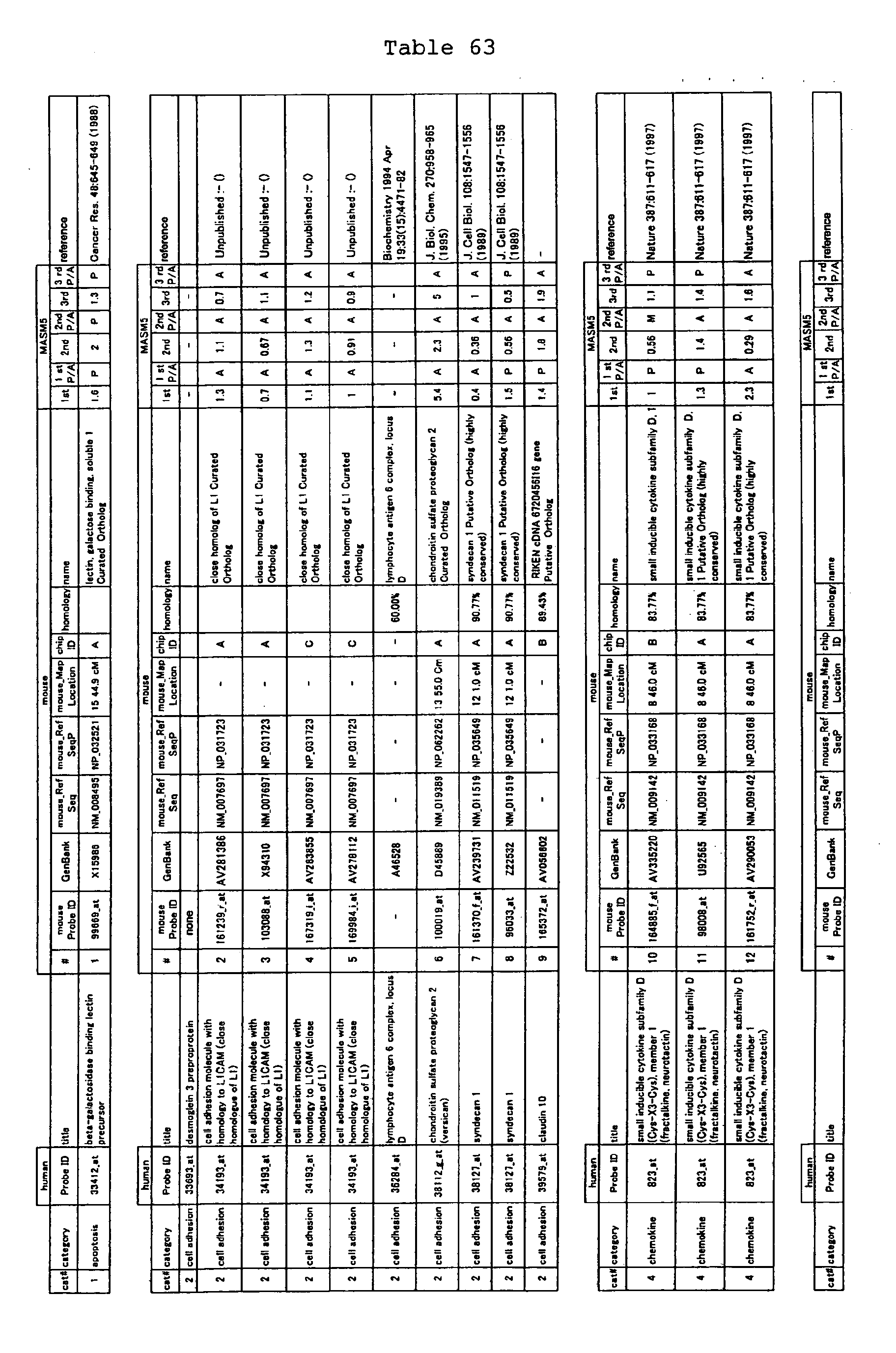

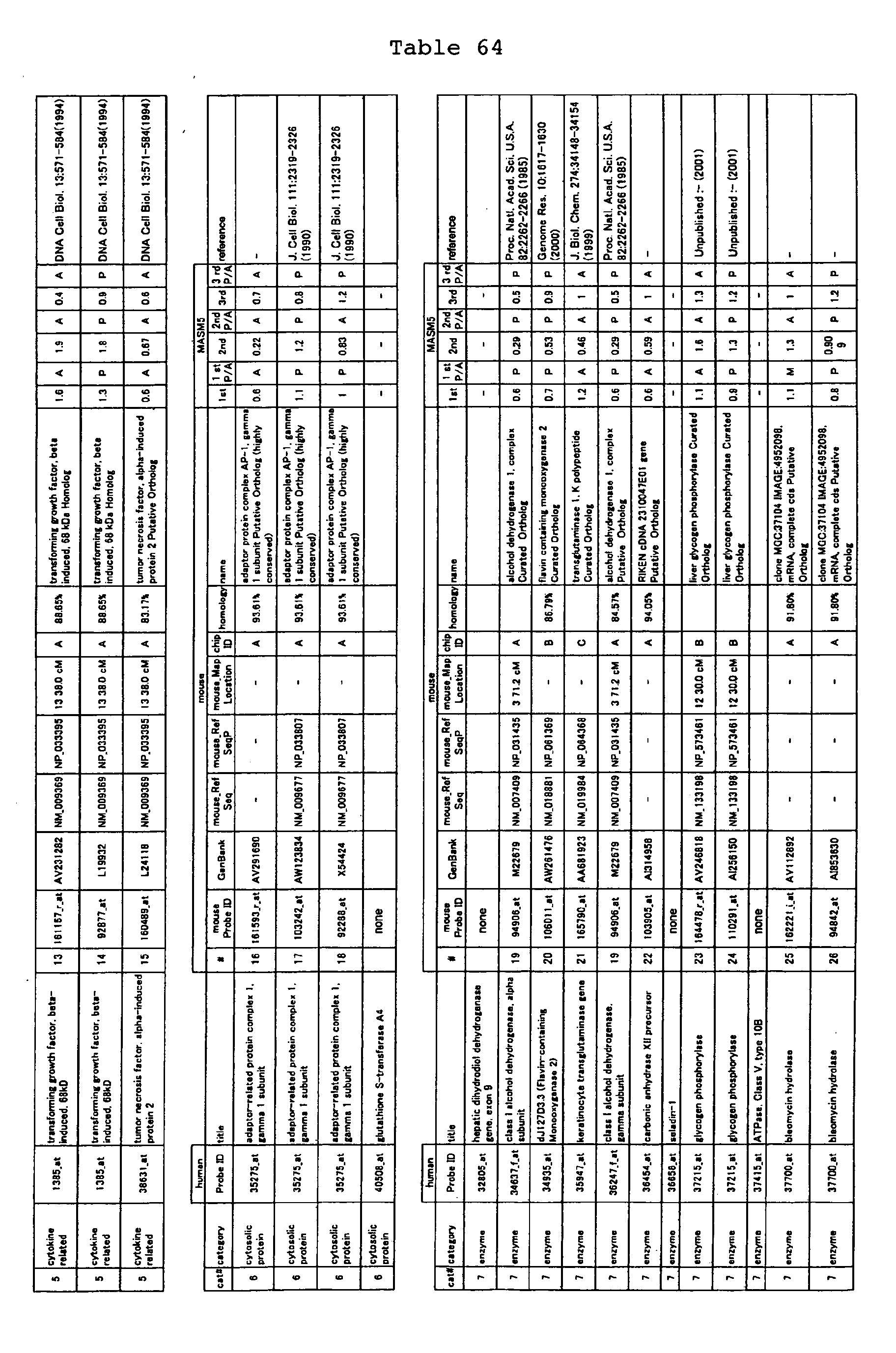

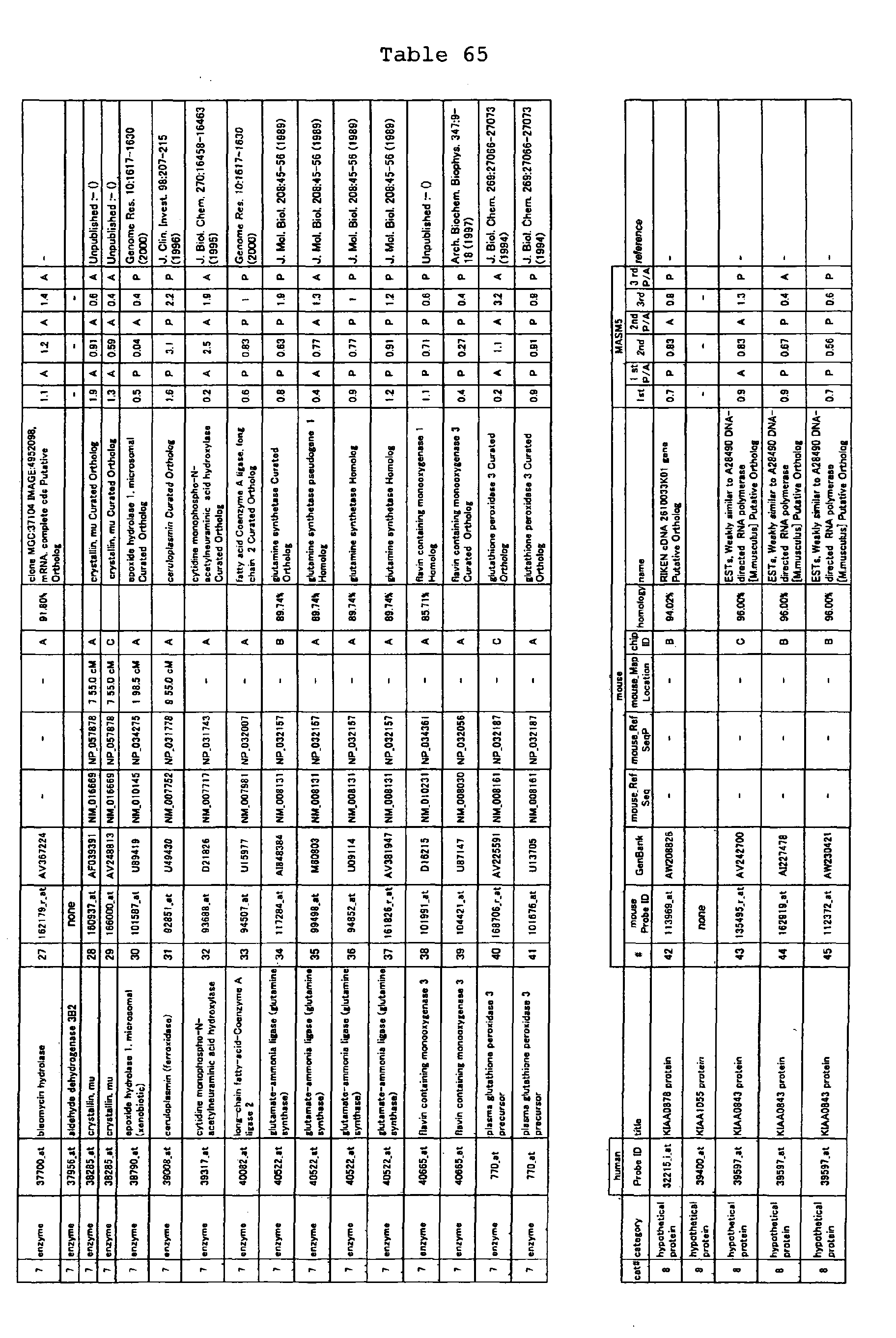

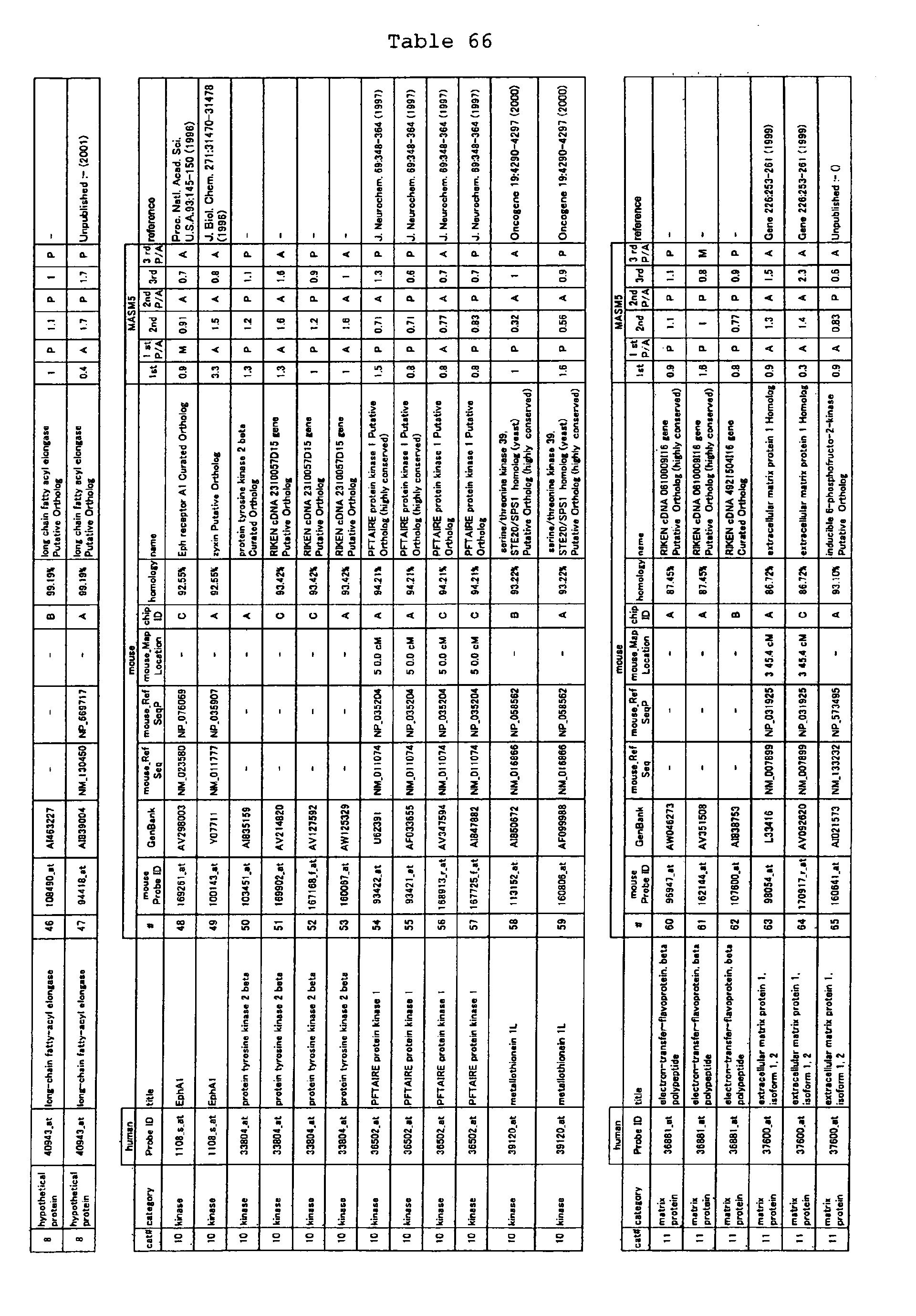

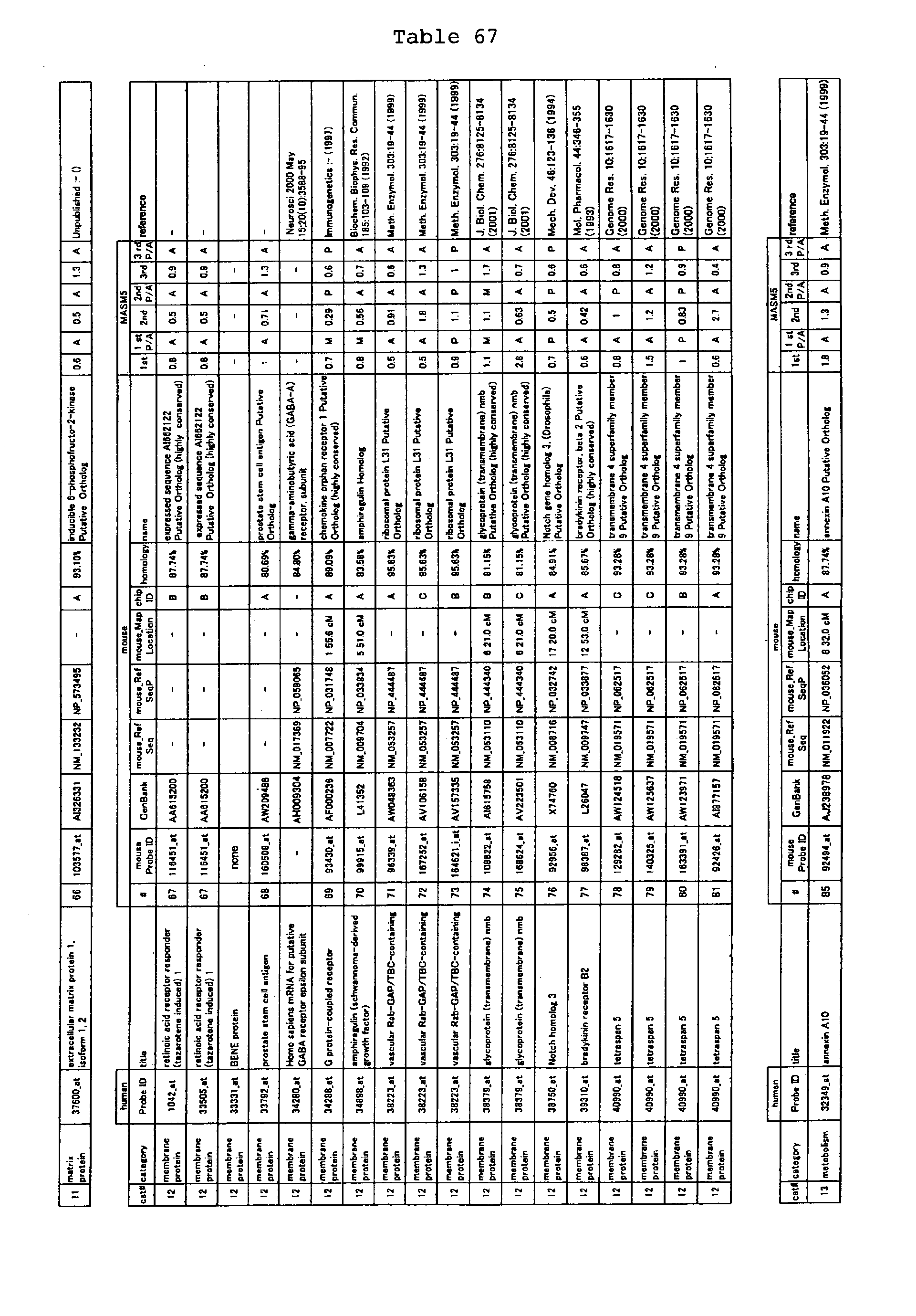

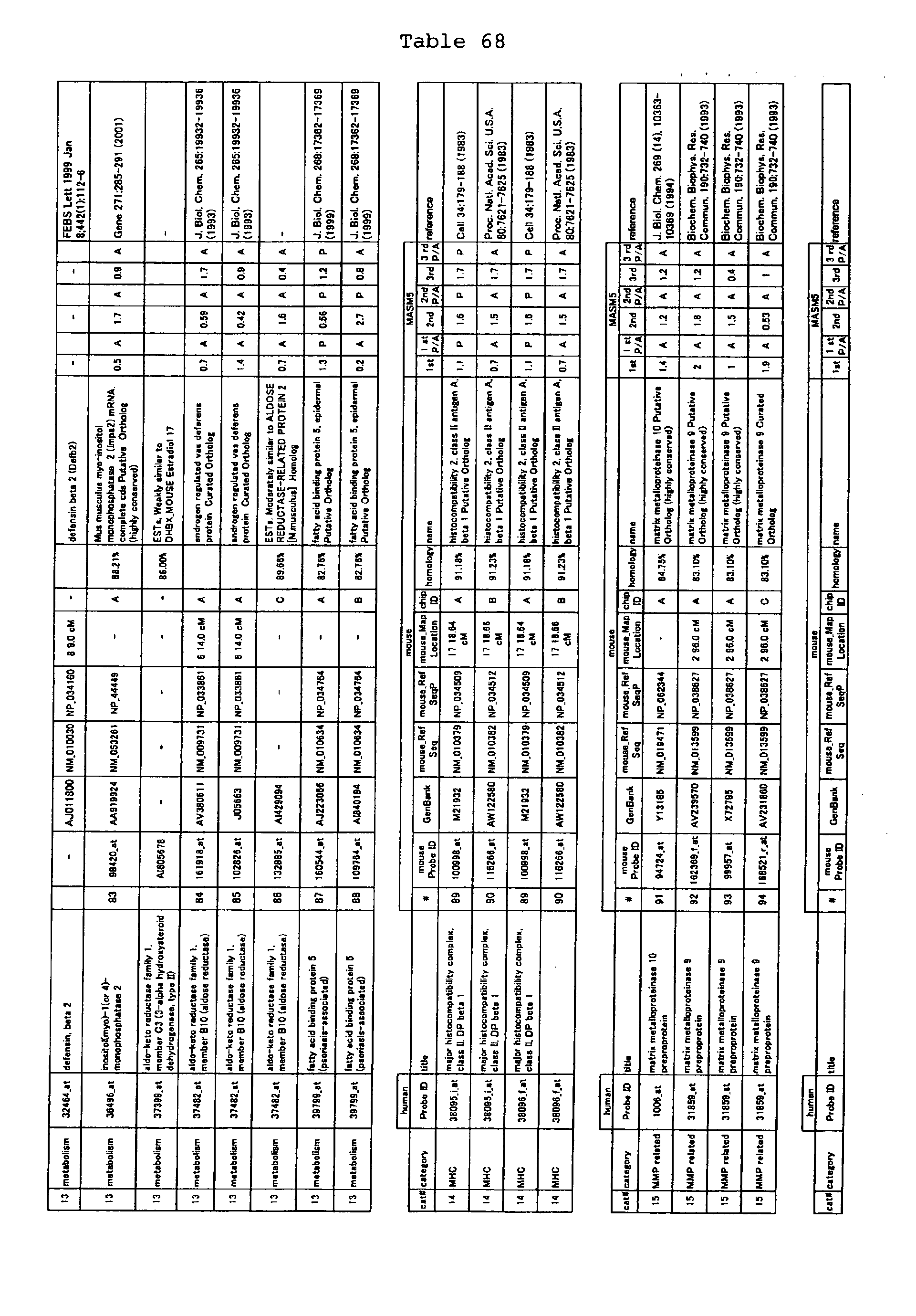

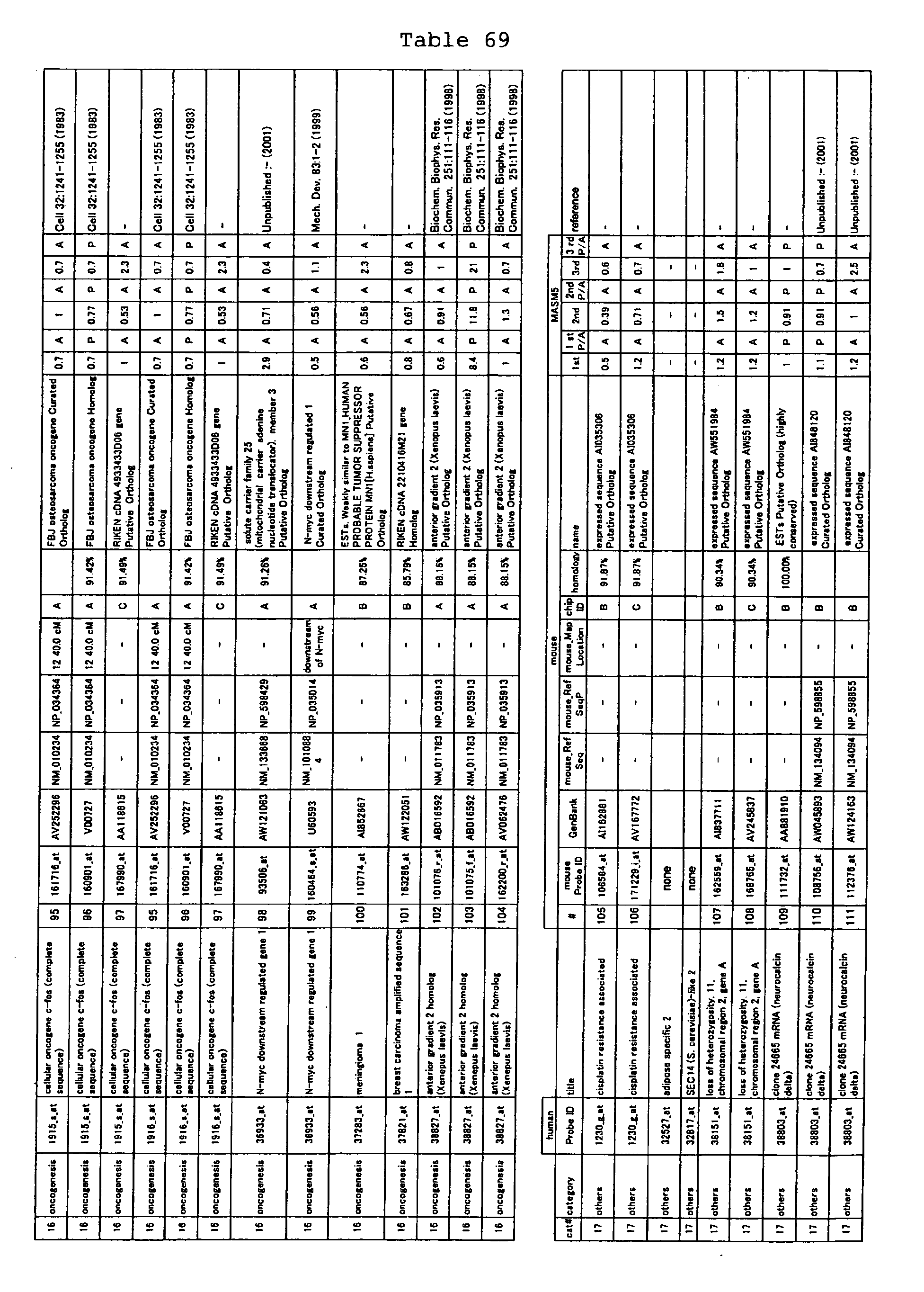

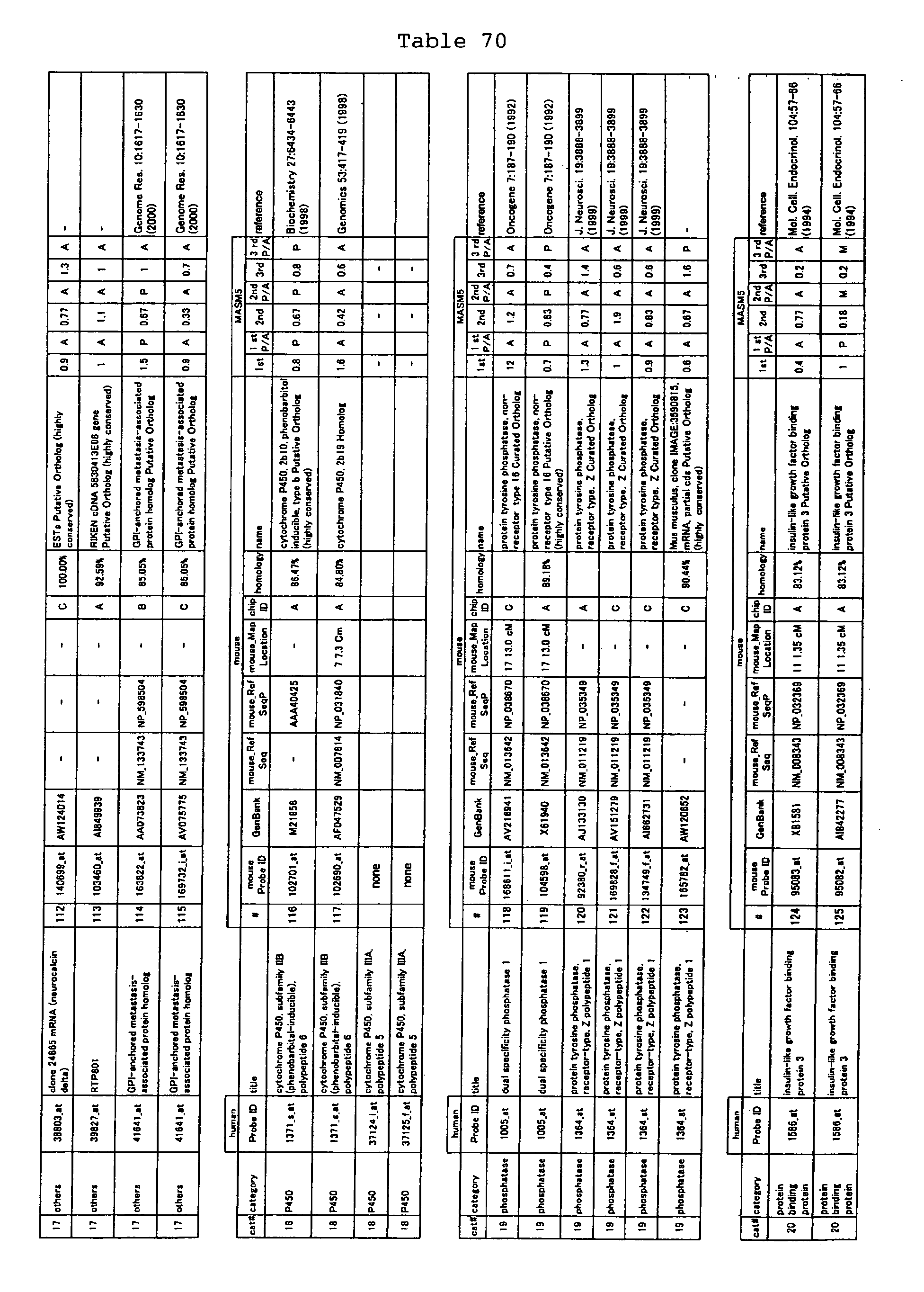

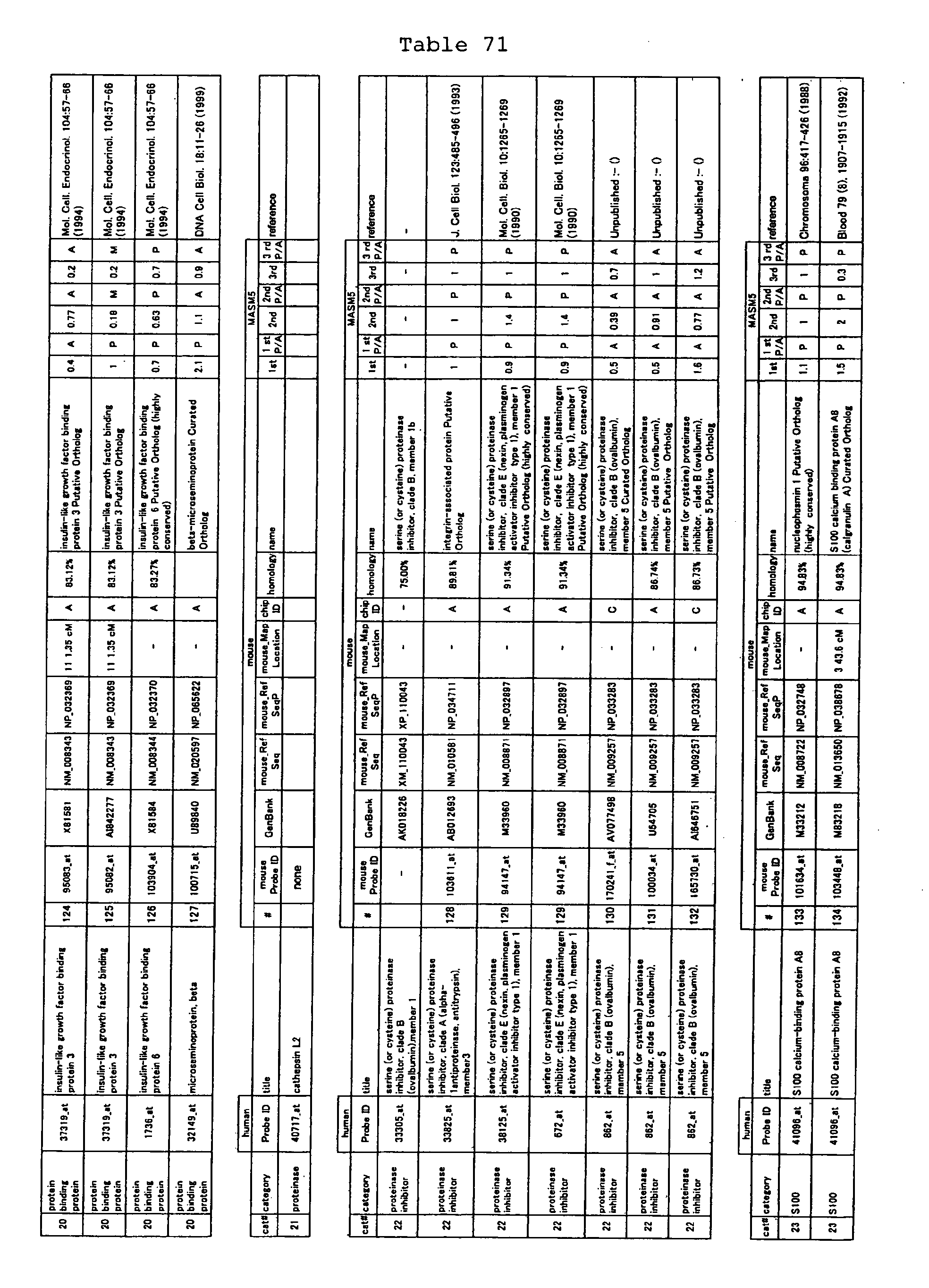

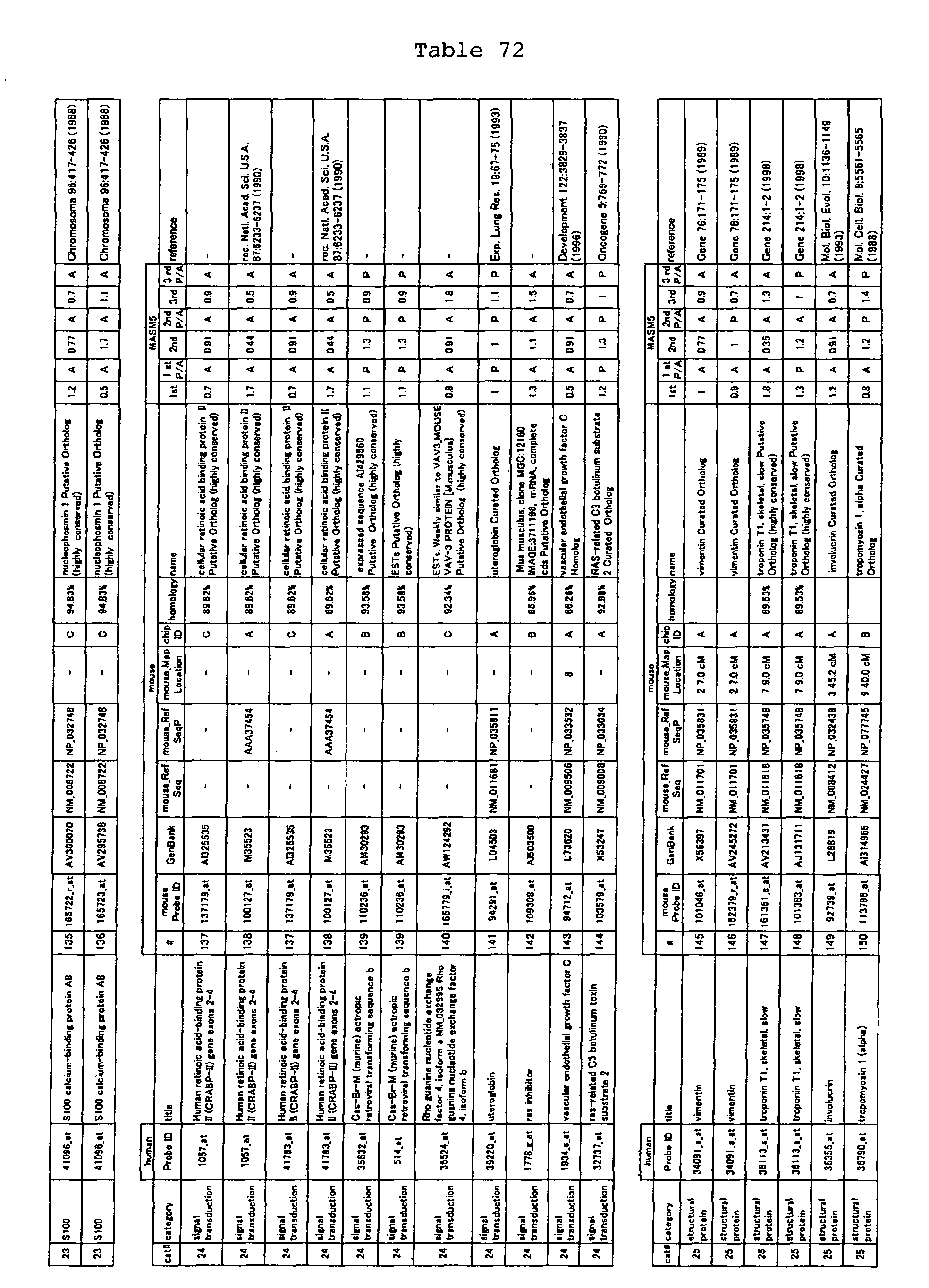

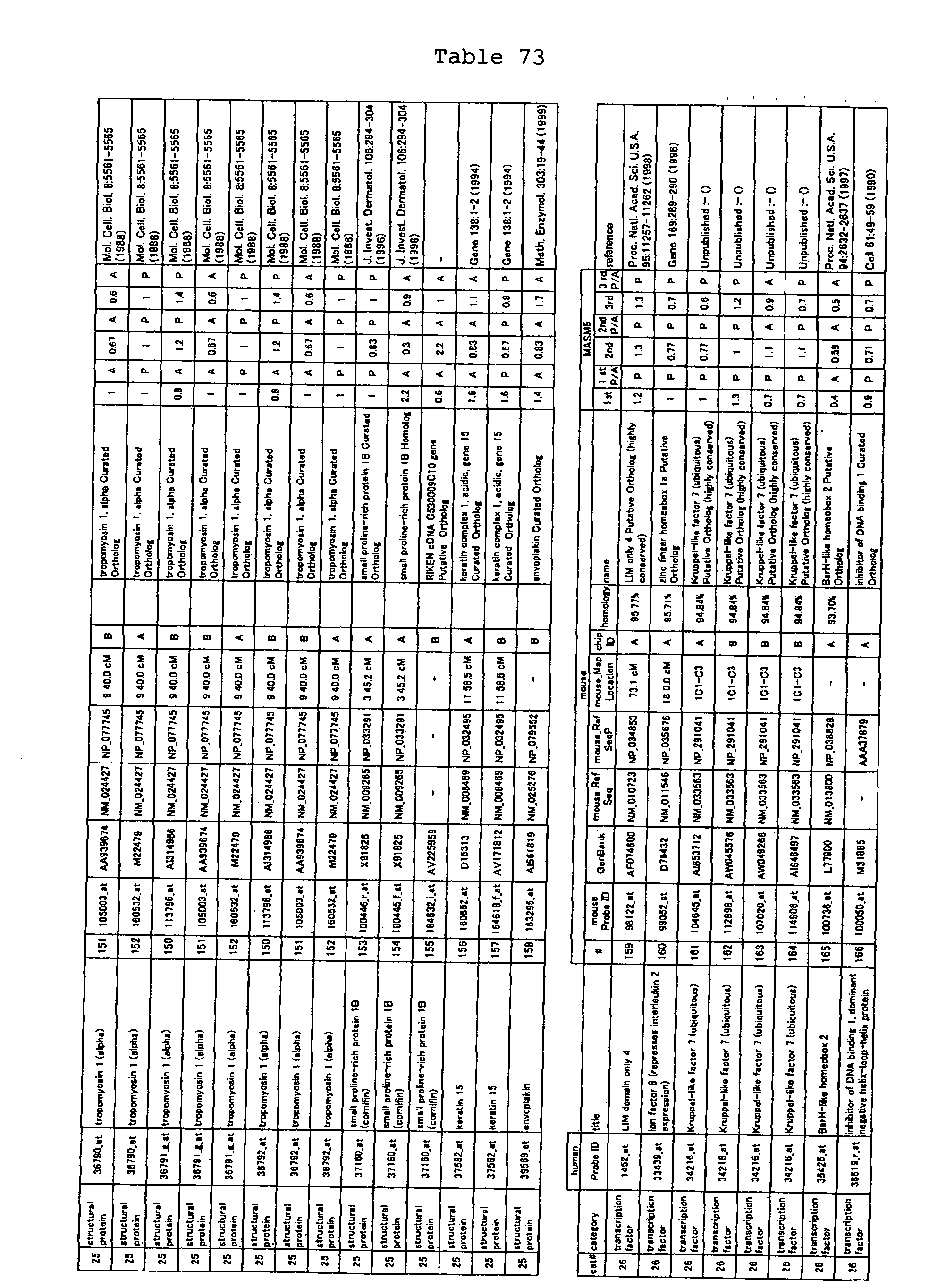

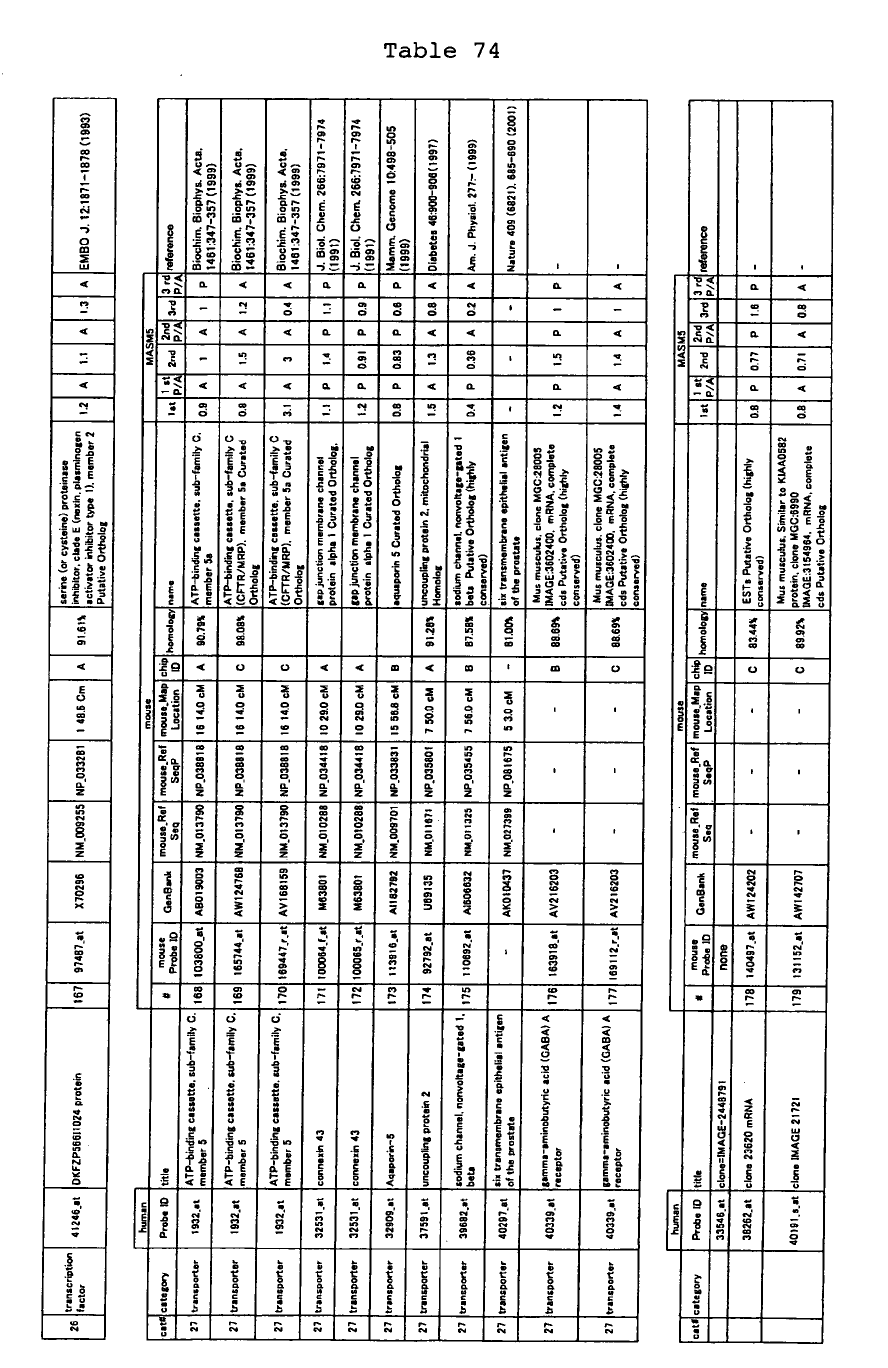

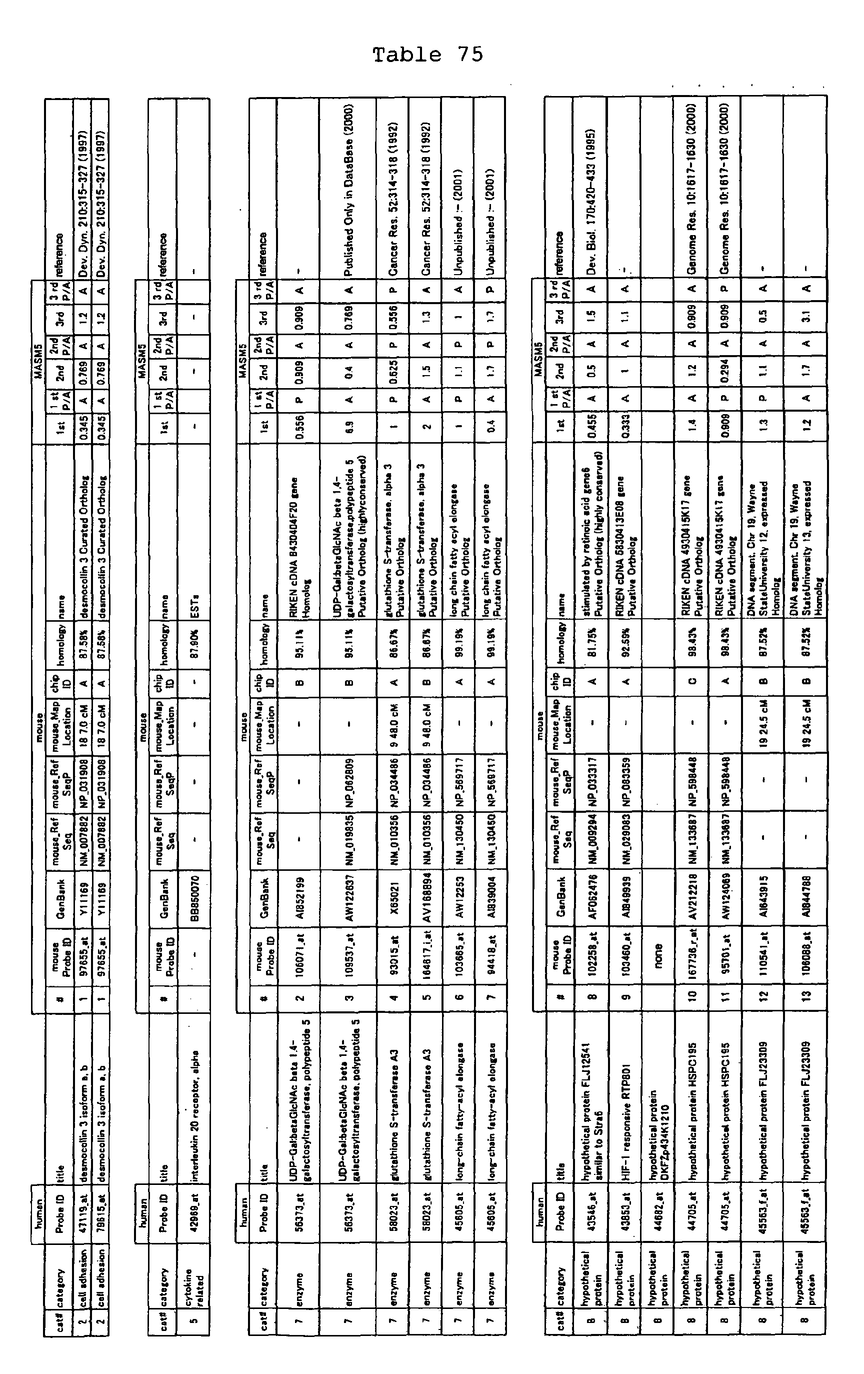

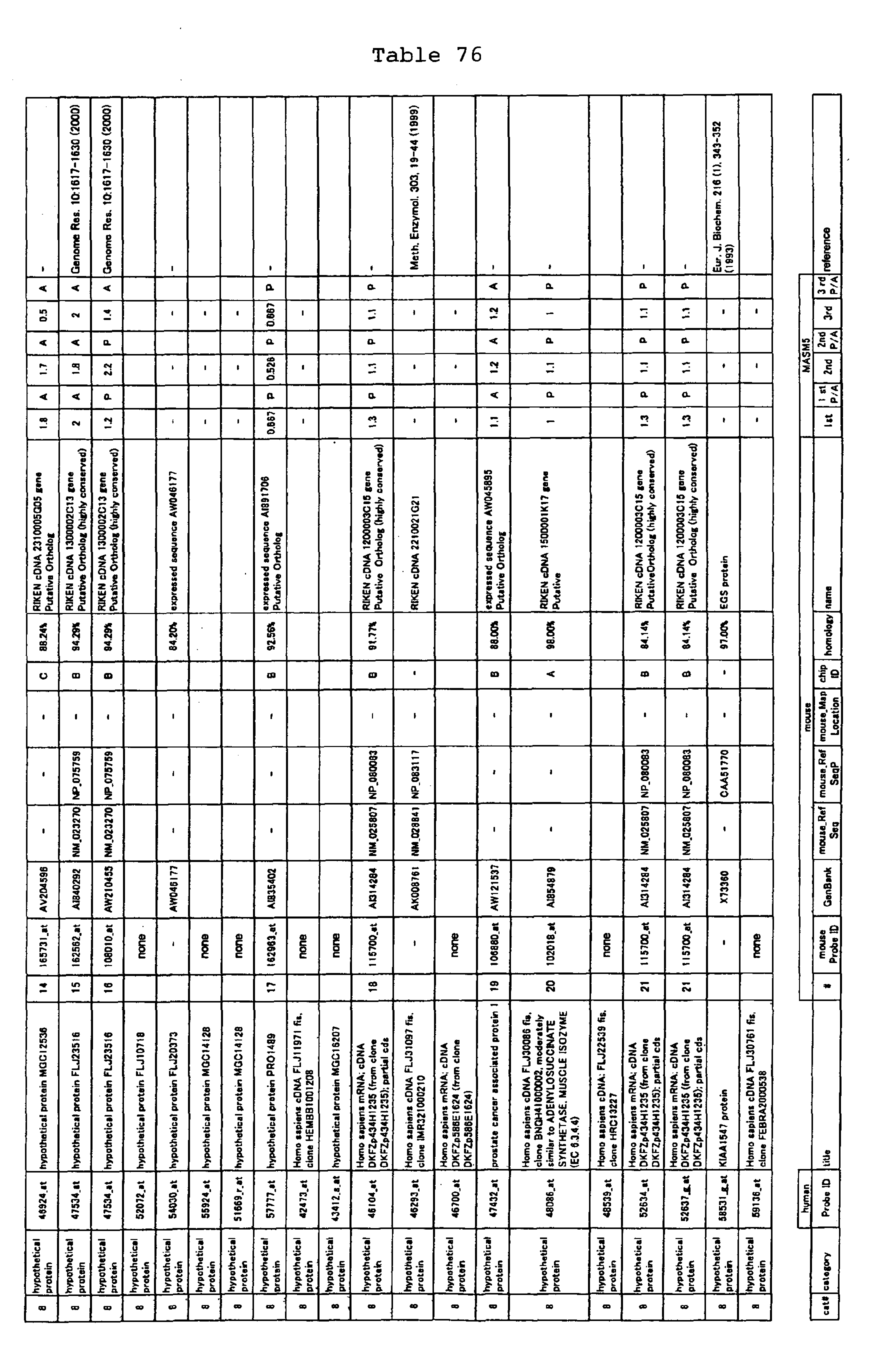

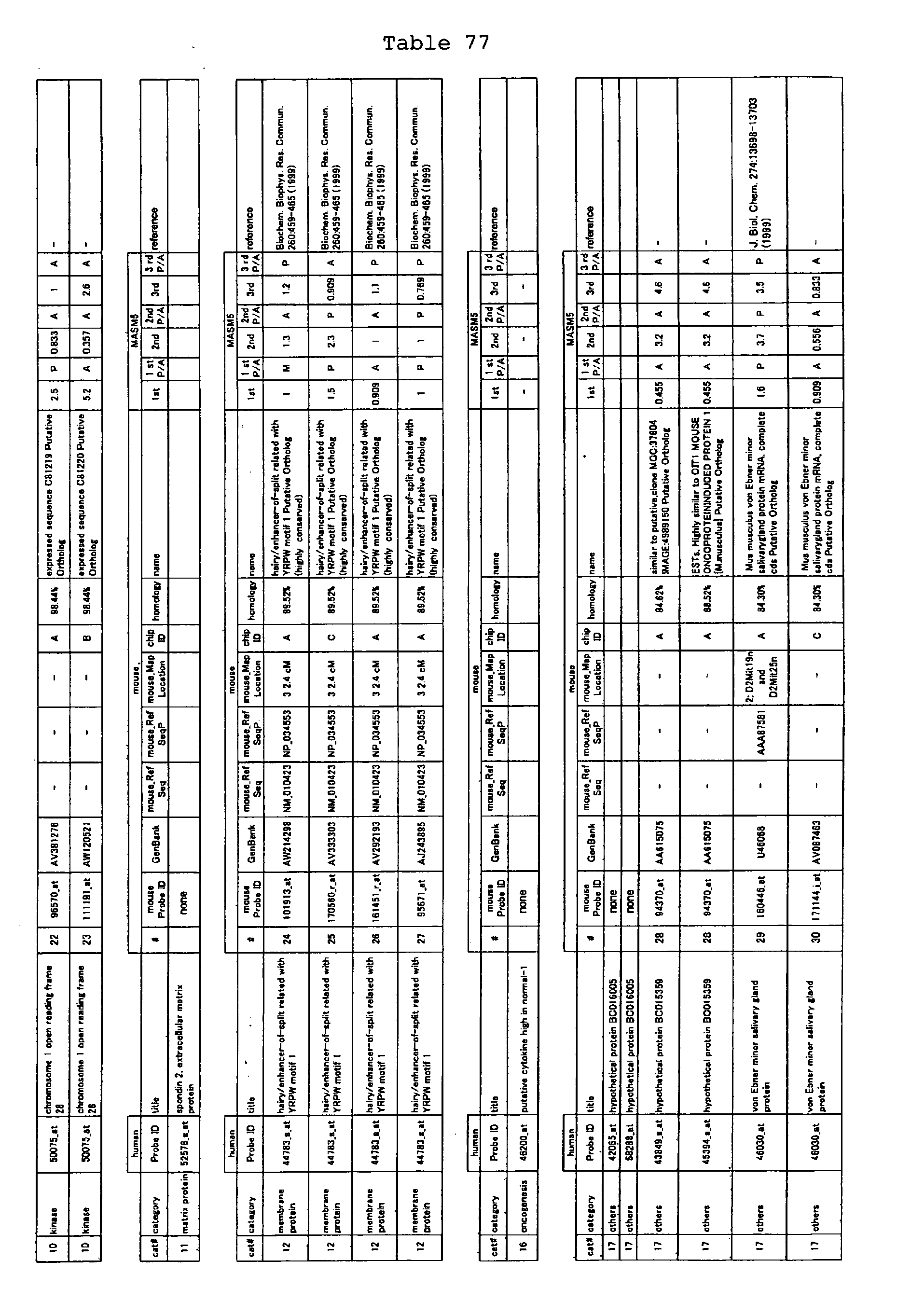

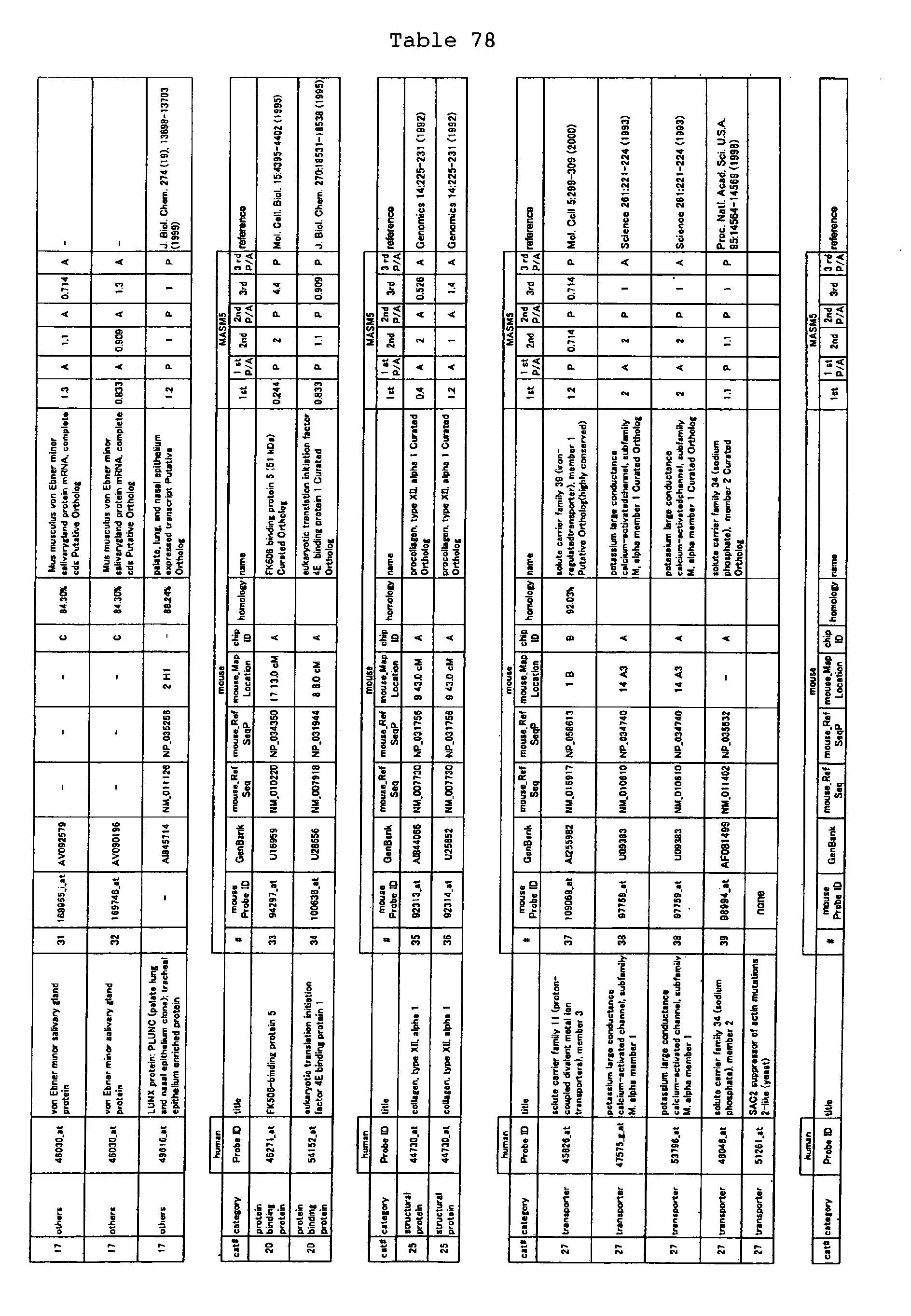

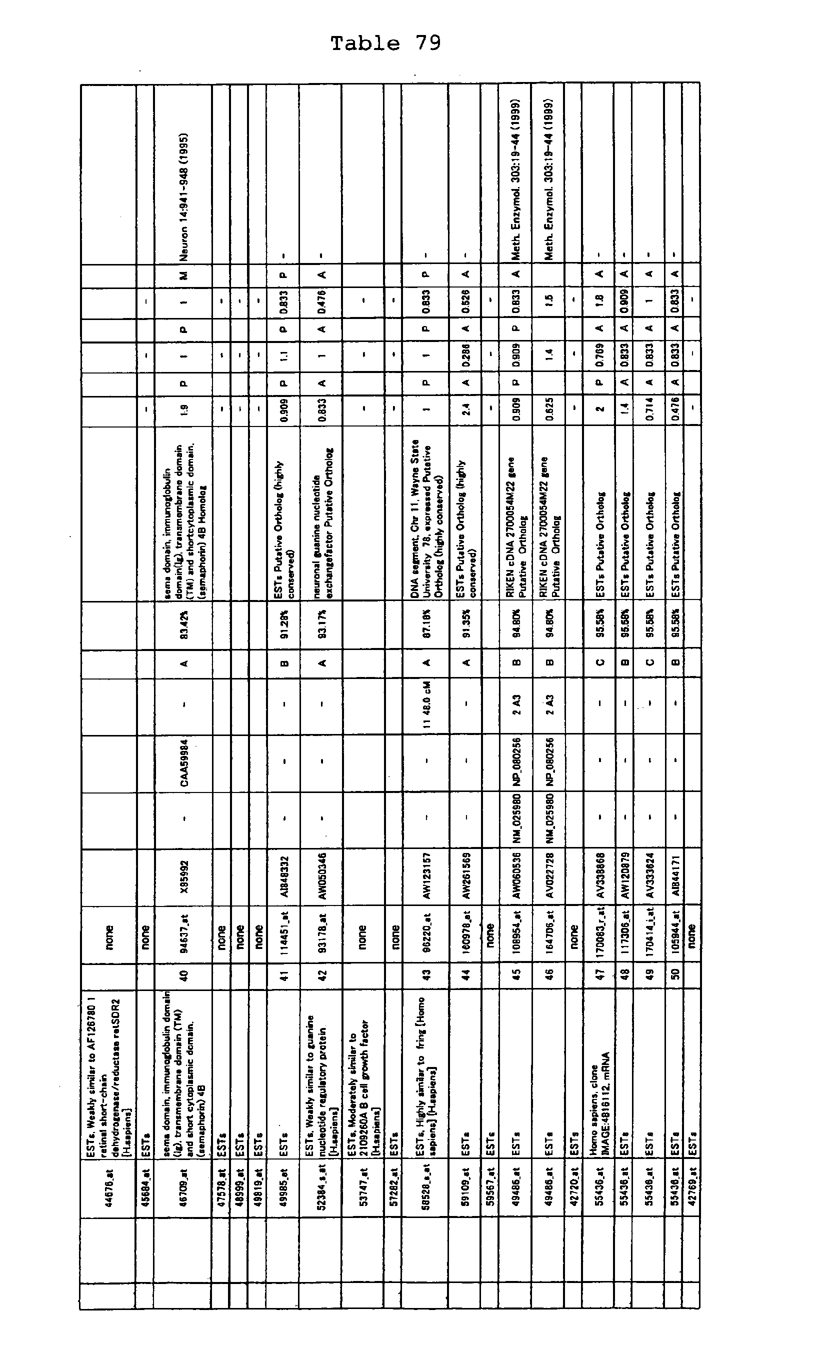

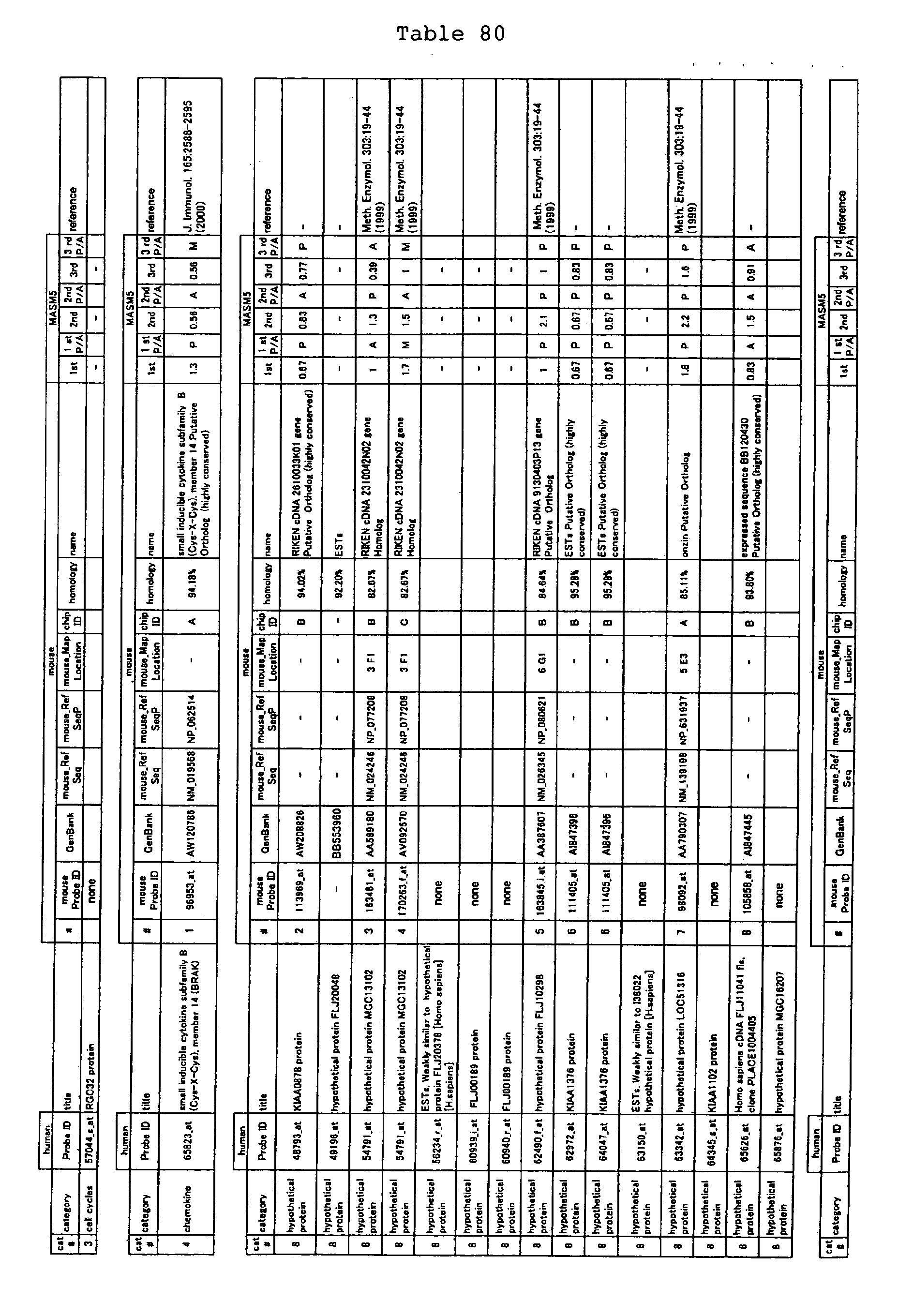

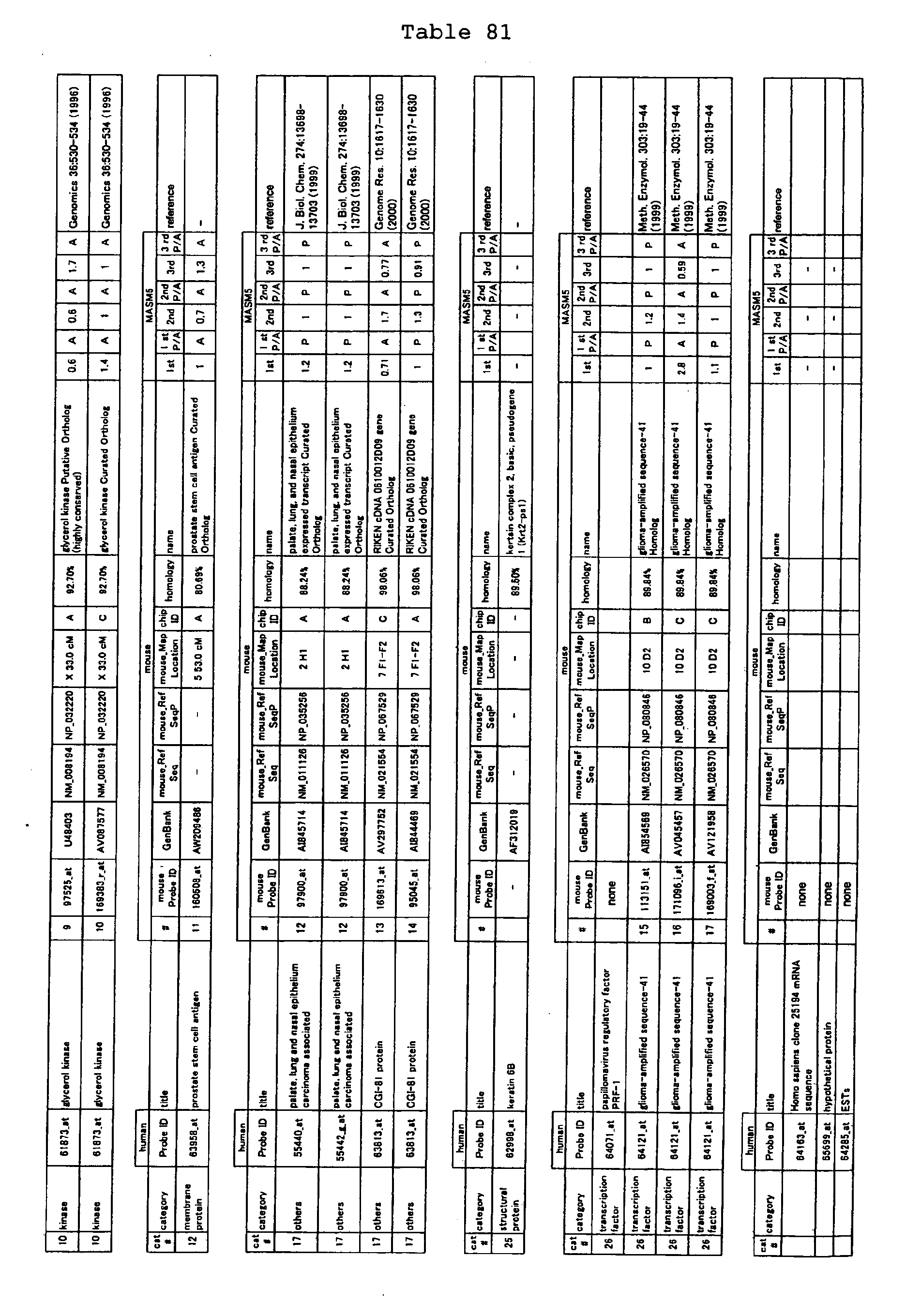

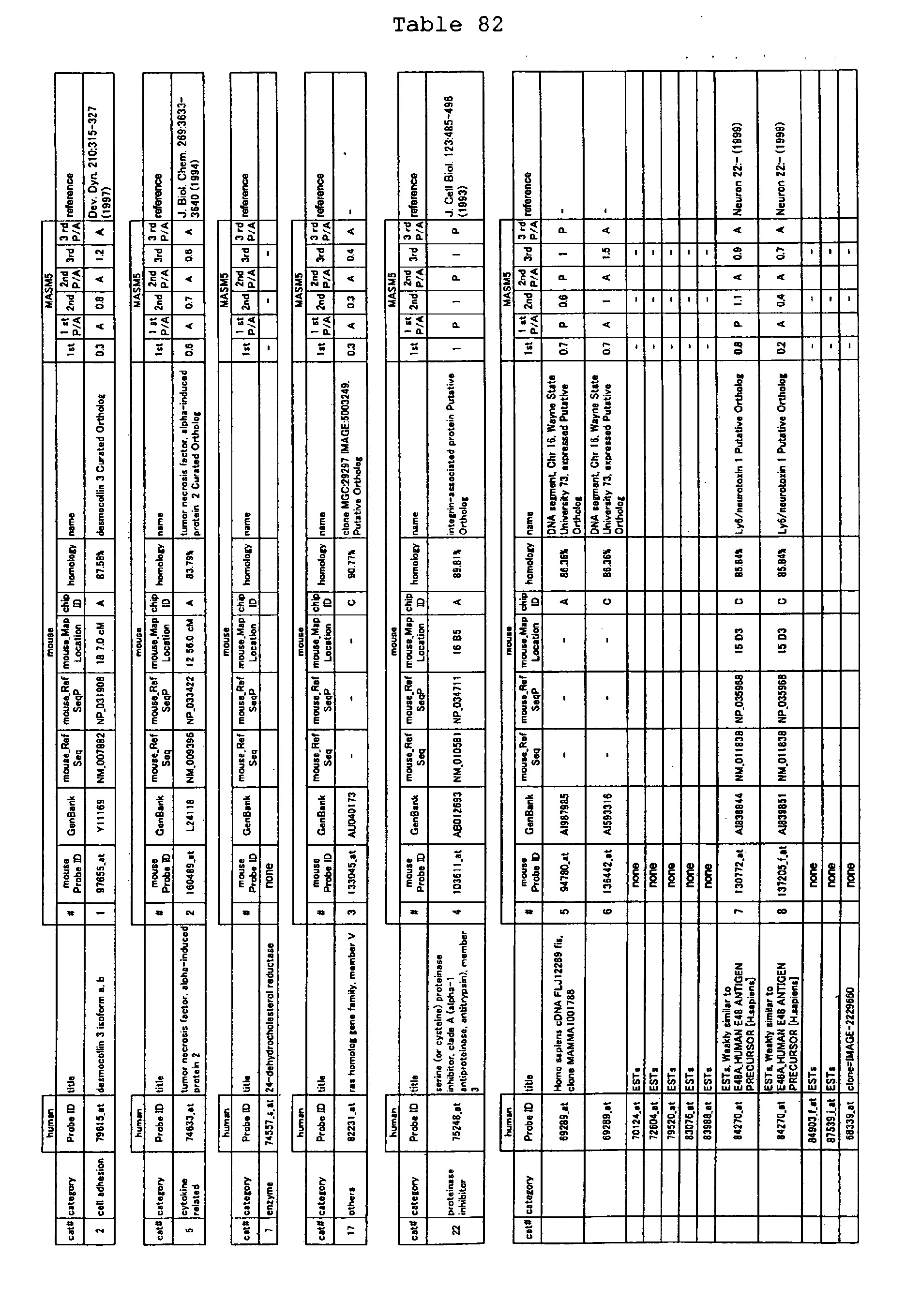

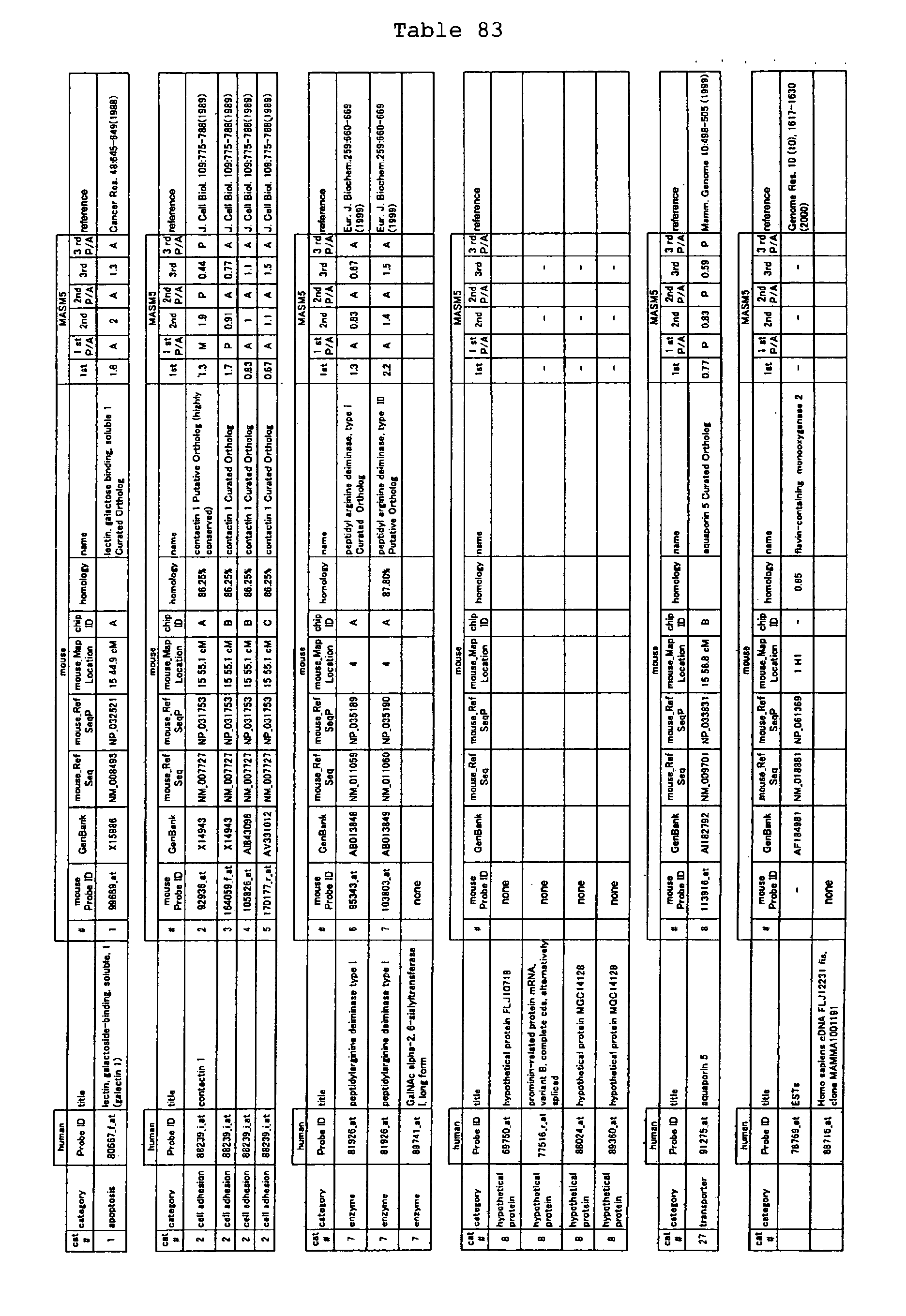

In addition, the nucleotide sequences and the amino acid sequences of the mouse counterparts are shown in SEQ ID NOs: 954 to 1635. The details are as follows.
The mouse counterparts of the human genes whose expression levels were increased by IL-13 (AI method):
954 to 1174 (nucleotide sequence)
1175 to 1375 (amino acid sequence)
The mouse counterparts of the human genes whose expression levels were decreased by IL-13 (IMM method):
1376 to 1505 (nucleotide sequence)
1506 to 1635 (amino acid sequence)
With respect to each mouse counterpart, Probe ID, GenBank Accession No. , Ref SEQ NO, and the corresponding SEQ ID NO in the Sequence Listing are shown in Tables 84 to 113.

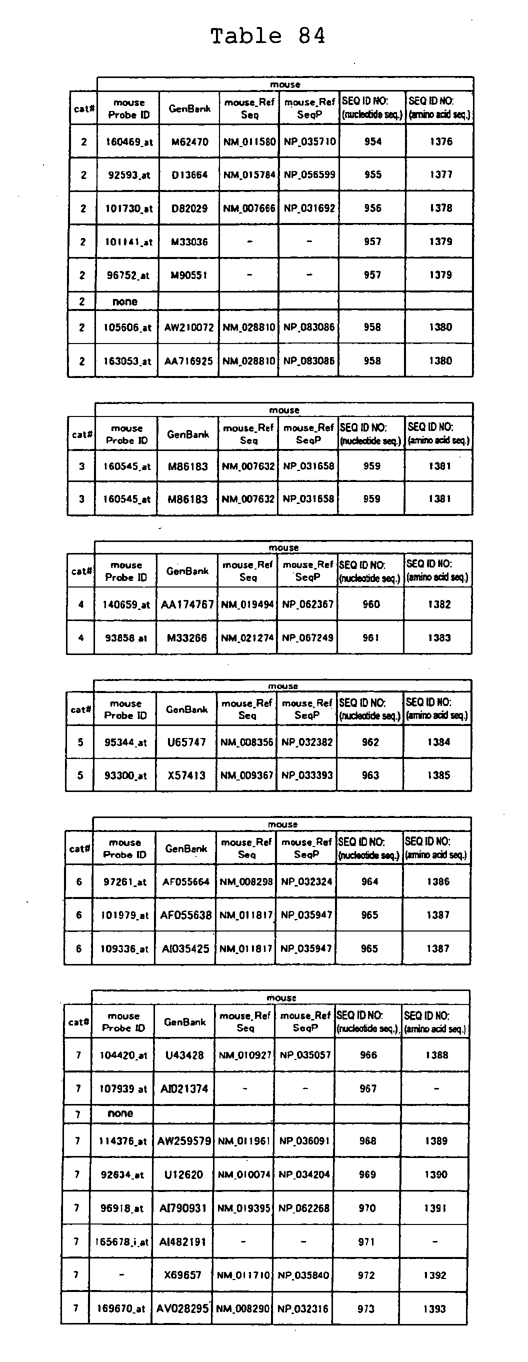

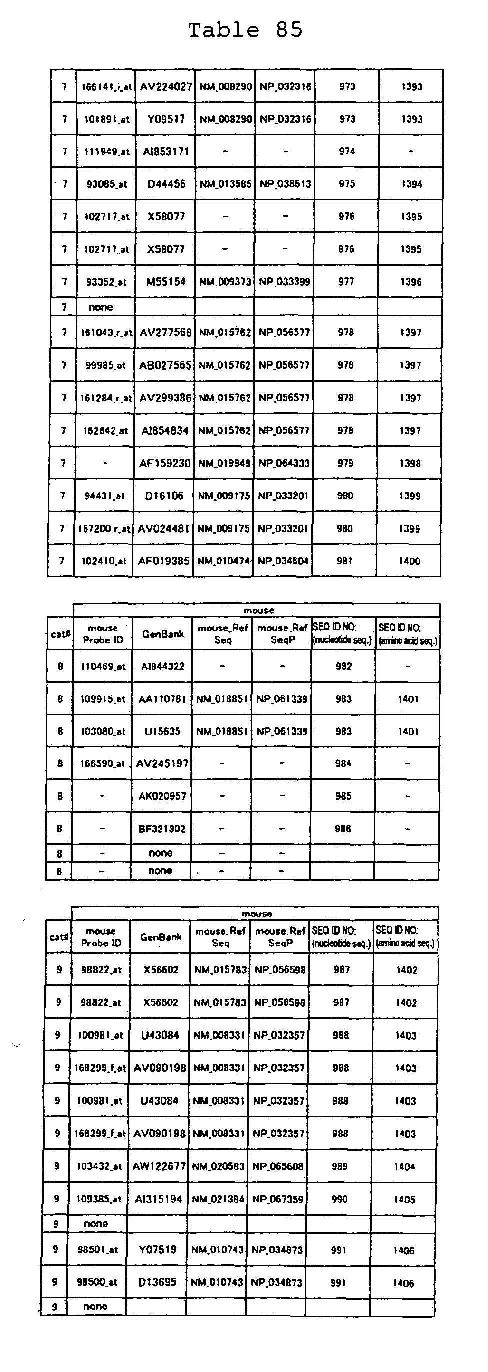

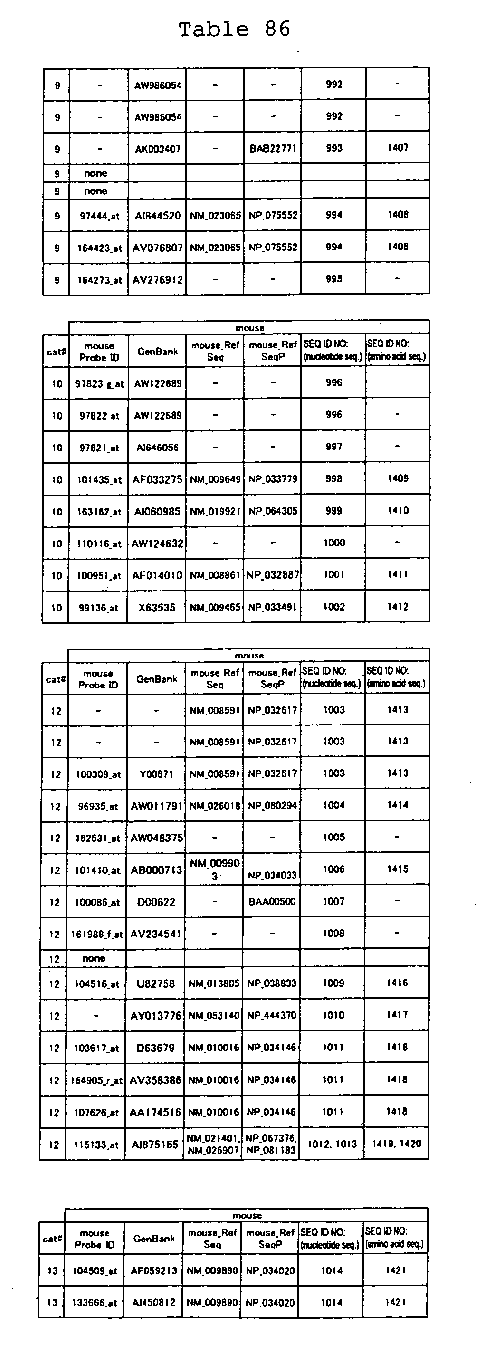

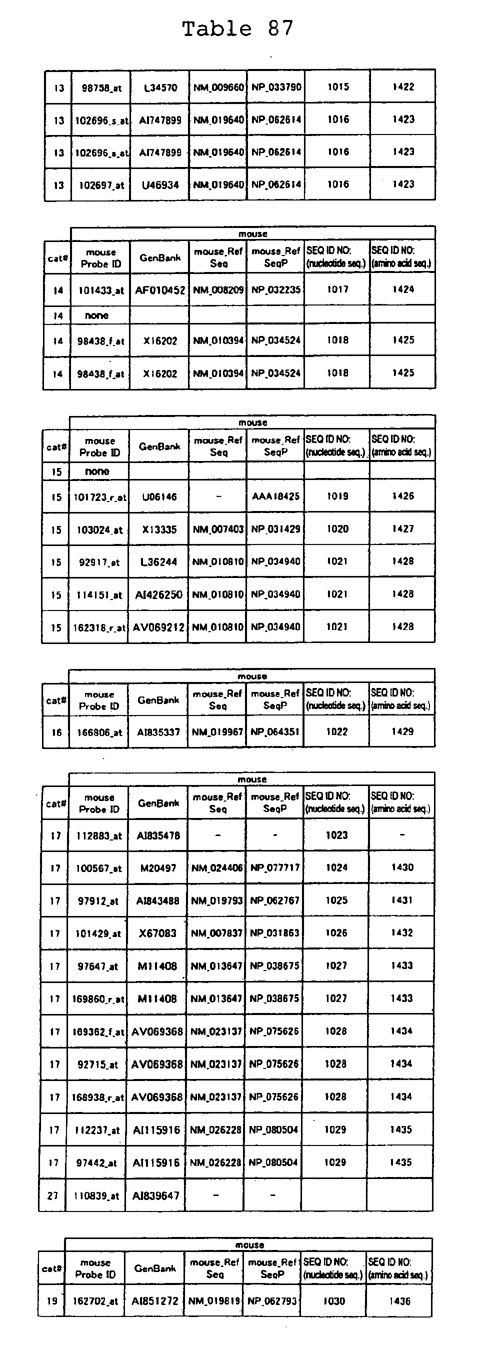

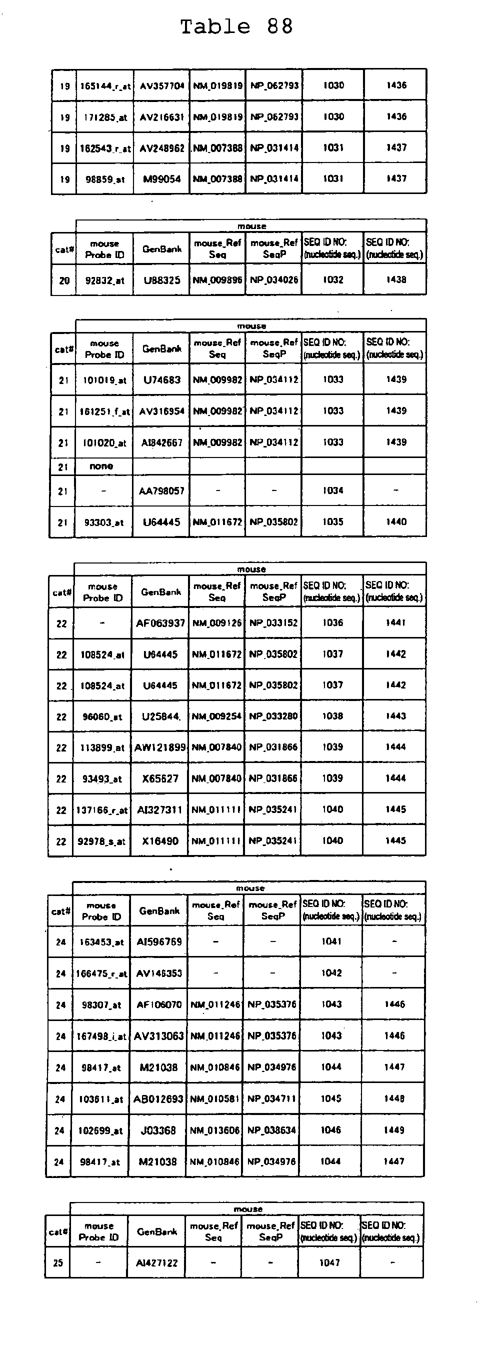

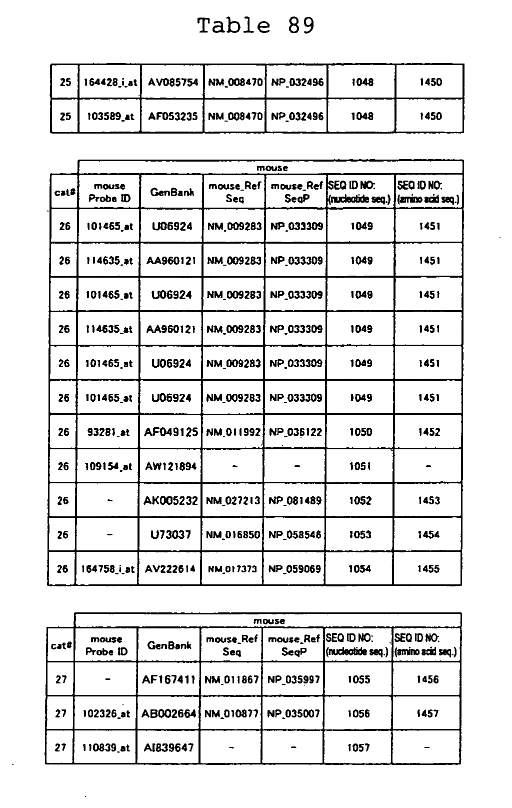

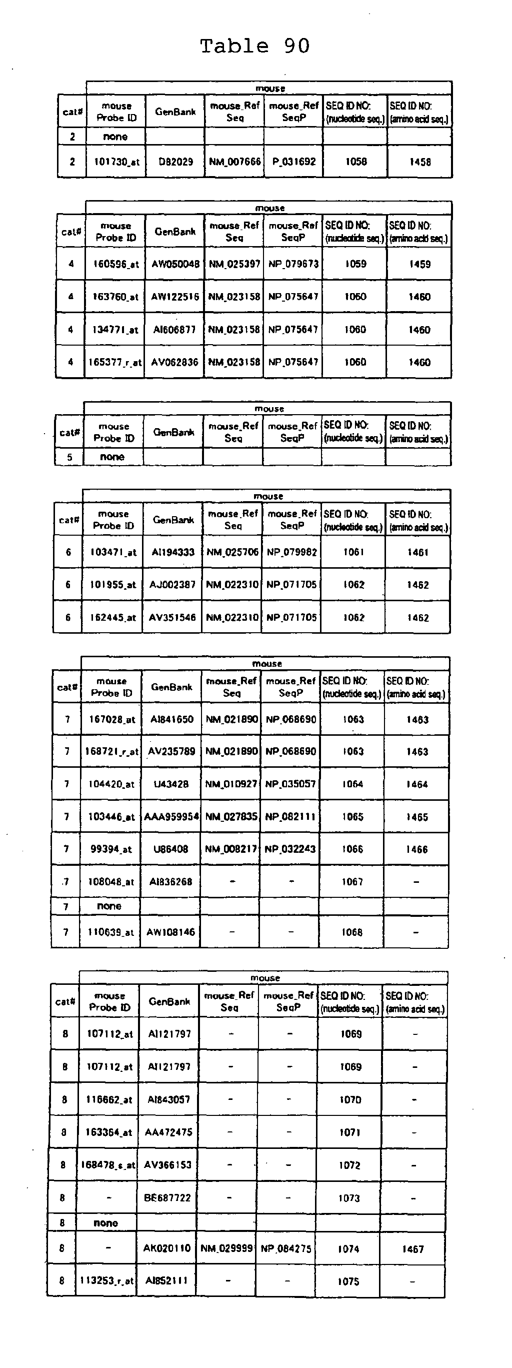

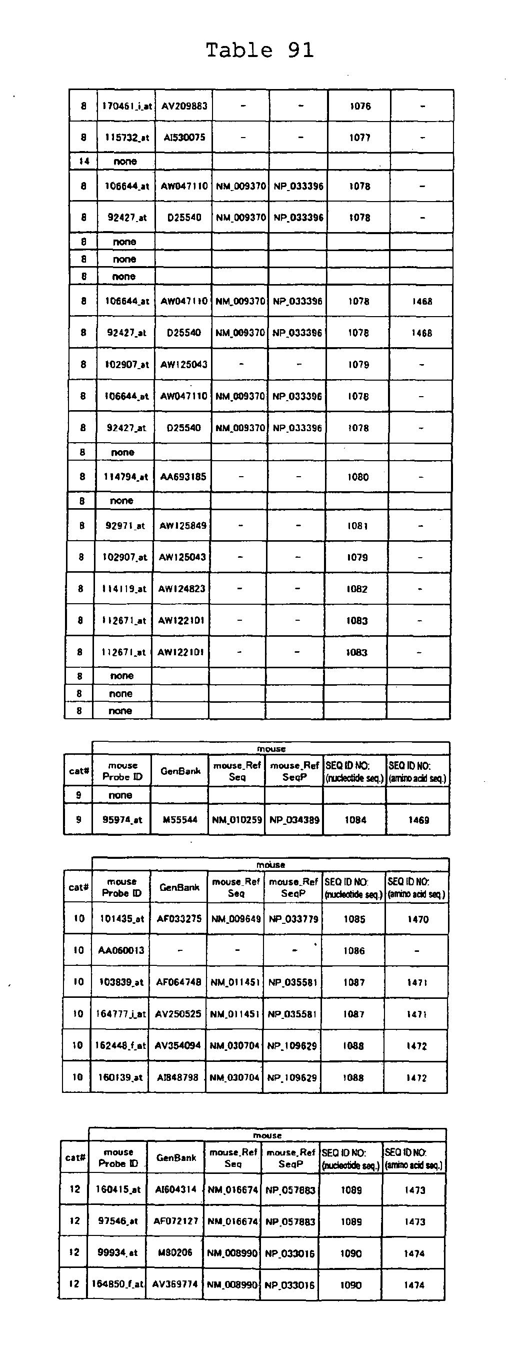

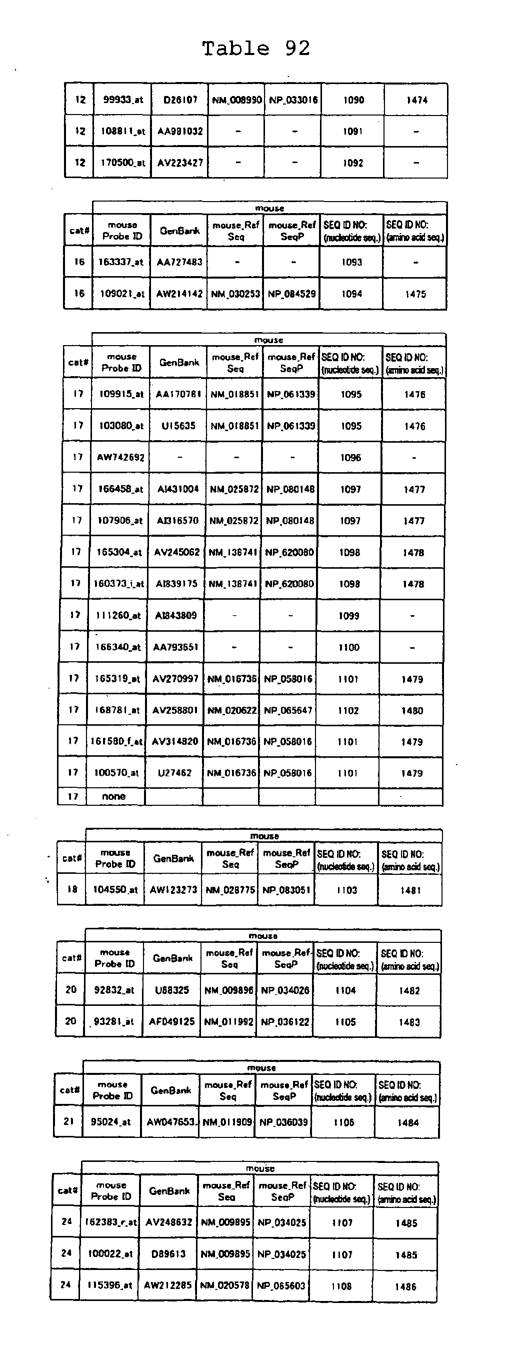

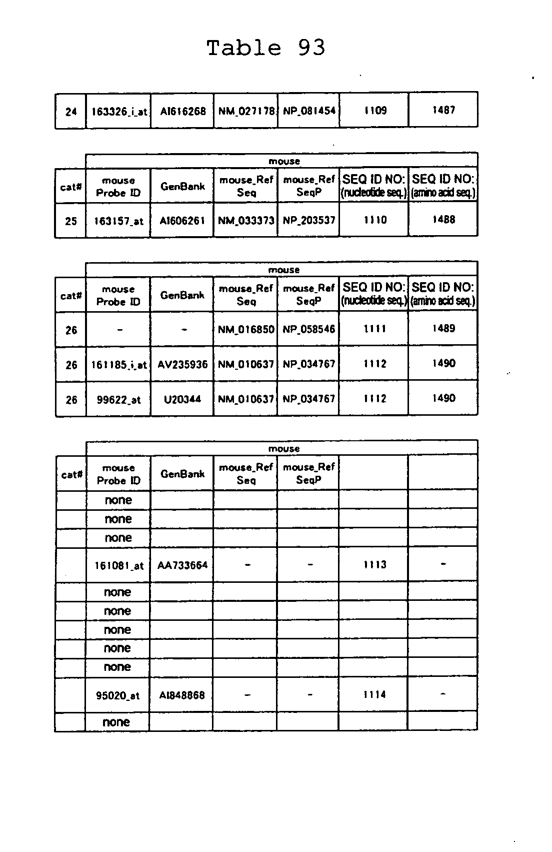

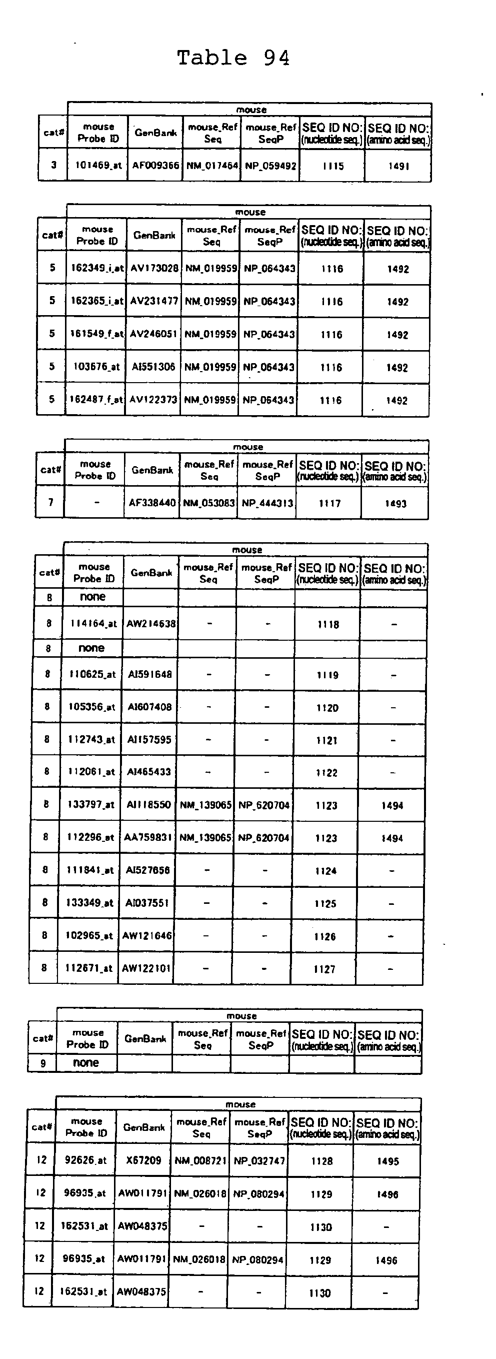

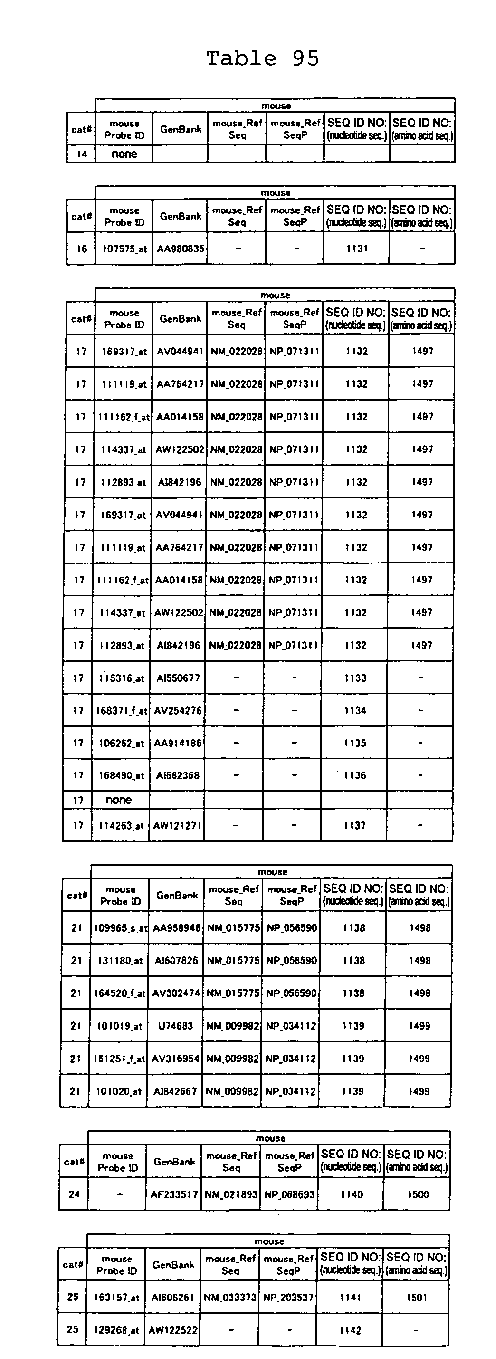

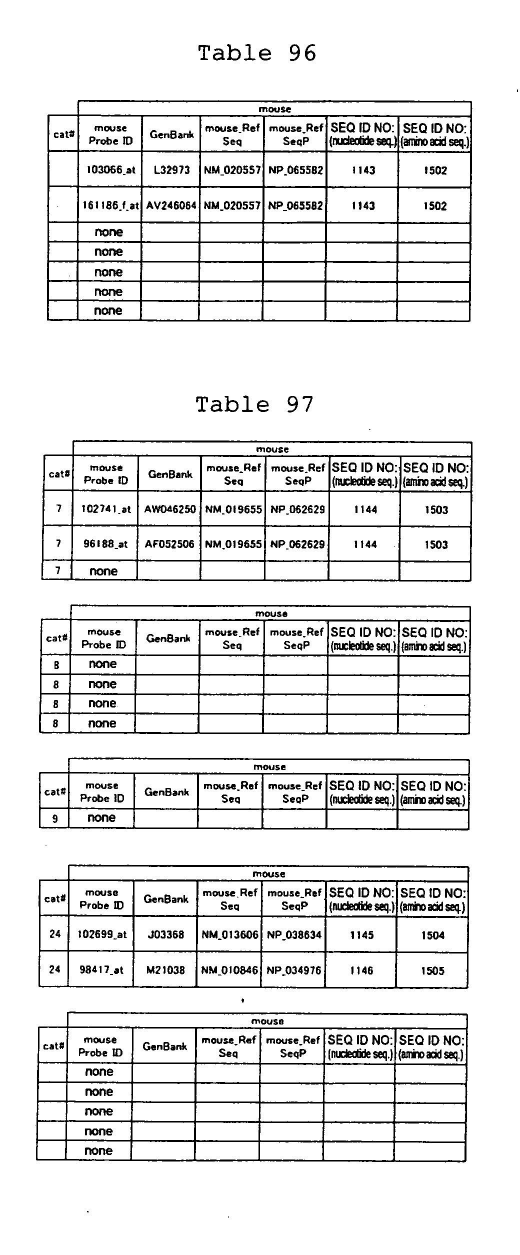

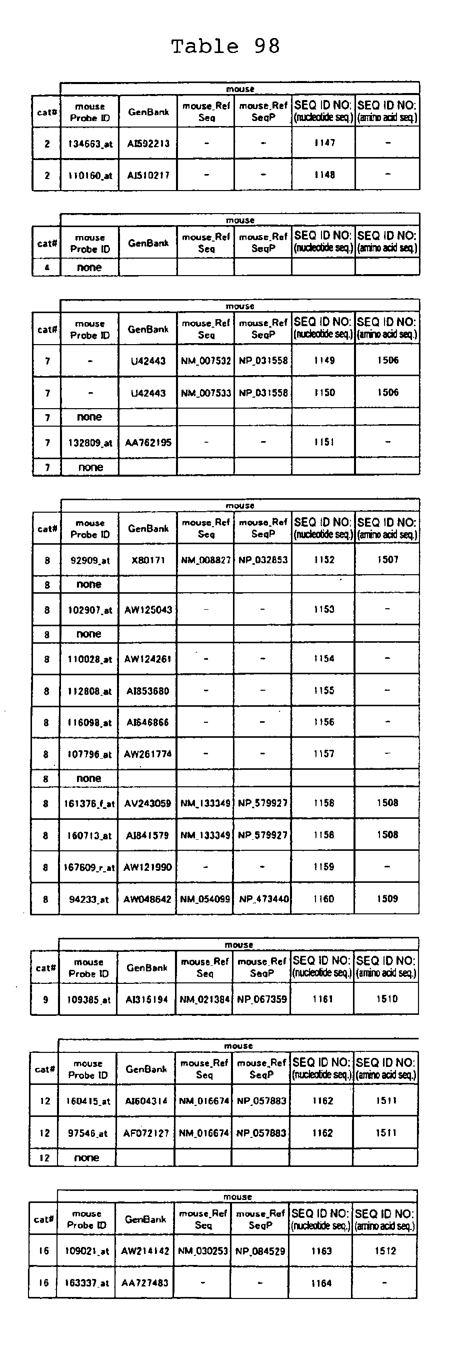

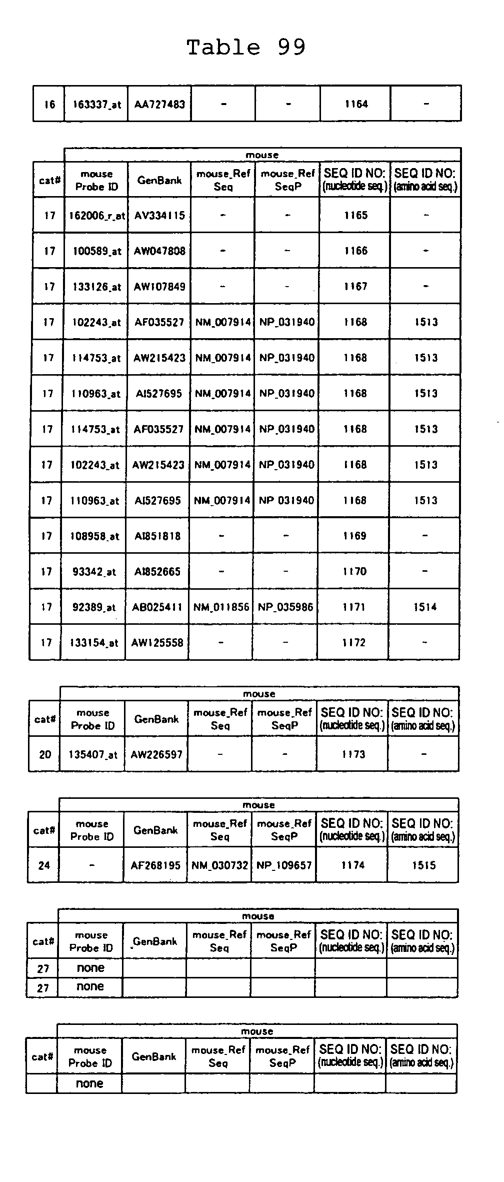

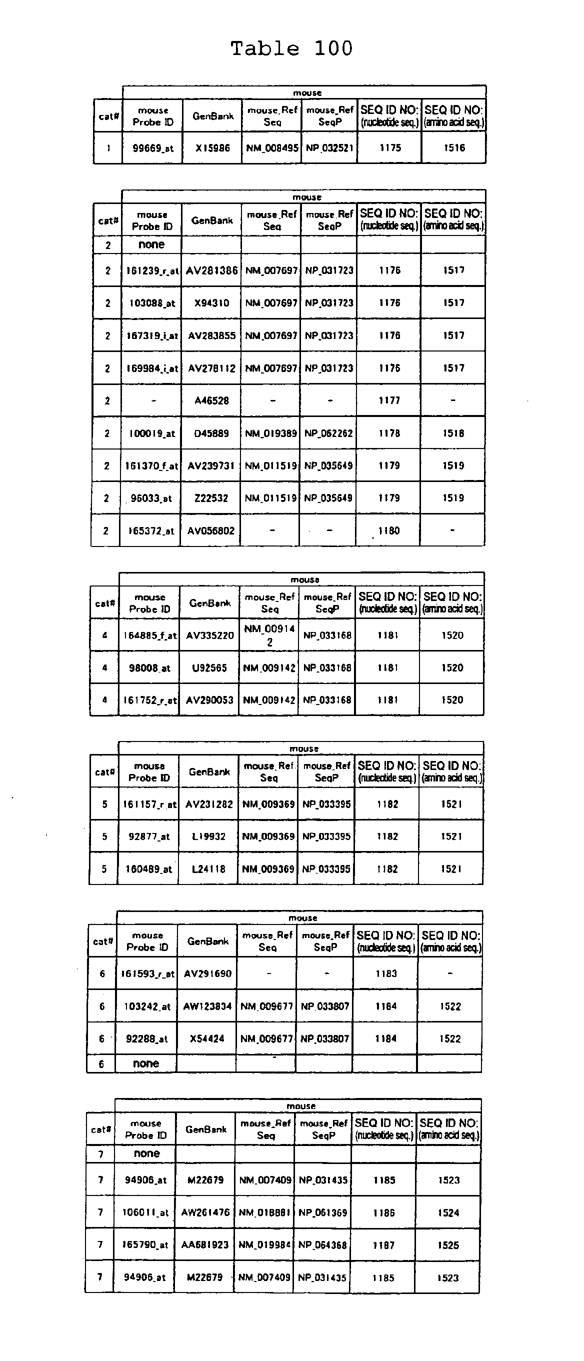

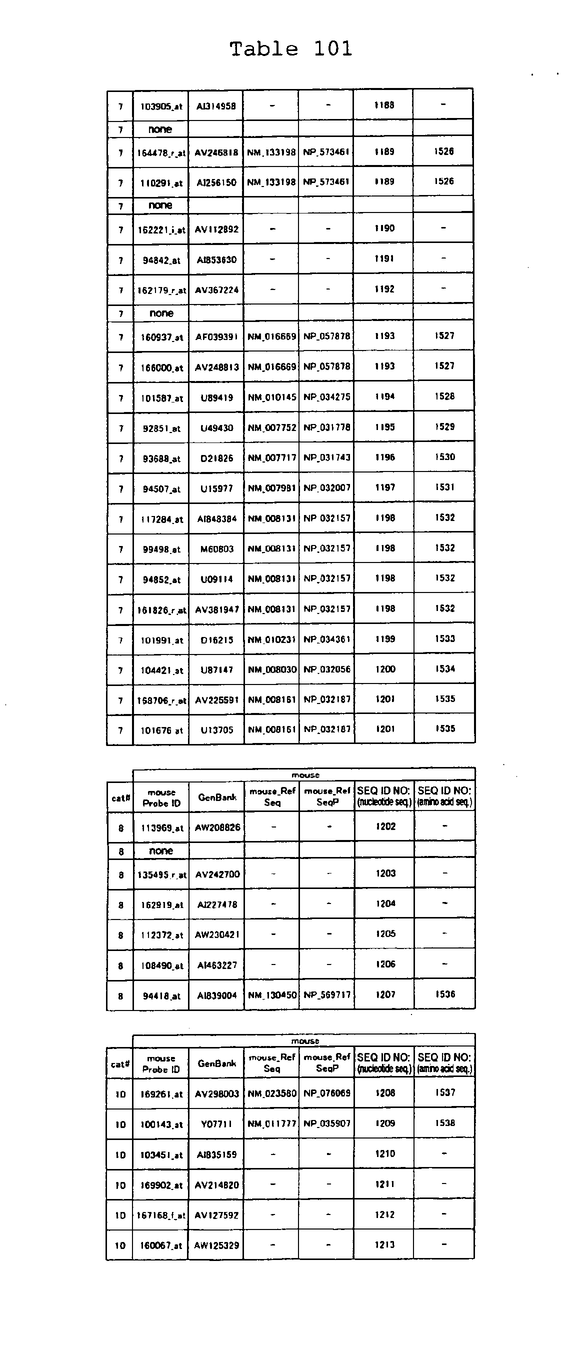

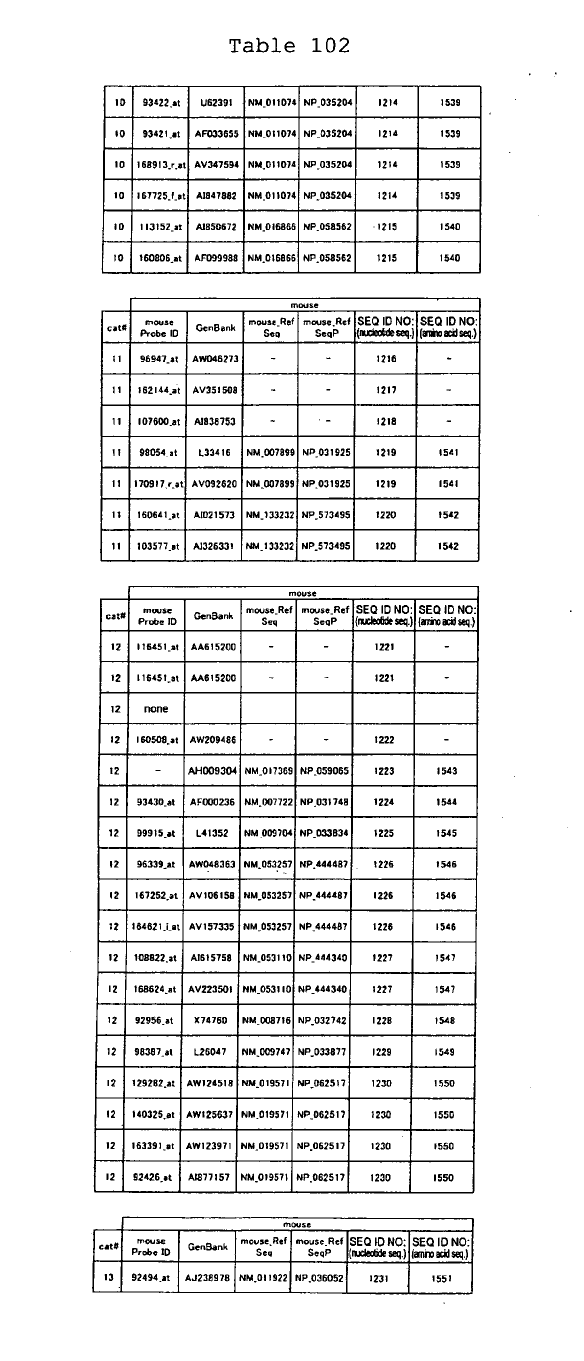

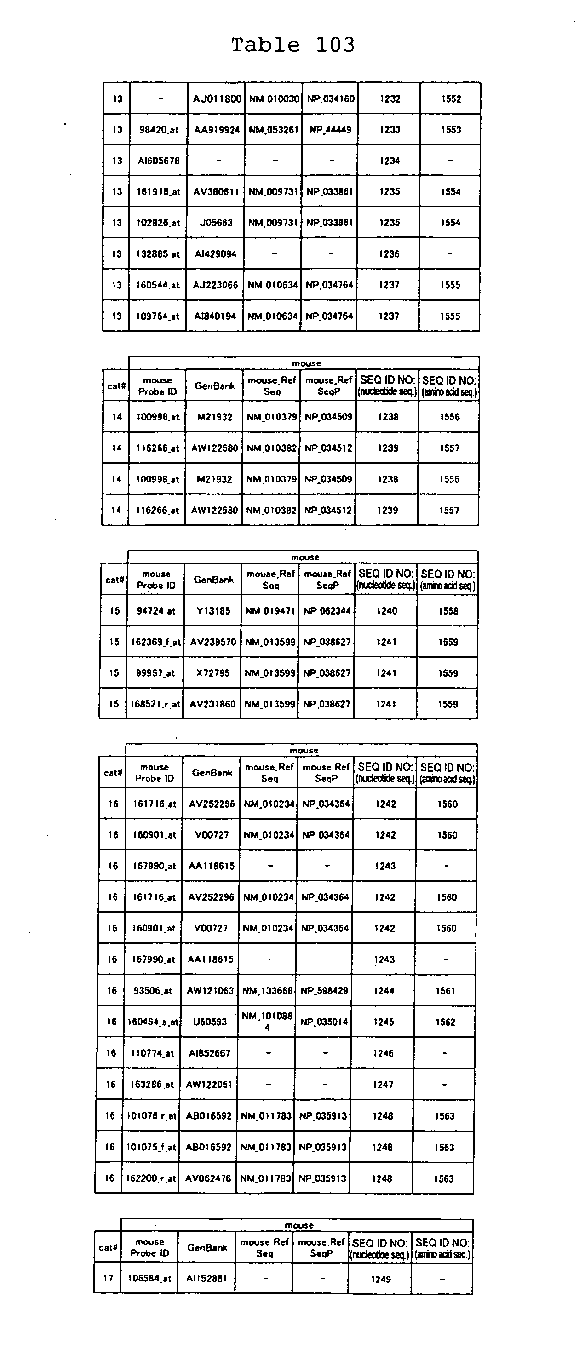

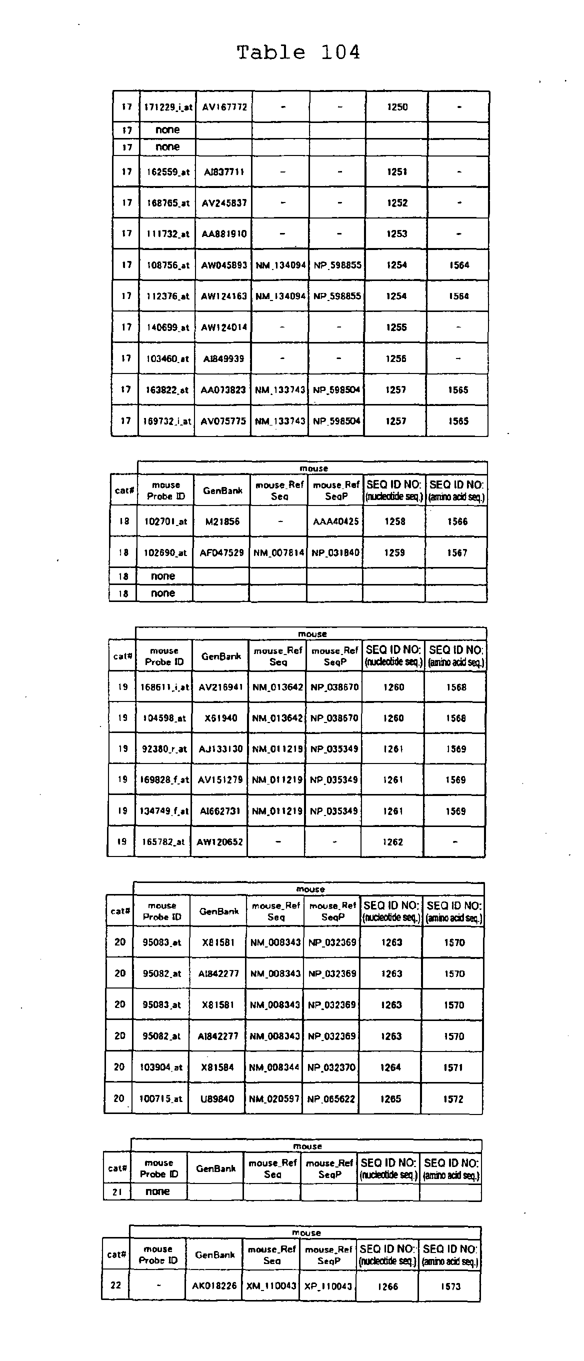

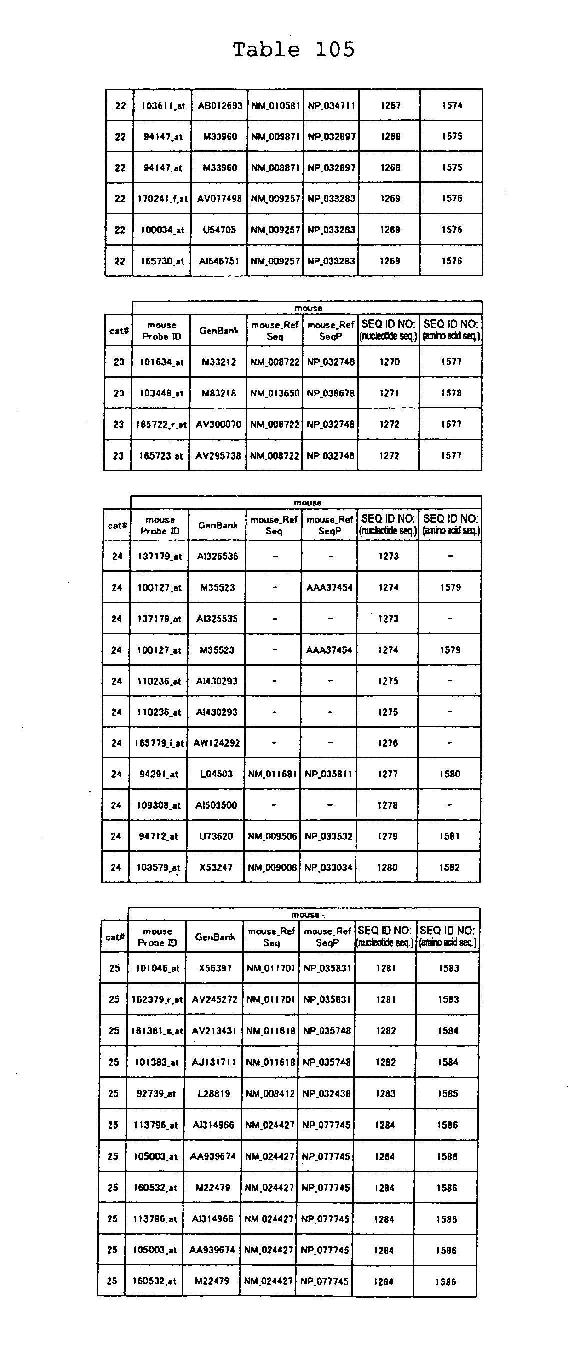

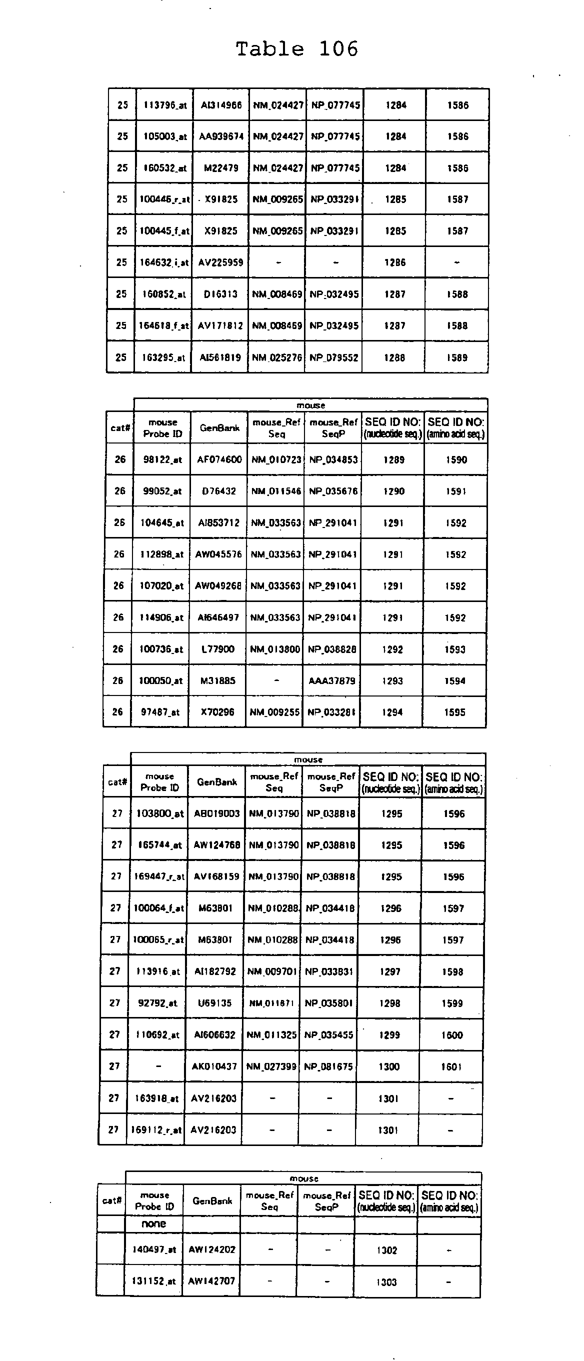

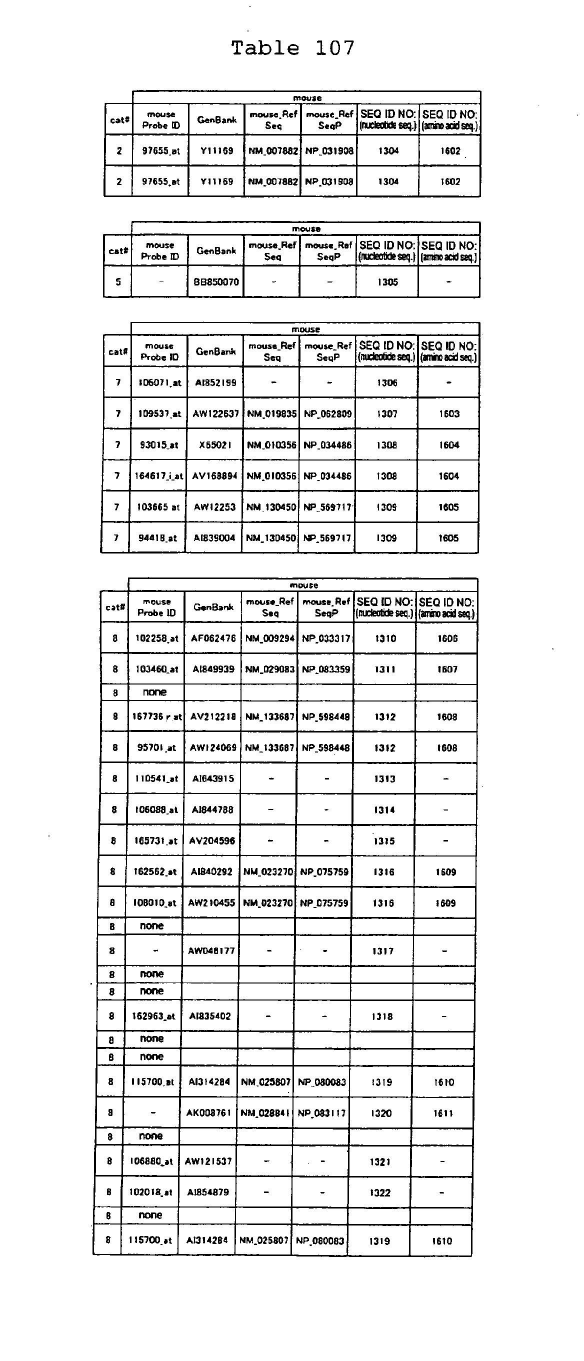

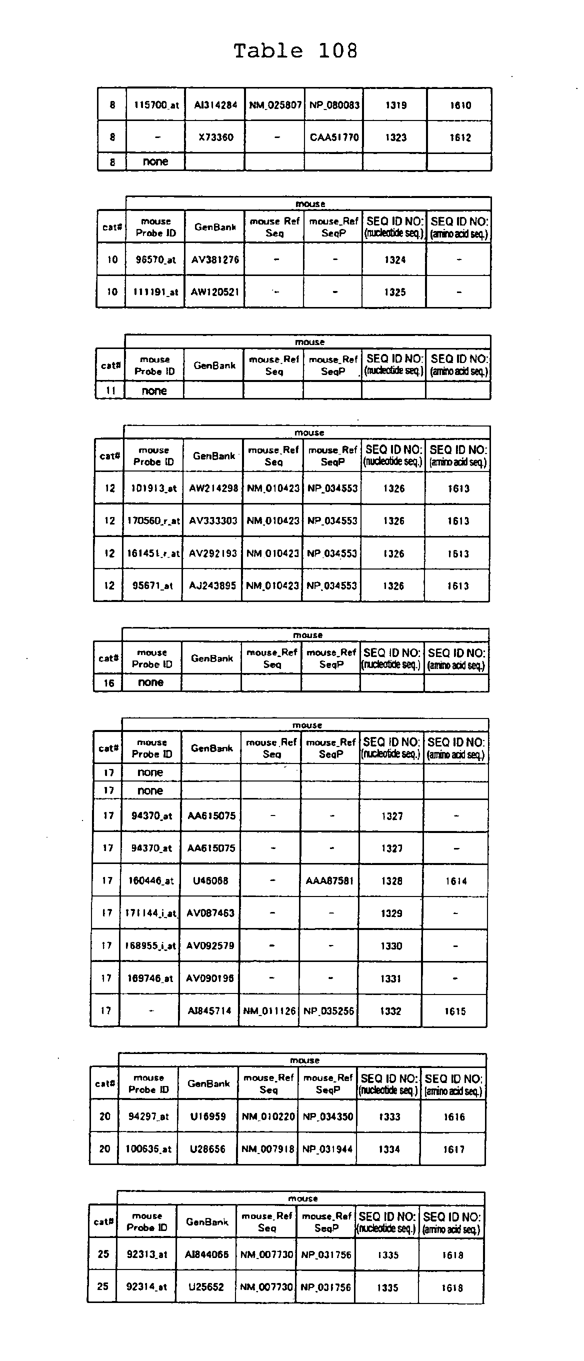

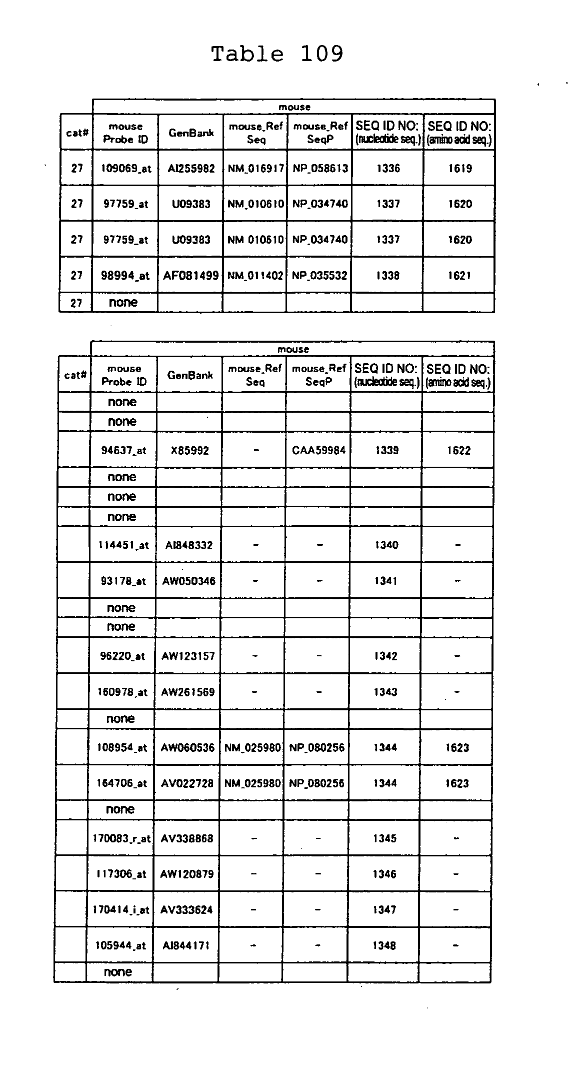

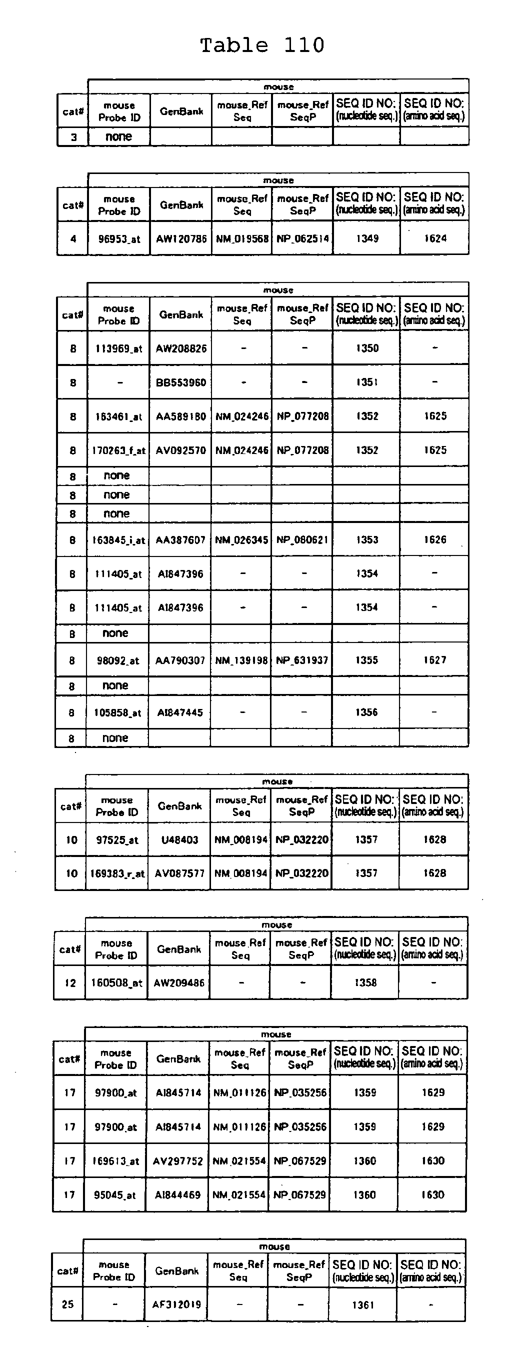

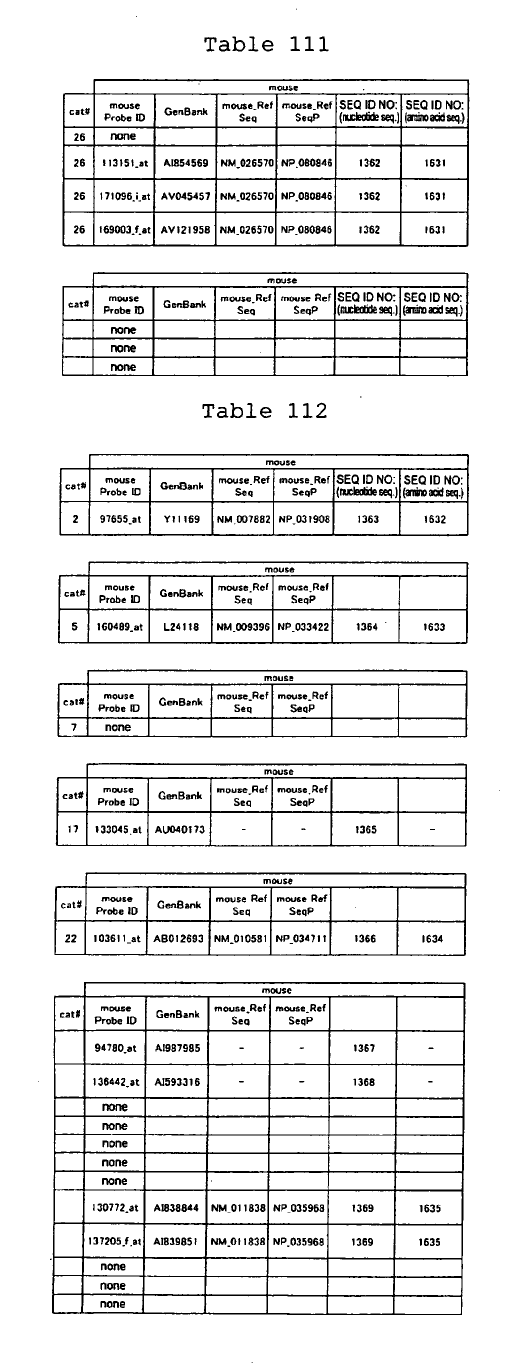

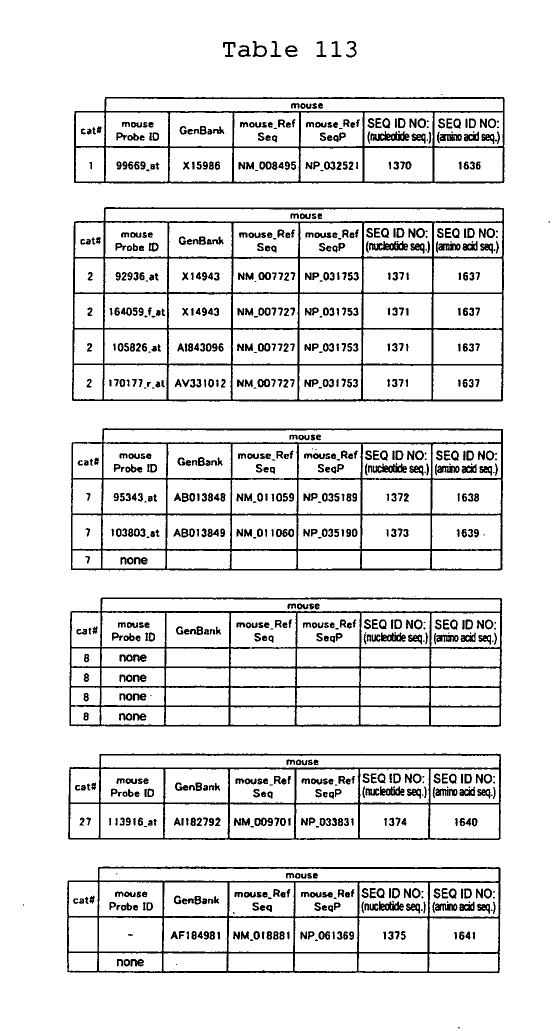

### 5. Determination of the expression levels of the genes narrowed down in Section 4 in the human goblet cell differentiation model and the mouse OVA antigen-exposed bronchial hypersensitivity model

Eighty-eight genes, most of which were recognized as genes whose expression levels were altered in human and mouse, were selected from the genes narrowed down in Section 4. A quantitative PCR assay was carried out with ABI 7700 using cDNA from the human goblet cell differentiation model and using cDNA from the mouse OVA antigen-exposed bronchial hypersensitivity model.

The primers and TaqMan probe used in the assay with ABI 7700 were designed based on the information on the sequence of each gene utilizing Primer Express (PE Biosystems). The 5' and 3' ends of the TaqMan probe were labeled with FAM (6-carboxy-fluorescein) and TAMRA (6-carboxy-N,N,N',N'-tetramethylrhodamine), respectively. The nucleotide sequences of oligonucleotides for the forward primer (F) , reverse primer (R), and TaqMan probe (TP) for each gene are shown below. The nucleotide sequences of the forward primer, TaqMan probe, and reverse primer used in the detection of each gene are indicated after probe ID, Accession No., symbol for each gene, and gene name, each of which are separated by //. The number in the parenthesis after each nucleotide sequence refers to the corresponding SEQ ID NO. The 5' and 3' ends of the TaqMan probe were labeled with FAM (6-carboxy-fluorescein) and TAMRA (6-carboxy-N,N,N',N'-tetramethylrhodamine), respectively.
Genes whose expression levels varied in both humans and mice: 
A1//NM_005409//SCYB11//"small inducible cytokine subfamily B
(Cys-X-Cys), member 11 precursor"
CCTTGGCTGTGATATTGTGTGC(1642)
ACGCTGTCTTTGCATAGGCCCT(1643)
CTCAATATCTGCCACTTTCACTGC(1644)
 
A4//U21931//FBP1//"fructose-1,6-biphosphatase (FBP1) gene, exon 7"
TGTCTCACACAGCAGTACCCTG(1645)
TGCTGTGCACCTTACATTCCTAGAGAGCAG(1646)
GTGCCAAGCATTCTACAGCATT(1647)
 A6//"NM_003856, NM_016232"//IL1RL1//interleukin 1 receptor-like 1 
TGACTGAGGACGCAGGTGATT(1648)
CCAGGTCCTTCACGGTCAAGGATGA(1649)
GGGCTCCGATTACTGGAAACA(1650)
 
A9//U88317//ALOX15//arachidonate 15-lipoxygenase
CTGCAGACCTGGTGTCGAGAG(1651)
TCACTGAAATCGGGCTGCAAGGG(1652)
ACAGGAAACCCTCGGTCCTG(1653)
 
A10//D26579//ADAM8//a disintegrin and metalloproteinase domain 8
precursor
  TGCTCCTCCGGTCACTGTG(1654)
  CAGCCCACCCTTCCCAGTTCCTG(1655)
  TTGATGACCTGCTTTGGTGC(1656)
 
A11//Y12653//diubiquitin//diubiquitin
TGTCCGGTCTAAGACCAAGGTTC(1657)
TGTGCAGGACCAGGTTCTTTTGCTGG(1658)
GGCTTCTCCGTGGCTTTAAGA(1659)
 
A19//NM_000120//EPHX1//epoxide hydrolase 1
TGAGGAGATCCACGACTTACACC(1660)
CGATAAGTTCCGTTTCACCCCACCTTTG(1661)
TCAGGTAGTTGGAGTTGAAGCCAT(1662)
 
A22//XM_051522//RDC1//G protein-coupled receptor
CGTGGACCGCTACCTCTCC(1663)
TCACCTACTTCACCAACACCCCCAGC(1664)
GGCGTACCATCTTCTTCCTGC(1665)
 
A24//NM_000598//IGFBP3//insulin-like growth factor-binding protein
3
CAGCGCTACAAAGTTGACTACGA(1666)
CCATATTCTGTCTCCCGCTTGGACTCG(1667)
CAGGTGATTCAGTGTGTCTTCCA(1668)
 
A25//m62402//IGFBP6//insulin-like growth factor-binding protein 6
CCAAGCAGGCACTGCCC(1669)
CCACAGGATGTGAACCGCAGAGACC(1670)
CGTGGTAGAGGTGCCTGGA(1671)
 
A26//NM_002964//S100A8//S100 calcium-binding protein A8
AGCTGGAGAAAGCCTTGAACTCT(1672)
TCCATGCCGTCTACAGGGATGACCTG(1673)
 CTGAGGACACTCGGTCTCTAGCA(1674) 
E1//NM_001843//CNTN1//contactin 1
GGTAGAGGAGAGCCCAGTATACCA(1675)
TGCTGCACCAAATGTGGCTCCTTC(1676)
GGCTTAAATGCCACTATGTAACCA(1677)
 
A57//NM_080657//cig5//vipirin
AAGAGGACATGACGGAACAGATC(1678)
AAGCACTAAACCCTGTCCGCTGGAAAGT(1679)
CCACAATTCTCACCCTCAATTAAGA(1680)
 
A59//u77643//SECTM1//secreted and transmembrane 1 precursor
TGGGACACCAGAGAAATAACAGAC(1681)
CACGCTGGAGGTTTCAGGTGCAGAAC(1682)
AGGCCAGAACCCAGTGTCAG(1683)
 
A68//NM_000096//CP//ceruloplasmin (ferroxidase)
TGGATGCTCAGCTGTCAGAATC(1684)
CATCTGAAAGCCGGTTTGCAAGCCT(1685)
TGTTACACTCCTGGACCTGGAA(1686)
 
B13//NM_012258//HEY1//hairy/enhancer-of-split related with YRPW
motif 1
CAATGCACTGAGCCCTTCAG(1687)
CCCACGCAGGCTGCAAACCTTG(1688)
TCCGTCCCCCAAGGTCTATAG(1689)
 
B14//NM_033197//MGC14597//von Ebner minor salivary gland protein
GGCTTCCTTCAATGGCATGT(1690)
CAGCATTGACCGTCTGGAGTTTGACCT(1691)
GTCACCCTTGATGGCAGGAT(1692)
 
A77//NM_003355//UCP2//uncoupling protein 2
CCCTACTGCCACTGTGAAGTTTCT(1693)
CACAGCTGCCTGCATCGCAGATCT(1694)
AGCAGTATCCAGAGGAAAGGTGAT(1695)
 
A78//NM_012449//STEAP//six transmembrane epithelial antigen of the
prostate
TGGAAAATGAAGCCTAGGAGAAAT(1696)
TGCTGGTCTCTCCCGTGTCCTTATGC(1697)
TCTGAAGGGCAGTCAAATTCATC(1698)
 B21//"NM_016583, NM_130852"//LOC51297//LUNX protein; PLUNC (palate 
lung and nasal epithelium clone); tracheal epithelium enriched
protein
TGGCCACCGTCTCTATGTCA(1699)
CTCGGCATAAAGCTCCAAGTGAATACGCC(1700)
CCAGCCTCAACAGACTTGCA(1701)
 
B23//NM_006424//SLC34A2//"solute carrier family 34 (sodium
phosphate), member 2"
CACTGTTCCCTCGACTGCTAACT(1702)
CTACAAGGAGAACATCGCCAAATGCCA(1703)
AAGATCCGGGAGGTGGAAATT(1704)
 
A83//u46569//AQP5//aquaporin 5 (exon4)
TTTCTGGGTAGGGCCCATC(1705)
CTGGCTGCCATCCTTTACTTCTACCTGCTC(1706)
ATGGCCACACGCTCACTCA(1707)
 
A84//AF030880//SLC26A4//"PDS(pendrin) mRNA, solute carrier family
26, member 4"
TTTGCCTCCTGAACTTCCACC(1708)
CTTGTTCTCGGAGATGCTGGCTGCAT(1709)
CCTACTGACACTGCAATAGCATAAGC(1710)
 
A89//x87159//SCNN1B//amiloride-sensitive sodium channel
ATTGATGAACGGAACCCCC(1711)
CACCCCATGGTCCTTGATCTCTTTGGA(1712)
TGCTGAGCTGCTTGTTAAGCC(1713)
 
A115//U70981//IL13RA2//"interleukin 13 receptor, α2"
TGCTCAGATGACGGAATTTGG(1714)
TGAGTGGAGTGATAAACAATGCTGGGAAGG(1715)
TGGTAGCCAGAAACGTAGCAAAG(1716)
 Mouse genes; 
A27//NM_019494 //SCYB11//"small inducible cytokine subfamily B
(Cys-X-Cys), member 11 precursor"
TGGCAGAGATCGAGAAAGCTTC(1717)
ACCCGAGTAACGGCTGCGACAAAGTT(1718)
TCCAGGCACCTTTGTCGTTT(1719)
 
A30//NM_019395//FBP1//"fructose-1,6-biphosphatase  (FBP1)  gene,
exon 7"
 CCTCTGAAGATGTGCAGGAGTTC(1720)
 
CACAAAGCCAAGTGAAGGCCAGCC(1721)
CAGAATGGAGTAGCGTCACTTGA(1722)
 
A32//NM_010743//IL1RL1//interleukin 1 receptor-like 1
TCCTAGGTGGCCAGAGTTGTG(1723)
CCCAAGACCTCACTGATCACAACAGCA(1724)
CACCCGGAGTAACACCATTATCA(1725)
 
A35//NM_009660//ALOX15//arachidonate 15-lipoxygenase
TACCCCACCGCCGATTT(1726)
CACGCCCTTGGATCCCCCAATG(1727)
CCCAGCATTTGGCCAGG(1728)
 
A36//x13335//ADAM8//a disintegrin and metalloproteinase domain 8
precursor
GGCTCTCCAACCCCCTATTCTA(1729)
AGACAGTTTCTACCAACCAGCCCCCAAG(1730)
GCCTCTTTGGTTTCACTATGGG(1731)
 
A37//NM_0023137//diubiquitin//diubiquitin
TGACAAGGAAACCACTATCCACC(1732)
CCTGAAGGTGGTGAAGCCCAGTGATG(1733)
CCAGAAACAAGGGCAGCTCT(1734)
 
A45//NM_010145//EPHX1//epoxide hydrolase 1
CCTGGCTGCCTACATCTTAGAGAA(1735)
CTGGACCAAGTCAGAATACCGTGAACTGGA(1736)
TTAGTCAGCAGATCTTCCAGGGAG(1737)
 
A48//NM_007722//RDC1//G protein-coupled receptor
TGGGAGCATCTTCTTCCTCG(1738)
TGCATGAGCGTGGACCGCTATCTC(1739)
GCCGGTGAAGTAGGTGATGG(1740)
 
A50//NM_008343//IGFBP3//insulin-like growth factor-binding protein
3
GCAGGCAGCCTAAGCACCTA(1741)
CCTCCCAACCTGCTCCAGGAAACA(1742)
TGCTCCTCCTCGGACTCACT(1743)
 
A51//NM_008344//IGFBP6//insulin-like growth factor-binding protein
6
GGAGAGCAAACCCCAAGGAG(1744)
TGCCTCCCGCTCTCGTGACACAA(1745)
 TCTTCTGCCGGTCTCTGTGG(1746) 
A52//NM_013650//S100A8//S100 calcium-binding protein A8
GAGTGTCCTCAGTTTGTGCAGAA(1747)
CACCCACTTTTATCACCATCGCAAGGAA(1748)
CTTGTGGCTGTCTTTGTGAGATG(1749)
 
E2//NM_007727//CNTN1//contactin 1
CCCAGGAGGCCTGAGAATAGA(1750)
TGGTTCCGACAATCACAGCCCTATCTCT(1751)
GAATCGTCTTGGTCTGGATCGT(1752)
 
A64//NM_021384//cig5//vipirin
GACAGCTTCGATGAGCAGGTT(1753)
CCTTGACCACGGCCAATCAGAGCAT(1754)
CTGCACCACCTCCTCAGCTT(1755)
 
A66//AF210700//SECTM1//secreted and transmembrane 1 precursor
AAGGAGTCCAGGCCCAGC(1756)
CAGATGCTCAGGACAAACACTCAGGGAACT(1757)
TCCATGCAGCTTCCAGGAG(1758)
 
A72//NM_007752//CP//ceruloplasmin (ferroxidase)
ACAGCAACAACCTGTGCCTACA(1759)
TCAACCTGTTCCCTGCCACCCTAATTG(1760)
TGCAACCCAGCTTTCAGATG(1761)
 
B18//NM_010423//HEY1//hairy/enhancer-of-split related with YRPW
motif 1
CACTCTCAGTCTCACGGATTTCA(1762)
CCAGTGTCGACCTGCGTAAGCGATC(1763)
TTCACAGGCACCAAGCTACTTTC(1764)
 
B19//U46068//MGC14597//von Ebner minor salivary gland protein
CACCCTGACCAAGATCCTTGA(1765)
TACACACTGCTGCCCAATGAGAATGGC(1766)
ACCCTTGCTCACAGACCACAT(1767)
 
A81//NM_011671//UCP2//uncoupling protein 2
GCATTGGCCTCTACGACTCTGT(1768)
CCTGCATGCTCTGAGCCCTTGGTGTA(1769)
GCCTGGAAGCGGACCTTTA(1770)
 
A82//NM_027399//STEAP//six transmembrane epithelial antigen of the
prostate
 
AGTGACGATGTTACAAACCCAGAA(1771)
TGCTCGTCTCTCCCGAGTCCTTAGTCG(1772)
GAATTCCTGCGTGTGCTGAAG(1773)
 
B24//NM_011126//LOC51297//LUNX protein; PLUNC (palate lung and nasal
epithelium clone); tracheal epithelium enriched protein
CAGCTTGCTCAATGGAGTCACT(1774)
AGGACATACCTTGCCCTGGATCAGCT(1775)
ACCAGGGTGACATCCAAACC(1776)
 
B26//NM_011402//SLC34A2//"solute carrier family 34 (sodium
phosphate), member 2"
CTCCAGCACCTCTTCCTCCA(1777)
CCGAACCGTCAGCAATGAAGAAGCAA(1778)
TGTTAGCGCCCATGATGATG(1779)
 
A98//AF087654//AQP5//aquaporin 5 (exon4)
GAACCCAGCCCGATCTTTC(1780)
CCCTGCGGTGGTCATGAATCGGT(1781)
CCCAGAAGACCCAGTGAGAGG(1782)
 
A99//AF167411//SLC26A4//"PDS(pendrin) mRNA, solute carrier family
26, member 4"
GGTTCTTGCCTCCTGTCCTG(1783)
CATCTGTGGGCCTGTTTTCGGACATG(1784)
AATGGAAAAGGATGCAGCCA(1785)
 
A104//AF112186//SCNN1B//amiloride-sensitive sodium channel
TGGTCCTTATTGATGAGCGGA(1786)
TGACCACCCGGTGGTTCTCAATTTGTT(1787)
CGGGTTGCTGCTGTTGTG(1788)
 
A127//U65747//IL13RA2//"interleukin 13 receptor, α2"
ACACAGGGCCAGACTCAAAGAT(1789)
AACCTGAACCCACATTGAGCCTCCATG(1790)
GCACACACTTCTTTGTTCAGATCC(1791)
 Genes whose expression levels tend to vary in both humans and mice:
Human genes; 
A2//NM_006705//GADD45G//"growth arrest and DNA damage inducible, γ"
CCCAGCATCACCCTCCCCGA(1792)
CCCAGCATCACCCTCCCCGA(1793)
GCGTCACCACGTCGATCAG(1794)
 
A20//d00632//GPX3//glutathione peroxidase 3
GGACACATTAATATCACCCGGA(1795)
ACAGCCTCATTCATGGTTTCACGTGC(1796)
CCCGAGATTAGGAGTTGCTGTT(1797)
 
A53//NM_005168//ARHE//"ras homolog gene family, member E"
CCACAAAGCGGATTTCACACATGCC(1798)
CCACAAAGCGGATTTCACACATGCC(1799)
TCCTTTCGTAAGTCCGTAGCAACT(1800)
 
A67//NM_002305//LGALS1//β-galactosidase binding lectin precursor
TCCTGACGCTAAGAGCTTCGTGCTGAA(1801)
TCCTGACGCTAAGAGCTTCGTGCTGAA(1802)
AAGCGAGGGTTGAAGTGCA(1803)
 
C7//NM_005672//PSCA//prostate stem cell antigen
AGGCACTGCCCTGCTGTGCTACTCCT(1804)
AGGCACTGCCCTGCTGTGCTACTCCT(1805)
GCTCACCTGGGCTTTGCA(1806)
 
A93//NM_002659//UTPR//urokinase-type plasminogen receptor
ACACCACCAAATGCAACGAGG(1807)
TTGAAAATCTGCCGCAGAATGGCCG(1808)
TCCCCTTGCAGCTGTAACACTG(1809)
 
A96//j05070//MMP9//type IV collagenase
ACCTCGAACTTTGACAGCGAC(1810)
TGCCCGGACCAAGGATACAGTTTGTT(1811)
GAGGAATGATCTAAGCCCAGC(1812)
 
A120//S78825//ID1//"inhibitor of DNA-binding 1, dominant negative
helix-loop-helix protein"
ATGAACGGCTGTTACTCACG(1813)
TGGAGATTCTCCAGCACGTCATCGACT(1814)
GATTCCGAGTTCAGCTCCAA(1815)
 Mouse genes; 
A28//NM_011817//GADD45G//"growth arrest and DNA-damage-inducible,
γ"
GCATTGCATCCTCATTTCGAAT(1816)
TGAGGACACATGGAAGGACCCTGCC(1817)
CCTCGCAGAACAAACTGAGCTT(1818)
 A46//u13705//GPX3//glutathione peroxidase 3 
AGAAGAACTTGGGCCATTTGG(1819)
TTCTGGGCTTCCCTTCCAACCAATTTG(1820)
TCTCGCCTGGCTCCTGTTT(1821)
 
A60//NM_028810//ARHE//"ras homolog gene family, member E"
GGGATGGTGCCCCTAGACTAG(1822)
CTGTCGTCTGGTGCCACTTCCTTCAA(1823)
GGGTTTTGCCAGAACAGCATT(1824)
 
A71//NM_008495//LGALS1//β-galactosidase-binding lectin precursor
ACAGCAACAACCTGTGCCTACA(1825)
CCCATGGAGACGCCAACACCATTG(1826)
CCCATCTTCCTTGGTGTTACA(1827)
 
C8//AW209486//PSCA//prostate stem cell antigen
CATCCCATCTCAGCCTTTACCA(1828)
CCTACTCTCCAGGGCCTGAGCCAGTG(1829)
GCCCTACCAAGTTTTGCTCAGA(1830)
 
A108//NM_011113//UTPR//urokinase-type plasminogen receptor
CAATGGTGGCCCAGTTCTG(1831)
AGCTTTCCACCGAATGGCTTCCAGTGT(1832)
GGGTATTGTTCCCCTCACAGC(1833)
 
A111//NM_013599//MMP9//type IV collagenase
CCATGCACTGGGCTTAGATCA(1834)
AGCGTGCCGGAAGCGCTCAT(1835)
TCGAGGTAGCTATACAGCGGG(1836)
 
A132//U43884//ID1//"inhibitor of DNA-binding 1, dominant negative
helix-loop-helix protein"
  CGACATGAACGGCTGCTACTC(1837)
  CGCCTCAAGGAGCTGGTGCCC(1838)
  CTTGCTCACTTTGCGGTTCTG(1839)
 Genes whose expression levels varied in humans:
Human genes; 
A3//NM_000625//NOS2A//"nitric oxide synthase 2A (inducible,
hepatocytes)"
ACCCTGAGCTCTTCGAAATCC(1840)
TTAGCTCCAGTTCCCGAAACC(1841)
TTAGCTCCAGTTCCCGAAACC(1842)
 A5//NM_005101//ISG15//"interferon-stimulated protein, 15 kDa" 
GGGACCTGACGGTGAAGATG(1843)
CTGACACCGACATGGAGCTGCTCAG(1844)
GCCAATCTTCTGGGTGATCTG(1845)
 
A8//NM_003956//CH25H//cholesterol 25-hydroxylase
ACGTGGTCAACATCTGGCTTTC(1846)
TCCGGCTACAACTTCCCTTGGTCCA(1847)
GGAGCGAAGTTGCAGTTAAAGTG(1848)
 
A12//U19557//SERPINB4 (SCCA2)//"serine (or cysteine) proteinase
inhibitor, clade B (ovalbumin), member 4"
AGCCACGGTCTCTCAG(1849)
AAGGCCTTTGTGGAGGTCACTGAGGAGGGA(1850)
GCAGCTGCAGCTTCCA(1851)
 
A13//NM_002575//SERPINB2//"serine (or cysteine) proteinase
inhibitor, clade B (ovalbumin), member 2"
ATGGTCCTGGTGAATGCTGTCTA(1852)
TGTAAACTCGGCTCAGCGCACACCT(1853)
GCTTTTCACGCAAGTACATCATCT(1854)
 
A15//NM_000433//NCF2//neutrophil cytosolic factor 2
TAGCATTGGCCACGAGCAT(1855)
TGAGCCCAGACATTCCAAAATCGACA(1856)
GATCACCACTGGCTCATATAGCTTCT(1857)
 
A23//NM_000435//NOTCH3//Notch homolog 3
ACTTTGCCAACCGTGAGATCA(1858)
TCCTGGTGCAGTCTCTCCTGGGCTA(1859)
ATCCAGCAAGCGCACGAT(1860)
 
B1//NM_022168//MDA5//melanoma differentiation associated protein-5
GACCCCAGAATTCAAGGAACTTT(1861)
CAAGCCTGGCCACATTTGCAGATGA(1862)
GCCTTTGTGCACCATCATTGT(1863)
 
B2//NM_052942//GBP5//guanylate binding protein 5
AAAATTGGCTGGCAGAGCAA(1864)
CTGCACAGCTCAGCACAACATTCCAA(1865)
CGTGCTGGAGCTCACTGAGA(1866)
 
B3//NM_018584//PRO1489//hypothetical protein PRO1489
AGAGGAGCCCAGAGCCTTCT(1867)
TCATCTGTCTCCCGGCCTGATACCA(1868)
 CCCACGATGAAATCAACAACCT(1869) 
C2//NM_032323//MGC13102//hypothetical protein MGC13102
CCAGTCGGTCCAGCTCTTTATT(1870)
TCAACCTGGCCGTGCTTTCCACTT(1871)
TCAACCTGGCCGTGCTTTCCACTT(1872)
 
A54//NM_003238//TGFB2//"transforming growth factor, β2"
CCTGAACAACGGATTGAGCTATATC(1873)
CCCAGCGCTACATCGACAGCAAAGT(1874)
AACAGCATCAGTTACATCGAAGGA(1875)
 
A55//NM_001539//DNAJA1//"DnaJ (Hsp40) homolog, subfamily A, member
1"
CCAAGTAGAACTGGTGGACTTTGA(1876)
CCAAATCAGGAAAGACGGCGCCA(1877)
CATCCTCATATGCTTCTCCATTGT(1878)
 
A56//NM_003032//SIAT1//"sialyltransferase 1 (β-galactoside
α-2,6-sialytransferase)"
ACGCAGTCCTGAGGTTTAATGG(1879)
CACCCACAGCCAACTTCCAACAAGATGT(1880)
GCACAAAAACTACCATTCGCCT(1881)
 
B9//NM_013324//CISH //cytokine-inducible SH2-containing protein
TGTGCATAGCCAAGACCTTCTC(1882)
CCAATACCAGCCAGATTCCCGAAGGTA(1883)
CTGGCATCTTCTGCAGGTGTT(1884)
 
A69//NM_006408//AGR2//anterior gradient 2 homolog (Xenepus laevis)
CAGTTTGTCCTCCTCAATCTGGTT(1885)
TGTCCCCAGGATTATGTTTGTTGACCCA(1886)
TTCCAGTGATATCGGCTCTAACTGT(1887)
 
A70//NM_002443 NM_138634//MSMB//"microseminoprotein, β-, isoform a,
b"
ACCTGTCTATAAGGAGTCCTGCTTATC(1888)
CAATGAATGTTCTCCTGGGCAGCGTT(1889)
AAGTCACGAAGGTGGCAAAGAT(1890)
 
B11//NM_024539//FLJ23516//hypothetical protein FLJ23516
CTGCTCGAAGGCTACGGAAT(1891)
TCTGCCTTTAATTGCCTCTGCTTCCTG(1892)
TGCGTAGTTGAAGCCTTCCA(1893)
 
B15//NM_002247//KCNMA1//"potassium large conductance
calcium-activated channel, subfamily M, α member 1"
CCGTGCCAGCAACTTTCATT(1894)
CCAAAGTGTCCATATTGCCTGGTACGCC(1895)
CCCTTAAATCAGCCCGACTTAA(1896)
 
C5//NM_018050//FLJ10298//hypothetical protein FLJ10298
CGAGGAAGCCTGTCCATTGA(1897)
TGACCAGAAATTTGCCAAGCCAAGAGTT(1898)
GCTTGTGAAAATTGGCCATGT(1899)
 
A75//NM_003246//THBS1//throbospondin 1
TCCAGCATGGTCCTGGAACT(1900)
TCTTCAGTCACTTTGCGGATGCTGTCCT(1901)
TGAACTCCGTTGTGATAGCATAGG(1902)
 
A76//NM_005688//ABCC5//"ATP-binding cassette, sub-family C, member
5"
GGACACTGCACAGCATCGAT(1903)
CCGCAGATTCCAACCAGTTTACCCTCTT(1904)
CGAAGGTTCCACTGATTGCAA(1905)
 
E3//NM_016354//SLC21A12//"solute carrier family 21 (organic anion
transporter), member 12"
GCGTCACCTACCTGGATGAGA(1906)
TACATTGCCATCTTCTACACAGCGGCC(1907)
GCCCATTTCCGTGTAGATATTCA(1908)
 
E4//NM_012434//SLC17A5//"solute carrier family 17 (anion/sugar
transporter), member 5"
TGCCACTATTCCAGGAATGGTT(1909)
CACGGTTTGCCATTCTCCAACAGTGTTA(1910)
CTTCACCTTTGGCGAATAGTGTAA(1911)
 
A87//x52947//GJA1//"cardiac gap junction protein, connexin 43"
GGTTACTGGCGACAGAAACAATTC(1912)
CGCAATTACAACAAGCAAGCAAGTGAGC(1913)
TGCCCCATTCGATTTTGTTC(1914)
 
A90//d28137//BST2//BST2
CAGTGATGGAGTGTCGCAATG(1915)
CATCTCCTGCAACAAGAGCTGACCGA(1916)
CACATCCTGAAAGCCCTTCTG(1917)
 
A94//j04164//IFI9-27//interferon-inducible protein9-27
CCTCTTCTTGAACTGGTGCTGT(1918)
TGGGCTTCATAGCATTCGCCTACTCC(1919)
CCATCTTCCTGTCCCTAGACTTC(1920)
 
A97//m24283//ICAM1//major group rhinovirus receptor (ICAM1)
GCTGACGTGTGCAGTAATACTGG(1921)
CAGACAGTGACCATCTACAGCTTTCCGG(1922)
TTCTGAGACCTCTGGCTTCGT(1923)
 
A113//D13666//OSF-2//osteoblast  specific  factor  2  (fasciclin
I-like)
  AGCAAACCACCTTCACGGATC(1924)
  AATTAGGCTTGGCATCTGCTCTGAGGCC(1925)
  GGTGCCAGCAAAGTGTATTCTCC(1926)
 
A114//D31784//CDH-6//"cadherin 6, type 2 preproprotein"
CGCAGTTCTGTAGTTGAGTTTCAAGG(1927)
TTAGCAGGGTTGATGTGGAGCGTGAAG(1928)
ACCAAGAACAGAATGCCCAGG(1929)
 
A116//U21049//DD96//"epithelial protein upregulated in carcinoma,
membrane associate"
GCCTTTGCAGTCAACCACTTCTG(1930)
ATGATCCTGACCGTCGGAAACAAGGC(1931)
TCTGTTCCCACCAGGACTCCAT(1932)
 
A117//X87212//CTSC//cathepsin C
TCTCAGACCCCAATCCTAAGCC(1933)
TCTTGTAGCCAGTATGCTCAAGGCTGTGAA(1934)
CTGCAATAAGGTATGGAAGCC(1935)
 
A118//U17077//BENE//BENE protein
TGCCCGAGCTGATATTTGG(1936)
TAGCCGCCACCCACATAGTATACCCCTT(1937)
CATACATCACCCATCCTTGCAG(1938)
 
A121//AI979079//FLJ10261//hypothetical protein FLJ10261
TTTGTCACTGAGCTCCGAAGG(1939)
TAGCTGTCAGAGCCAAAGACATCGGAATCT(1940)
TCCCAATGCCTCTGAGGATATT(1941)
 
A122//M87434//OAS2//2'-5'-oligoadenylate synthetase 2 (69-71 kD)
CATCAGGAACATCCTGCTGCA(1942)
 
CAGCTCCAATCAGCGAGGCCAGTAATCT(1943)
CACATTATTGGTTGGGTCAACTGG(1944)
 
A123//AB032953//Odz2//"odd Oz/ten-m homolog 2 (Drosophila, mouse)"
AGGCATGGTCAATGCCAGGT(1945)
TCATGACAACAGCTTCCGCATCGCAA(1946)
AGTCTCACTTATGACGGGCTTGATG(1947)
 
A124//X82693//E48//"lymphocyte antigen 6 complex, locus D"
AAGCATTCTGTGGTCTGCCC(1948)
CTCGCTTCTGCAAGACCACGAACACA(1949)
TTCACCAGATTCCCCCTCAGAG(1950)
 
A137//AF061812//KRT16//"keratin type 16 gene, exon 8"
CACCATTGAGAATGCGCAG(1951)
TTTTGCAGATTGACAATGCCAGGCTG(1952)
ACTTGGTCCTGAAGTCATCGG(1953)
 Mouse genes; 
A29//m84373//NOS2A//"nitric oxide synthase 2A (inducible,
hepatocytes)"
TGACGGCAAACATGACTTCAG(1954)
AATTCACAGCTCATCCGGTACGCTGG(1955)
GCCATCGGGCATCTGGTA(1956)
 
A38//NM_009126//SERPINB4 (SCCA2)//"serine (or cysteine) proteinase
inhibitor, clade B (ovalbumin), member 4"
ATGACCTCCCAATTCCATTGG(1957)
ACATGGGAATGGTCGATGCCTTTGA(1958)
ACCAGAGAAGTCAGCCTTCTGTG(1959)
 
A39//NM_011111//SERPINB2//"serine (or cysteine) proteinase
inhibitor, clade B (ovalbumin), member 2"
CACATGAGGTTTTGTAGCATGAACT(1960)
AGCCTCAGAATTGCATCTTCAAGTGCCA(1961)
GCACTGAAGACTGCTATACAATTGC(1962)
 
A41//NM_010877//NCF2//neutrophil cytosolic factor 2
ACCACCTCCTAATTCTAGCCCC(1963)
AGTTGTCACCAGGTCACAAGCAAAAAGAGC(1964)
CATGTAAGGCATAGGCACGCT(1965)
 
B5//AA959954//MDA5//melanoma differentiation associated protein-5
GAGAGCAAATGTGGACTCAGCTAGT(1966)
 
TGTAGCCCGAGATCACCCACAGAGAAC(1967)
AATGCCCATGAGGTATTGTCCTA(1968)
 
B6//NM_010259//GBP5//guanylate binding protein 5
GCAGCAAATAGAGCATTGGC(1969)
AGCATGAGATGCTGATGGAACAGAAGGA(1970)
TGCTCCATCTTCTCAGTCAGC(1971)
 
C4//NM_024246//MGC13102//hypothetical protein MGC13102
GGGCTGGCGAGATATTGAAC(1972)
CCATTCAAAGAGGATGCCAACCTGCTC(1973)
CGCTCGATGCACTGTAGATCA(1974)
 
A61//NM_009367//TGFB2//"transforming growth factor, β2"
TTACCCTAAGCGAGAAAGTGCAA(1975)
CGCAGCCAACGCGCCCA(1976)
CCTTAACCCCTGTGGAACAACA(1977)
 
A62//NM_008298//DNAJA1//"DnaJ (Hsp40) homolog, subfamily A, member
1"
TGTCTAGTTATATGAAGTGAACCAATTGTG(1978)
TGCCTTTGCATTGTATTGCCTCAGCC(1979)
CGAAATGTATTATGCCACCTTCTAGTAA(1980)
 
A63//D16106//SIAT1//"sialyltransferase 1 (β-galactoside
α-2,6-sialytransferase)"
GGGTTACCTGCCCAAAGAGAC(1981)
TTCAGAACCAAGGCTGGGCCTTGG(1982)
CAGAAGACACGACGGCACAC(1983)
 
B10//NM_009895//CISH //cytokine-inducible SH2-containing protein
CAGTGCCCGCAGCTTACAA(1984)
CTGTGTCGGCTAGTCATCAACCGTCTGG(1985)
TCGGAGGTAGTCGGCCATAC(1986)
 
B16//NM_023270//FLJ23516//hypothetical protein FLJ23516
TCGCAGTGAGACTGCATCATC(1987)
CTTCAGTACAAGGAGCAGATGAGCCACCTC(1988)
TTTGCTGACTGCGCATGTTC(1989)
 
B20//NM_010610//KCNMA1//"potassium large conductance
calcium-activated channel, subfamily M, α member 1"
TGGTAACGTGGACACCCTTGA(1990)
TAATGATTGCTCCACCAGTTTCCGTGC(1991)
 GTTGGCGGCTGCTCATCTT(1992) 
C6//NM_026345//FLJ10298//hypothetical protein FLJ10298
GTCCTCTGCATGCTAGGCAAG(1993)
AGCCATCCCTCAGTCCAACCACTTTCTG(1994)
ACCCTTCTTCTCTTCCTCTTTAAAAAA(1995)
 
A79//NM_011580//THBS1//throbospondin 1
GGTGCTGCAGAATGTGAGGTT(1996)
AGGCTGCTCCAGCTCTACCAACGTCCT(1997)
AACCGTTCACCACGTTGTTGT(1998)
 
A80//NM_013790//ABCC5//"ATP-binding cassette, sub-family C, member
5"
TGGAGGCTGCATCAAGATTG(1999)
TCAGTGGCACTGTCAGATCAAACCTGG(2000)
TCTTCCGTGTACTGGTTGAAAGG(2001)
 
A102//M61896//GJA1//"cardiac gap junction protein, connexin 43"
CGAGCAAAACTGGGCGAA(2002)
ACAGCGCAGAGCAAAATCGAATGGG(2003)
ATGGTGCTTCCGGCCTG(2004)
 
A109//AK003407//IFI9-27//interferon-inducible protein9-27
AGGTGTCGGTGCCTGACC(2005)
TGGTCTGGTCCCTGTTCAATACACTCTTCA(2006)
GCCCAGGCAGCAGAAGTTC(2007)
 
A112//m31585//ICAM1//major group rhinovirus receptor (ICAM1)
AGTCCGCTGTGCTTTGAGAAC(2008)
TGGCACCGTGCAGTCGTCCG(2009)
CCGGAAACGAATACACGGTG(2010)
 
A125//D13664//OSF-2//osteoblast specific factor 2 (fasciclin
I-like)
TAGCCCAATTAGGCTTGGCATCC(2011)
TAGCACCTGTGAACAATGCGTTCTCTGATG(2012)
TAAGAAGGCGTTGGTCCATGCT(2013)
 
A126//D82029//CDH-6//"cadherin 6, type 2 preproprotein"
TTTAAGACCCCCGAGTCCTCTC(2014)
CCAATTGGCAGGATCAAAGCCAGTGA(2015)
CTCCGCATTTTCTCCCACATC(2016)
 A128//AW01791//DD96//"epithelial protein up-regulated in carcinoma, 
membrane associate"
GATGCAAGGCCTCATTGCTG(2017)
CGCTGTGTTCTTGGTCCTTGTTGCAA(2018)
AGAAGTGGTTGACGGCGAAGAC(2019)
 
A129//U74683//CTSC//cathepsin C
TCTCAGACACCAATCCTGAGTC(2020)
TCTTGCAGCCCCTATGCCCAAGGTTGTGAT(2021)
CTGCAATGAGGTATGGGAATCC(2022)
 
A130//BC012256//BENE//BENE protein
CGGGTTCTGGGTGTGGACT(2023)
CTGCTACACACGTCGCATACCCCTTG(2024)
CATACAGCACCCATCCCTGC(2025)
 
A133//BC006062//FLJ10261//hypothetical protein FLJ10261
CGGCATCTGGTATAACATCCTCA(2026)
AGGTGTTGGGAAGCTGGCTGTCATCA(2027)
GATGAAGTCAGACGTGAAGGAGATC(2028)
 
A135//NM_011856//Odz2//"odd Oz/ten-m homolog 2 (Drosophila, mouse)"
GAATGATCAACGCCAGGTTTG(2029)
ACCTATCACGACAATAGCTTCCGCATTGC(2030)
CGCTAATGACGGGTTTGATGC(2031)
 
A136//X53782//E48//"lymphocyte antigen 6 complex, locus D"
GGTCTGCCCGTCCAACTTC(2032)
TTCTGCAAAACCGTCACCTCAGTGGAG(2033)
TCACCAGGTTCCCATTCAGAG(2034)
 
A138//AF053235//KRT16//"keratin type 16 gene, exon 8"
TCAAGACCATTGAGGACCTGA(2035)
ACACGATCACCTACTCACTCCTCAAGCA(2036)
AGCCTGGCATTGTCAATCTG(2037)
 Genes whose expression levels tend to vary in humans:
Human genes; 
A16//NM_002997//SDC1//syndecan 1
TGGTGGGTTTCATGCTGTACC(2038)
TGAAGAAGAAGGACGAAGGCAGCT(2039)
GCATAGAATTCCTCCTGTTTGGTG(2040)
 
A21//NM_024090//LCE//hypothetical protein MGC5487
TCTCTGACCCTTGCAGTCTTCA(2041)
 
CATTTTGATGACCAAAGGCCTGAAGCA(2042)
GAATTTGCTGACAGGTCCATTG(2043)
 
A88//u17986//SLC6A8//SLC6A8
TCCTACTACTTCCGTTTCCAAAGG(2044)
CCTCTGTTGTGCCCTCTGCTTTGTCAT(2045)
CTCACATCAGTCACCATGGAGAG(2046)
 Mouse genes; 
A42//NM_011519//SDC1//syndecan 1
GGCTTTCATGCTGTACCGGAT(2047)
TGGAGGAGCCCAAACAAGCCAATG(2048)
AGGCGTAGAACTCCTCCTGCTT(2049)
 
A47//NM_130450//LCE//hypothetical protein MGC5487
AGCTGTACTTTGATTGCAGGTCAA(2050)
CTCACCAGTTGTCCATGTCCACCCAC(2051)
GGACCAATCAGCTAGGACAACTTG(2052)
 Genes whose expression levels varied in mice:
Human genes; 
A17//NM_000667//ADH1A//"class I alcohol dehydrogenase, α subunit"
TTTCCCTTGTGGCAGTCTTCA(2053)
CCTCTACCCTACATGATCTGGAGCAACAGC(2054)
TTGGAAAGCCCCCAAATGT(2055)
 
A58//NM_014375//FETUB//fetuin B
CCGAGTCTCTTGCGAAATACAA(2056)
ACAACCCACTGGCTAGAAGCCCTGGT(2057)
CGGAGGACTGAAGTGAACAGCT(2058)
 
B22//NM_014585//SLC11A3//"solute carrier family 11 (proton-coupled
divalent metal ion transporters), member 3"
AACCGCCAGAGAGGATGCT(2059)
TGGATCCTTGGCCGACTACCTGACCT(2060)
CACATCCGATCTCCCCAAGTA(2061)
 
A119//V01512//c-fos//cellular oncogene c-fos (complete sequence)
GGCAAGGTGGAACAGTTATCTCC(2062)
TCCGAAGGGAAAGGAATAAGATGGCTGCA(2063)
AGTGTATCAGTCAGCTCCCTCCTC(2064)
 Mouse genes;

A43//NM_007409//ADH1A//"class I alcohol dehydrogenase, α subunit"

TGTGGTGTAAGCGTCGTCGTA(2065)
CCAATGCCCAGAACCTCTCCATGAAC(2066)
CGCCAAATATTGCTCCCTTC(2067)

A44//NM_008030//FMO3//Flavin-containing Monooxygenase 3
CTTGCAGCCCCTACCAGTTC(2068)
CCCGGAACGCCATCCTAACACAGTG(2069)
TGACGACACGCGTCTTCATAG(2070)

A65//NM_021564//FETUB//fetuin B
CTCGTCAAAGTCACCAAGGCTAT(2071)
CCATGTACCAAATCCCAGGCCAGCT(2072)
AATACCAACGGGCTCAGAGTCA(2073)

B25//NM_016917//SLC11A3//"solute carrier family 11 (proton-coupled
divalent metal ion transporters), member 3"
  CTATTCTCAGGACTAGCCCAGCTT(2074)
  TCCAGGCATGAATACGGAGATCACACA(2075)
  CCTAGAACGGATATCTTCAAATGGA(2076)

A131//V00727//c-fos//cellular oncogene c-fos (complete sequence)
CCTGAAGAGGAAGAGAAACGGAG(2077)
CGAAGGGAACGGAATAAGATGGCTGC(2078)
CGATTCCGGCACTTGGC(2079)

The total RNAs extracted by the method described above were treated with DNase (Nippon Gene Co. , Ltd.). Then, the cDNAs prepared by reverse transcription were used as templates. The primer used was random hexamer (GIBCO BRL). A plasmid clone for each gene, which contained the nucleotide sequence region amplified with the pair of primers, was prepared for a standard curve to determine the copy number. A dilution series of the plasmid was used as templates in the PCR assay. The composition of the reaction solution used to monitor PCR amplification was the same as that shown in Table 39.

Furthermore, similar quantitative analyses for the β-actin gene and the glyceraldehyde-3-phosphate dehydrogenase (GAPDH) gene as internal standards for correction were carried out to correct the difference of cDNA concentration in a sample. The copy number of the gene of interest was determined by correcting based on the determined copy numbers for the genes.

The nucleotide sequences of primers and probes used in the assays for human and mouse β-actin, and human and mouse GAPDH, are the same as shown in Example 6 (human: SEQ ID Nos: 7 to 12) and Example 9 (mouse: SEQ ID NOs: 18 to 23). The expression levels (copy/ng RNA) of the respective genes corrected with the level of β-actin are shown in Figs 7 to 31 (altered in both human and mouse) and Figs 32 to 69 (altered in human). In the OVA-administered group, the respective genes showed significant variations in expression levels. Specifically, the expression levels of genes belonging to groups (A) and (B) were confirmed to be increased and decreased, respectively.

### 6. Determination of the localization of each mRNA in the lung of OVA antigen-exposed bronchial hypersensitivity model by in situ hybridization (hereinafter referred to as "ISH")

A32/IL-1R-1, A36/ADAM 8, A37/diubiqutin, A42/SDC1, A50/IGFBP3, and A129/CTSC were analyzed for the localization pattern. After perfusion fixation with 10% buffered neutral formalin, the pulmonary tissues were removed from three mice from the naive group and each of the other three groups (S-Sal group, Pred group and S-OVA group) 24 hours after the final exposure to the antigen. The tissues were fixed with 10% buffered neutral formalin, and then embedded in paraffin to prepare tissue blocks.

All paraffin blocks from the mouse lung samples were sliced into 3 µm sections. Then, the sections were treated with hematoxylin for nuclear staining. Among them, sections exhibiting good tissue morphology were selected from a single individual each of the S-Sal group and S-OVA group for carrying out ISH. The nucleotide sequences of the ISH probes are shown in the following SEQ ID NOs: CTSC (SEQ ID NO: 2080, 2081); IL-1 receptor 1 (SEQ ID NO: 2082); ADAM8 (SEQ ID NO: 2083); Diubiquitin (SEQ ID NO: 2084); SDC1 (SEQ ID NO: 2085); and

IGFBP3 (SEQ ID NO: 2086).

The paraffin sections of mouse lung tissues from the S-Sal group and the S-OVA group were rehydrated by deparaffinization (washed with water after treatment with xylene, 100%, 90%, 80%, and 70% alcohol). Then, the sections were treated with the ISH probe described above. After the staining, the sections were treated for nuclear staining. The conditions used for the ISH experiments are described below. The ISH result is shown in Table 158.
Probe concentration: 250 ng/ml
Hybridization temperature: 60°C
Duration of hybridization: 6 hours
Post-hybridization wash: 0.1x SSC/70°C /6 minutes/3 times
Coloring reagents: NBT/BCIP
Duration of color development: 7 hours

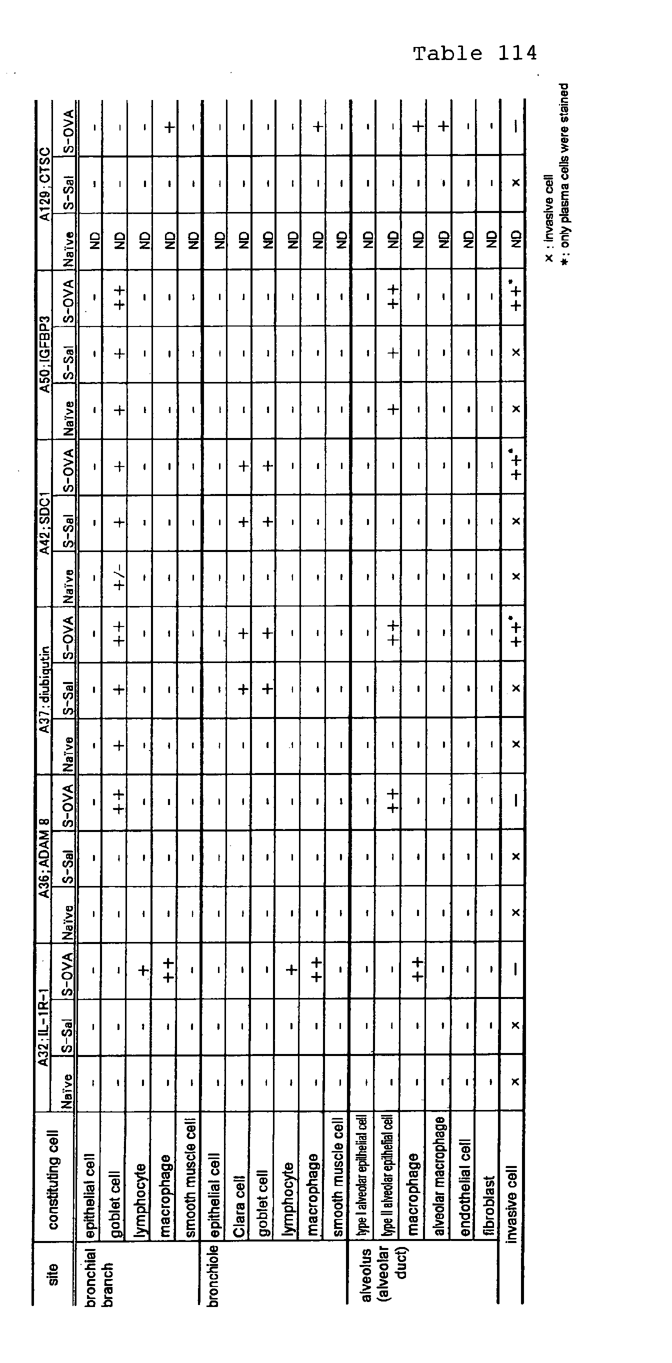

## Claims

1. A method of testing for bronchial asthma or chronic obstructive pulmonary disease, which comprises the steps of:
(1) determining the expression level of a marker gene in a biological sample from a subject;
(2) comparing the expression level determined in step (1) with the expression level of the marker gene in a biological sample from a healthy subject; and
(3) judging the subject to have bronchial asthma or chronic obstructive pulmonary disease when the result of the comparison in step (2) indicates that (i) the expression level of the marker gene in the subject is higher than that in the control when the marker gene is a gene according to (a) or (ii) when the expression level of the marker gene in the subject is lower than that in the control when said marker gene is a gene according to (b);
wherein the marker gene is any one selected from the group according to (a) or (b):
(a) a group of genes whose expression levels increase when respiratory epithelial cells are stimulated with interleukin-13, and comprise any one of the nucleotide sequences of SEQ ID NOs: 25 to 310;
(b) a group of genes whose expression levels decrease when respiratory epithelial cells are stimulated with interleukin-13, and comprise any one of the nucleotide sequences of SEQ ID NOs: 311 to 547.

2. The testing method according to claim 1, wherein the biological sample is a respiratory epithelial cell.

3. The testing method according to claim 1, wherein the gene expression level is measured by PCR analysis of the cDNA.

4. The testing method according to claim 1, wherein the gene expression level is measured by detecting the protein encoded by the marker gene.

5. A reagent for testing for bronchial asthma or chronic obstructive pulmonary disease, wherein the reagent comprises a polynucleotide comprising the nucleotide sequence of a marker gene, or an oligonucleotide having at least 15 nucleotides and comprising a nucleotide sequence complementary to the complementary strand of the nucleotide sequence of the marker gene, and wherein, the marker gene is any one selected from the group according to (a) or (b) in claim 1.

6. A reagent for testing for bronchial asthma or chronic obstructive pulmonary disease, wherein the reagent comprises an antibody that recognizes a protein encoded by a marker gene, and wherein the marker gene is any one selected from the group according to (a) or (b) in claim 1.

7. A method of screening for a therapeutic agent for bronchial asthma or chronic obstructive pulmonary disease, wherein the marker gene is any one selected from the group according to (a) or (b) in claim 1, and wherein the method comprises the steps of:
(1) contacting a candidate compound with a cell expressing the marker gene;
(2) measuring the expression level of said gene; and
(3) selecting a compound that decreases the expression level of a marker gene belonging to group (a) or increases the expression level of a marker gene belonging to group (b), as compared to that in a control with which the compound has not been contacted.

8. The method according to claim 7, wherein the cell is a respiratory epithelial cell or a goblet cell.

9. The method according to claim 8, which comprises the step of culturing the respiratory epithelial cells under the condition in which culture medium is removed from the apical side of said cells and the culture medium is supplied from the basolateral side of the cells.

10. A kit for screening for a candidate compound for a therapeutic agent to treat bronchial asthma or chronic obstructive pulmonary disease, wherein the kit comprises (i) a polynucleotide comprising the nucleotide sequence of a marker gene, or an oligonucleotide having at least 15 nucleotides and comprising a nucleotide sequence that is complementary to the complementary strand of the polynucleotide, and (ii) a cell expressing the marker gene, and wherein the marker gene is any one selected from the group according to (a) or (b) in claim 1.

11. A kit for screening for a candidate compound for a therapeutic agent to treat bronchial asthma or chronic obstructive pulmonary disease, wherein the kit comprises (i) an antibody that recognize a protein encoded by a marker gene, and (ii) a cell expressing the marker gene, wherein the marker gene is selected from the group according to (a) or (b) in claim 1.

12. The kit according to claim 10 or 11, which further comprises a cell-supporting material to culture respiratory epithelial cells under conditions in which the culture medium is supplied from the basolateral side of the cells.

13. The kit according to claim 12, which further comprises respiratory epithelial cells.

14. An animal model for bronchial asthma or chronic obstructive pulmonary disease, wherein the animal is a transgenic nonhuman vertebrate wherein the expression level of a marker gene, or a gene functionally equivalent to the marker gene, has been increased in the respiratory tissue, wherein the marker gene is any one selected from the group according to (a) in claim 1 or the following (A):
(A) a group of genes whose expression levels increase in the lung of an animal model for bronchial hypersensitivity induced by an exposure to the ovalbumin antigen, wherein the genes comprise any one of the nucleotide sequences of SEQ ID NOs: 954 to 1174.

15. The animal model according to claim 14, wherein the nonhuman vertebrate is a mouse.

16. An animal model for bronchial asthma or chronic obstructive pulmonary disease, wherein the animal is a transgenic nonhuman vertebrate wherein the expression level of a marker gene, or a gene functionally equivalent to the marker gene, has been decreased in the respiratory tissue, wherein the marker gene is any one selected from the group according to (b) in claim 1 or the following (B) :
(B) a group of genes whose expression levels decrease in the lung of an animal model for bronchial hypersensitivity induced by an exposure to the ovalbumin antigen, wherein the genes comprise any one of the nucleotide sequences of SEQ ID NOs: 1376 to 1515.

17. The animal model according to claim 16, wherein the nonhuman vertebrate is a mouse.

18. A method for producing an animal model for bronchial asthma or chronic obstructive pulmonary disease, which comprises the step of administering to a mouse any one of (i) to (iv):
(i) a polynucleotide comprising the nucleotide sequence constituting any one of the genes selected from the gene group according to (A) in claim 14;
(ii) a protein encoded by a polynucleotide comprising the nucleotide sequence constituting any one of the genes selected from the gene group according to (A) in claim 14;
(iii) an antisense nucleic acid of a polynucleotide comprising the nucleotide sequence constituting any one of the genes selected from the gene group according to (B) in claim 16, a ribozyme, or a polynucleotide that suppresses the expression of a gene through an RNAi (RNA interference) effect; and
(iv) an antibody that binds to a protein encoded by a polynucleotide comprising the nucleotide sequence constituting any one of the genes selected from the gene group according to (B) in claim 16, or a fragment comprising an antigen-binding region thereof.

19. An inducer that induces bronchial asthma in a mouse, wherein said inducer comprises as an active ingredient any one of (i) to (iv) in claim 18.

20. A method of screening for a therapeutic agent for bronchial asthma or chronic obstructive pulmonary disease comprising the steps of:
(1) administering a candidate compound to an animal subject,
(2) assaying the expression level of the marker gene in a biological sample obtained from the animal subject, and
(3) selecting a compound that decreases the expression level of a marker gene belonging to group (a) or (A) , or a compound that increases the expression level of a marker gene belonging to group (b) or (B) , as compared to that in a control with which the candidate compound has not been contacted,
wherein the marker gene is any one selected from the group consisting of (a) or (b) in claim 1, (A) in claim 14, and (B) in claim 16, or a gene functionally equivalent to said marker gene.

21. A method of screening for a therapeutic agent for bronchial asthma or chronic obstructive pulmonary disease comprising the steps of:
(1) contacting a candidate compound with a cell into which a vector has been introduced, wherein the vector comprises a transcriptional regulatory region of a marker gene and a reporter gene that is expressed under the control of the transcriptional regulatory region,
(2) measuring the activity of the reporter gene, and
(3) selecting a compound that decreases the expression level of the reporter gene when the marker gene belongs to group (a), or a compound that increases the expression level of the reporter gene when the marker gene belongs to group (b) , as compared to that in a control with which the candidate compound has not been contacted,
wherein the marker gene is any one selected from the group according to (a) or (b) in claim 1, or a gene functionally equivalent to the marker gene.

22. A method of screening for a therapeutic agent for bronchial asthma or chronic obstructive pulmonary disease comprising the steps of:
(1) contacting a candidate compound with a protein encoded by a marker gene,
(2) measuring the activity of the protein, and
(3) selecting a compound that decreases the activity when the marker gene belongs to group (a) , or a compound that increases the activity when the marker gene belongs to the group (b) , as compared to that in a control where the candidate compound has not been contacted,
wherein the marker gene is any one selected from the group according to (a) or (b) in claim 1, or a gene functionally equivalent to the marker gene.

23. A therapeutic agent for bronchial asthma or chronic obstructive pulmonary disease, which comprises as an active ingredient a compound being obtainable by any one of the screening methods according to claims 7, 20, 21, and 22.

24. A therapeutic agent for bronchial asthma or chronic obstructive pulmonary disease, which comprises as an active ingredient a marker gene or an antisense nucleic acid corresponding to a portion of the marker gene, a ribozyme, or a polynucleotide that suppresses the expression of the gene through an RNAi effect, wherein the marker gene is any one selected from the group according to (a) in claim 1.

25. A therapeutic agent for bronchial asthma or chronic obstructive pulmonary disease, which comprises as an active ingredient an antibody recognizing a protein encoded by a marker gene, wherein the marker gene is any one selected from the group according to (a) in claim 1.

26. A therapeutic agent for bronchial asthma or chronic obstructive pulmonary disease, which comprises as an active ingredient a marker gene, or a protein encoded by a marker gene, wherein the marker gene is any one selected from the group according to (b) in claim 1.

27. A DNA chip for testing for bronchial asthma or a chronic obstructive pulmonary disease, on which a probe has been immobilized to assay a marker gene, and wherein the marker gene comprises at least a single type of gene selected from group (a) and (b) in claim 1.
